# EUROPEAN PATENT SPECIFICATION

(11) **EP 3 325 505 B1**
(45) Date of publication and mention of the grant of the patent: **19.08.2020**
(21) Application number: 16747683.7
(22) Date of filing: 22.07.2016
(51) Int. Cl.: A61K 38/14, A61K 38/57, A61K 47/62, A61K 47/69, A61P 35/00, A61P 35/02, C07K 14/81, A61K 38/17

(54) **MESSENGER RIBONUCLEIC ACIDS FOR THE PRODUCTION OF INTRACELLULAR BINDING POLYPEPTIDES AND METHODS OF USE THEREOF**
BOTEN-RIBONUKLEINSÄUREN (MRNA) ZUR HERSTELLUNG INTRAZELLULÄRER BINDUNGSPOLYPEPTIDE UND VERFAHREN ZUR VERWENDUNG DAVON
ACIDES RIBONUCLÉIQUES MESSAGERS POUR LA PRODUCTION DE POLYPEPTIDES DE LIAISON INTRACELLULAIRES ET LEURS PROCÉDÉS D'UTILISATION

(30) Priority: 23.07.2015 US 201562196236 P; 23.07.2015 US 201562196240 P; 22.01.2016 US 201662286276 P; 22.01.2016 US 201662286245 P
(43) Date of publication of application: 30.05.2018
(73) Proprietor: Modernatx, Inc., Cambridge, MA 02139 (US)
(72) Inventor: HUANG, Eric, Yi-chun, Boston, MA 02118 (US); FREDERICK, Josh, Charlestown, MA 02129 (US); MCKINNEY, Kristine, Cambridge, MA 02139 (US); HENDERSON, Christina, Stow, MA 01775 (US); CHEUNG-ONG, Kahlin, Boston, MA 02215 (US); BOLEN, Joseph, Cambridge, MA 02139 (US); KELSEY, Stephen, Michael, Brookline, MA 02445 (US); MORIN, Michael, Cambridge, MA 02139 (US); GURUMURTHY, Sushma, Sharon, MA 02067 (US)
(74) Representative: Hoffmann Eitle
(86) International application number: PCT/US2016/043744
(87) International publication number: WO 2017/015630

(56) References cited:
- WO-A1-2009/136182
- WO-A1-2015/042705
- WO-A2-2013/143700
- US-A1- 2013 123 199
- L K J STADLER ET AL: "The use of a neutral peptide aptamer scaffold to anchor BH3 peptides constitutes a viable approach to studying their function", CELL DEATH AND DISEASE, vol. 5, no. 1, E1037, 30 January 2014 (2014-01-30), pages 1-9, XP055315981, DOI: 10.1038/cddis.2013.564
- BRITTA VALLAZZA ET AL: "Recombinant messenger RNA technology and its application in cancer immunotherapy, transcript replacement therapies, pluripotent stem cell induction, and beyond", WILEY INTERDISCIPLINARY REVIEWS: RNA, vol. 6, no. 5, 9 June 2015 (2015-06-09), pages 471-499, XP055316739, United Kingdom ISSN: 1757-7004, DOI: 10.1002/wrna.1288
- JOPLING C L ET AL: "Positive and negative modulation of viral and cellular mRNAs by liver-specific microRNA miR-122", COLD SPRING HARBOR SYMPOSIA ON QUANTITATIVE BIOLOGY, BIOLOGICAL LABORATORY, COLD SPRING HARBOR, NY, US, vol. 71, 1 January 2006 (2006-01-01), pages 369-376, XP008100797, ISSN: 0091-7451, DOI: 10.1101/SQB.2006.71.022
- LIN CHEN ET AL: "Differential Targeting of Prosurvival Bcl-2 Proteins by Their BH3-Only Ligands Allows Complementary Apoptotic Function", MOLECULAR CELL, vol. 17, no. 3, 1 February 2005 (2005-02-01), pages 393-403, XP055051154, ISSN: 1097-2765, DOI: 10.1016/j.molcel.2004.12.030 cited in the application
- CHIARA BRACONI ET AL: "The Role of MicroRNAs in Human Liver Cancers", SEMINARS IN ONCOLOGY, vol. 38, no. 6, 1 December 2011 (2011-12-01), pages 752-763, XP055340641, US ISSN: 0093-7754, DOI: 10.1053/j.seminoncol.2011.08.001
- ELIZABETH J KELLY ET AL: "MicroRNAs and the Regulation of Vector Tropism", MOLECULAR THERAPY, vol. 17, no. 3, 1 March 2009 (2009-03-01), pages 409-416, XP055007015, ISSN: 1525-0016, DOI: 10.1038/mt.2008.288
- T. HOFFMANN ET AL: "Structure-function studies of an engineered scaffold protein derived from stefin A. I: Development of the SQM variant", PROTEIN ENGINEERING, DESIGN AND SELECTION, vol. 23, no. 5, 23 February 2010 (2010-02-23), pages 403-413, XP055317441, GB ISSN: 1741-0126, DOI: 10.1093/protein/gzq012

## Description

### Related Applications

This application claims the benefit of U.S. Provisional Patent Application Serial No. 62/196,236 filed July 23, 2015; U.S. Provisional Patent Application Serial No. 62/196,240 filed July 23, 2015; U.S. Provisional Patent Application Serial No. 62/286,245 filed January 22, 2016; and U.S. Provisional Patent Application Serial No. 62/286,276 filed January 22, 2016.

### Background of the Invention

A number of therapeutic tools exist for modulating the function of biological pathways and/or molecules that are involved in disease. These tools include, for example, small molecule inhibitors and therapeutic antibodies. However, many biological molecules (e.g., proteins) that are known to be involved in diseases, e.g., cancers, are not readily druggable using small molecule inhibitors or therapeutic antibodies. In particular, some intracellular targets, such as Bcl-2 family members (e.g., anti-apoptotic Bcl-2 family members), are difficult to target with small molecule inhibitors, and are also not accessible to therapeutic antibodies administered into the blood stream due to the permeability barrier of the cell's plasma membrane.

Clearly, therefore, there exists a need for new therapeutic compositions capable of modulating intracellular disease targets, including anti-apoptotic Bcl-2 family members, and related methods for delivery these agents to cells to treat and prevent diseases such as cancers.

### Summary of invention

The present invention provides a lipid nanoparticle comprising a messenger RNA encoding a polypeptide comprising a Stefin A (SteA) mutant polypeptide scaffold comprising at least one binding polypeptide domain, wherein optionally said mRNA comprises one or more modified nucleobases. Other aspects of the invention are provided in the claims.

The present disclosure provides compositions including isolated mRNAs encoding a polypeptide comprising a Stefin A (SteA) scaffold polypeptide and one or more binding polypeptides. The SteA scaffold polypeptide acts to present one or more binding polypeptides, for example intracellularly, to thereby modulate the activity of a target to which the binding polypeptide(s) binds. In one embodiment, the binding polypeptide is a BH3 domain. In another embodiment, the binding polypeptide is a Bcl-2-like polypeptide. In various other embodiments, the binding polypeptide binds a target selected from the group consisting of an oncoprotein, a viral protein, an immune-related protein, a transcription factor, a cytokine, a receptor, an enzyme, DNA or RNA. In various other embodiments, the binding polypeptide is from a protein selected from the group consisting of an oncoprotein, an antibody or antigen-binding portion thereof, a ligand-binding protein, a DNA-binding protein and an RNA-binding protein. Non-limiting examples of binding polypeptides include anti-Ras peptides, SALL4-inhibitory peptides, TOPK-inhibitory peptides and p53-inhibitory peptides.

In particular embodiments, the isolated mRNAs include one or more modified nucleobase and are referred to as a modified mRNAs (mmRNAs). In particular embodiments, the isolated mRNAs are present in pharmaceutical compositions. In particular embodiments, the mRNAs are present in nanoparticles, e.g. lipid nanoparticles.

Accordingly, in one embodiment, the invention pertains to a modified messenger RNA (mmRNA) encoding a polypeptide comprising a Stefin A (SteA) mutant polypeptide scaffold comprising at least one binding polypeptide domain, wherein said mmRNA comprises one or more modified nucleobases. In one embodiment, the SteA mutant polypeptide scaffold is Stefin A Quadruple Mutant - Tracy (SQT) having an N-terminal insertion site, a loop 1 insertion site and a loop 2 insertion site, wherein the binding polypeptide domain is located at the N-terminal insertion site, the loop 1 insertion site, or the loop 2 insertion site. In one embodiment, the SteA mutant polypeptide scaffold comprises first and second binding polypeptides, wherein the first and the second binding polypeptides comprise the same amino acid sequence. In one embodiment, the SteA mutant polypeptide scaffold comprises first and second binding polypeptides, wherein the first and the second binding polypeptides comprise different amino acid sequences. In one embodiment, the polypeptide comprises a binding polypeptide located at the N-terminal insertion site. In another embodiment, the polypeptide comprises a binding polypeptide located at the loop 1 insertion site. In another embodiment, the polypeptide comprises a binding polypeptide located at the loop 2 insertion site. In another embodiment, the polypeptide further comprises a third binding polypeptide, wherein the polypeptide comprises a binding polypeptide located at each of the N-terminal insertion site, the loop 1 insertion site, and the loop 2 insertion site. In one embodiment, the first, second, and third binding polypeptides comprise the same amino acid sequence. In another embodiment, the first, second, and third binding polypeptides comprise at least two different amino acid sequences. In still another embodiment, each of the first, second and third binding polypeptides has a different amino acid sequence.

In another embodiment, the disclosure provides compositions including isolated mRNAs encoding a polypeptide comprising a Stefin A (SteA) scaffold polypeptide and one or more BH3 domains, as well as methods of using such compositions, for example, for inducing apoptosis and/or treating cancer (e.g., liver cancer or colorectal cancer).

In a first aspect, the disclosure features an isolated mRNA encoding a polypeptide comprising a SteA scaffold polypeptide, the SteA scaffold polypeptide including an N-terminal insertion site, a loop 1 insertion site, and a loop 2 insertion site, and a first Bcl-2 homology 3 (BH3) domain, wherein the first BH3 domain is located within the SteA scaffold polypeptide at the N-terminal insertion site, the loop 1 insertion site, or the loop 2 insertion site. In some embodiments, the isolated mRNA is formulated in a composition suitable for administration to a human. In one embodiment, the first BH3 domain is a PUMA BH3 domain. In another embodiment, the first BH3 domain is a Bim BH3 domain. In another embodiment, the first BH3 domain is a BH3 mimic. In one embodiment, the SteA scaffold polypeptide is an SQT scaffold polypeptide. In some embodiments, the isolated mRNA is formulated in a composition suitable for administration to a human.

In a second aspect, the disclosure features a nanoparticle, e.g., a lipid nanoparticle (LNP), including a messenger RNA (mRNA) encoding a polypeptide comprising a SteA scaffold polypeptide, the SteA scaffold polypeptide including an N-terminal insertion site, a loop 1 insertion site, and a loop 2 insertion site, and a first BH3 domain, wherein the first BH3 domain is located at the N-terminal insertion site, the loop 1 insertion site, or the loop 2 insertion site. In certain embodiments, the mRNA is a modified mRNA. In one embodiment, the first BH3 domain is a PUMA BH3 domain. In another embodiment, the first BH3 domain is a Bim BH3 domain. In another embodiment, the first BH3 domain is a BH3 mimic. In one embodiment, the SteA scaffold polypeptide is an SQT scaffold polypeptide.

In certain embodiments of the first and second aspects, the polypeptide further includes a second BH3 domain, wherein the first and the second BH3 domains are located at different insertion sites selected from the N-terminal insertion site, the loop 1 insertion site, and the loop 2 insertion site. In some embodiments, the first and the second BH3 domains include the same amino acid sequence. In some embodiments, the first and the second BH3 domains include different amino acid sequences. In some embodiments, the polypeptide includes a BH3 domain located at the N-terminal insertion site. In some embodiments, the polypeptide includes a BH3 domain located at the loop 1 insertion site. In some embodiments, the polypeptide includes a BH3 domain located at the loop 2 insertion site.

In some embodiments of the first and second aspects, the polypeptide further includes a third BH3 domain, wherein the polypeptide includes a BH3 domain located at each of the N-terminal insertion site, the loop 1 insertion site, and the loop 2 insertion site. In some embodiments, the first, second, and third BH3 domains include the same amino acid sequence. In some embodiments, the first, second, and third BH3 domains include at least two different amino acid sequences. In some embodiments, each BH3 domain has a different amino acid sequence.

In any of the preceding embodiments of the first and second aspects, the first, second, or third BH3 domain may include the amino acid sequence of X₁X₂X₃X₄X₅X₆X₇X₈X₉DX₁₀X₁₁X₁₂, wherein X₁, X₅, X₈, and X₁₁ are any hydrophobic amino acid residue, X₂ and X₉ are Gly, Ala, or Ser, X₃, X₄, X₆, and X₇ are any amino acid residue, X₁₀ is Asp or Glu, and X₁₂ is Asn, His, Asp, or Tyr. In some embodiments, X₅ is leucine.

In any of the preceding embodiments of the first and second aspects, the first, second, or third BH3 domain may be an amino acid sequence having at least 90% identity to any one of SEQ ID NOs: 1-26. In some embodiments, the BH3 domain includes the amino acid sequence of any one of SEQ ID NOs: 1-26. In some embodiments, the BH3 domain includes an amino acid sequence having at least 90% identity to any one of SEQ ID NOs: 27-30. In some embodiments, the BH3 domain includes the amino acid sequence of any one of SEQ ID NOs: 27-30. In some embodiments, the BH3 domain includes the amino acid sequence of EEQWAREIGAQLRRMADDLNAQYERR (SEQ ID NO: 27) or DMRPEIWIAQELRRIGDEFNAYYARR (SEQ ID NO: 28).

In particular embodiments of the SteA fusion polypeptides, the SteA scaffold includes an amino acid sequence having at least 90% identity to any one of SEQ ID NOs: 53-71. In some embodiments, the SteA scaffold includes an amino acid sequence selected from any one of SEQ ID NOs: 53-71. In some embodiments, the SteA scaffold includes an amino acid sequence of SEQ ID NO: 70. In some embodiments, the fusion polypeptide includes a BH3 domain including the amino acid sequence of SEQ ID NO: 27 or SEQ ID NO: 28 inserted into the N-terminal insertion site of a SteA scaffold including an amino acid sequence having at least 90% identity to any one of SEQ ID NOs: 53-71 or including an amino acid sequence of any one of SEQ ID NOs: 53-71.

In any of the preceding embodiments, the mRNA may include a microRNA (miRNA) binding site. In some embodiments, the miRNA binding site is a microRNA-122 (miR-122) binding site. In some embodiments, the miR-122 binding site includes a miR-122 seed sequence. In some embodiments, the miR-122 binding site includes a nucleotide sequence of SEQ ID NO: 31 or SEQ ID NO: 32.

In certain embodiments, the lipid nanoparticle may include a cationic and/or ionizable lipid. In some embodiments, the cationic and/or ionizable lipid is DLin-KC2-DMA or DLin-MC3-DMA. In some embodiments, the lipid nanoparticle is a liposome.

In certain embodiments, the lipid nanoparticle may further include a targeting moiety conjugated to the outer surface of the lipid nanoparticle.

In certain embodiments of the second aspect, the lipid nanoparticle may further comprise an mRNA encoding a Bcl-2-like polypeptide, or a variant or fragment thereof. In certain embodiments, the mRNA is a modified mRNA comprising one or more modified nucleobases. In particular embodiments, the Bcl-2-like polypeptide is Bcl-2, Bcl-x_{L}, Bcl-w, Mcl-1 or A1. In particular embodiments, a variant has at least 90% amino acid sequence homology to a Bcl-2-like polypeptide, such as Bcl-2, Bcl-x_{L}, Bcl-w, Mcl-1 or A1. In some embodiments, the Bcl-2-like polypeptide is a variant selected from Mcl-ldel.N/C, Bcl-2 (C295/A128E), Bcl-2 (D34A) del.C32, Bcl-xL del.C24, Mcl-1 del.N/C(2010), and Bcl-xL (D61A) del.C24. In certain embodiments, a BCL-2-like polypeptide fragment binds a BH3 domain. In certain embodiments, the mRNA encoding the Bcl-2-like polypeptide or variant or fragment thereof comprises a microRNA (miRNA) binding site. In certain embodiments, the miRNA binding site is a microRNA-21 (miR-21) binding site. In certain embodiments, the miR-21 binding site comprises a miR-21 seed sequence. In particular embodiments, the miR-21 binding site comprises a nucleotide sequence of SEQ ID NO: 33, SEQ ID NO: 34 or SEQ ID NO: 106.

In another aspect, the present disclosure provides an mRNA encoding a Bcl-2-like polypeptide or variant or fragment thereof. In certain embodiments, the mRNA is a modified mRNA comprising one or more modified nucleobases. In certain embodiments, the Bcl-2-like polypeptide is Bcl-2, Bcl-x_{L}, Bcl-w, Mcl-1 or A1, and in certain embodiments, the Bcl-2-like polypeptide is a variant selected from Mcl-ldel.N/C, Bcl-2 (C295/A128E), Bcl-2 (D34A) del.C32, Bcl-xL del.C24, Mcl-1 del.N/C(2010), and Bcl-xL (D61A) del.C24. In particular embodiments, the mRNA encoding the Bcl-2-like polypeptide or variant thereof comprises a microRNA (miRNA) binding site, and in certain embodiments, the miRNA binding site is a microRNA-21 (miR-21) binding site. In various embodiments, the miR-21 binding site comprises a miR-21 seed sequence, and in certain embodiments, the miR-21 binding site comprises a nucleotide sequence of SEQ ID NO: 33, SEQ ID NO: 34 or SEQ ID NO: 106.

In another aspect, the invention features a pharmaceutical composition comprising any one of the preceding mRNAs or nanoparticles, e.g., lipid nanoparticles, and a pharmaceutically acceptable diluent, carrier or excipient. In particular embodiments, the pharmaceutical composition comprises an mRNA encoding a fusion polypeptide comprising a SteA scaffold polypeptide and a BH3 domain. In particular embodiments, the pharmaceutical composition comprises an mRNA encoding a Bcl-2-like polypeptide or variant or fragment thereof. In particular embodiments, the pharmaceutical composition comprises an mRNA encoding a fusion polypeptide comprising a SteA scaffold polypeptide and a BH3 domain, and an mRNA encoding a Bcl-2-like polypeptide or variant or fragment thereof. In other embodiments, one pharmaceutical composition can comprise an mRNA encoding a fusion polypeptide comprising a SteA scaffold polypeptide and a BH3 domain (or an LNP encapsulating said mRNA) and a second pharmaceutical composition can comprise an mRNA encoding a Bcl-2-like polypeptide or variant or fragment thereof (or LNP encapsulating said mRNA) and the two pharmaceutical compositions can be co-administered. In certain embodiments, the mRNA is a modified mRNA comprising one or more modified nucleobases. In particular embodiments, the Bcl-2-like polypeptide is Bcl-2, Bcl-x_{L}, Bcl-w, Mcl-1 or A1. In some embodiments, the Bcl-2-like polypeptide is a functional variant selected from Mcl-ldel.N/C, Bcl-2 (C295/A128E), Bcl-2 (D34A) del.C32, Bcl-xL del.C24, Mcl-1 del.N/C(2010), and Bcl-xL (D61A) del.C24. In certain embodiments, the mRNA encoding the Bcl-2-like polypeptide or functional variant thereof comprises a microRNA (miRNA) binding site. In certain embodiments, the miRNA binding site is a microRNA-21 (miR-21) binding site. In certain embodiments, the miR-21 binding site comprises a miR-21 seed sequence. In particular embodiments, the miR-21 binding site comprises a nucleotide sequence of SEQ ID NO: 33, SEQ ID NO: 34 or SEQ ID NO: 106.

In another aspect, the disclosure features a method for inducing apoptosis in a cell, the method including contacting the cell with any one of the preceding fusion polypeptides, or a nanoparticle (e.g., a lipid nanoparticle) or pharmaceutical composition comprising any of the preceding SteA fusion polypeptides comprising one or more BH3 domains, whereby the mRNA enters the cell under conditions permissive for expression of the fusion polypeptide, thereby inducing apoptosis. In some embodiments, the cell is a cancer cell. In some embodiments, the cancer cell is a liver cancer cell. In some embodiments, the liver cancer cell is a hepatocellular carcinoma cell. In some embodiments, the cancer cell is a colorectal cancer cell. In some embodiments, the colorectal cancer cell is in a primary tumor or a metastasis. In some embodiments, the cancer cell is a hematopoietic cell. In some embodiments, the cancer cell is a myeloid cell. In some embodiments, the cancer cell is a hematopoietic stem cell (e.g., a hematopoietic stem cell from bone marrow, an erythroid stem cell, a myeloid stem cell, a thrombocytic stem cell). In any of the preceding embodiments, the cell may be a human cell.

In another aspect, the invention features a method for treating a subject having cancer, the method including providing or administering an effective amount of any one of the preceding SteA fusion polypeptides, or a nanoparticle (e.g., a lipid nanoparticle) or pharmaceutical composition comprising any of the preceding SteA fusion polypeptides comprising one or more BH3 domains, to the subject. In some embodiments, the cancer is liver cancer or colorectal cancer. In some embodiments, the liver cancer is hepatocellular carcinoma. In some embodiments, the colorectal cancer is a primary tumor or a metastasis. In some embodiments, the cancer is a hematopoietic cancer. In some embodiments, the cancer is an acute myeloid leukemia, a chronic myeloid leukemia, a chronic myelomonocytic leukemia, a myelodystrophic syndrome (including refractory anemias and refractory cytopenias) or a myeloproliferative neoplasm or disease (including polycythemia vera, essential thrombocytosis and primary myelofibrosis). In some embodiments, the lipid nanoparticle or isolated mRNA (or pharmaceutical composition) is administered to the patient parenterally.

In certain embodiments, the cell is also contacted with, or the subject is also provided with an mRNA encoding a Bcl-2-like polypeptide or functional variant thereof. In certain embodiments, the mRNA is a modified mRNA comprising one or more modified nucleobases. In particular embodiments, the Bcl-2-like polypeptide is Bcl-2, Bcl-x_{L}, Bcl-w, Mcl-1 or A1. In some embodiments, the Bcl-2-like polypeptide is a functional variant selected from Mcl-ldel.N/C, Bcl-2 (C295/A128E), Bcl-2 (D34A) del.C32, Bcl-xL del.C24, Mcl-1 del.N/C(2010), and Bcl-xL (D61A) del.C24. In certain embodiments, the mRNA encoding the Bcl-2-like polypeptide or functional variant thereof comprises a microRNA (miRNA) binding site. In certain embodiments, the miRNA binding site is a microRNA-21 (miR-21) binding site. In certain embodiments, the miR-21 binding site comprises a miR-21 seed sequence. In particular embodiments, the miR-21 binding site comprises a nucleotide sequence of SEQ ID NO: 33, SEQ ID NO: 34 or SEQ ID NO: 106. In particular embodiments, the pharmaceutical composition or nanoparticle, e.g., lipid nanoparticle, comprising the mRNA encoding the fusion polypeptide also comprises the mRNA encoding a Bcl-2-like polypeptide or variant or fragment thereof. In particular embodiments, the mRNA encoding the fusion polypeptide and the mRNA encoding the Bcl-2-like polypeptide are in separate pharmaceutical compositions or nanoparticles, e.g., lipid nanoparticles. In particular embodiments, the cell is contacted with, or the subject is provided with, the mRNA encoding a Bcl-2-like polypeptide or functional variant thereof at the same time or sequentially with the mRNA encoding the fusion polypeptide.

In certain embodiments, the cell is also contacted with, or the subject is also provided with an mRNA that selectively inhibits MCL1, or a pharmaceutical composition comprising the mmRNA that selectively inhibits MCL1, wherein the mRNA in the pharmaceutical composition is optionally in a lipid nanoparticle. In certain embodiments, the mRNA that selectively inhibits MCL1 encodes an amino acid sequence selected from the group consisting of SEQ ID NOs: 107-110.

In another aspect, the invention features a lipid nanoparticle encapsulating a modified mRNA of the invention, wherein the lipid nanoparticle comprises a cationic lipid, a PEG-modified lipid, a sterol and a non-cationic lipid. In one embodiment, the cationic lipid is selected from the group consisting of 2,2-dilinoleyl-4-methylaminoethyl-[1,3]-dioxolane (DLin-KC2-DMA), dilinoleyl-methyl-4-dimethylaminobutyrate (DLin-MC3-DMA) and di((Z)-non-2-en-1-yl) 9-((4-(dimethylamino)butanoyl)oxy)heptadecanedioate (L319). In one embodiment, the cationic lipid nanoparticle has a molar ratio of about 20-60% cationic lipid, about 5-25% non-cationic lipid, about 25-55% sterol and about 0.5-15% PEG-modified lipid. In one embodiment, the cationic lipid is an ionizable cationic lipid and the non-cationic lipid is a neutral lipid, and the sterol is cholesterol. In one embodiment, the open reading frame of the encapsulated mmRNA is codon-optimized. In one embodiment, the nanoparticle has a polydiversity value of less than 0.4. In one embodiment, the nanoparticle has a net neutral charge at a neutral pH. In one embodiment, the nanoparticle has a mean diameter of 50-200 nm. In one embodiment, at least 80% of the uracils in the open reading frame of the encapsulated mmRNA have a chemical modification. In one embodiment, 100% of the uracils in the open reading frame of the encapsulated mmRNA have a chemical modification. In one embodiment, the chemical modification is in the 5-position of the uracils.

In certain embodiments, the disclosure features an isolated mRNA having a 5' and 3' UTR and encoding a polypeptide comprising SteA scaffold polypeptide and a BH3 domain. In certain embodiments, an isolated mRNA has a 5'UTR set forth in SEQ ID NO: 72, a 3' UTR set forth in SEQ ID NO: 73, and a nucleotide sequence encoding SQT-PUMA-BH3, as set forth in SEQ ID NO: 74. In certain embodiments, an isolated mRNA has a 5'UTR set forth in SEQ ID NO: 72, a 3' UTR set forth in SEQ ID NO: 73, and a nucleotide sequence encoding SQT-Bim-BH3, as set forth in SEQ ID NO: 75. In certain embodiments, an isolated mRNA has a 5'UTR set forth in SEQ ID NO: 72, a 3' UTR set forth in SEQ ID NO: 73, and a nucleotide sequence encoding SQT-BAD-BH3, as set forth in SEQ ID NO: 76. In certain embodiments, an isolated mRNA has a 5'UTR set forth in SEQ ID NO: 72, a 3' UTR set forth in SEQ ID NO: 73, and a nucleotide sequence encoding SQT-Noxa-BH3, as set forth in SEQ ID NO: 77.

In certain embodiments, the disclosure features an isolated mRNA having a 5' and 3' UTR and encoding a polypeptide comprising SteA scaffold polypeptide and a Bcl-2-like polypeptide domain. In certain embodiments, an isolated mRNA has a 5'UTR set forth in SEQ ID NO: 72, a 3' UTR set forth in SEQ ID NO: 73, and a nucleotide sequence set forth in SEQ ID NO: 92. In certain embodiments, an isolated mRNA has a 5'UTR set forth in SEQ ID NO: 72, a 3' UTR set forth in SEQ ID NO: 73, and a nucleotide sequence set forth in SEQ ID NO: 93. In certain embodiments, an isolated mRNA has a 5'UTR set forth in SEQ ID NO: 72, a 3' UTR set forth in SEQ ID NO: 73, and a nucleotide sequence set forth in SEQ ID NO: 94. In certain embodiments, an isolated mRNA has a 5'UTR set forth in SEQ ID NO: 72, a 3' UTR set forth in SEQ ID NO: 73, and a nucleotide sequence set forth in SEQ ID NO: 95. In certain embodiments, an isolated mRNA has a 5'UTR set forth in SEQ ID NO: 72, a 3' UTR set forth in SEQ ID NO: 73, and a nucleotide sequence set forth in SEQ ID NO: 96. In certain embodiments, an isolated mRNA has a 5'UTR set forth in SEQ ID NO: 72, a 3' UTR set forth in SEQ ID NO: 73, and a nucleotide sequence set forth in SEQ ID NO: 97. In certain embodiments, an isolated mRNA has a 5'UTR set forth in SEQ ID NO: 72, a 3' UTR set forth in SEQ ID NO: 73, and a nucleotide sequence set forth in SEQ ID NO: 98. In certain embodiments, an isolated mRNA has a 5'UTR set forth in SEQ ID NO: 72, a 3' UTR set forth in SEQ ID NO: 73, and a nucleotide sequence set forth in SEQ ID NO: 99. In certain embodiments, an isolated mRNA has a 5'UTR set forth in SEQ ID NO: 72, a 3' UTR set forth in SEQ ID NO: 73, and a nucleotide sequence set forth in SEQ ID NO: 100. In certain embodiments, an isolated mRNA has a 5'UTR set forth in SEQ ID NO: 72, a 3' UTR set forth in SEQ ID NO: 73, and a nucleotide sequence set forth in SEQ ID NO: 101. In certain embodiments, an isolated mRNA has a 5'UTR set forth in SEQ ID NO: 72, a 3' UTR set forth in SEQ ID NO: 73, and a nucleotide sequence set forth in SEQ ID NO: 102. In certain embodiments, an isolated mRNA has a 5'UTR set forth in SEQ ID NO: 72, a 3' UTR set forth in SEQ ID NO: 73, and a nucleotide sequence set forth in SEQ ID NO: 103. In certain embodiments, an isolated mRNA has a 5'UTR set forth in SEQ ID NO: 72, a 3' UTR set forth in SEQ ID NO: 73, and a nucleotide sequence set forth in SEQ ID NO: 104. In certain embodiments, an isolated mRNA has a 5'UTR set forth in SEQ ID NO: 72, a 3' UTR set forth in SEQ ID NO: 73, and a nucleotide sequence set forth in SEQ ID NO: 105.

In certain embodiments, the disclosure features an isolated mRNA having a 5' and 3' UTR and encoding a polypeptide comprising SteA scaffold polypeptide and an anti-Mcl-1 domain. In certain embodiments, an isolated mRNA has a 5'UTR set forth in SEQ ID NO: 72, a 3' UTR set forth in SEQ ID NO: 73, and a nucleotide sequence set forth in SEQ ID NO: 111. In certain embodiments, an isolated mRNA has a 5'UTR set forth in SEQ ID NO: 72, a 3' UTR set forth in SEQ ID NO: 73, and a nucleotide sequence set forth in SEQ ID NO: 112.

In another embodiment, the disclosure features an isolated mRNA encoding SQT-PUMA-BH3 comprising a nucleotide sequence set forth in SEQ ID NO: 166. In another embodiment the invention features an isolated mRNA encoding SQT-Bim-BH3 comprising a nucleotide sequence set forth in SEQ ID NO: 167.

In another embodiment, the invention features lipid nanoparticles comprising any of the aforementioned mmRNAs encoding a polypeptide comprising a Stefin A (SteA) mutant polypeptide scaffold comprising at least one binding polypeptide domain. Pharmaceutical compositions comprising any of the mmRNAs, or a lipid nanoparticle comprising any of the mmRNAs, and a pharmaceutically acceptable carrier, diluent or excipient, are also disclosed. Methods of inducing apoptosis in a cell, the method comprising contacting the cell with any of the mmRNAs, or a lipid nanoparticle comprising any of the mmRNAs or a pharmaceutical composition comprising any of the mmRNAs, are also disclosed. Methods of treating cancer in a subject in need thereof, the method comprising providing an effective amount of any of the mmRNAs, or a lipid nanoparticle comprising any of the mmRNAs or a pharmaceutical composition comprising any of the mmRNAs, are also disclosed.

In another embodiment, the invention provides compositions including isolated mRNAs encoding a polypeptide comprising a Stefin A (SteA) scaffold polypeptide and one or more peptides selected from the group consisting of: a TOPK inhibiting peptide, a SALL4 inhibiting peptide, an anti-Ras peptide and a p53-inhibitory peptide. The invention also relates to methods of using such compositions, for example, for treating cancer.

In a first aspect, the invention features an isolated mRNA encoding a polypeptide comprising a SteA scaffold polypeptide, the SteA scaffold polypeptide including an N-terminal insertion site, a loop 1 insertion site, and a loop 2 insertion site, and one or more peptides selected from the group consisting of: a TOPK inhibiting peptide, a SALL4 inhibiting peptide, an anti-Ras peptide and a p53-inhibitory peptide, wherein the peptide is located within the SteA scaffold polypeptide at the N-terminal insertion site, the loop 1 insertion site, or the loop 2 insertion site. In some embodiments, the isolated mRNA is formulated in a composition suitable for administration to a human.

In a second aspect, the invention features a nanoparticle, e.g., a lipid nanoparticle (LNP), including a messenger RNA (mRNA) encoding a polypeptide comprising a SteA scaffold polypeptide, the SteA scaffold polypeptide including an N-terminal insertion site, a loop 1 insertion site, and a loop 2 insertion site, and one or more peptides selected from the group consisting of: a TOPK inhibiting peptide, a SALL4 inhibiting peptide, an anti-Ras peptide and a p53-inhibitory peptide, wherein the peptide is located at the N-terminal insertion site, the loop 1 insertion site, or the loop 2 insertion site. In certain embodiments, the mRNA is a modified mRNA.

In another embodiment, the disclosure features a modified messenger RNA (mmRNA) encoding a polypeptide comprising a Stefin A (SteA) mutant polypeptide scaffold comprising at least one binding polypeptide domain, wherein the binding domain polypeptide inhibits the interaction of p53 with MDM2, and wherein said mmRNA comprises one or more modified nucleobases. In one embodiment, the SteA mutant polypeptide scaffold is Stefin A Quadruple Mutant - Tracy (SQT) having an N-terminal insertion site, a loop 1 insertion site and a loop 2 insertion site, wherein the binding polypeptide domain is located at the N-terminal insertion site, the loop 1 insertion site, or the loop 2 insertion site. In one embodiment, the binding domain polypeptide comprises an amino acid sequence selected from the group consisting of SEQ ID NOs: 130-141. In one embodiment, the binding domain polypeptide comprising an amino acid sequence shown in SEQ ID NOs: 130-141 is located at the N-terminal insertion site. In another embodiment, the binding domain polypeptide comprising an amino acid sequence shown in SEQ ID NOs: 130-141 is located at the loop 2 insertion site. In one embodiment, the polypeptide encoded by the mmRNA comprises an amino acid sequence selected from the group consisting of SEQ ID NOs: 142-148.

In another embodiment, the invention features lipid nanoparticles comprising any of the aforementioned mmRNAs encoding a polypeptide comprising a Stefin A (SteA) mutant polypeptide scaffold comprising at least one binding polypeptide domain, wherein the binding domain polypeptide inhibits the interaction of p53 with MDM2. Pharmaceutical compositions comprising any of the mmRNAs, or a lipid nanoparticle comprising any of the mmRNAs, and a pharmaceutically acceptable carrier, diluent or excipient, are also disclosed. Methods of activating p53 in a cell, the method comprising contacting the cell with any of the mmRNAs, or a lipid nanoparticle comprising any of the mmRNAs or a pharmaceutical composition comprising any of the mmRNAs, are also disclosed. Methods of treating cancer in a subject in need thereof, the method comprising providing an effective amount of any of the mmRNAs, or a lipid nanoparticle comprising any of the mmRNAs or a pharmaceutical composition comprising any of the mmRNAs, are also disclosed.

### Brief Description of the Drawings

FIGURE 1 is a diagram showing an alignment of the indicated SteA scaffold polypeptide amino acid sequences.
FIGURE 2 is a graph showing that HepG2 human hepatocellular carcinoma cells transfected with mRNA encoding SQT-PUMA-BH3 or SQT-Bim-BH3 underwent cell death almost immediately following transfection. In this figure and others, SQT-PUMA-BH3 is labeled as "SQT PUMA," SQT-Noxa-BH3 is labeled as "SQT Noxa," and SQT-Bim-BH3 is labeled as "SQT Bim."
FIGURE 3 is a graph showing that MC3-LNP-formulated mRNA encoding SQT-PUMA-BH3 or SQT-Bim-BH3 kills Hep3B cells at higher concentrations. In this figure and others, SQT-Bad-BH3 is labeled as "SQT Bad."
FIGURE 4 is a graph showing that MC3-LNP-formulated mRNA encoding SQT-PUMA-BH3 or SQT-Bim-BH3 kills HepG2 cells.
FIGURE 5 is a graph showing that MC3-LNP-formulated SQT-PUMA-BH3 and SQT-Bim-BH3 induce apoptosis (as judged by YOYO-3 dye uptake and fluorescence) as a function of time when administered to primary hepatocytes.
FIGURE 6 is a graph showing the IC50 determination of the indicated SQT-BH3 constructs introduced into Hep3B cells in lipid nanoparticles (LNPs).
FIGURE 7 is a graph showing the IC50 determination of the indicated SQT-BH3 constructs introduced into HepG2luc cells in lipid nanoparticles (LNPs).
FIGURE 8 is a graph showing increased survival in animals treated with 0.1-1 mg/kg of MC3-formulated lipid nanoparticles containing SQT-PUMA-BH3 mRNA that includes a mIR-122 binding site as compared to SQT-PUMA-BH3 mRNA that does not include a mIR-122 binding site.
FIGURE 9 is a graph showing that Bcl-2-like trap constructs inhibited SQT-Bim-BH3-induced death in Hep3B cells. The graph indicates the apoptosis signal in Hep3B cells treated with the indicated SQT-Bim-BH3 and the indicated trap constructs at the 12 hour time point.
FIGURE 10 is a graph showing that a linked miR-21 sequence reduced expression of desCitrine in Hep3B cells. The graph shows an approximately 15-fold difference in higher expression from miR122 vs. miR21-linked desCitrine in Hep3B cells.
FIGURE 11 is a schematic diagram showing miR-21-linked BH3 trap expression preventing cell death by SQT-BH3 in normal hepatocytes (miR122+/miR21-) but not hepatic carcinoma cells (HCC; miR122-/miR21+).
FIGURE 12 is a graph showing the intracellular expression of the SQT scaffold polypeptide in cells *in vitro* for half-life determination.
FIGURE 13 is a graph showing intracellular expression of the SQT scaffold polypeptide in liver cells *in vivo* after two successive doses of lipid nanoparticles carrying an mmRNA encoding the construct.
FIGURE 14 is a graph showing intracellular expression of the SQT scaffold in liver cells *in vivo* within the first 150 hours post-injection of lipid nanoparticles carrying an mmRNA encoding the construct.
FIGURE 15A-B are graphs showing apoptosis of Hep3B cells treated with increasing amounts of sorafenib (in µM) alone (no LNP) or in combination with LNPs containing mRNA constructs encoding either SQT-PUMA-BH3 (68pM) (Figure 15A) or SQT-BAD-BH3 (550pM) (Figure 15B).
FIGURE 16 is a graph showing tumor growth inhibition (TGI) in mice bearing subcutaneous Hep3B tumors treated with LNPs containing SQT-PUMA-BH3 (at 2 mg/kg or 3 mg/kg) or with PBS alone.
FIGURE 17 is a graph showing tumor growth inhibition (TGI) in mice bearing subcutaneous Hep3B tumors treated with sorafenib alone, LNPs containing SQT-PUMA-BH3 alone, or the combination of sorafenib and LNPs containing SQT-PUMA-BH3.
FIGURE 18 is a bar graph showing tumor growth inhibition (TGI) in mice bearing orthotopic Hep3B xenografts treated with LNPs containing increasing amounts of SQT-PUMA-BH3, as compared to treatment with PBS or with LNPs containing SQT alone.
FIGURE 19 is a graph showing tumor growth inhibition (TGI) in mice bearing systemic TFla tumors treated with LNPs containing SQT-PUMA-BH3, as compared to treatment with PBS alone, cytarabine alone or LNPs containing GFP.
FIGURES 20A-B are graphs showing tumor growth inhibition (TGI) in mice bearing systemic HEL tumors treated with LNPs containing SQT-PUMA-BH3, as compared to treatment with PBS alone or LNPs containing GFP. Figure 20A shows treatment at a dosage of 1.5 mg/kg for SQT-PUMA-BH3 and GFP. Figure 20B shows treatment at a dosage of 3 mg/kg for SQT-PUMA-BH3 and GFP.
FIGURES 21A-D are graphs showing the synergistic pro-apoptotic effect of targeting MCL1 in combination with SQT-PUMA-BH3 or SQT-Bad-BH3 in hepatocellular carcinoma cells (HCC) but not in primary hepatocytes. Figures 21A and 21C show Hep3B HCC cells. Figures 21B and 21D show primary hepatocytes. Figures 21A and 21B show SQT-PUMA-BH3. Figures 21C and 21D show SQT-Bad-BH3.
FIGURES 22A-B are bar graphs showing the synergistic pro-apoptotic effects of an anti-MCLl mRNA construct in combination with SQT-PUMA-BH3. Figure 22A shows anti-MCLl mRNA at 50 ng. Figure 22B shows anti-MCL mRNA at 12.5 ng.
FIGURES 23A-B are graphs showing mean BLI (photons/second) on Day 33 (Figure 23A) or Day 61 (Figure 23B) in mice bearing systemic TFla tumors treated with LNPs containing mRNA encoding SQT-PUMA-BH3, as compared to treatment with PBS alone, cytarabine alone or LNPs containing mRNA encoding GFP.
FIGURE 24 is a graph showing percent survival over time of mice bearing systemic TFla tumors treated with LNPs containing mRNA encoding SQT-PUMA-BH3, as compared to treatment with PBS alone, cytarabine alone or LNPs containing mRNA encoding GFP.
FIGURE 25 is a photograph of Western blot gels from experiments in which HCT116 cells were transfected with the indicated SQT-p53-inhibitory peptide constructs (SQT-MDM2 constructs), showing increased p53 and p21 levels in the cells as a result of the treatment. The bar graph below the gels shows p53 levels (normalized to vinculin) relative to the 10 µM nutlin-3a sample (positive control).
FIGURE 26 is a photograph of immunoprecipitation gels from experiments in which HCT116 cells were transfected with the indicated SQT-p53-inhibitory peptide constructs (SQT-MDM2 constructs), demonstrating binding of the SQT-p53-inhibitory peptide fusion proteins to MDM2.

### Detailed Description

Intracellular delivery of relatively small therapeutic polypeptides that can specifically bind to an intracellular target (e.g., scaffold polypeptides that present specific domains or motifs) is one approach to modulate such intracellular targets. For example, inhibition of anti-apoptotic Bcl-2 family proteins in cancer cells may induce apoptosis in cancer cells, including cancer cells that are resistant to conventional chemotherapies. In principle, introduction of an mRNA encoding such a therapeutic polypeptide into the cell may lead to translation of the therapeutic polypeptide within the cell, allowing it to modulate its intracellular target(s). Delivery of mRNA encoding such a therapeutic polypeptide has advantages over other nucleic acid delivery approaches known in the art, such as viruses (e.g., retroviruses), because delivery of mRNA typically does not lead to integration of the nucleic acid into the host cell's genome, allowing transient expression of the nucleic acid. However, the delivery of therapeutic RNAs to cells is generally considered difficult, for example, due to the relative instability and low cell permeability of RNAs.

The present disclosure includes compositions such as isolated mRNAs encoding a fusion polypeptide comprising a Stefin A scaffold polypeptide and one or more binding polypeptides, such as one or more BH3 domains. In addition, the present invention includes nanoparticles, e.g., lipid nanoparticles, that contain mRNAs encoding a fusion polypeptide comprising a Stefin A scaffold polypeptide and one or more binding polypeptides, such as one or more BH3 domains, as well as pharmaceutical compositions comprising any of these mRNAs or nanoparticles, e.g., lipid nanoparticles. In particular embodiments, the one or more binding polypeptides, e.g., BH3 domains, are located within the Stefin A scaffold polypeptide at an N-terminal insertion site, a loop 1 insertion site, and/or a loop 2 insertion site. This is achieved by inserting or fusing in-frame the binding polypeptide-encoding sequence(s) into the N-terminal insertion site, the loop 1 insertion site and/or the loop 2 insertion site of the Stefin A scaffold polypeptide-encoding sequence.

The disclosure further features methods of modulating a target to which a binding polypeptide binds in a cell by contacting the cell with a composition of the invention (e.g., an isolated mRNA or lipid nanoparticle). Regarding embodiments comprising a SteA fusion polypeptide comprising one or more BH3 domains, the invention further features methods of inducing apoptosis in a cell by contacting the cell with a composition of the invention (e.g., an isolated mRNA or a lipid nanoparticle). The invention also features methods of treating a patient suffering from cancer that involve administration of a composition of the invention, e.g., in a pharmaceutical composition further comprising one or more pharmaceutically acceptable carriers, diluents or excipients.

### mRNA

The invention provides isolated mRNAs, for example, mRNAs that encode a fusion polypeptide comprising a SteA scaffold polypeptide and one or more binding polypeptides, such as BH3 domains, as well as mRNAs that encode a Bcl-2-like polypeptide or a variant or fragment thereof. In certain embodiments, an isolated mRNA of the disclosure encodes both the fusion polypeptide comprising a SteA scaffold polypeptide and one or more BH3 domains, and the Bcl-2-like polypeptide or variant or fragment thereof.

An mRNA may be a naturally or non-naturally occurring mRNA. An mRNA may include one or more modified nucleobases, nucleosides, or nucleotides, as described below, in which case it may be referred to as a "modified mRNA" or "mmRNA." As described herein "nucleoside" is defined as a compound containing a sugar molecule (e.g., a pentose or ribose) or derivative thereof in combination with an organic base (e.g., a purine or pyrimidine) or a derivative thereof (also referred to herein as "nucleobase"). As described herein, "nucleotide" is defined as a nucleoside including a phosphate group.

An mRNA may include a 5' untranslated region (5'-UTR), a 3' untranslated region (3'-UTR), and/or a coding region (e.g., an open reading frame). An mRNA may include any suitable number of base pairs, including tens (e.g., 10, 20, 30, 40, 50, 60, 70, 80, 90 or 100), hundreds (e.g., 200, 300, 400, 500, 600, 700, 800, or 900) or thousands (e.g., 1000, 2000, 3000, 4000, 5000, 6000, 7000, 8000, 9000, 10,000) of base pairs. Any number (e.g., all, some, or none) of nucleobases, nucleosides, or nucleotides may be an analog of a canonical species, substituted, modified, or otherwise non-naturally occurring. In certain embodiments, all of a particular nucleobase type may be modified.

In some embodiments, an mRNA as described herein may include a 5' cap structure, a chain terminating nucleotide, optionally a Kozak sequence (also known as a Kozak consensus sequence), a stem loop, a polyA sequence, and/or a polyadenylation signal.

A 5' cap structure or cap species is a compound including two nucleoside moieties joined by a linker and may be selected from a naturally occurring cap, a non-naturally occurring cap or cap analog, or an anti-reverse cap analog (ARCA). A cap species may include one or more modified nucleosides and/or linker moieties. For example, a natural mRNA cap may include a guanine nucleotide and a guanine (G) nucleotide methylated at the 7 position joined by a triphosphate linkage at their 5' positions, e.g., m⁷G(5')ppp(5')G, commonly written as m⁷GpppG. A cap species may also be an anti-reverse cap analog. A non-limiting list of possible cap species includes m⁷GpppG, m⁷Gpppm⁷G, m⁷3ʹdGpppG, m₂^{7,O3ʹ} GpppG, m₂^{7,O3ʹ}GppppG, m₂^{7,O2ʹ}GppppG, m⁷Gpppm⁷G, m⁷ 3ʹdGpppG, m₂^{7,O3'}GpppG, m₂^{7,O3ʹ}GppppG, and m₂^{7,O2ʹ}GppppG.

An mRNA may instead or additionally include a chain terminating nucleoside. For example, a chain terminating nucleoside may include those nucleosides deoxygenated at the 2' and/or 3' positions of their sugar group. Such species may include 3'-deoxyadenosine (cordycepin), 3'-deoxyuridine, 3'-deoxycytosine, 3'-deoxyguanosine, 3'-deoxythymine, and 2',3'-dideoxynucleosides, such as 2',3'-dideoxyadenosine, 2',3'-dideoxyuridine, 2',3'-dideoxycytosine, 2',3'-dideoxyguanosine, and 2',3'-dideoxythymine. In some embodiments, incorporation of a chain terminating nucleotide into an mRNA, for example at the 3'-terminus, may result in stabilization of the mRNA, as described, for example, in International Patent Publication No. WO 2013/103659.

An mRNA may instead or additionally include a stem loop, such as a histone stem loop. A stem loop may include 2, 3, 4, 5, 6, 7, 8, or more nucleotide base pairs. For example, a stem loop may include 4, 5, 6, 7, or 8 nucleotide base pairs. A stem loop may be located in any region of an mRNA. For example, a stem loop may be located in, before, or after an untranslated region (i.e., a 5' untranslated region or a 3' untranslated region), a coding region, or a polyA sequence or tail. In some embodiments, a stem loop may affect one or more function(s) of an mRNA, such as initiation of translation, translation efficiency, and/or transcriptional termination.

An mRNA may instead or additionally include a polyA sequence and/or polyadenylation signal. A polyA sequence may be comprised entirely or mostly of adenine nucleotides or analogs or derivatives thereof. A polyA sequence may be a tail located adjacent to a 3' untranslated region of an mRNA. In some embodiments, a polyA sequence may affect the nuclear export, translation, and/or stability of an mRNA.

An mRNA may instead or additionally include a microRNA binding site.

In some embodiments, an mRNA is a bicistronic mRNA comprising a first coding region and a second coding region with an intervening sequence comprising an internal ribosome entry site (IRES) sequence that allows for internal translation initiation between the first and second coding regions, or with an intervening sequence encoding a self-cleaving peptide, such as a 2A peptide. IRES sequences and 2A peptides are typically used to enhance expression of multiple proteins from the same vector. A variety of IRES sequences are known and available in the art and may be used, including, e.g., the encephalomyocarditis virus IRES.

In one embodiment, the polynucleotides of the present invention may include a sequence encoding a self-cleaving peptide. The self-cleaving peptide may be, but is not limited to, a 2A peptide. A variety of 2A peptides are known and available in the art and may be used, including e.g., the foot and mouth disease virus (FMDV) 2A peptide, the equine rhinitis A virus 2A peptide, the *Thosea asigna* virus 2A peptide, and the porcine teschovirus-1 2A peptide. 2A peptides are used by several viruses to generate two proteins from one transcript by ribosome-skipping, such that a normal peptide bond is impaired at the 2A peptide sequence, resulting in two discontinuous proteins being produced from one translation event. As a non-limiting example, the 2A peptide may have the protein sequence: GSGATNFSLLKQAGDVEENPGP (SEQ ID NO: 35), fragments or variants thereof. In one embodiment, the 2A peptide cleaves between the last glycine and last proline. As another non-limiting example, the polynucleotides of the present invention may include a polynucleotide sequence encoding the 2A peptide having the protein sequence GSGATNFSLLKQAGDVEENPGP (SEQ ID NO: 35) fragments or variants thereof. One example of a polynucleotide sequence encoding the 2A peptide is:
GGAAGCGGAGCTACTAACTTCAGCCTGCTGAAGCAGGCTGGAGACGTGGAGGAG AACCCTGGACCT (SEQ ID NO: 36). In one illustrative embodiment, a 2A peptide is encoded by the following sequence: 5'-TCCGGACTCAGATCCGGGGATCTCAAAATTGTCGCTCCTGTCAAACAAACTCTTA ACTTTGATTTACTCAAACTGGCTGGGGATGTAGAAAGCAATCCAGGTCCACTC-3' (SEQ ID NO: 37). The polynucleotide sequence of the 2A peptide may be modified or codon optimized by the methods described herein and/or are known in the art.

In one embodiment, this sequence may be used to separate the coding regions of two or more polypeptides of interest. As a non-limiting example, the sequence encoding the F2A peptide may be between a first coding region A and a second coding region B (A-F2Apep-B). The presence of the F2A peptide results in the cleavage of the one long protein between the glycine and the proline at the end of the F2A peptide sequence (NPGP is cleaved to result in NPG and P) thus creating separate protein A (with 21 amino acids of the F2A peptide attached, ending with NPG) and separate protein B (with 1 amino acid, P, of the F2A peptide attached). Likewise, for other 2A peptides (P2A, T2A and E2A), the presence of the peptide in a long protein results in cleavage between the glycine and proline at the end of the 2A peptide sequence (NPGP is cleaved to result in NPG and P). Protein A and protein B may be the same or different peptides or polypeptides of interest. In particular embodiments, protein A and protein B are a fusion polypeptide comprising a SteA scaffold and a BH3 domain, and a Bcl-2-like polypeptide, in either order. In certain embodiments, the first coding region and the second coding region encode a fusion polypeptide comprising a SteA scaffold and a BH3 domain, and a Bcl-2-like polypeptide, in either order.

### Modified mRNAs

In some embodiments, an mRNA of the invention comprises one or more modified nucleobases, nucleosides, or nucleotides (termed "modified mRNAs" or "mmRNAs"). In some embodiments, modified mRNAs may have useful properties, including enhanced stability, intracellular retention, enhanced translation, and/or the lack of a substantial induction of the innate immune response of a cell into which the mRNA is introduced, as compared to a reference unmodified mRNA. Therefore, use of modified mRNAs may enhance the efficiency of protein production, intracellular retention of nucleic acids, as well as possess reduced immunogenicity.

In some embodiments, an mRNA includes one or more (e.g., 1, 2, 3 or 4) different modified nucleobases, nucleosides, or nucleotides. In some embodiments, an mRNA includes one or more (e.g., 1, 2, 3, 4, 5, 6, 7, 8, 9, 10, 20, 30, 40, 50, 60, 70, 80, 90, 100, or more) different modified nucleobases, nucleosides, or nucleotides. In some embodiments, the modified mRNA may have reduced degradation in a cell into which the mRNA is introduced, relative to a corresponding unmodified mRNA.

In some embodiments, the modified nucleobase is a modified uracil. Exemplary nucleobases and nucleosides having a modified uracil include pseudouridine (ψ), pyridin-4-one ribonucleoside, 5-aza-uridine, 6-aza-uridine, 2-thio-5-aza-uridine, 2-thio-uridine (s²U), 4-thio-uridine (s⁴U), 4-thio-pseudouridine, 2-thio-pseudouridine, 5-hydroxy-uridine (ho⁵U), 5-aminoallyl-uridine, 5-halo-uridine (e.g., 5-iodo-uridineor 5-bromo-uridine), 3-methyl-uridine (m³U), 5-methoxy-uridine (mo⁵U), uridine 5-oxyacetic acid (cmo⁵U), uridine 5-oxyacetic acid methyl ester (mcmo⁵U), 5-carboxymethyl-uridine (cm⁵U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm⁵U), 5-methoxycarbonylmethyl-2-thio-uridine (mcm⁵s²U), 5-aminomethyl-2-thio-uridine (nm⁵s²U), 5-methylaminomethyl-uridine (mnm⁵U), 5-methylaminomethyl-2-thio-uridine (mnm⁵s²U), 5-methylaminomethyl-2-seleno-uridine (mnm⁵se²U), 5-carbamoylmethyl-uridine (ncm⁵U), 5-carboxymethylaminomethyl-uridine (cmnm⁵U), 5-carboxymethylaminomethyl-2-thio-uridine (cmnm⁵s²U), 5-propynyl-uridine, 1-propynyl-pseudouridine, 5-taurinomethyl-uridine (τm⁵U), 1-taurinomethyl-pseudouridine, 5-taurinomethyl-2-thio-uridine(τm⁵s²U), 1-taurinomethyl-4-thio-pseudouridine, 5-methyl-uridine (m⁵U, i.e., having the nucleobase deoxythymine), 1-methyl-pseudouridine (m¹ψ), 5-methyl-2-thio-uridine (m⁵s²U), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m⁵D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp³U), 1-methyl-3-(3-amino-3-carboxypropyl)pseudouridine (acp³ ψ), 5-(isopentenylaminomethyl)uridine (inm⁵U), 5-(isopentenylaminomethyl)-2-thio-uridine (inm⁵s²U), α-thio-undine, 2'-O-methyl-uridine (Um), 5,2'-O-dimethyl-uridine (m⁵Um), 2'-O-methyl-pseudouridine (ψm), 2-thio-2'-O-methyl-uridine (s²Um), 5-methoxycarbonylmethyl-2'-O-methyl-uridine (mcm⁵Um), 5-carbamoylmethyl-2'-O-methyl-uridine (ncm⁵Um), 5-carboxymethylaminomethyl-2'-O-methyl-uridine (cmnm⁵Um), 3,2'-O-dimethyl-uridine (m³Um), and 5-(isopentenylaminomethyl)-2'-O-methyl-uridine (inm⁵Um), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino)]uridine.

In some embodiments, the modified nucleobase is a modified cytosine. Exemplary nucleobases and nucleosides having a modified cytosine include 5-aza-cytidine, 6-aza-cytidine, pseudoisocytidine, 3-methyl-cytidine (m³C), N4-acetyl-cytidine (ac⁴C), 5-formyl-cytidine (f⁵C), N4-methyl-cytidine (m⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, pyrrolo-cytidine, pyrrolo-pseudoisocytidine, 2-thio-cytidine (s²C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, lysidine (k₂C), α-thio-cytidine, 2'-O-methyl-cytidine (Cm), 5,2'-O-dimethyl-cytidine (m⁵Cm), N4-acetyl-2ʹ-O-methyl-cytidine (ac⁴Cm), N4,2'-O-dimethyl-cytidine (m⁴Cm), 5-formyl-2'-O-methyl-cytidine (f⁵Cm), N4,N4,2'-O-trimethyl-cytidine (m⁴₂Cm), 1-thio-cytidine, 2'-F-ara-cytidine, 2'-F-cytidine, and 2'-OH-ara-cytidine.

In some embodiments, the modified nucleobase is a modified adenine. Exemplary nucleobases and nucleosides having a modified adenine include α-thio-adenosine, 2-amino-purine, 2, 6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m¹A), 2-methyl-adenine (m²A), N6-methyl-adenosine (m⁶A), 2-methylthio-N6-methyl-adenosine (ms²m⁶A), N6-isopentenyl-adenosine (i⁶A), 2-methylthio-N6-isopentenyl-adenosine (ms²i⁶A), N6-(cis-hydroxyisopentenyl)adenosine (io⁶A), 2-methylthio-N6-(cis-hydroxyisopentenyl)adenosine (ms²io⁶A), N6-glycinylcarbamoyl-adenosine (g⁶A), N6-threonylcarbamoyl-adenosine (t⁶A), N6-methyl-N6-threonylcarbamoyl-adenosine (m⁶t⁶A), 2-methylthio-N6-threonylcarbamoyl-adenosine (ms²g⁶A), N6,N6-dimethyl-adenosine (m⁶₂A), N6-hydroxynorvalylcarbamoyl-adenosine (hn⁶A), 2-methylthio-N6-hydroxynorvalylcarbamoyl-adenosine (ms²hn⁶A), N6-acetyl-adenosine (ac⁶A), 7-methyl-adenine, 2-methylthio-adenine, 2-methoxy-adenine, α-thio-adenosine, 2'-O-methyl-adenosine (Am), N6,2'-O-dimethyl-adenosine (m⁶Am), N6,N6,2ʹ-O-trimethyl-adenosine (m⁶₂Am), 1,2ʹ-O-dimethyl-adenosine (m¹Am), 2'-O-ribosyladenosine (phosphate) (Ar(p)), 2-amino-N6-methyl-purine, 1-thio-adenosine, 8-azido-adenosine, 2'-F-ara-adenosine, 2'-F-adenosine, 2'-OH-ara-adenosine, and N6-(19-amino-pentaoxanonadecyl)-adenosine.

In some embodiments, the modified nucleobase is a modified guanine. Exemplary nucleobases and nucleosides having a modified guanine include α-thio-guanosine, inosine (I), 1-methyl-inosine (m¹I), wyosine (imG), methylwyosine (mimG), 4-demethyl-wyosine (imG-14), isowyosine (imG2), wybutosine (yW), peroxywybutosine (o₂yW), hydroxywybutosine (OhyW), undermodified hydroxywybutosine (OhyW*), 7-deaza-guanosine, queuosine (Q), epoxyqueuosine (oQ), galactosyl-queuosine (galQ), mannosylqueuosine (manQ), 7-cyano-7-deaza-guanosine (preQ₀), 7-aminomethyl-7-deaza-guanosine (preQ₁), archaeosine (G⁺), 7-deaza-8-aza-guanosine, 6-thio-guanosine, 6-thio-7-deaza-guanosine, 6-thio-7-deaza-8-aza-guanosine, 7-methyl-guanosine (m⁷G), 6-thio-7-methyl-guanosine, 7-methyl-inosine, 6-methoxy-guanosine, 1-methyl-guanosine (m¹G), N2-methyl-guanosine (m²G), N2,N2-dimethyl-guanosine (m²₂G), N2,7-dimethyl-guanosine (m^{2,7}G), N2, N2,7-dimethyl-guanosine (m^{2,2,7}G), 8-oxo-guanosine, 7-methyl-8-oxo-guanosine, 1-methyl-6-thio-guanosine, N2-methyl-6-thio-guanosine, N2,N2-dimethyl-6-thio-guanosine, α-thio-guanosine, 2'-O-methyl-guanosine (Gm), N2-methyl-2'-O-methyl-guanosine (m²Gm), N2,N2-dimethyl-2ʹ-O-methyl-guanosine (m²₂Gm), 1-methyl-2'-O-methyl-guanosine (m¹Gm), N2,7-dimethyl-2'-O-methyl-guanosine (m^{2,7}Gm), 2'-O-methyl-inosine (Im), 1,2'-O-dimethyl-inosine (m¹Im), 2'-O-ribosylguanosine (phosphate) (Gr(p)), 1-thio-guanosine, O6-methyl-guanosine, 2'-F-ara-guanosine, and 2'-F-guanosine.

In some embodiments, an mRNA of the invention includes a combination of one or more of the aforementioned modified nucleobases (e.g., a combination of 2, 3 or 4 of the aforementioned modified nucleobases.)

In some embodiments, the modified nucleobase is pseudouridine (ψ), N1-methylpseudouridine (m¹ψ), 2-thiouridine, 4'-thiouridine, 5-methylcytosine, 2-thio-1-methyl-1-deaza-pseudouridine, 2-thio-1-methyl-pseudouridine, 2-thio-5-aza-uridine , 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 5-methoxyuridine, or 2'-O-methyl uridine. In some embodiments, an mRNA of the invention includes a combination of one or more of the aforementioned modified nucleobases (e.g., a combination of 2, 3 or 4 of the aforementioned modified nucleobases.)

In some embodiments, the modified nucleobase is a modified cytosine. Exemplary nucleobases and nucleosides having a modified cytosine include N4-acetyl-cytidine (ac⁴C), 5-methyl-cytidine (m⁵C), 5-halo-cytidine (e.g., 5-iodo-cytidine), 5-hydroxymethyl-cytidine (hm⁵C), 1-methyl-pseudoisocytidine, 2-thio-cytidine (s²C), 2-thio-5-methyl-cytidine. In some embodiments, an mRNA of the invention includes a combination of one or more of the aforementioned modified nucleobases (e.g., a combination of 2, 3 or 4 of the aforementioned modified nucleobases.)

In some embodiments, the modified nucleobase is a modified adenine. Exemplary nucleobases and nucleosides having a modified adenine include 7-deaza-adenine, 1-methyl-adenosine (m¹A), 2-methyl-adenine (m²A), N6-methyl-adenosine (m⁶A). In some embodiments, an mRNA of the invention includes a combination of one or more of the aforementioned modified nucleobases (e.g., a combination of 2, 3 or 4 of the aforementioned modified nucleobases.)

In some embodiments, the modified nucleobase is a modified guanine. Exemplary nucleobases and nucleosides having a modified guanine include inosine (I), 1-methyl-inosine (m¹I), wyosine (imG), methylwyosine (mimG), 7-deaza-guanosine, 7-cyano-7-deaza-guanosine (preQ₀), 7-aminomethyl-7-deaza-guanosine (preQ₁), 7-methyl-guanosine (m⁷G), 1-methyl-guanosine (m¹G), 8-oxo-guanosine, 7-methyl-8-oxo-guanosine. In some embodiments, an mRNA of the invention includes a combination of one or more of the aforementioned modified nucleobases (e.g., a combination of 2, 3 or 4 of the aforementioned modified nucleobases.)

In some embodiments, the modified nucleobase is 1-methyl-pseudouridine (m¹ψ), 5-methoxy-uridine (mo⁵U), 5-methyl-cytidine (m⁵C), pseudouridine (ψ), α-thio-guanosine, or α-thio-adenosine. In some embodiments, an mRNA of the invention includes a combination of one or more of the aforementioned modified nucleobases (e.g., a combination of 2, 3 or 4 of the aforementioned modified nucleobases.)

In some embodiments, the mRNA comprises pseudouridine (ψ). In some embodiments, the mRNA comprises pseudouridine (ψ) and 5-methyl-cytidine (m⁵C). In some embodiments, the mRNA comprises 1-methyl-pseudouridine (m¹ψ). In some embodiments, the mRNA comprises 1-methyl-pseudouridine (m¹ψ) and 5-methyl-cytidine (m⁵C). In some embodiments, the mRNA comprises 2-thiouridine (s²U). In some embodiments, the mRNA comprises 2-thiouridine and 5-methyl-cytidine (m⁵C). In some embodiments, the mRNA comprises 5-methoxy-uridine (mo⁵U). In some embodiments, the mRNA comprises 5-methoxy-uridine (mo⁵U) and 5-methyl-cytidine (m⁵C). In some embodiments, the mRNA comprises 2'-O-methyl uridine. In some embodiments, the mRNA comprises 2'-O-methyl uridine and 5-methyl-cytidine (m⁵C). In some embodiments, the mRNA comprises comprises N6-methyl-adenosine (m⁶A). In some embodiments, the mRNA comprises N6-methyl-adenosine (m⁶A) and 5-methyl-cytidine (m⁵C).

In certain embodiments, an mRNA of the invention is uniformly modified (i.e., fully modified, modified through-out the entire sequence) for a particular modification. For example, an mRNA can be uniformly modified with 5-methyl-cytidine (m⁵C), meaning that all cytosine residues in the mRNA sequence are replaced with 5-methyl-cytidine (m⁵C). Similarly, mRNAs of the invention can be uniformly modified for any type of nucleoside residue present in the sequence by replacement with a modified residue such as those set forth above.

In some embodiments, an mRNA of the invention may be modified in a coding region (e.g., an open reading frame encoding a polypeptide). In other embodiments, an mRNA may be modified in regions besides a coding region. For example, in some embodiments, a 5'-UTR and/or a 3'-UTR are provided, wherein either or both may independently contain one or more different nucleoside modifications. In such embodiments, nucleoside modifications may also be present in the coding region.

Examples of nucleoside modifications and combinations thereof that may be present in mmRNAs of the present invention include, but are not limited to, those described in PCT Patent Application Publications: WO2012045075, WO2014081507, WO2014093924, WO2014164253, and WO2014159813.

The mmRNAs of the invention can include a combination of modifications to the sugar, the nucleobase, and/or the internucleoside linkage. These combinations can include any one or more modifications described herein.

Examples of modified nucleosides and modified nucleoside combinations are provided below in Table 1 and Table 2. These combinations of modified nucleotides can be used to form the mmRNAs of the invention. In certain embodiments, the modified nucleosides may be partially or completely substituted for the natural nucleotides of the mRNAs of the invention. As a non-limiting example, the natural nucleotide uridine may be substituted with a modified nucleoside described herein. In another non-limiting example, the natural nucleoside uridine may be partially substituted (e.g., about 0.1%, 1%, 5%, 10%, 15%, 20%, 25%, 30%, 35%, 40%, 45%, 50%, 55%, 60%, 65%, 70%, 75%, 80%, 85%, 90%, 95% or 99.9% of the natural uridines) with at least one of the modified nucleoside disclosed herein.

**Table 1 Combinations of Nucleoside Modifications**

| **Modified Nucleotide** | **Modified Nucleotide Combination** |
|---|---|
| α-thio-cytidine | α-thio-cytidine/5-iodo-uridine |
| | α-thio-cytidine/N1-methyl-pseudouridine |
| | α-thio-cytidine/α-thio-uridine |
| | α-thio-cytidine/5-methyl-uridine |
| | α-thio-cytidine/pseudo-uridine |
| | about 50% of the cytosines are α-thio-cytidine |
| pseudoisocytidine | pseudoisocytidine/5-iodo-uridine |
| | pseudoisocytidine/N1-methyl-pseudouridine |
| | pseudoisocytidine/α-thio-uridine |
| | pseudoisocytidine/5-methyl-uridine |
| | pseudoisocytidine/pseudouridine |
| | about 25% of cytosines are pseudoisocytidine |
| | pseudoisocytidine/about 50% of uridines are N1-methyl-pseudouridine and about 50% of uridines are pseudouridine |
| | pseudoisocytidine/about 25% of uridines are N1-methyl-pseudouridine and about 25% of uridines are pseudouridine |
| pyrrolo-cytidine | pyrrolo-cytidine/5-iodo-uridine |
| | pyrrolo-cytidine/N 1-methyl-pseudouridine |
| | pyrrolo-cytidine/α-thio-uridine |
| | pyrrolo-cytidine/5-methyl-uridine |
| | pyrrolo-cytidine/pseudouridine |
| | about 50% of the cytosines are pyrrolo-cytidine |
| 5-methyl-cytidine | 5-methyl-cytidine/5-iodo-uridine |
| | 5-methyl-cytidine/N1-methyl-pseudouridine |
| | 5-methyl-cytidine/α-thio-uridine |
| | 5-methyl-cytidine/5-methyl-uridine |
| | 5-methyl-cytidine/pseudouridine |
| | about 25% of cytosines are 5-methyl-cytidine |
| | about 50% of cytosines are 5-methyl-cytidine |
| | 5-methyl-cytidine/5-methoxy-uridine |
| | 5-methyl-cytidine/5-bromo-uridine |
| | 5-methyl-cytidine/2-thio- uridine |
| | 5-methyl-cytidine/about 50% of uridines are 2-thio- uridine |
| | about 50% of uridines are 5-methyl-cytidine/ about 50% of uridines are 2-thio-uridine |
| N4-acetyl-cytidine | N4-acetyl-cytidine /5-iodo-uridine |
| | N4-acetyl-cytidine /N1-methyl-pseudouridine |
| | N4-acetyl-cytidine /α-thio-uridine |
| | N4-acetyl-cytidine /5-methyl-uridine |
| | N4-acetyl-cytidine /pseudouridine |
| | about 50% of cytosines are N4-acetyl-cytidine |
| | about 25% of cytosines are N4-acetyl-cytidine |
| | N4-acetyl-cytidine /5-methoxy-uridine |
| | N4-acetyl-cytidine /5 - bromo- uridine |
| | N4-acetyl-cytidine /2-thio-uridine |
| | about 50% of cytosines are N4-acetyl-cytidine/ about 50% of uridines are 2-thio-uridine |

**Table 2. Modified Nucleosides and Combinations Thereof**

| |
|---|
| 1-(2,2,2-Trifluoroethyl)pseudo-UTP |
| 1-Ethyl-pseudo-UTP |
| 1-Methyl-pseudo-U-alpha-thio-TP |
| 1-methyl-pseudouridine TP, ATP, GTP, CTP |
| 1-methyl-pseudo-UTP/5-methyl-CTP/ATP/GTP |
| 1-methyl-pseudo-UTP/CTP/ATP/GTP |
| 1-Propyl-pseudo-UTP |
| 25 % 5-Aminoallyl-CTP + 75 % CTP/25 % 5-Methoxy-UTP + 75 % UTP |
| 25 % 5-Aminoallyl-CTP + 75 % CTP/75 % 5-Methoxy-UTP + 25 % UTP |
| 25 % 5-Bromo-CTP + 75 % CTP/ 25 % 5-Methoxy-UTP + 75 % UTP |
| 25 % 5-Bromo-CTP + 75 % CTP/ 75 % 5-Methoxy-UTP + 25 % UTP |
| 25 % 5-Bromo-CTP + 75 % CTP/1-Methyl-pseudo-UTP |
| 25 % 5-Carboxy-CTP + 75 % CTP/ 25 % 5-Methoxy-UTP + 75 % UTP |
| 25 % 5-Carboxy-CTP + 75 % CTP/ 75 % 5-Methoxy-UTP + 25 % UTP |
| 25 % 5-Ethyl-CTP + 75 % CTP/ 25 % 5-Methoxy-UTP + 75 % UTP |
| 25 % 5-Ethyl-CTP + 75 % CTP/ 75 % 5-Methoxy-UTP + 25 % UTP |
| 25 % 5-Ethynyl-CTP + 75 % CTP/ 25 % 5-Methoxy-UTP + 75 % UTP |
| 25 % 5-Ethynyl-CTP + 75 % CTP/ 75 % 5-Methoxy-UTP + 25 % UTP |
| 25 % 5-Fluoro-CTP + 75 % CTP/ 25 % 5-Methoxy-UTP + 75 % UTP |
| 25 % 5-Fluoro-CTP + 75 % CTP/ 75 % 5-Methoxy-UTP + 25 % UTP |
| 25 % 5-Formyl-CTP + 75 % CTP/ 25 % 5-Methoxy-UTP + 75 % UTP |
| 25 % 5-Formyl-CTP + 75 % CTP/ 75 % 5-Methoxy-UTP + 25 % UTP |
| 25 % 5-Hydroxymethyl-CTP + 75 % CTP/ 25 % 5-Methoxy-UTP + 75 % UTP |
| 25 % 5-Hydroxymethyl-CTP + 75 % CTP/ 75 % 5-Methoxy-UTP + 25 % UTP |
| 25 % 5-Iodo-CTP + 75 % CTP/ 25 % 5-Methoxy-UTP + 75 % UTP |
| 25 % 5-Iodo-CTP + 75 % CTP/ 75 % 5-Methoxy-UTP + 25 % UTP |
| 25 % 5-Methoxy-CTP + 75 % CTP/ 25 % 5-Methoxy-UTP + 75 % UTP |
| 25 % 5-Methoxy-CTP + 75 % CTP/ 75 % 5-Methoxy-UTP + 25 % UTP |
| 25 % 5-Methyl-CTP + 75 % CTP/25 % 5-Methoxy-UTP + 75 % 1-Methyl-pseudo-UTP |
| 25 % 5-Methyl-CTP + 75 % CTP/25 % 5-Methoxy-UTP + 75 % UTP |
| 25 % 5-Methyl-CTP + 75 % CTP/50 % 5-Methoxy-UTP + 50 % 1-Methyl-pseudo-UTP |
| 25 % 5-Methyl-CTP + 75 % CTP/50 % 5-Methoxy-UTP + 50 % UTP |
| 25 % 5-Methyl-CTP + 75 % CTP/5-Methoxy-UTP |
| 25 % 5-Methyl-CTP + 75 % CTP/75 % 5-Methoxy-UTP + 25 % 1-Methyl-pseudo-UTP |
| 25 % 5-Methyl-CTP + 75 % CTP/75 % 5-Methoxy-UTP + 25 % UTP |
| 25 % 5-Phenyl-CTP + 75 % CTP/ 25 % 5-Methoxy-UTP + 75 % UTP |
| 25 % 5-Phenyl-CTP + 75 % CTP/ 75 % 5-Methoxy-UTP + 25 % UTP |
| 25 % 5-Trifluoromethyl-CTP + 75 % CTP/ 25 % 5-Methoxy-UTP + 75 % UTP |
| 25 % 5-Trifluoromethyl-CTP + 75 % CTP/ 75 % 5-Methoxy-UTP + 25 % UTP |
| 25 % 5-Trifluoromethyl-CTP + 75 % CTP/1-Methyl-pseudo-UTP |
| 25 % N4-Ac-CTP + 75 % CTP/ 25 % 5-Methoxy-UTP + 75 % UTP |
| 25 % N4-Ac-CTP + 75 % CTP/ 75 % 5-Methoxy-UTP + 25 % UTP |
| 25 % N4-Bz-CTP + 75 % CTP/ 25 % 5-Methoxy-UTP + 75 % UTP |
| 25 % N4-Bz-CTP + 75 % CTP/ 75 % 5-Methoxy-UTP + 25 % UTP |
| 25 % N4-Methyl-CTP + 75 % CTP/ 25 % 5-Methoxy-UTP + 75 % UTP |
| 25 % N4-Methyl-CTP + 75 % CTP/ 75 % 5-Methoxy-UTP + 25 % UTP |
| 25 % Pseudo-iso-CTP + 75 % CTP/ 25 % 5-Methoxy-UTP + 75 % UTP |
| 25 % Pseudo-iso-CTP + 75 % CTP/ 75 % 5-Methoxy-UTP + 25 % UTP |
| 25% 5-Bromo-CTP/75% CTP/ Pseudo-UTP |
| 25% 5-methoxy-UTP/25% 5-methyl-CTP/ATP/GTP |
| 25% 5-methoxy-UTP/5-methyl-CTP/ATP/GTP |
| 25% 5-methoxy-UTP/75% 5-methyl-CTP/ATP/GTP |
| 25% 5-methoxy-UTP/CTP/ATP/GTP |
| 25% 5-metoxy-UTP/50% 5-methyl-CTP/ATP/GTP |
| 2-Amino-ATP |
| 2-Thio-CTP |
| 2-thio-pseudouridine TP, ATP, GTP, CTP |
| 2-Thio-pseudo-UTP |
| 2-Thio-UTP |
| 3-Methyl-CTP |
| 3-Methyl-pseudo-UTP |
| 4-Thio-UTP |
| 50 % 5-Bromo-CTP + 50 % CTP/1-Methyl-pseudo-UTP |
| 50 % 5-Hydroxymethyl-CTP + 50 % CTP/1-Methyl-pseudo-UTP |
| 50 % 5-methoxy-UTP/5-methyl-CTP/ATP/GTP |
| 50 % 5-Methyl-CTP + 50 % CTP/25 % 5-Methoxy-UTP + 75 % 1-Methyl-pseudo-UTP |
| 50 % 5-Methyl-CTP + 50 % CTP/25 % 5-Methoxy-UTP + 75 % UTP |
| 50 % 5-Methyl-CTP + 50 % CTP/50 % 5-Methoxy-UTP + 50 % 1-Methyl-pseudo-UTP |
| 50 % 5-Methyl-CTP + 50 % CTP/50 % 5-Methoxy-UTP + 50 % UTP |
| 50 % 5-Methyl-CTP + 50 % CTP/5-Methoxy-UTP |
| 50 % 5-Methyl-CTP + 50 % CTP/75 % 5-Methoxy-UTP + 25 % 1-Methyl-pseudo-UTP |
| 50 % 5-Methyl-CTP + 50 % CTP/75 % 5-Methoxy-UTP + 25 % UTP |
| 50 % 5-Trifluoromethyl-CTP + 50 % CTP/1-Methyl-pseudo-UTP |
| 50% 5-Bromo-CTP/ 50% CTP/Pseudo-UTP |
| 50% 5-methoxy-UTP/25% 5-methyl-CTP/ATP/GTP |
| 50% 5-methoxy-UTP/50% 5-methyl-CTP/ATP/GTP |
| 50% 5-methoxy-UTP/75% 5-methyl-CTP/ATP/GTP |
| 50% 5-methoxy-UTP/CTP/ATP/GTP |
| 5-Aminoallyl-CTP |
| 5-Aminoallyl-CTP/ 5-Methoxy-UTP |
| 5-Aminoallyl-UTP |
| 5-Bromo-CTP |
| 5-Bromo-CTP/ 5-Methoxy-UTP |
| 5-Bromo-CTP/1-Methyl-pseudo-UTP |
| 5-Bromo-CTP/Pseudo-UTP |
| 5-bromocytidine TP, ATP, GTP, UTP |
| 5-Bromo-UTP |
| 5-Carboxy-CTP/ 5-Methoxy-UTP |
| 5-Ethyl-CTP/5-Methoxy-UTP |
| 5-Ethynyl-CTP/5-Methoxy-UTP |
| 5-Fluoro-CTP/ 5-Methoxy-UTP |
| 5-Formyl-CTP/ 5-Methoxy-UTP |
| 5-Hydroxymethyl-CTP/ 5-Methoxy-UTP |
| 5-Hydroxymethyl-CTP |
| 5-Hydroxymethyl-CTP/1-Methyl-pseudo-UTP |
| 5-Hydroxymethyl-CTP/5-Methoxy-UTP |
| 5-hydroxymethyl-cytidine TP, ATP, GTP, UTP |
| 5-Iodo-CTP/ 5-Methoxy-UTP |
| 5-Me-CTP/5-Methoxy-UTP |
| 5-Methoxy carbonyl methyl-UTP |
| 5-Methoxy-CTP/5-Methoxy-UTP |
| 5-methoxy-uridine TP, ATP, GTP, UTP |
| 5-methoxy-UTP |
| 5-Methoxy-UTP |
| 5-Methoxy-UTP/ N6-Isopentenyl-ATP |
| 5-methoxy-UTP/25% 5-methyl-CTP/ATP/GTP |
| 5-methoxy-UTP/5-methyl-CTP/A TP/GTP |
| 5-methoxy-UTP/75% 5-methyl-CTP/ATP/GTP |
| 5-methoxy-UTP/CTP/ATP/GTP |
| 5-Methyl-2-thio-UTP |
| 5-Methylaminomethyl-UTP |
| 5-Methyl-CTP/ 5-Methoxy-UTP |
| 5-Methyl-CTP/ 5-Methoxy-UTP(cap 0) |
| 5-Methyl-CTP/ 5-Methoxy-UTP(No cap) |
| 5-Methyl-CTP/25 % 5-Methoxy-UTP + 75 % 1-Methyl-pseudo-UTP |
| 5-Methyl-CTP/25 % 5-Methoxy-UTP + 75 % UTP |
| 5-Methyl-CTP/50 % 5-Methoxy-UTP + 50 % 1-Methyl-pseudo-UTP |
| 5-Methyl-CTP/50 % 5-Methoxy-UTP + 50 % UTP |
| 5-Methyl-CTP/5-Methoxy-UTP/N6-Me-ATP |
| 5-Methyl-CTP/75 % 5-Methoxy-UTP + 25 % 1-Methyl-pseudo-UTP |
| 5-Methyl-CTP/75 % 5-Methoxy-UTP + 25 % UTP |
| 5-Phenyl-CTP/ 5-Methoxy-UTP |
| 5-Trifluoromethyl-CTP/ 5-Methoxy-UTP |
| 5-Trifluoromethyl-CTP |
| 5-Trifluoromethyl-CTP/ 5-Methoxy-UTP |
| 5-Trifluoromethyl-CTP/1-Methyl-pseudo-UTP |
| 5-Trifluoromethyl-CTP/Pseudo-UTP |
| 5-Trifluoromethyl-UTP |
| 5-trifluromethylcytidine TP, ATP, GTP, UTP |
| 75 % 5-Aminoallyl-CTP + 25 % CTP/25 % 5-Methoxy-UTP + 75 % UTP |
| 75 % 5-Aminoallyl-CTP + 25 % CTP/75 % 5-Methoxy-UTP + 25 % UTP |
| 75 % 5-Bromo-CTP + 25 % CTP/ 25 % 5-Methoxy-UTP + 75 % UTP |
| 75 % 5-Bromo-CTP + 25 % CTP/ 75 % 5-Methoxy-UTP + 25 % UTP |
| 75 % 5-Carboxy-CTP + 25 % CTP/ 25 % 5-Methoxy-UTP + 75 % UTP |
| 75 % 5-Carboxy-CTP + 25 % CTP/ 75 % 5-Methoxy-UTP + 25 % UTP |
| 75 % 5-Ethyl-CTP + 25 % CTP/ 25 % 5-Methoxy-UTP + 75 % UTP |
| 75 % 5-Ethyl-CTP + 25 % CTP/ 75 % 5-Methoxy-UTP + 25 % UTP |
| 75 % 5-Ethynyl-CTP + 25 % CTP/ 25 % 5-Methoxy-UTP + 75 % UTP |
| 75 % 5-Ethynyl-CTP + 25 % CTP/ 75 % 5-Methoxy-UTP + 25 % UTP |
| 75 % 5-Fluoro-CTP + 25 % CTP/ 25 % 5-Methoxy-UTP + 75 % UTP |
| 75 % 5-Fluoro-CTP + 25 % CTP/ 75 % 5-Methoxy-UTP + 25 % UTP |
| 75 % 5-Formyl-CTP + 25 % CTP/ 25 % 5-Methoxy-UTP + 75 % UTP |
| 75 % 5-Formyl-CTP + 25 % CTP/ 75 % 5-Methoxy-UTP + 25 % UTP |
| 75 % 5-Hydroxymethyl-CTP + 25 % CTP/25 % 5-Methoxy-UTP + 75 % UTP |
| 75 % 5-Hydroxymethyl-CTP + 25 % CTP/75 % 5-Methoxy-UTP + 25 % UTP |
| 75 % 5-Iodo-CTP + 25 % CTP/25 % 5-Methoxy-UTP + 75 % UTP |
| 75 % 5-Iodo-CTP + 25 % CTP/75 % 5-Methoxy-UTP + 25 % UTP |
| 75 % 5-Methoxy-CTP + 25 % CTP/25 % 5-Methoxy-UTP + 75 % UTP |
| 75 % 5-Methoxy-CTP + 25 % CTP/75 % 5-Methoxy-UTP + 25 % UTP |
| 75 % 5-methoxy-UTP/5-methyl-CTP/ATP/GTP |
| 75 % 5-Methyl-CTP + 25 % CTP/25 % 5-Methoxy-UTP + 75 % 1-Methyl-pseudo- UTP |
| 75 % 5-Methyl-CTP + 25 % CTP/25 % 5-Methoxy-UTP + 75 % UTP |
| 75 % 5-Methyl-CTP + 25 % CTP/50 % 5-Methoxy-UTP + 50 % 1-Methyl-pseudo-UTP |
| 75 % 5-Methyl-CTP + 25 % CTP/50 % 5-Methoxy-UTP + 50 % UTP |
| 75 % 5-Methyl-CTP + 25 % CTP/5-Methoxy-UTP |
| 75 % 5-Methyl-CTP + 25 % CTP/75 % 5-Methoxy-UTP + 25 % 1-Methyl-pseudo- UTP |
| 75 % 5-Methyl-CTP + 25 % CTP/75 % 5-Methoxy-UTP + 25 % UTP |
| 75 % 5-Phenyl-CTP + 25 % CTP/ 25 % 5-Methoxy-UTP + 75 % UTP |
| 75 % 5-Phenyl-CTP + 25 % CTP/ 75 % 5-Methoxy-UTP + 25 % UTP |
| 75 % 5-Trifluoromethyl-CTP + 25 % CTP/ 25 % 5-Methoxy-UTP + 75 % UTP |
| 75 % 5-Trifluoromethyl-CTP + 25 % CTP/ 75 % 5-Methoxy-UTP + 25 % UTP |
| 75 % 5-Trifluoromethyl-CTP + 25 % CTP/1-Methyl-pseudo-UTP |
| 75 % N4-Ac-CTP + 25 % CTP/25 % 5-Methoxy-UTP + 75 % UTP |
| 75 % N4-Ac-CTP + 25 % CTP/75 % 5-Methoxy-UTP + 25 % UTP |
| 75 % N4-Bz-CTP + 25 % CTP/25 % 5-Methoxy-UTP + 75 % UTP |
| 75 % N4-Bz-CTP + 25 % CTP/75 % 5-Methoxy-UTP + 25 % UTP |
| 75 % N4-Methyl-CTP + 25 % CTP/25 % 5-Methoxy-UTP + 75 % UTP |
| 75 % N4-Methyl-CTP + 25 % CTP/75 % 5-Methoxy-UTP + 25 % UTP |
| 75 % Pseudo-iso-CTP + 25 % CTP/25 % 5-Methoxy-UTP + 75 % UTP |
| 75 % Pseudo-iso-CTP + 25 % CTP/75 % 5-Methoxy-UTP + 25 % UTP |
| 75% 5-Bromo-CTP/25% CTP/ 1-Methyl-pseudo-UTP |
| 75% 5-Bromo-CTP/25% CTP/ Pseudo-UTP |
| 75% 5-methoxy-UTP/25% 5-methyl-CTP/ATP/GTP |
| 75% 5-methoxy-UTP/50% 5-methyl-CTP/ATP/GTP |
| 75% 5-methoxy-UTP/75% 5-methyl-CTP/ATP/GTP |
| 75% 5-methoxy-UTP/CTP/ATP/GTP |
| 8-Aza-ATP |
| Alpha-thio-CTP |
| CTP/25 % 5-Methoxy-UTP + 75 % 1-Methyl-pseudo-UTP |
| CTP/25 % 5-Methoxy-UTP + 75 % UTP |
| CTP/50 % 5-Methoxy-UTP + 50 % 1-Methyl-pseudo-UTP |
| CTP/50 % 5-Methoxy-UTP + 50 % UTP |
| CTP/5-Methoxy-UTP |
| CTP/5-Methoxy-UTP (cap 0) |
| CTP/5-Methoxy-UTP(No cap) |
| CTP/75 % 5-Methoxy-UTP + 25 % 1-Methyl-pseudo-UTP |
| CTP/75 % 5-Methoxy-UTP + 25 % UTP |
| CTP/UTP(No cap) |
| Nl-Me-GTP |
| N4-Ac-CTP |
| N4Ac-CTP/1-Methyl-pseudo-UTP |
| N4Ac-CTP/5-Methoxy- UTP |
| N4-acetyl-cytidine TP, ATP, GTP, UTP |
| N4-Bz-CTP/ 5-Methoxy-UTP |
| N4-methyl CTP |
| N4-Methyl-CTP/ 5-Methoxy-UTP |
| Pseudo-iso-CTP/ 5-Methoxy-UTP |
| PseudoU-alpha-thio-TP |
| pseudouridine TP, ATP, GTP, CTP |
| pseudo-UTP/5-methyl-CTP/ ATP/GTP |
| UTP-5-oxyacetic acid Me ester |
| Xanthosine |

According to the invention, polynucleotides of the invention may be synthesized to comprise the combinations or single modifications of Table 1 or Table 2.

Where a single modification is listed, the listed nucleoside or nucleotide represents 100 percent of that A, U, G or C nucleotide or nucleoside having been modified. Where percentages are listed, these represent the percentage of that particular A, U, G or C nucleobase triphosphate of the total amount of A, U, G, or C triphosphate present. For example, the combination: 25 % 5-Aminoallyl-CTP + 75 % CTP/ 25 % 5-Methoxy-UTP + 75 % UTP refers to a polynucleotide where 25% of the cytosine triphosphates are 5-Aminoallyl-CTP while 75% of the cytosines are CTP; whereas 25% of the uracils are 5-methoxy UTP while 75% of the uracils are UTP. Where no modified UTP is listed then the naturally occurring ATP, UTP, GTP and/or CTP is used at 100% of the sites of those nucleotides found in the polynucleotide. In this example all of the GTP and ATP nucleotides are left unmodified.

The mRNAs of the present invention, or regions thereof, may be codon optimized. Codon optimization methods are known in the art and may be useful for a variety of purposes: matching codon frequencies in host organisms to ensure proper folding, bias GC content to increase mRNA stability or reduce secondary structures, minimize tandem repeat codons or base runs that may impair gene construction or expression, customize transcriptional and translational control regions, insert or remove proteins trafficking sequences, remove/add post translation modification sites in encoded proteins (e.g., glycosylation sites), add, remove or shuffle protein domains, insert or delete restriction sites, modify ribosome binding sites and mRNA degradation sites, adjust translation rates to allow the various domains of the protein to fold properly, or to reduce or eliminate problem secondary structures within the polynucleotide. Codon optimization tools, algorithms and services are known in the art; non-limiting examples include services from GeneArt (Life Technologies), DNA2.0 (Menlo Park, CA) and/or proprietary methods. In one embodiment, the mRNA sequence is optimized using optimization algorithms, e.g., to optimize expression in mammalian cells or enhance mRNA stability.

In certain embodiments, the present disclosure includes polynucleotides having at least 80%, at least 85%, at least 90%, at least 95%, at least 98%, or at least 99% sequence identity to any of the polynucleotide sequences described herein.

mRNAs of the present invention may be produced by means available in the art, including but not limited to *in vitro* transcription (IVT) and synthetic methods. Enzymatic (IVT), solid-phase, liquid-phase, combined synthetic methods, small region synthesis, and ligation methods may be utilized. In one embodiment, mRNAs are made using IVT enzymatic synthesis methods. Methods of making polynucleotides by IVT are known in the art and are described in International Application PCT/US2013/30062. Accordingly, the present disclosure also includes polynucleotides, e.g., DNA, constructs and vectors that may be used to *in vitro* transcribe an mRNA described herein.

Non-natural modified nucleobases may be introduced into polynucleotides, e.g., mRNA, during synthesis or post-synthesis. In certain embodiments, modifications may be on internucleoside linkages, purine or pyrimidine bases, or sugar. In particular embodiments, the modification may be introduced at the terminal of a polynucleotide chain or anywhere else in the polynucleotide chain; with chemical synthesis or with a polymerase enzyme. Examples of modified nucleic acids and their synthesis are disclosed in PCT application No. PCT/US2012/058519. Synthesis of modified polynucleotides is also described in Verma and Eckstein, Annual Review of Biochemistry, vol. 76, 99-134 (1998).

Either enzymatic or chemical ligation methods may be used to conjugate polynucleotides or their regions with different functional moieties, such as targeting or delivery agents, fluorescent labels, liquids, nanoparticles, etc. Conjugates of polynucleotides and modified polynucleotides are reviewed in Goodchild, Bioconjugate Chemistry, vol. 1(3), 165-187 (1990).

### microRNA Binding Sites

microRNAs (or miRNA) are 19-25 nucleotide long (commonly 19-23 nucleotides long, most typically 22 nucleotides long) noncoding RNAs that bind to the 3'UTR of nucleic acid molecules and post-translationally down-regulate gene expression either by reducing nucleic acid molecule stability or by inhibiting translation. The mRNAs of the invention may comprise one or more microRNA target sequences or sites, microRNA binding sequences or sites, sequence complementary to a microRNA sequences, or sequence complementary to a microRNA seed region or sequence. Such sequences may correspond to any known microRNA such as those taught in US Publication US2005/0261218 and US Publication US2005/0059005. A microRNA sequence comprises a "seed" region or sequence, i.e., a sequence in the region of positions 2-8 of the mature microRNA, which sequence has perfect Watson-Crick complementarity to the miRNA target sequence. The bases of the microRNA seed region or sequence have complete complementarity with the target sequence. microRNAs derive enzymatically from regions of RNA transcripts that fold back on themselves to form short hairpin structures often termed a pre-miRNA (precursor-miRNA). The pre-miRNA typically has a two-nucleotide overhang at its 3' end, and has 3' hydroxyl and 5' phosphate groups. This precursor-mRNA is processed in the nucleus and subsequently transported to the cytoplasm where it is further processed by DICER (a RNase III enzyme), to form a mature microRNA of approximately 22 nucleotides. The mature microRNA is then incorporated into a ribonuclear particle to form the RNA-induced silencing complex, RISC, which mediates gene silencing. Art-recognized nomenclature for mature miRNAs typically designates the arm of the pre-miRNA from which the mature miRNA derives; "5p" means the microRNA is from the 5 prime arm of the pre-miRNA hairpin and "3p" means the microRNA is from the 3 prime end of the pre-miRNA hairpin.

In some embodiments, an mRNA of the invention may include one or more microRNA (miRNA) binding sites. In some embodiments, a miRNA binding site includes a sequence that has complementarity (e.g., partial or complete complementarity) with an miRNA seed sequence. In particular embodiments, the miRNA binding site includes a sequence that has complete complementarity with a miRNA seed sequence. In some embodiments, a miRNA binding site includes a sequence that has complementarity (e.g., partial or complete complementarity) with an miRNA sequence. In particular embodiments, the miRNA binding site includes a sequence that has complete complementarity with a miRNA sequence. In some embodiments, a miRNA binding site has complete complementarity with a miRNA sequence but for 1, 2 or 3 nucleotide substitutions, terminal additions, and/or truncations. Incorporation of one or more miRNA binding sites into an mRNA of the invention (e.g., an mRNA encoding a SteA scaffold polypeptide that includes one or more BH3 domains) may target the molecule for degradation or reduced translation, provided the miRNA in question is available (e.g., expressed in a target cell or tissue.) In some embodiments, incorporation of one or more miRNA binding site into an mRNA of the invention may reduce the hazard of off-target effects upon nucleic acid molecule delivery and/or enable tissue-specific regulation of expression of a polypeptide encoded by the mRNA.

In some embodiments, an mRNA of the invention (e.g., an mRNA encoding a SteA scaffold polypeptide that includes one or more BH3 domains or a Bcl-2-like polypeptide) may include one or more microRNA binding sites. For example, an mRNA may include 1, 2, 3, 4, 5, 6, 7, 8, 9, or 10 miRNA binding sites. In some embodiments, the miRNA binding sites are the same. In other embodiments, the mRNA may include one or more (e.g., 1, 2, 3, 4, 5, 6, 7, 8, 9, or 10) different miRNA binding sites, for example, miRNA binding sites of different miRNAs. In some embodiments, the miRNA binding site may be present in a 3'-UTR of an mRNA of the invention. For example, in some embodiments, an mRNA may include 1, 2, 3, 4, 5, 6, 7, 8, 9, or 10 miRNA binding sites in a 3'-UTR.

Examples of tissues where microRNA are known to regulate mRNA, and thereby protein expression, include, but are not limited to, liver (e.g., miR-122), muscle (e.g., miR-133, miR-206, and miR-208), endothelial cells (e.g., miR-17-92, and miR-126), myeloid cells (e.g., miR-142-3p, miR-142-5p, miR-16, miR-21, miR-223, miR-24, and miR-27), adipose tissue (e.g., let-7, and miR-30c), heart (e.g., miR-1d and miR-149), kidney (e.g., miR-192, miR-194, and miR-204), and lung epithelial cells (e.g., let-7, miR-133, and miR-126).

In some embodiments, the therapeutic window and/or differential expression (e.g., tissue-specific expression) of a polypeptide of the invention (e.g., a SteA scaffold polypeptide that includes one or more BH3 domains or a Bcl-2-like polypeptide) may be altered by incorporation of a miRNA binding site into an mRNA encoding the polypeptide. In one example, an mRNA may include one or more miRNA binding sites that are bound by miRNAs that have higher expression in one tissue type as compared to another. In another example, an mRNA may include one or more miRNA binding sites that are bound by miRNAs that have lower expression in a cancer cell as compared to a non-cancerous cell of the same tissue of origin. When present in a cancer cell that expresses low levels of such an miRNA, the polypeptide encoded by the mRNA typically will show increased expression. If the polypeptide is able to induce apoptosis, for example, by inhibiting an anti-apoptotic Bcl-2 family member and/or by activating a pro-apoptotic Bcl-2 family member, this may result in preferential cell killing of cancer cells as compared to normal cells.

Liver cancer cells (e.g., hepatocellular carcinoma cells) typically express low levels of miR-122 as compared to normal liver cells. Therefore, an mRNA encoding a polypeptide (e.g., an mRNA encoding a SteA scaffold polypeptide) that includes at least one miR-122 binding site (e.g., in the 3'-UTR of the mRNA) will typically express comparatively low levels of the polypeptide in normal liver cells and comparatively high levels of the polypeptide in liver cancer cells. If the polypeptide is able to induce apoptosis (such as a SteA scaffold polypeptide including one or more BH3 domains, as described herein), this can cause preferential cell killing of liver cancer cells (e.g., hepatocellular carcinoma cells) as compared to normal liver cells.

Liver cancer cells (e.g., hepatocellular carcinoma cells) typically express high levels of miR-21 as compared to normal liver cells. Therefore, an mRNA encoding a polypeptide (e.g., a Bcl-2-like polypeptide) that includes at least one miR-21 binding site (e.g., in the 3'-UTR of the mRNA) will typically express comparatively high levels of the polypeptide in normal liver cells and comparatively low levels of the polypeptide in liver cancer cells. If the polypeptide is able to inhibit apoptosis (e.g., by inhibiting activity of a fusion polypeptide including one or more BH3 domains, as described herein), this can further cause preferential cell killing of liver cancer cells (e.g., hepatocellular carcinoma cells) as compared to normal liver cells. For example, in normal liver cells, the Bcl-2-like-polypeptide (or BH3-trap) will be expressed and inhibit apoptosis induced by SteA-BH3 fusion polypeptide expressed in the normal liver cells.

In particular embodiments, the present disclosure contemplates the use of two or more mRNAs, wherein the first mRNA encodes SteA-BH3 fusion polypeptide and the second mRNA encodes an inhibitor of the SteA-BH3 polypeptide (e.g., a BH3-trap polypeptide), such as a Bcl-2-like polypeptide, or a variant or fragment thereof. In particular embodiments, when expressed in the same cell, the BH3-trap polypeptide binds the BH3 domain of the SteA-BH3 fusion polypeptide, thus preventing it from binding or inhibiting anti-apoptotic Bcl-2 family proteins present in the cell. In particular embodiments, the first mRNA encoding the SteA-BH3 polypeptide comprises one or more regulatory sequences to enhance expression in cancer cells as compared to normal cells. In particular embodiments, the second mRNA encoding the BH3-trap polypeptide comprises one or more regulatory sequences to reduce expression in cancer cells as compared to normal cells. In particular embodiments, the first mRNA comprises at least one first microRNA binding site, wherein the cognate microRNA that binds the first microRNA binding site is preferentially expressed in normal cells as compared to cancer cells. In particular embodiments, the second mRNA comprises at least one second microRNA binding site, wherein the cognate microRNA that binds the second microRNA binding site is preferentially expressed in cancer cells as compared to normal cells. Thus, the expression of the SteA-BH3 polypeptide, which induces apoptosis, is increased in cancer cells as compared to normal cells, and the expression of the BH3-trap polypeptide that inhibits SteA-BH3-induced apoptosis is increased in normal cells as compared to cancer cells, thus specifically targeting cancer cells for apoptosis. In certain embodiments, the first microRNA binding site is a miR-122 binding site, and the second microRNA binding site is a miR-21 binding site. In certain instances, the present disclosure contemplates the use of a first mRNA that encodes a fusion polypeptide comprising a SteA scaffold polypeptide and one or more BH3 domains, wherein this first mRNA contains one or more miR-122 binding sites, and a second mRNA that encodes a BH3-trap polypeptide, e.g., a Bcl-2-like polypeptide, where this second mRNA contains one or more miR-21 binding sites.

As a non-limiting example, mRNAs of the invention (e.g., those encoding SteA-BH3 fusion polypeptides) may include at least one miR-122 binding site. For example, a mRNA of the invention may include a miR-122 binding site that includes a sequence with partial or complete complementarity with a miR-122 seed sequence. In some embodiments, a miR-122 seed sequence may correspond to nucleotides 2-7 of a miR-122. In some embodiments, a miR-122 seed sequence may be 5'-GGAGUG-3'. In some embodiments, a miR-122 seed sequence may be nucleotides 2-8 of a miR-122. In some embodiments, a miR-122 seed sequence may be 5'-GGAGUGU-3'. In some embodiments, the miR-122 binding site includes a nucleotide sequence of 5' - UAUUUAGUGUGAUAAUGGCGUU - 3' (SEQ ID NO: 31) or 5' - CAAACACCAUUGUCACACUCCA - 3' (SEQ ID NO: 32) or a complement thereof. In some embodiments, inclusion of at least one miR-122 binding site in an mRNA may dampen expression of a polypeptide encoded by the mRNA in a normal liver cell as compared to other cell types that express low levels of miR-122. In other embodiments, inclusion of at least one miR-122 binding site in an mRNA may allow increased expression of a polypeptide encoded by the mRNA in a liver cancer cell (e.g., a hepatocellular carcinoma cell) as compared to a normal liver cell. In some embodiments, an mRNA that encodes a SteA scaffold polypeptide that includes one or more BH3 domains contains one or more miR-122 binding sites.

As a further non-limiting example, mRNAs of the invention, e.g., those encoding BH3-trap polypeptides, such as a Bcl-2-like polypeptide may include at least one miR-21 binding site. For example, an mRNA of the invention may include a miR-21 binding site that includes a sequence with partial or complete complementarity with a miR-21 seed sequence. In some embodiments, a miR-21 sequence may be 5'-UAGCUUAUCAGACUGAUGUUGA-3' (SEQ ID NO: 33) or 5' - CAACACCAGUCGAUGGGCUGU - 3' (SEQ ID NO: 34) or a complement thereof. In some embodiments, a miR-21 seed sequence may correspond to nucleotides 1-8 or 2-8 of a miR-21. In some embodiments, a miR-21 seed sequence may be 5'-UAGCUUAU-3' or 5'-AGCUUAU-3' or a complement thereof. An additional miR-21 seed sequence is shown in SEQ ID NO: 106.

In some embodiments, inclusion of at least one miR-21 binding site in an mRNA may increase expression of a polypeptide encoded by the mRNA in a normal liver cell as compared to other cell types that express low levels of miR-21, such as liver cancer cells. In other embodiments, inclusion of at least one miR-21 binding site in an mRNA may allow reduced expression of a polypeptide encoded by the mRNA in a liver cancer cell (e.g., a hepatocellular carcinoma cell) as compared to a normal liver cell. In some embodiments, an mRNA that encodes a BH3-trap polypeptide (e.g., a Bcl-2-like polypeptide or variant there) contains one or more miR-21 binding sites.

### Stefin A Scaffold Polypeptides

In various embodiments, an mRNA of the invention encodes a fusion polypeptide comprising a Stefin A (SteA) scaffold polypeptide, wherein the SteA scaffold polypeptide comprises one or more binding polypeptides (e.g., a BH3 domains) located at the N-terminal insertion site, the loop 1 insertion site and/or the loop 2 insertion site such that the binding polypeptide(s) are presented on the SteA scaffold polypeptide. Stefin A (also known in the art as cystatin A) is the founding member of the cystatin family of protein inhibitors of cysteine cathepsins, which are lysosomal peptidases of the papain family. The Stefin subgroup of the cystatin family are relatively small (e.g., around 100 amino acid residues long) single domain proteins. SteA is characterized as a monomeric, single chain, single domain polypeptide of 98 amino acids long. The structure of SteA has been solved, enabling rational engineering of the protein to allow for insertion and display of binding polypeptide amino acid sequences at defined sites. SteA contains a structural loop called "loop 1" at amino acid positions 48-50, inclusive, and a loop called "loop 2" at amino acid positions 71-79, inclusive. Both loop 1 and loop 2 are sandwiched by amino acids that form beta-sheets. Wild-type SteA is considered in the art to have one known biological activity, which is inhibition of cathepsin activity. Wild-type SteA typically interacts with cathepsins using three binding interfaces: the N-terminus, the loop 1 region, and the loop 2 region, with key contacts being made by glycine at position 4, valine at position 48, and lysine at position 73. In some embodiments, a SteA scaffold polypeptide includes one or more mutations that reduces or abrogates cathepsin inhibitory activity.

In some embodiments, a SteA scaffold polypeptide of the invention is derived from a SteA sequence (for example, a wild-type SteA sequence, for instance, human SteA), or from a derivative of SteA known in the art and/or as described below, for example, any derivative of SteA described in U.S. Patent Nos. 8,063,019 and 8,853,131. Non-limiting exemplary SteA scaffold polypeptides which may be used in the compositions and methods of the invention include wild-type SteA (e.g., human SteA), STM ("Stefin A Triple Mutant," as described, e.g., in U.S. Patent No. 8,063,019 and in Woodman et al., J. Mol. Biol. 352: 1118-1133, 2005), SQM ("Stefin A Quadruple Mutant," as described, e.g., in U.S. Patent No. 8,853,131), SQT ("Stefin A Quadruple Mutant, Tracy," as described, e.g., in U.S. Patent No. 8,853,131 and Stadler et al., Protein Eng. Des. Sel. 24(9): 751-763, 2011), and other SteA scaffold polypeptides described in U.S. Patent No. 8,853,131 and Hoffman et al., Protein Eng. Des. Sel. 23(5): 403-413, 2010, including SDM ("Stefin A Double Mutant"), SUC ("Stefin A Unique C-terminus"), SUM ("Stefin A Unique Middle"), SUN ("Stefin A Unique N-terminus"), or fragments thereof, including SDM-, SDM--, SQM-, SQM--, SUC-, SUC--, SUM-, SUM--, SUN-, SUN--, and SQL.

The amino acid sequence of wild-type human SteA is:

MIPGGLSEAKPATPEIQEIVDKVKPQLEEKTNETYGKLEAVQYKTQVVAGTNYYIKV
RAGDNKYMHLKVFKSLPGQNEDLVLTGYQVDKNKDDELTGF (SEQ ID NO: 53).

The amino acid sequence of STM is: 
MIPWGLSEAKPATPEIQEIVDKVKPQLEEKTNETYGKLEAVQYKTQVDAGTNYYIKV
RAGDNKYMHLKVFNGPPGQNEDLVLTGYQVDKNKDDELTGF (SEQ ID NO: 54).
 Compared to wild-type SteA, STM contains a G4W mutation, which disrupts the interaction of STM with cathepsins, a V48D mutation that disrupts the interaction of STM with cathepsins and reduces dimer formation through domain swapping, and a mutation to introduce a unique RsrII restriction enzyme site at codons 71-73. In some embodiments, a polynucleotide sequence encoding a binding polypeptide may be inserted into the RsrII site of STM, thereby introducing a binding polypeptide amino acid sequence into loop 2.

An exemplary amino acid sequence of SDM is: 
MIPGGLSEAKPATPEIQEIVDKVKPQLEEKTNETYGKLEAVQYKTQVLASTNYYIKV
RAGDNKYMHLKVFNGPPGQNEDLVRSGYQVDKNKDDELTGF (SEQ ID NO: 55).
 SDM contains a Leu residue at position 48 as a result of an engineered NheI restriction enzyme site added to the open reading frame at codons 48-50, inclusive, as compared to wild-type SteA or STM. In some embodiments, a polynucleotide sequence encoding a binding polypeptide may be inserted at the NheI site of SDM, thereby introducing a binding polypeptide amino acid sequence into loop 1. SDM also contains the sequence Asn-Gly-Pro at positions 71-73 as a result of an engineered RsrII site added to the open reading frame at codons 71-73, inclusive, as compared to wild type SteA and STM. SDM also contains the sequence Arg-Ser at positions 82-83 as a result of an engineered RsrII site added to the open reading frame at codons 82-83, inclusive, as compared to wild type SteA or STM. In some embodiments, a polynucleotide sequence encoding a binding polypeptide may be inserted between the RsrII sites of SDM, thereby replacing loop 2 with a binding polypeptide amino acid sequence.

The amino acid sequence of SQM is: 
MIPRGLSEAKPATPEIQEIVDKVKPQLEEKTNETYGKLEAVQYKTQVLASTNYYIKVR
AGDNKYMHLKVFNGPPGQNEDLVRSGYQVDKNKDDELTGF (SEQ ID NO: 56).
 SQM contains an Arg residue at position 4 as a result of an engineered AvrII restriction enzyme site added to the open reading frame as compared to STM or wild-type SteA. In some embodiments, a polynucleotide sequence encoding a binding polypeptide may be inserted at the AvrII site of SQM, thereby introducing a binding polypeptide amino acid sequence into the N-terminus of SQM. SQM also contains a Leu residue at position 48 as a result of an engineered NheI restriction enzyme site added to the open reading frame at codons 48-50, inclusive, as compared to wild-type SteA or STM. In some embodiments, a polynucleotide sequence encoding a binding polypeptide may be inserted at the NheI site of SQM, thereby introducing a binding polypeptide amino acid sequence into loop 1. SQM also contains the sequence Asn-Gly-Pro at positions 71-73 as a result of an engineered RsrII site added to the open reading frame at codons 71-73, inclusive, as compared to wild type SteA and STM. SQM also contains the sequence Arg-Ser at positions 82-83 as a result of an engineered RsrII site added to the open reading frame at codons 82-83, inclusive, as compared to wild type SteA or STM. In some embodiments, a polynucleotide sequence encoding a binding polypeptide may be inserted between the RsrII sites of SQM, thereby replacing loop 2 with a binding polypeptide amino acid sequence.

The amino acid sequence of SUC is: 
MIPGGLSEAKPATPEIQEIVDKVKPQLEEKTNETYGKLEAVQYKTQVVAGTNYYIKV
RAGDNKYMHLKVF NGPPGQNEDLVRSGYQVDKNKDDELTGF (SEQ ID NO: 57).
 SUC also contains the sequence Asn-Gly-Pro at positions 71-73 as a result of an engineered RsrII site added to the open reading frame at codons 71-73, inclusive, as compared to wild type SteA. SUC also contains the sequence Arg-Ser at positions 82-83 as a result of an engineered RsrII site added to the open reading frame at codons 82-83, inclusive, as compared to wild type SteA or STM.

The amino acid sequence of SUM is: 
MIPGGLSEAKPATPEIQEIVDKVKPQLEEKTNETYGKLEAVQYKTQVLASTNYYIKV
RAGDNKYMHLKVFKSLPGQNEDLVLTGYQVDKNKDDELTGF (SEQ ID NO: 58).
 SUM contains a Leu residue at position 48 as a result of an engineered NheI restriction enzyme site added to the open reading frame at codons 48-50, inclusive, as compared to wild-type SteA or STM. In some embodiments, a polynucleotide sequence encoding a binding polypeptide may be inserted at the NheI site of SUM, thereby introducing a binding polypeptide amino acid sequence into loop 1.

The amino acid sequence of SUN is: 
MIPRGLSEAKPATPEIQEIVDKVKPQLEEKTNETYGKLEAVQYKTQVVAGTNYYIKV
RAGDNKYMHLKVFKSLPGQNEDLVLTGYQVDKNKDDELTGF (SEQ ID NO: 59).
 SUN contains an Arg residue at position 4 as a result of an engineered AvrII restriction enzyme site added to the open reading frame as compared to STM or wild-type SteA. In some embodiments, a polynucleotide sequence encoding a binding polypeptide may be inserted into the AvrII site of SUN, thereby introducing a binding polypeptide amino acid sequence into the N-terminus of SUN.

The amino acid sequence of SDM- is:

MIPGGLSEAKPATPEIQEIVDKVKPQLEEKTNETYGKLEAVQYKTQVL
ASTNYYIKVRAGDNKYMHLKVFNGPPGQNEDLVRS (SEQ ID NO: 60).

The amino acid sequence of SDM-- is:

MIPGGLSEAKPATPEIQEIVDKVKPQLEEKTNETYGKLEAVQYKTQVLASTNYYIKV
RAGDNKYMHLKVFNGP (SEQ ID NO: 61).

The amino acid sequence of SQM- is:

MIPRGLSEAKPATPEIQEIVDKVKPQLEEKTNETYGKLEAVQYKTQVLASTNYYIKVR
AGDNKYMHLKVFNGPPGQNEDLVRS (SEQ ID NO: 62).

The amino acid sequence of SQM-- is:

MIPRGLSEAKPATPEIQEIVDKVKPQLEEKTNETYGKLEAVQYKTQVLASTNYYIKVR
AGDNKYMHLKVFNGP (SEQ ID NO: 63).

The amino acid sequence of SUC- is:

MIPGGLSEAKPATPEIQEIVDKVKPQLEEKTNETYGKLEAVQYKTQVVAGTNYYIKV
RAGDNKYMHLKVF NGPPGQNEDLVRS (SEQ ID NO: 64).

The amino acid sequence of SUC-- is:

MIPGGLSEAKPATPEIQEIVDKVKPQLEEKTNETYGKLEAVQYKTQVVAGTNYYIKV
RAGDNKYMHLKVFNGP (SEQ ID NO: 65).

The amino acid sequence of SUM- is:

MIPGGLSEAKPATPEIQEIVDKVKPQLEEKTNETYGKLEAVQYKTQVLASTNYYIKV
RAGDNKYMHLKVFKSLPGQNEDLVLT (SEQ ID NO: 66).

The amino acid sequence of SUM-- is:

MIPGGLSEAKPATPEIQEIVDKVKPQLEEKTNETYGKLEAVQYKTQVLASTNYYIKV
RAGDNKYMHLKVFKSL (SEQ ID NO: 67).

The amino acid sequence of SUN- is:

MIPRGLSEAKPATPEIQEIVDKVKPQLEEKTNETYGKLEAVQYKTQVVAGTNYYIKV
RAGDNKYMHLKVFKSLPGQNEDLVLT (SEQ ID NO: 68).

The amino acid sequence of SUN-- is:

MIPRGLSEAKPATPEIQEIVDKVKPQLEEKTNETYGKLEAVQYKTQVVAGTNYYIKV
RAGDNKYMHLKVFKSL (SEQ ID NO: 69).

The amino acid sequence of SQT is: 
MIPRGLSEAKPATPEIQEIVDKVKPQLEEKTNETYGKLEAVQYKTQVLASTNYYIKVR
AGDNKYMHLKVFNGPPGQNADRVLTGYQVDKNKDDELTGF (SEQ ID NO: 70).
 SQT contains an Arg residue at position 4 as a result of an engineered AvrII restriction enzyme site added to the open reading frame as compared to STM or wild-type SteA. In some embodiments, a polynucleotide sequence encoding a binding polypeptide may be inserted at the AvrII site of SQT, thereby introducing a binding polypeptide amino acid sequence into the N-terminus of SQT. SQT also contains a Leu residue at position 48 as a result of an engineered NheI restriction enzyme site added to the open reading frame at codons 48-50, inclusive, as compared to wild-type SteA or STM. In some embodiments, a polynucleotide sequence encoding a binding polypeptide may be inserted at the NheI site of SQT, thereby introducing a binding polypeptide amino acid sequence into loop 1. SQT also contains the sequence Asn-Gly-Pro at positions 71-73 as a result of an engineered RsrII site added to the open reading frame as compared to wild-type SteA or STM. SQT also contains the sequence Ala-Asp-Arg at positions 78-80 as a result of an engineered RsrII site added to the open reading frame as compared to wild type SteA or STM. In some embodiments, a polynucleotide sequence encoding a binding polypeptide may be inserted between the RsrII sites of SQT, thereby replacing loop 2 with a binding polypeptide amino acid sequence.

The amino acid sequence of SQL is:

MIPRGLSEAKPATPEIQEIVDKVKPQLEEKTNETYGKLEAVQYKTQVLALASTNYYIK
VRAGDNKYMHLKVFNGPPGQNADRVLTGYQVDKNKDDELTGF (SEQ ID NO: 71).

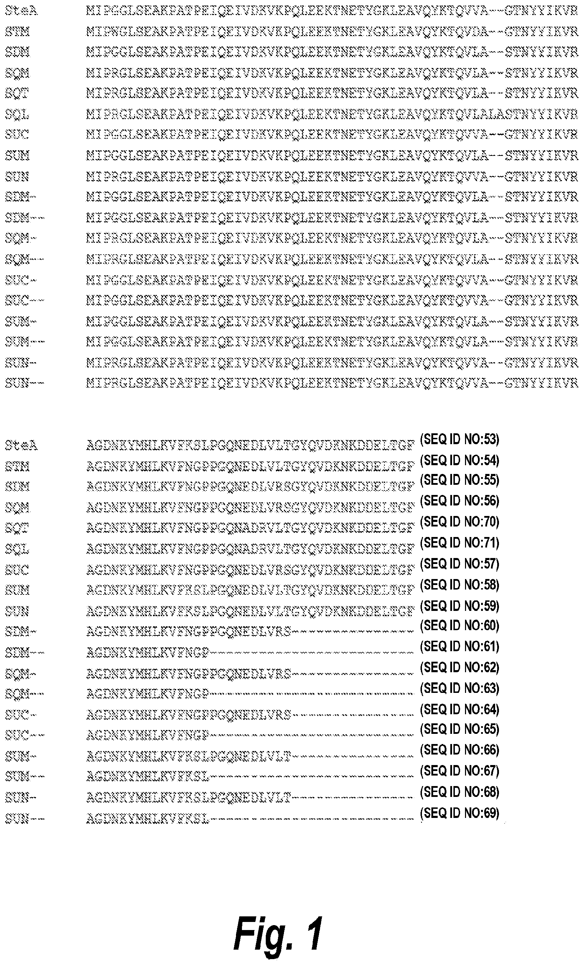

Figure 1 shows an alignment of the SteA scaffold polypeptide amino acid sequences described above.

In some embodiments, a SteA scaffold polypeptide as used in the compositions and methods of the invention comprises an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99% identity to an amino acid sequence selected from the group consisting of SEQ ID NOs: 53-71. In some embodiments, a SteA scaffold polypeptide of the invention includes an amino acid sequence selected from the group consisting of SEQ ID NOs: 53-71. In particular embodiments, a SteA scaffold polypeptide of the disclosure comprises an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%), identity to the amino acid sequence of SEQ ID NO: 70. In some embodiments, a SteA scaffold polypeptide of the invention comprises an amino acid sequence of SEQ ID NO: 70.

A fusion polypeptide or SteA scaffold polypeptide of the invention may be derived from any SteA scaffold polypeptide known in the art. For example, a SteA scaffold polypeptide may include one or more mutational changes, e.g., amino acid insertions, deletions or substitutions, as compared to any SteA scaffold polypeptide described herein or known in the art. In some embodiments, a SteA scaffold polypeptide may include one or more mutational changes in one or more (e.g., 1, 2, or 3) of the following three regions: the N-terminus (e.g., a mutational change in the one or more of the first 8 codons that encode the first 8 amino acids of a SteA scaffold polypeptide), the loop 1 region (e.g., a mutational change in one or more (e.g., 1, 2, 3, 4, 5, 6, 7, 8, or 9 mutational changes) of codons 46 to 54 inclusive that encode amino acids within or adjacent to loop 1 of a SteA scaffold polypeptide), and/or the loop 2 region (e.g., a mutational change in one or more (e.g., 1, 2, 3, 4, 5, 6, 7, 8, 9, 10, 11, 12, 13, 14, 15, 16, 17, or 18 mutational changes) of codons 67 to 84 inclusive that encode amino acids within or adjacent to loop 2 of a SteA scaffold polypeptide), for example, as described in U.S. Patent No. 8,853,131. In some embodiments, a SteA scaffold polypeptide may include one or more mutational changes in the N-terminus as compared to a reference SteA scaffold polypeptide. In some embodiments, a SteA scaffold polypeptide may include one or more mutational changes in the loop 1 region as compared to a reference SteA scaffold polypeptide. In some embodiments, a SteA scaffold polypeptide may include one or more mutational changes in the loop 2 region as compared to a reference SteA scaffold polypeptide. In some embodiments, a SteA scaffold polypeptide may include one or more mutational changes in the N-terminus and the loop 1 region as compared to a reference SteA scaffold polypeptide. In some embodiments, a SteA scaffold polypeptide may include one or more mutational changes in the N-terminus and the loop 2 region as compared to a reference SteA scaffold polypeptide. In some embodiments, a SteA scaffold polypeptide may include one or more mutational changes in the loop 1 region and the loop 2 region as compared to a reference SteA scaffold polypeptide. In some embodiments, a SteA scaffold polypeptide may include one or more mutational changes in the N-terminus, the loop 1 region, and the loop 2 region as compared to a reference SteA scaffold polypeptide.

The effects of a mutational change, if any, on the conformational stability, expression level, secondary structure, ability to display a binding polypeptide amino acid sequence inserted in an insertion site, or other characteristics of a SteA scaffold polypeptide can readily be determined by a person of ordinary skill in the art, for example, using methods described in U.S. Patent Nos. 8,063,019 and 8,853,131. For example, in some embodiments, a SteA scaffold polypeptide retains a substantially similar conformational stability as compared to any of the SteA scaffold polypeptides described herein, for example, wild-type SteA, STM, SQM, or SQT. Conformational stability may be determined for example, by methods including but not limited to differential scanning fluorimetry, circular dichroism, spectroscopy, or other methods known in the art. Secondary structure may be determined, for example, by circular dichroism or other methods known in the art. In some embodiments, a SteA scaffold polypeptide retains a substantially similar expression level as compared to any of the SteA scaffold polypeptides described herein, for example, wild-type SteA, STM, SQM, or SQT. Expression levels may be determined, for example, by methods including but not limited to Western blot, immunohistochemistry (IHC), mass spectrometry, enzyme-linked immunosorbent assay (ELISA), or by other methods known in the art. In some embodiments, a SteA scaffold polypeptide retains a substantially similar ability to display a binding polypeptide amino acid sequence as compared to any of the SteA scaffold polypeptides described herein, for example, wild-type SteA, STM, SQM, or SQT. The ability of a SteA scaffold polypeptide to display a binding polypeptide amino acid sequence may be determined by testing whether a known binding partner of a binding polypeptide amino acid sequence is able to physically interact with the binding polypeptide amino acid sequence when presented in the context of a SteA scaffold polypeptide, for example, by co-immunoprecipitation, yeast two-hybrid, or other methods known in the art.

In some embodiments, a fusion polypeptide of the invention comprises a SteA scaffold polypeptide and one or more binding polypeptide amino acid sequence(s) located at a N-terminal insertion site, a loop 1 insertion site, and/or a loop 2 insertion site. In some embodiments, a fusion polypeptide includes a binding polypeptide amino acid sequence located at an N-terminal insertion site of a SteA scaffold polypeptide. In some embodiments, an N-terminal insertion site includes one or more of positions 1-8 inclusive (e.g., position 1, 2, 3, 4, 5, 6, 7, and/or 8) of a SteA scaffold polypeptide. In particular embodiments, the N-terminal insertion site may be position 4 of a SteA scaffold polypeptide. In some embodiments, a fusion polypeptide includes a binding polypeptide amino acid sequence located at a loop 1 insertion site. In some embodiments, a loop 1 insertion site includes one or more of positions 46 to 54 inclusive (e.g., position 46, 47, 48, 49, 50, 51, 52, 53, and/or 54) of a SteA scaffold polypeptide. In particular embodiments, the loop 1 insertion site may include positions 48-50, e.g., position 48, 49, and/or 50 of a SteA scaffold polypeptide. In some embodiments, a fusion polypeptide includes a binding polypeptide amino acid sequence located at a loop 2 insertion site. In particular embodiments, the loop 2 insertion site includes one or more of positions 71-83 inclusive (e.g., position 71, 72, 73, 74, 75, 76, 77, 78, 79, 80, 81, 82, and/or 83) of a SteA scaffold polypeptide. In further embodiments, the loop 2 insertion site may include positions 71-73, 78-80, and/or 82-83 of a SteA scaffold polypeptide. In some embodiments, a SteA scaffold polypeptide may include more than one (e.g., 1, 2, 3, 4, 5, or more) binding polypeptide amino acid sequence located at the same insertion site (e.g., an N-terminal insertion site, a loop 1 insertion site, or a loop 2 insertion site). In some embodiments, the binding polypeptide amino acid sequence is an amino acid sequence of a BH3 domain or fragment thereof, for example, a BH3 domain or fragment thereof as described herein.

In some embodiments, a fusion polypeptide comprising a SteA scaffold polypeptide may include one or more binding polypeptide amino acid sequence(s) located at multiple insertion sites (e.g., 2 or 3 insertion sites) selected from an N-terminal insertion site, a loop 1 insertion site, and/or a loop 2 insertion site. In some embodiments, the same binding polypeptide amino acid sequence may be located at two or more insertion sites. In other embodiments, different binding polypeptide amino acid sequences may be located at two or more insertion sites. In some embodiments, the binding polypeptide amino acid sequence is an amino acid sequence of a BH3 domain or fragment thereof, as described herein.

In some embodiments, a binding polypeptide amino acid sequence may include between about 1 and about 50 amino acids. For example, in some embodiments, a binding polypeptide amino acid sequence may include, for example, 1 to 50 amino acids, 1 to 40 amino acids, 1 to 30 amino acids, 1 to 20 amino acids, 1 to 10 amino acids, or 1 to 5 amino acids. In particular embodiments, a binding polypeptide amino acid sequence may include, for example, between about 1 and about 26 amino acids (e.g., 1, 2, 3, 4, 5, 6, 7, 8, 9, 10, 11, 12, 13, 14, 15, 16, 17, 18, 19, 20, 21, 22, 23, 24, 25, or 26 amino acids). In other particular embodiments, a binding polypeptide amino acid sequence may include, for example, between about 1 and about 13 amino acids (e.g., 1, 2, 3, 4, 5, 6, 7, 8, 9, 10, 11, 12, or 13 amino acids). In some embodiments, a binding polypeptide amino acid sequence may be an amino acid sequence of a BH3 domain. In particular embodiments, a BH3 domain may include about 10 to about 40 amino acid residues, e.g., about 10, 12, 14, 16, 18, 20, 22, 24, 26, 28, 30, 32, 34, 36, 38, or 40 residues.

It is to be understood that in addition to a SteA scaffold polypeptide and one or more binding polypeptide amino acid sequences, a fusion polypeptide of the present disclosure may include additional elements, including linkers and epitope tags. Functions of a linker region can include introduction of restriction enzyme sites into the nucleotide sequence, introduction of a flexible component or space-creating region between two protein domains, or creation of an affinity tag for specific molecular interaction. A linker may be any suitable length, for example, 1, 2, 3, 4, 5, 6, 7, 8, 9, 10, or more amino acids long. An epitope tag may be included to facilitate detection and/or purification of a fusion polypeptide. Exemplary non-limiting epitope tags include FLAG, V5, HA, myc, GFP, and His.

In particular embodiments, the binding polypeptide amino acid sequence present in a fusion polypeptide comprising a SteA scaffold polypeptide encodes one or more BH3 domain. In some embodiments, the fusion polypeptide comprises one, two, three, or more BH3 domains. In some embodiments, the fusion polypeptide includes a BH3 domain located at a N-terminal insertion site. In some embodiments, the fusion polypeptide includes a BH3 domain located at a loop 1 insertion site. In some embodiments, the fusion polypeptide includes a BH3 domain located at a loop 2 insertion site. In some embodiments, the fusion polypeptide includes a BH3 domain located at a N-terminal insertion site and a loop 1 insertion site. In some embodiments, the fusion polypeptide includes a BH3 domain located at a N-terminal insertion site and a loop 2 insertion site. In some embodiments, the fusion polypeptide includes a BH3 domain located at a loop 1 insertion site and a loop 2 insertion site. In some embodiments, the fusion polypeptide includes a BH3 domain located at a N-terminal insertion site, a loop 1 insertion site, and a loop 2 insertion site. In embodiments where a fusion polypeptide includes more than one BH3 domain, the BH3 domains may be the same or different.

### BH3 Domains

In some embodiments, an mRNA of the invention may encode one or more BH3 domains. In particular embodiments, an mRNA of the invention may encode a SteA scaffold polypeptide comprising one or more BH3 domains. In some embodiments, the BH3 domain is a human BH3 domain. In other embodiments, the BH3 domain may be from a non-human species, e.g., *Caenorhabditis elegans,* rodents (e.g., mice and rats), or non-human primates. Typically, a BH3 domain is derived from a pro-apoptotic Bcl-2 family member, including from an effector pro-apoptotic Bcl-2 family member (e.g., BAK or BAX) or from a BH3-only family member (e.g., BID, BIM, BAD, BIK, BMF, bNIP3, HRK, Noxa, and PUMA). In particular embodiments, the BH3 domain is a BH3 domain derived from a BH3-only family member. Without wishing to be bound by theory, it is known in the art that the balance of pro-apoptotic Bcl-2 family proteins and anti-apoptotic Bcl-2 family proteins in a cell is important for regulation of apoptosis. Structural studies have shown that the BH3 domain of BH3-only proteins can bind as an amphipathic helix in a surface-exposed hydrophobic groove of an anti-apoptotic Bcl-2 family member (see, for example, Day et al., J. Mol. Biol. 380:958-971, 2008). The disclosure features methods of inducing apoptosis that involve introducing an mRNA encoding a SteA scaffold polypeptide including one or more BH3 domains into a cell under conditions permissive for expression of the SteA scaffold polypeptide.

In some embodiments, a BH3 domain may directly bind to a Bcl-2 family protein. For example, in some embodiments, a BH3 domain may directly bind to a pro-apoptotic Bcl-2 family protein. In some embodiments, the pro-apoptotic Bcl-2 family protein is Bax and/or Bak. In some embodiments, a BH3 domain may directly interact with an anti-apoptotic Bcl-2 family protein. In some embodiments, the anti-apoptotic Bcl-2 family protein may BCL-2, BCL-XL, BCL-w, MCL-1 or BCL2-related protein A1 (BCL2A1).

In some embodiments, a BH3 domain is derived from a BH3-only family member. In some embodiments, a BH3 domain as used herein may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to the amino acid sequence of X₁X₂X₃X₄X₅X₆X₇X₈X₉DX₁₀X₁₁X₁₂, wherein X₁, X₅, X₈, and X₁₁ are, independently, any hydrophobic residue, X₂ and X₉ are, independently, Gly, Ala, or Ser, X₃, X₄, X₆, and X₇ are, independently, any amino acid residue, X₁₀ is Asp or Glu, and X₁₂ is Asn, His, Asp, or Tyr. In some embodiments, a hydrophobic residue is Leu, Ala, Val, Ile, Pro, Phe, Met or Trp. In some embodiments, X₅ is Leu.

In some embodiments, a BH3 domain as used herein may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to any one of any one of SEQ ID NOs: 1-26. In some embodiments, the BH3 domain includes an amino acid sequence selected from the group consisting of SEQ ID NOs: 1-26, as shown in Table 3, which also indicates the name, UniProt sequence identifier, and amino acid residues. Illustrative BH3 domains that may be used according to the present invention are also described in Lomonosova and Chinnadurai, Oncogene (2009) 27, S2-S19.

**Table 3. Illustrative BH3 Domains**

| **SEQ ID NO** | **BH3 Domain Sequence** | **Source Protein (UniProt)** |
|---|---|---|
| 1 | LALRLACIGDEMDVS | BIK>Q13323157-71 |
| 2 | EKAELLQGGDLLRQR | BNIP1>Q12981\|110-124 |
| 3 | VESILKKNSDWIWDW | BNIP3>Q12983\|106-120 |
| 4 | EVEALKKSADWVSDW | PNIP3L>O60238\|120-134 |
| 5 | TAARLKALGDELHQR | HRK>O00198\|33-47 |
| 6 | IAQELRRIGDEFNAY | BIMe1>O43521\|148-162 |
| 7 | YGRELRRMSDEFVDS | BAD>Q92934\|110-124 |
| 8 | IARHLAQVGDSMDRS | BID>P55957\|86-100 |
| 9 | IARKLQCIADQFHRL | BMF>Q96LC9\|133-147 |
| 10 | CATQLRRFGDKLNFR | NOXA>Q13794\|25-39 |
| 11 | IGAQLRRMADDLNAQ | PUMA>Q9BXH1\|137-151 |
| 12 | LSRRLKVTGDLFDIM | Beclin1>Q14457\|112-126 |
| 13 | IVELLKYSGDQLERK | BCL-Gs>Q9BZR8-2\|212-226 |
| 14 | ALETLRRVGDGVQRN | MCL-1S>Q07820-2\|209-223 |
| 15 | IGSKLAAMCDDFDAQ | EGL-1>O61667\|69-83 |
| 16 | IGRKLTVMCDEFDSE | CED-13>Q9TYO6\|55-69 |
| 17 | NIRRLRALADGVQKV | ApoL1>O14791\|154-168 |
| 18 | NIDKLRALADDIDKT | ApoL6>Q9BWW8\|56-70 |
| 19 | MVTLLPIEGQEIHFF | BRCC2>Q8IZY5\|1-115 |
| 20 | PTVPLPSETDGYVAP | HER2>P0462611116-1130 |
| 21 | PQRYLVIQGDDRMKL | HER4>Q153031981-995 |
| 22 | TVGELSRALGHENGS | MAP1>Q96BY2\|116-130 |
| 23 | VGQLLQDMGDDVYQQ | MULE>Q7Z6Z711976-1990 |
| 24 | LHEVLNGLLDRPDWE | SPHK2>Q9NRA0\|250-264 |
| 25 | AVHSLSRIGDELYLE | RAD9>Q99638116-30 |
| 26 | NPKFLKNAGRDCSRR | TGM2>P21980\|200-214 |

In some embodiments, the BH3 domain may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to any one of any one of SEQ ID NOs: 27-30. In some embodiments, the BH3 domain includes the amino acid sequence of any one of SEQ ID NOs: 27-30. In some embodiments, the BH3 domain includes the amino acid sequence of SEQ ID NO: 27. In some embodiments, the BH3 domain includes the amino acid sequence of SEQ ID NO: 28. Illustrative BH3 domains that may be used according to the present invention are described in Stadler et al, Cell Death and Disease (2014) 5, e1037, 1-9. The BH3 domain of Puma has the amino acid sequence: EEQWAREIGAQLRRMADDLNAQYERR (SEQ ID NO: 27); the BH3 domain of Bim has the amino acid sequence: DMRPEIWIAQELRRIGDEFNAYYARR (SEQ ID NO: 28); the BH3 domain of Bad has the amino acid sequence:
NLWAAQRYGRELRRMSDEFVDSFKKG (SEQ ID NO: 29); and the BH3 domain of Noxa has the amino acid sequence: PAELEVECATQLRRFGKLNFRQKLL (SEQ ID NO: 30).

In some embodiments, a BH3 domain may be able to induce apoptosis, for example, when presented in the context of a SteA scaffold polypeptide. A person of ordinary skill in the art can readily determine if a BH3 domain is able to induce apoptosis using a variety of methods, for example, caspase activation assays (e.g., caspase-3/7 activation assays), stains and dyes (e.g., CELLTOX™, MITOTRACKER® Red, propidium iodide, and YOYO3), cell viability assays, cell morphology, and PARP-1 cleavage.

### Bcl-2-like Polypeptides

The present disclosure also includes mRNAs encoding a polypeptide that inhibits a BH3 domain present in a fusion polypeptide described herein. In some embodiments, an mRNA of the invention may encode one or more Bcl-2-like polypeptides or a variant or fragment thereof. In particular embodiments, an mRNA of the invention may encode a prosurvival Bcl-2-like polypeptide, such as Bcl-2, Bcl-X_{L}, Bcl-w, Mcl-1 or A1 polypeptide, or a variant or fragment thereof. Structural studies have shown that the hydrophobic face of the amphipathic helix present in BH3 domains inserts into a hydrophobic groove formed by the BH1, BH2 and BH3 domains of the prosurvival Bcl-2-like polypeptides, such as Bcl-2, Bcl-X_{L}, Bcl-w, Mcl-1 and A1, thus neutralizing the prosurvival Bcl-2-like polypeptides. In particular embodiments, the Bcl-2-like polypeptides and variants thereof comprise BH1, BH2 and BH3 domains. In particular embodiments, variants may include one or more N-terminal or C-terminal deletion. For example, in particular embodiments, soluble, monomeric prosurvival Bcl-2-like polypeptides have a deletion of their hydrophobic C-terminal domain. In certain embodiments, Mcl-1 and other Bcl-2-like polypeptides have a deletion of an N-terminal PEST region. In some embodiments, the Bcl-2-like polypeptide is a human polypeptide. In other embodiments, the Bcl-2-like polypeptide may be from a non-human species, e.g., *Caenorhabditis elegans,* rodents (e.g., mice and rats), or non-human primates. Without wishing to be bound by theory, it is believed that when expressed in the same cell, the exogenous Bcl-2-like polypeptide or variant thereof binds to the BH3 domain of the SteA-BH3 fusion polypeptide, thus sequestering it and preventing it from inducing apoptosis (see, for example, Day et al., J. Mol. Biol. 380:958-971, 2008).

In some embodiments, a Bcl-2-like polypeptide or variant or fragment thereof may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to the amino acid sequence of a human Bcl-2, Bcl-X_{L}, Bcl-w, Mcl-1 or A1 polypeptide, the amino acid sequences of which are shown in SEQ ID NOs: 38-42, respectively. In some embodiments, the a Bcl-2-like polypeptide or variant or fragment thereof comprises an amino acid sequence shown in SEQ ID NOs: 38-42.

In particular embodiments, a Bcl-2-like polypeptide variant is a soluble Bcl-2-like polypeptide variant, such as a Bcl-2 polypeptide comprising a C-terminal truncation (e.g., deletion of the C-terminal 22-32 amino acid residues, e.g., the C-terminal 22 or 43 amino acid residues), a Bcl-X_{L} polypeptide comprising a C-terminal truncation (e.g., deletion of the C-terminal 24 amino acid residues), a Bcl-w polypeptide comprising a C-terminal truncation (e.g., deletion of the C-terminal 29 amino acid residues), a Mcl-1 polypeptide comprising C-terminal and N-terminal truncations (e.g., deletion of the C-terminal 23 amino acid residues and the N-terminal 151 amino acid residues), or an A1 polypeptide comprising a C-terminal truncation (e.g., deletion of the C-terminal 20 amino acid residues). In particular embodiments, a Bcl-2-like polypeptide may in addition or alternatively comprise one or more amino acid substitutions as compared to its corresponding wild type Bcl-2-like polypeptide. In certain embodiments, a variant includes a Bcl-2 polypeptide having one or more C29S, D34A, or A128E amino acid substitutions. In certain embodiments, a variant includes a Bcl-X_{L} polypeptide having a D61A amino acid substitution. In certain embodiments, BH3-trap polypeptides (e.g., Bcl-2-like polypeptides) of the present disclosure include: Mcl-1 del.N/C; Bcl-w (C29S/A128E); Bcl-2 (D34A) del.C32; Bcl-X_{L} del.C24, Mcl-1 del.N/C(2010), and Bcl-xL (D61A) del.C24 (the amino acid sequences of which are shown in SEQ ID NOs: 94-99, respectively) and wild type Bcl-2-like polypeptides. Illustrative soluble monomeric prosurvival proteins are also described in Chen et al., Molecular Cell (2005) 17, 393-403.

In some embodiments, a Bcl-2-like polypeptide or variant thereof as used herein may be encoded by a nucleotide sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to any one of any one of SEQ ID NOs: 94-99. In some embodiments, the Bcl-2-like polypeptide is encoded by a nucleotide sequence selected from the group consisting of SEQ ID NOs: 94-99.

In some embodiments, a Bcl-2-like polypeptide may be able to inhibit apoptosis induced by a BH3 polypeptide, such as a SteA-BH3 fusion polypeptide described herein. A person of ordinary skill in the art can readily determine if a Bcl-2-like polypeptide is able to inhibit BH3-induced apoptosis using a variety of methods, for example, caspase activation assays (e.g., caspase-3/7 activation assays), stains and dyes (e.g., CELLTOX™, MITOTRACKER® Red, propidium iodide, and YOYO3), cell viability assays, cell morphology, and PARP-1 cleavage. In particular embodiments, the Bcl-2-like polypeptide is Bcl-2, Bcl-x_{L}, Bcl-w, Mcl-1 or A1. In some embodiments, the Bcl-2-like polypeptide is a functional variant selected from Mcl-ldel.N/C, Bcl-w (C29S/A128E), Bcl-2 (D34A) del.C32, Bcl-xL del.C24, Mcl-1 del.N/C(2010), and Bcl-xL (D61A) del.C24.

The mRNA constructs encoding the Bcl-2-like polypeptides can be used in combination with an mRNA construct encoding a SteA-BH3 fusion polypeptide by co-transfection of both constructs into cells (described further in the Examples). Alternatively, the Bcl-2-like polypeptide constructs can be introduced into cells as a single agent, wherein they can act to inhibit the activity of endogenous BH3 domains.

### Anti-MCLl Constructs

The present disclosure also includes mRNAs encoding a polypeptide that targets MCL1, referred to herein as anti-MCL1 constructs, which can be used in combination with an mRNA construct encoding a SteA-BH3 fusion polypeptide to synergistically promote apoptosis. Example 15 describes in detail anti-MCL1 constructs that exhibit synergistic pro-apoptotic effects when used in combination with an SQT-BH3 construct. While not intending to be limited by mechanism, it is thought that by neutralizing MCL1 in tumor cells, the tumor then reverts to sole reliance on BCLXL, BCL2 and/or other prosurvival members of the family as the pro-survival mechanism/pathway. Accordingly, use of SQT-BH3, which specifically destroys BCLXL, BCL2 and/or other prosurvival members of the family then leads to better tumor killing when used in combination with an anti-MCL1 agent.

Non-limiting examples of an amino acid sequences that can be used as an anti-MCL1 construct are shown in SEQ ID NOs: 107 and 108 (with an epitope tag) and in SEQ ID NOs: 109 and 110 (without an epitope tag). The nucleotide sequence encoding the open reading frames of SEQ ID NOs: 107 and 108 is shown in SEQ ID NOs: 111 and 112, respectively. Sequences that target MCL1 also have been described in the art (see e.g., Lee, E.F. et al. (2008) J. Cell. Biol. 180:341-355; Foight, G.W. et al. (2014) ACS Chem. Biol. 9:1962-1968; Placzek, W.J. et al. (2011) J. Biol. Chem. 286:39829-39835).

Furthermore, an anti-MCL1 construct can include one or more microRNA binding sites. Such binding sites are described hereinbefore. For example, in one embodiment, an anti-MCL1 construct includes an miR122 binding site.

An anti-MCL1 construct also can include an epitope tag, such as a FLAG, His or V5 epitope tag.

In certain embodiments, an anti-MCL1 construct comprises a scaffold polypeptide. Thus, the construct can encode a fusion polypeptide of the scaffold polypeptide and the anti-MCL1 polypeptide(s). Suitable scaffold polypeptides include the SteA scaffolds described herein, such as an SQT scaffold. The amino acid sequence of a non-limiting example of an SQT scaffold/anti-MCL1 fusion polypeptide is shown in SEQ ID NO: 108, in which a single mutated BH3 domain has been inserted into the N-terminal loop of the SQT scaffold protein. The nucleotide sequence encoding this ORF used in the mRNA construct is shown in SEQ ID NO: 112. The amino acid sequence of this ORF without the V5 epitope tag is shown in SEQ ID NO: 110.

In certain embodiments, an anti-MCL1 construct comprises a scaffold polypeptide. Thus, the construct can encode a fusion polypeptide of the scaffold polypeptide and the anti-MCL1 polypeptide(s). Suitable scaffold polypeptides include the SteA scaffolds described herein, such as an SQT scaffold. The amino acid sequence of a non-limiting example of an SQT scaffold/anti-MCL1 fusion polypeptide is shown in SEQ ID NO: 107, in which a single mutated Bim BH3 domain has been inserted into the N-terminal loop of the SQT scaffold protein. The nucleotide sequence encoding this ORF used in the mRNA construct is shown in SEQ ID NO: 111. The amino acid sequence of this ORF without the V5 epitope tag is shown in SEQ ID NO: 109.

For use of an anti-MCL1 construct in combination with an SQT-BH3 construct, both constructs can be incorporated into the same mmRNA construct and introduced into cells as a single construct. Alternatively, the two mmRNAs can be prepared as two separate constructs and they can be used in combination by introducing both constructs into the same cells. Either or both can be delivered to cells in a lipid nanoparticle as described herein.

### Other Binding Polypeptides

Provided herein are isolated mRNAs (e.g., modified mRNAs) encoding a fusion protein comprising a SteA polypeptide and one or more binding polypeptides located at the N-terminal insertion site, the loop 1 insertion site and/or the loop 2 insertion site. In various embodiments, the fusion protein comprises a first binding polypeptide or a first and a second binding polypeptide or a first, second and third binding polypeptide.

In various embodiments, the first, second and/or third binding polypeptide binds to a target selected from the group consisting of an oncoprotein, a viral protein, an immune-related protein, a transcription factor, a cytokine, a receptor, an enzyme, DNA or RNA.

In various embodiments, the first, second and/or third binding polypeptide is from a protein selected from the group consisting of an oncoprotein, an antibody or antigen-binding portion thereof, a ligand-binding protein, a DNA-binding protein and an RNA-binding protein. Non-limiting examples of antibodies or antigen-binding portions that can be used as the binding polypeptide in the fusion protein include a VHH domain (also referred to as a single antibody domain, containing only the VH region) or a CH3 domain. Non-limiting examples of oncoproteins include MYC, MAX, and MDM2. Non-limiting examples of ligand-binding proteins include cytokines, growth factors and the like that bind to receptors, as well as enzymes that bind to substrates, including for example Cyclin E1 (CCNE1). Non-limiting examples of DNA-binding proteins include transcription factors such as MYC, MAX, STAT3 and SALL4A. Non-limiting examples of RNA-binding proteins include proteins that bind to miRNAs.

In one embodiment, the binding polypeptide may bind to the DNA binding domain of V-myc myelocytomatosis viral oncogene homolog (MYC) to prevent its association with MYC regulatory elements within the regulatory or promoter region of a target gene, thereby reducing gene expression controlled by MYC. In one embodiment, the binding polypeptide may bind to the DNA binding domain of MYC and/or myc-associated factor X (MAX) to prevent the association of a MYC homodimer or MYC/MAX heterodimer with its regulatory element within the regulatory region or promoter of a target gene, thereby reducing gene expression controlled by MYC. In one embodiment, the binding polypeptide may bind to the DNA binding domain of Signal transducer and activator of transcription 3 (STAT3) to prevent its association to its DNA regulatory element, termed gamma-activated sites (GAS), within a regulatory or promoter region of a target gene, thereby reducing gene expression controlled by STAT3.

In another embodiment, the binding polypeptide binds to the dimerization domain of at least one transcription factor, preventing dimerization with a partner to form either a homo-or a heterodimer. In one embodiment, the transcription factor is a transcriptional activator. In one embodiment, the transcription factor is a transcriptional repressor. In one embodiment the binding polypeptide binds to the dimerization domain of MYC to prevent MYC homo-dimerization. In one embodiment, the binding polypeptide binds to the dimerization domains of MYC and/or MAX, preventing MYC/MAX heterodimer formation. In one embodiment the binding polypeptide may bind to the dimerization domain of STAT3 to prevent homo-dimerization.

In another embodiment, the binding polypeptide is from SALL4A, a stem cell factor that represses the transcription of NuRD/PTEN and SALL1 through an epigenetic repressor (Lu.J. et al. (2009) PLoS One 4:10.1371). In another embodiment, the binding polypeptide is from an antibody or antigen binding fragment thereof that binds to RAS, such as an anti-Ras single antibody domain (VHH) or other intracellular antibody that binds to RAS. Examples of such antibodies are known in the art (e.g., Montano, X. and Jimenez, A. (1995) Cell Growth Differ. 6:597-605). In another embodiment, the binding polypeptide is from MDM2, a negative regulator of the tumor suppressor p53 (Nag, S. et al. (2014) Curr. Med. Chem. 21:553-574).

### Anti-Ras Peptides and Constructs

In various embodiments, an mRNA of the invention encodes one or more anti-Ras peptides or anti-Ras peptide constructs (e.g., one copy or multiple copies of an anti-Ras peptide) as a fusion protein comprising a SteA polypeptide and one or more anti-Ras peptides located at the N-terminal insertion site, the loop 1 insertion site and/or the loop 2 insertion site. It is known in the art that unregulated activity of RAS gene products can cause cancer (see, e.g., Goodsell, DS Oncologist 4: 263-4, 1999). Anti-Ras peptide ligands which bind anti-Ras have been described (see, e.g., Gareiss, PC ChemBioChem 11: 517-522, 2010).

In one embodiment, the isolated mRNA encodes a fusion protein comprising a Ste A scaffold (such as SQT) and one copy of an anti-Ras peptide. In other embodiments, the isolated mRNAs encode a fusion protein comprising a Ste A scaffold (such as SQT) and multiple copies of an anti-Ras peptide. In one embodiment, the mRNA encodes a Ste A fusion protein comprising at least three anti-Ras peptides. In one embodiment, the mRNA encodes a Ste A fusion protein comprising two to ten anti-Ras peptides. In one embodiment, the mRNA encodes a SteA fusion protein comprising three anti-Ras peptides. In other embodiments, the mRNA encodes a Ste A fusion protein comprising 2, 4, 5, 6, 7, 8, 9 or 10 anti-Ras peptides. In some embodiments, the mRNA construct encodes one or more linkers as described herein.

When a construct contains multiple anti-Ras peptides, the construct can contain multiple copies of the same anti-Ras peptide or, alternatively, can contain a combination of two or more different anti-Ras peptides. In certain embodiments, the anti-Ras peptide has the amino acid sequence shown in SEQ ID NO: 115 in Table 4 below. In Tables 4 and 5 below, SEQ ID NO: 115 shows the amino acid sequence of a representative anti-Ras peptide, whereas SEQ ID NOs: 116-118 show the amino acid sequences of this anti-Ras peptide inserted into the N-terminus (SEQ ID NO: 116), Loop 1 (SEQ ID NO: 117) or Loop 2 (SEQ ID NO: 118) of the SQT scaffold. Without wishing to be bound by theory, it is known in the art that unregulated activity of RAS gene products can cause cancer (see, e.g., Goodsell, DS Oncologist 4: 263-4, 1999). Thus, the disclosure features methods of altering anti-Ras activity to treat or prevent cancer.

In some embodiments, a Ste A scaffold-anti-Ras peptide construct (containing one or multiple copies of an anti-Ras peptide) as used herein may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to the amino acid sequence shown in SEQ ID NO: 115. In some embodiments, a Ste A scaffold-anti-Ras peptide construct may have any one of the following sequences in Table 5 or in SEQ ID NOs: 116-118 but for having at least 1, 2, or 3 substitutions, C-terminal or N-terminal additions or deletions as compared to said sequences.

In some embodiments, the anti-Ras peptide or scaffold construct thereof includes an amino acid sequence selected from the group consisting of SEQ ID NOs 115-118 below, as shown in Table 4 or 5 or the Sequence Listing. The sequence of the anti-Ras peptide within an SQT scaffold construct is underlined and bolded in Table 5 below.

**Table 4 Illustrative Anti-RAS Peptide**

| **SEQ ID NO** | **Anti-RAS Sequence** | **Description** |
|---|---|---|
| 115 | HYPWFKARLYPL | Anti-RAS peptide |

**Table 5 . Illustrative Anti-Ras Scaffold Constructs**

| **SEQ ID NO** | **Anti-Ras-SQT Construct** | **Source Protein (UniProt)** |
|---|---|---|
| 116 | MIPRHYPWFKARLYPLGLSEAKPATPEIQEIVDKVKPQLEEKTNETYGK | Anti-Ras |
| | LEAVQYKTQVLASTNYYIKVRAGDNKYMHLKVFNGPPGQNADRVLTGY QVDKNKDDELTGF | inserted in N-term of scaffold |
| 117 | MIPRGLSEAKPATPEIQEIVDKVKPQLEEKTNETYGKLEAVQYKTQVL HYPWFKARLYPLASTNYYIKVRAGDNKYMHLKVFNGPPGQNADRVLTGY QVDKNKDDELTGF | Anti-Ras inserted in Loop 1 of scaffold |
| 118 | MIPRGLSEAKPATPEIQEIVDKVKPQLEEKTNETYGKLEAVQYKTQVL ASTNYYIKVRAGDNKYMHLKVFNGPHYPWFKARLYPLPGQNADRVLTGY QVDKNKDDELTGF | Anti-Ras inserted in Loop 2 of scaffold |

In some embodiments, a Ste A scaffold-anti-Ras peptide construct may be able to alter cell growth and proliferation. A person of ordinary skill in the art can readily determine if a Ste A scaffold-anti-Ras peptide construct is able to affect cell growth and proliferation using a variety of methods known in the art.

### SALL4-inhibitory Peptides

In various embodiments, an mRNA of the invention encodes one or more SALL4-inhibitory peptides or peptide constructs (e.g., one copy or multiple copies of a SALL4-inhibitory peptide) as a fusion protein comprising a SteA polypeptide and one or more SALL4-inhibitory peptides located at the N-terminal insertion site, the loop 1 insertion site and/or the loop 2 insertion site.

In one embodiment, the isolated mRNA encodes a fusion protein comprising a Ste A scaffold (such as SQT) and one copy of a SALL4-inhibitory peptide. In other embodiments, an mRNA of the invention encodes a fusion protein comprising a Ste A scaffold (such as SQT) and multiple copies of a SALL4-inhibitory peptide. SALL4 encodes a zinc-finger transcription factor that is not normally expressed in adult tissue but is expressed in a subset of hepatocellular carcinomas (WO2013043128; Yong, New Engl. J. Med. 368:2266-2276, 2013). Consequently, Ste A scaffold-SALL4-inhibitory peptide constructs of the disclosure may be used to treat or prevent cancer.

In particular embodiments, the isolated mRNAs encode a fusion protein comprising a Ste A scaffold (such as SQT) and multiple copies of a SALL4-inhibitory peptide. In one embodiment, the mRNA encodes a Ste A fusion protein comprising at least three SALL4-inhibitory peptides. In one embodiment, the mRNA encodes a Ste A fusion protein comprising two to ten SALL4-inhibitory peptides. In one embodiment, the mRNA encodes a Ste A fusion protein comprising three SALL4-inhibitory peptides. In other embodiments, the mRNA encodes a Ste A fusion protein comprising 2, 4, 5, 6, 7, 8, 9 or 10 SALL4-inhibitory peptides. In some embodiments, the mRNA construct encodes one or more linkers as described herein.

When a construct contains multiple SALL4-inhibitory peptides, the construct can contain multiple copies of the same SALL4-inhibitory peptide or, alternatively, can contain a combination of two or more different SALL4-inhibitory peptides. In certain embodiments, the SALL4-inhibitory peptide comprises an amino acid sequence set forth in SEQ ID NO: 125.

In some embodiments, a SALL4-inhibitory peptide as used herein may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to an amino acid sequence set forth in SEQ ID NO: 125. In some embodiments, a peptide or scaffold construct may have any one of the following sequences set forth in Table 6 or in SEQ ID NOs: 119-125 but for having at least 1, 2, or 3 substitutions, C-terminal or N-terminal additions or deletions as compared to said sequences.

SEQ ID NO: 125 shows the amino acid sequence of a representative SALL4-inhibitory peptide, whereas SEQ ID NOs: 119-124 show the amino acid sequences of this SALL4-inhibitory peptide inserted into the N-terminus (SEQ ID NO: 119), Loop 1 (SEQ ID NO: 120) or Loop 2 (SEQ ID NO: 121) of the SQT scaffold. SEQ ID NOs: 122-124 show the equivalent constructs to SEQ ID NOs: 119-121, but with a V5 tag at the C-terminal end. The sequence of the SALL4-inhibitory peptide within an SQT scaffold construct is underlined in Table 6 below.

**Table 6. Illustrative SALL4-inhibitory Peptides and Scaffold Constructs**

| **SEQ ID NO** | **SALL4-inhibitory Sequence** | **Source Protein (UniProt)** |
|---|---|---|
| 119 | MIPRMSRRKQAKPQHIGLSEAKPATPEIQEIVDKVK PQLEEKTNETYGKLEAVQYKTQVLASTNYYIKVRAG DNKYMHLKVFNGPPGQNADRVLTGYQVDKNKDDEL TGF | SALL4-inhibitory peptide inserted in N-term of scaffold |
| 120 | MIPRGLSEAKPATPEIQEIVDKVKPQLEEKTNETYG | SALL4-inhibitory peptide inserted in Loop 1 of scaffold |
| | KLEAVQYKTQVLMSRRKQAKPQHIASTNYYIKVRAG DNKYMHLKVFNGPPGQNADRVLTGYQVDKNKDDEL TGF | |
| 121 | MIPRGLSEAKPATPEIQEIVDKVKPQLEEKTNETYG KLEAVQYKTQVLASTNYYIKVRAGDNKYMHLKVFN GPMSRRKQAKPQHIPGQNADRVLTGYQVDKNKDDEL TGF | SALL4-inhibitory peptide inserted in Loop 2 of scaffold |
| 122 | MIPRMSRRKQAKPQHIGLSEAKPATPEIQEIVDKVK PQLEEKTNETYGKLEAVQYKTQVLASTNYYIKVRAG DNKYMHLKVFNGPPGQNADRVLTGYQVDKNKDDEL TGF GKPIPNPLLGLDST | SALL4-inhibitory peptide inserted in N-term of scaffold, +V5 flag |
| 123 | MIPRGLSEAKPATPEIQEIVDKVKPQLEEKTNETYG KLEAVQYKTQVLMSRRKQAKPQHIASTNYYIKVRAG DNKYMHLKVFNGPPGQNADRVLTGYQVDKNKDDEL TGF GKPIPNPLLGLDST | SALL4-inhibitory peptide inserted in Loop 1 of scaffold, +V5 flag |
| 124 | MIPRGLSEAKPATPEIQEIVDKVKPQLEEKTNETYG KLEAVQYKTQVLASTNYYIKVRAGDNKYMHLKVFN GPMSRRKQAKPQHIPGQNADRVLTGYQVDKNKDDEL TGF GKPIPNPLLGLDST | SALL4-inhibitory peptide inserted in Loop 2 of scaffold, +V5 flag |
| 125 | MSRRKQAKPQHI | isolated SALL4-inhibitory peptide |

In some embodiments, a Ste A scaffold-SALL4-inhibitory peptide construct may be able to inhibit cell proliferation. A person of ordinary skill in the art can readily determine if a Ste A scaffold-SALL4-inhibitory peptide construct is able to inhibit cell proliferation using a variety of methods known in the art.

### TOPK-inhibitory Peptides

In various embodiments, an mRNA of the invention encodes one or more inhibitory peptides of T-lymphokine-activated killer cell-originated protein kinase (TOPK) (e.g., one copy or multiple copies of a TOPK-inhibitory peptide) as a fusion protein comprising a SteA polypeptide and one or more TOPK-inhibitory peptides located at the N-terminal insertion site, the loop 1 insertion site and/or the loop 2 insertion site.

In one embodiment, the isolated mRNA encodes a fusion protein comprising a Ste A scaffold (such as SQT) and one copy of the TOPK-inhibitory peptide. In other embodiments, an mRNA of the invention encodes a fusion protein comprising a Ste A scaffold (such as SQT) and multiple copies of a TOPK-inhibitory peptide. TOPK is critical for mitosis of breast cancer cells (see, e.g,. Matsuo et al., Science Translation Medicine, 6(259): 259ra145, and US Patent No. 8,673,548). Consequently, Ste A scaffold-TOPK-inhibitory peptide constructs of the disclosure can be used to treat or preventing cancer.

In particular embodiments, the isolated mRNAs encode a fusion protein comprising a Ste A scaffold (such as SQT) and multiple copies of a TOPK-inhibitory peptide. In one embodiment, the mRNA encodes a fusion protein comprising a Ste A scaffold and at least three TOPK-inhibitory peptides. In one embodiment, the mRNA encodes a fusion protein comprising a Ste A scaffold and two to ten TOPK-inhibitory peptides. In one embodiment, the mRNA encodes a fusion protein comprising a Ste A scaffold and three TOPK-inhibitory peptides. In other embodiments, the mRNA encodes a fusion protein comprising a Ste A scaffold and 2, 4, 5, 6, 7, 8, 9 or 10 TOPK-inhibitory peptides. In some embodiment, the mRNA encodes one or more linkers as described herein.

When a construct contains multiple TOPK-inhibitory peptides, the construct can contain multiple copies of the same TOPK-inhibitory peptide or, alternatively, can contain a combination of two or more different TOPK-inhibitory peptides. In certain embodiments, the TOPK-inhibitory peptide comprises an amino acid sequence set forth in SEQ ID NO: 129.

In some embodiments, a TOPK-inhibitory peptide as used herein may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to the amino acid sequence set forth in SEQ ID NO: 129. In some embodiments, a peptide or scaffold construct may have any one of the following sequences in Table 7 or in SEQ ID NOs: 126-129 but for having at least 1, 2, or 3 substitutions, C-terminal or N-terminal additions or deletions as compared to said sequences. SEQ ID NO: 129 shows the amino acid sequence of a representative TOPK-inhibitory peptide, whereas SEQ ID NOs: 126-128 show the amino acid sequences of this TOPK-inhibitory peptide inserted into the N-terminus (SEQ ID NO: 126), Loop 1 (SEQ ID NO: 127) or Loop 2 (SEQ ID NO: 128) of the SQT scaffold. The sequence of the TOPK-inhibitory peptide within the SQT scaffold constructs is bolded in Table 7 below.

**Table 7. Illustrative TOPK-inhibitory peptides and Scaffolds**

| **SEQ ID NO** | | **Source Protein (UniProt)** |
|---|---|---|
| 126 | MIPRMEGISNFKTPSKLSEKKKGLSEAKPATPEIQEIV DKVKPQLEEKTNETYGKLEAVQYKTQVLASTNYYIKVRA GDNKYMHLKVFNGPPGQNADRVLTGYQVDKNKDDELTG F | TOPK-inhibitory peptide inserted in N-term of scaffold |
| 127 | MIPRGLSEAKPATPEIQEIVDKVKPQLEEKTNETYGKLE AVQYKTQVLMEGISNFKTPSKLSEKKKASTNYYIKVRA GDNKYMHLKVFNGPPGQNADRVLTGYQVDKNKDDELTG F | TOPK-inhibitory peptide inserted in Loop 1 of scaffold |
| 128 | MIPRGLSEAKPATPEIQEIVDKVKPQLEEKTNETYGKLE AVQYKTQVLASTNYYIKVRAGDNKYMHLKVFNGPMEGI SNFKTPSKLSEKKKPGQNADRVLTGYQVDKNKDDELTG F | TOPK-inhibitory peptide inserted in Loop 2 of scaffold |
| 129 | MEGISNFKTPSKLSEKKK | isolated TOPK-inhibitory peptide |

In some embodiments, a Ste A scaffold-TOPK-inhibitory peptide construct may be able to inhibit cell proliferation. A person of ordinary skill in the art can readily determine if a Ste A scaffold-TOPK-inhibitory peptide construct is able to inhibit cell proliferation using a variety of methods known in the art.

### p53-Inhibitory Peptides

In various embodiments, an mRNA of the invention encodes one or more p53-inhibitory peptides (e.g., one copy or multiple copies of a p53-inhibitory peptide) as a fusion protein comprising a SteA polypeptide and one or more p53-inhibitory peptides located at the N-terminal insertion site, the loop 1 insertion site and/or the loop 2 insertion site.

p53 is a tumor suppressor (see, e.g., Surget S et al., OncoTargets and Therapy 7: 57-68, 2013) implicated is a wide range of proliferative and/or tumorogenic disorders, in particular, cancer. MDM2 is a negative regulator of p53 and inhibition of the p53/MDM2 interaction activates the tumor suppressor activity of p53 and decreases p53 degradation. Consequently, p53-inhibitory peptides of the disclosure that disrupt the p53/MDM2 interaction can be used to treat or preventing proliferative and/or tumorogenic disorders, in particular, cancer.

In one embodiment, the isolated mRNA encodes a fusion protein comprising a Ste A scaffold (such as SQT) and one copy of a p53-inhibitory peptide. In other embodiments, the isolated mRNAs encode a fusion protein comprising a Ste A scaffold (such as SQT) and multiple copies of a p53-inhibitory peptide. In one embodiment, the mRNA encodes a Ste A fusion protein comprising at least three p53-inhibitory peptides. In one embodiment, the mRNA encodes a Ste A fusion protein comprising two to ten p53-inhibitory peptides. In one embodiment, the mRNA encodes a Ste A fusion protein comprising three p53-inhibitory peptides. In other embodiments, the mRNA encodes a Ste A fusion protein comprising 2, 4, 5, 6, 7, 8, 9 or 10 p53-inhibitory peptides. In some embodiments, the mRNA encodes one or more linkers as described herein.

When a construct contains multiple p53- inhibitory peptides, the construct can contain multiple copies of the same p53- inhibitory peptide or, alternatively, can contain a combination of two or more different p53- inhibitory peptides. In certain embodiments, the p53- inhibitory peptide is selected from SEQ ID NOs: 130-141 set forth in the Table 8 below.

In some embodiments, the p53-inhibitory peptide is a biologically-active portion, isolated from human p53. In exemplary aspects of the disclosure, the p53-inhibitory peptide (also referred to herein as a p53-inhibitory domain) is obtained from a full-length or naturally-occurring p53 protein or polypeptide, wherein the peptide or domain lacks the full function of p53, *e.g.,* a functionality and/or biological activity attributed to one or more p53 domains/peptides distinct from said inhibitory domain function. In other embodiments, the p53-inhibitory peptide may be from a non-human species, e.g., *Caenorhabditis elegans,* rodents (e.g., mice and rats), or non-human primates. In yet other embodiments, the p53-inhibitory peptide can be a p53-binding peptide isolated from a phage display library (e.g., as described in Pazgier, M. et al. (2009) Proc. Natl. Acad. Sci. USA 106:4665-4670; Phan, J. et al. (2010) J. Biol. Chem. 285:2174-2183).

In some embodiments, a p53-inhibitory peptide as used herein may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to any of the p53-inhibitory peptides shown below in Table 8 (SEQ ID NOs: 130-141). In some embodiments, a p53-inhibitory peptide within a scaffold construct may have any one of the following sequences in Table 8 or in SEQ ID NOs: 130-141 but for having at least 1, 2, or 3 substitutions, C-terminal or N-terminal additions or deletions as compared to said sequences.

In some embodiments, a p53-inhibitory peptide scaffold construct as used herein may include an amino acid sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to any of the p53-inhibitory peptide scaffold constructs shown below in Table 9. In some embodiments, a p53-inhibitory peptide scaffold construct may have any one of the following sequences in Table 9 or in SEQ ID NOs: 142-148 but for having at least 1, 2, or 3 substitutions, C-terminal or N-terminal additions or deletions as compared to said sequences. In some embodiments, a p53-inhibitory peptide scaffold construct comprises an amino acid sequence shown in SEQ ID NOs: 142-148.

In some embodiments, a p53-inhibitory peptide scaffold construct as used herein may be encoded by a nucleotide sequence having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to a nucleotide sequence shown in SEQ ID NOs: 149-155. In some embodiments, a p53-inhibitory peptide scaffold construct may be encoded by a nucleotide sequence shown in SEQ ID NOs: 149-155 but for having at least 1, 2, or 3 substitutions, C-terminal or N-terminal additions or deletions as compared to said sequences. In some embodiments, a p53-inhibitory peptide scaffold construct is encoded by a nucleotide sequence shown in SEQ ID NOs: 149-155.

In some embodiments, a p53-inhibitory peptide scaffold construct as used herein may be encoded by an mRNA construct having at least about 60% (e.g., about 60%, about 62%, about 64%, about 66%, about 68%, about 70%, about 72%, about 74%, about 76%, about 78%, about 80%, about 82%, about 84%, about 86%, about 88%, about 90%, about 92%, about 94%, about 96%, about 98%, or about 99%) identity to an mRNA constuct sequence shown in SEQ ID NOs: 157-163. In some embodiments, a p53-inhibitory peptide scaffold construct may be encoded by an mRNA construct sequence shown in SEQ ID NOs: 157-163 but for having at least 1, 2, or 3 substitutions, C-terminal or N-terminal additions or deletions as compared to said sequences. In some embodiments, a p53-inhibitory peptide scaffold construct is encoded by an mRNA construct sequence shown in SEQ ID NOs: 157-163.

In some embodiments, the p53-inhibitory peptide includes an amino acid sequence selected from those shown in Table 8 below.

**Table 8. Illustrative p53-inhibitory peptides**

| **SEQ ID NO** | **p53-inhibitory sequence** | **Description** |
|---|---|---|
| 130 | TSFAEYWNLLSP | pMI |
| 131 | ETFSDLWKLLPE | p53 |
| 132 | ETFEHWWSQLLS | pDIQ |
| 133 | ETFEHWW AQLTS | P1E6W |
| 134 | LTFEHSWAQLTS | p536S (6S) |
| 135 | LTFEHWW AQLTS | P6W |
| 136 | LTFEHYW AQLTS | P3848 |
| 137 | LTFEEYWAQLLSAA | P7041 |
| 138 | LTAEEYTAQLLSAA | P7342 |
| 139 | TNWYANLEKLLR | PMI-a |
| 140 | TAWYANFEKLLR | PMI-b |
| 141 | DWWPLAFEALLR | PMI-g |

In some embodiments, the p53-inhibitory peptide scaffold construct includes an amino acid sequence selected from those shown in Table 9 below. The sequence of the p53-inhibitory peptide within the SQT scaffold constructs is bolded in Table 9 below.

**Table 9. Illustrative p53-inhibitory peptide scaffold constructs**

| **SEQ ID NO** | **p53-inhibitory peptide scaffold construct sequences** | **Description** |
|---|---|---|
| 142 | MIPRLTFEHYWAQLTSGLSEAKPATPEIQEIVDKV KPQLEEKTNETYGKLEAVQYKTQVLASTNYYIKVRA GDNKYMHLKVFNGPPGQNADRVLTGYQVDKNKDDE LTGF | P53 inhibitory peptide p3848 inserted in N-term of scaffold |
| 143 | MIPRLTFEEYWAQLLSAAGLSEAKPATPEIQEIVD KVKPQLEEKTNETYGKLEAVQYKTQVLASTNYYIKV RAGDNKYMHLKVFNGPPGQNADRVLTGYQVDKNKD DELTGF | P53 inhibitory peptide p7041 inserted in N-term of scaffold |
| 144 | MIPRETFEHWWSQLLSGLSEAKPATPEIQEIVDKV KPQLEEKTNETYGKLEAVQYKTQVLASTNYYIKVRA GDNKYMHLKVFNGPPGQNADRVLTGYQVDKNKDDE LTGF | P53 inhibitory peptide pDIQ inserted in N-term of scaffold |
| 145 | MIPRTSFAEYWNLLSPGLSEAKPATPEIQEIVDKV KPQLEEKTNETYGKLEAVQYKTQVLASTNYYIKVRA GDNKYMHLKVFNGPPGQNADRVLTGYQVDKNKDDE | P53 inhibitory peptide pMI inserted in N-term of scaffold |
| | LTGF | |
| 146 | MIPRGLSEAKPATPEIQEIVDKVKPQLEEKTNETYG KLEAVQYKTQVLASTNYYIKVRAGDNKYMHLKVFN GPLTFEHYWAQLTSPGQNADRVLTGYQVDKNKDDE LTGF | P53 inhibitory peptide p3848 inserted in Loop 2 of scaffold |
| 147 | MIPRGLSEAKPATPEIQEIVDKVKPQLEEKTNETYG KLEAVQYKTQVLASTNYYIKVRAGDNKYMHLKVFN GPETFEHWWAQLTSPGQNADRVLTGYQVDKNKDD ELTGF | P53 inhibitory peptide p1E6W inserted in Loop 2 of scaffold |
| 148 | MIPRLTFEHWWAQLTSGLSEAKPATPEIQEIVDK VKPQLEEKTNETYGKLEAVQYKTQVLASTNYYIKVR AGDNKYMHLKVFNGPPGQNADRVLTGYQVDKNKDD ELTGF | P53 inhibitory peptide p6W inserted in N-term of scaffold |

In some embodiments, a Ste A scaffold-p53-inhibitory peptide construct may be able to inhibit cell proliferation. A person of ordinary skill in the art can readily determine if a Ste A scaffold-p53-inhibitory peptide construct is able to inhibit cell proliferation using a variety of methods known in the art.

### Screening of Binding Polypeptide Libraries

In one embodiment, a binding polypeptide of interest is selected by screening a library of binding polypeptide libraries that are presented on the SteA scaffold fusion polypeptide. That is, a library of nucleotides encoding binding polypeptides can be incorporated into mRNAs encoding the SteA scaffold fusion polypeptide, e.g., at the N-terminal insertion site, at the loop 1 insertion site and/or at the loop 2 insertion site, and the resultant SteA-binding polypeptide library can be screened for a binding polypeptide having the desired binding property of interest. For example, a library of binding polypeptides can be introduced into host cells that express MYC and a MYC-responsive reporter gene and binding polypeptides that bind to MYC and inhibit its association with MYC regulatory elements within the regulatory or promoter region of the reporter gene can be selected based on reduced expression of the reporter gene. Similarly, a library of binding polypeptides can be introduced into host cells that express STAT3 and a STAT3-responsive reporter gene and binding polypeptides that bind to STAT3 and inhibit its association with STAT3 regulatory elements within the regulatory or promoter region of the reporter gene can be selected based on reduced expression of the reporter gene.

The library of binding polypeptides can be, for example, a library of mutated versions of a known binding domain or can be a library of randomly generated polypeptides.

In one embodiment, the library of binding polypeptides is a library of BH3 domains. For example, a library of polypeptides having the amino acid sequence of X₁X₂X₃X₄X₅X₆X₇X₈X₉DX₁₀X₁₁X₁₂, wherein X₁, X₅, X₈, and X₁₁ are, independently, any hydrophobic residue, X₂ and X₉ are, independently, Gly, Ala, or Ser, X₃, X₄, X₆, and X₇ are, independently, any amino acid residue, X₁₀ is Asp or Glu, and X₁₂ is Asn, His, Asp, or Tyr, can be generated and screened for a BH3 domain having the desired functional property, such as activation of apoptosis. In some embodiments, a hydrophobic residue is Leu, Ala, Val, Ile, Pro, Phe, Met or Trp. In some embodiments, X₅ is Leu

In another embodiment, the library of binding polypeptides is a library of SteA polypeptides. For example, the wild type sequences of a SteA polypeptide within the loop 1 or loop 2 region can be mutagenized to create a library of modified SteA polypeptide sequences that then can be screened for a binding property of interest. Thus, "a binding polypeptide" of the invention within the SteA scaffold fusion polypeptide can be a modified form of the wild-type SteA sequence.

In one embodiment, the library of binding polypeptides is presented on a SteA scaffold fusion protein and screened for desired binding and/or functional properties. In another embodiment, the library of binding polypeptides is screened using a different expression system, such as phage display, yeast display or other library expression system well-established in the art, a binding polypeptide is selected having the desired binding and/or functional properties, the binding polypeptide sequence is determined and then a nucleotide sequence encoding the selected binding polypeptide sequence is introduced into an mRNA encoding a SteA scaffold fusion polypeptide, e.g., at the N-terminal insertion site, the loop 1 insertion site and/or the loop 2 insertion site such that the selected binding polypeptide can be presented by the SteA scaffold fusion polypeptide. General methodologies for screening libraries using scaffold proteins in systems such as phage display are described in, for example, PCT Publication WO 2014/125290.

### Nanoparticles

The mRNAs of the invention may be formulated in nanoparticles or other delivery vehicles, e.g., to protect them from degradation when delivered to a subject. Illustrative nanoparticles are described in Panyam, J. & Labhasetwar, V. Adv. Drug Deliv. Rev. 55, 329-347 (2003) and Peer, D. et al. Nature Nanotech. 2, 751-760 (2007). In certain embodiments, an mRNA of the invention is encapsulated within a nanoparticle. In particular embodiments, a nanoparticle is a particle having at least one dimension (e.g., a diameter) less than or equal to 1000 nM, less than or equal to 500 nM or less than or equal to 100 nM. In particular embodiments, a nanoparticle includes a lipid. Lipid nanoparticles include, but are not limited to, liposomes and micelles. Any of a number of lipids may be present, including cationic and/or ionizable lipids, anionic lipids, neutral lipids, amphipathic lipids, PEGylated lipids, and/or structural lipids. Such lipids can be used alone or in combination. In particular embodiments, a lipid nanoparticle comprises one or more mRNAs described herein, e.g., a mRNA encoding a SteA fusion polypeptide, *e.g.,* a SteA-BH3 fusion polypeptide, and/or a mRNA encoding a BH3-trap polypeptide.

In some embodiments, the lipid nanoparticle formulations of the mRNAs described herein may include one or more (e.g., 1, 2, 3, 4, 5, 6, 7, or 8) cationic and/or ionizable lipids. Such cationic lipids include, but are not limited to, 3-(didodecylamino)-N1,N1,4-tridodecyl-1-piperazineethanamine (KL10), N1- [2-(didodecylamino)ethyl] - N1,N4,N4-tridodecyl-1,4-piperazinediethanamine (KL22), 14,25-ditridecyl-15,18,21,24-tetraaza-octatriacontane (KL25), 1,2-dilinoleyloxy-N,N-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA), 2,2-dilinoleyl-4-(2-dimethylaminoethyl)-[1,3]-dioxolane (DLin-KC2-DMA), 2-({8-[(3p)-cholest-5-en-3-yloxy]octyl}oxy)-N,N-dimethyl-3-[(9Z,12Z)-octadeca-9,12-dien-1-yloxy]propan-1-amine (Octyl-CLinDMA),
(2R)-2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2R)),
(2S)-2-({8-[(3β)-cholest-5-en-3-yloxy]octyl}oxy)-N,N-dimethyl-3-[(9Z,12Z)-octadeca-9,12-dien-1-yloxy]propan-1-amine (Octyl-CLinDMA (2S)).N,N-dioleyl-N,N-dimethylammonium chloride ("DODAC"); N-(2,3-dioleyloxy)propyl-N,N--N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 1,2-Dioleyloxy-3-trimethylaminopropane chloride salt ("DOTAP.Cl"); 3-β-(N-(N',N'-dimethylaminoethane)-carbamoyl)cholesterol ("DC-Chol"), N-(1-(2,3-dioleyloxy)propyl)-N-2-(sperminecarboxamido)ethyl)-N,N-dimethyl- ammonium trifluoracetate ("DOSPA"), dioctadecylamidoglycyl carboxyspermine ("DOGS"), 1,2-dioleoyl-3-dimethylammonium propane ("DODAP"), N,N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, a number of commercial preparations of cationic and/or ionizable lipids can be used, such as, e.g., LIPOFECTIN® (including DOTMA and DOPE, available from GIBCO/BRL), and LIPOFECTAMINE® (including DOSPA and DOPE, available from GIBCO/BRL). KL10, KL22, and KL25 are described, for example, in U.S. Patent No. 8,691,750. In particular embodiments, the lipid is DLin-MC3-DMA or DLin-KC2-DMA.

Anionic lipids suitable for use in lipid nanoparticles of the invention include, but are not limited to, phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, and other anionic modifying groups joined to neutral lipids.

Neutral lipids suitable for use in lipid nanoparticles of the invention include, but are not limited to, diacylphosphatidylcholine, diacylphosphatidylethanolamine, ceramide, sphingomyelin, dihydrosphingomyelin, cephalin, and cerebrosides. Lipids having a variety of acyl chain groups of varying chain length and degree of saturation are available or may be isolated or synthesized by well-known techniques. Additionally, lipids having mixtures of saturated and unsaturated fatty acid chains can be used. In some embodiments, the neutral lipids used in the invention are DOPE, DSPC, DPPC, POPC, or any related phosphatidylcholine. In some embodiments, the neutral lipid may be composed of sphingomyelin, dihydrosphingomyeline, or phospholipids with other head groups, such as serine and inositol.

In some embodiments, amphipathic lipids are included in nanoparticles of the disclosure Exemplary amphipathic lipids suitable for use in nanoparticles of the invention include, but are not limited to, sphingolipids, phospholipids, and aminolipids. In some embodiments, a phospholipid is selected from the group consisting of 1,2-dilinoleoyl-sn-glycero-3-phosphocholine (DLPC), 1,2-dimyristoyl-sn-glycero-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine,1,2-dioleoyl-sn-glycero-3-phosphoetha nolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, 1,2-dioleoyl-sn-glycero-3-phospho-rac-(1-glycerol) sodium salt (DOPG), and sphingomyelin. Other phosphorus-lacking compounds, such as sphingolipids, glycosphingolipid families, diacylglycerols, and β-acyloxyacids, may also be used. Additionally, such amphipathic lipids can be readily mixed with other lipids, such as triglycerides and sterols.

In some embodiments, the lipid component of a nanoparticle of the invention may include one or more PEGylated lipids. A PEGylated lipid (also known as a PEG lipid or a PEG-modified lipid) is a lipid modified with polyethylene glycol. The lipid component may include one or more PEGylated lipids. A PEGylated lipid may be selected from the non-limiting group consisting of PEG-modified phosphatidylethanolamines, PEG-modified phosphatidic acids, PEG-modified ceramides, PEG-modified dialkylamines, PEG-modified diacylglycerols, and PEG-modified dialkylglycerols. For example, a PEGylated lipid may be PEG-c-DOMG, PEG-DMG, PEG-DLPE, PEG-DMPE, PEG-DPPC, or a PEG-DSPE lipid.

A lipid nanoparticle of the invention may include one or more structural lipids. Exemplary, non-limiting structural lipids that may be present in the lipid nanoparticles of the invention include cholesterol, fecosterol, sitosterol, campesterol, stigmasterol, brassicasterol, ergosterol, tomatidine, tomatine, ursolic acid, or alpha-tocopherol).

In some embodiments, one or more mRNAs of the invention may be formulated in a lipid nanoparticle having a diameter from about 1 nm to about 900 nm, e.g., about 1 nm to about 100 nm, about 1 nm to about 200 nm, about 1 nm to about 300 nm, about 1 nm to about 400 nm, about 1 nm to about 500 nm, about 1 nm to about 600 nm, about 1 nm to about 700 nm, about 1 nm to 800 nm, about 1 nm to about 900 nm. In some embodiments, the nanoparticle may have a diameter from about 10 nm to about 300 nm, about 20 nm to about 200 nm, about 30 nm to about 100 nm, or about 40 nm to about 80 nm. In some embodiments, the nanoparticle may have a diameter from about 30 nm to about 300 nm, about 40 nm to about 200 nm, about 50 nm to about 150 nm, about 70 to about 110 nm, or about 80 nm to about 120 nm. In one embodiment, an mRNA may be formulated in a lipid nanoparticle having a diameter from about 10 to about 100 nm including ranges in between such as, but not limited to, about 10 to about 20 nm, about 10 to about 30 nm, about 10 to about 40 nm, about 10 to about 50 nm, about 10 to about 60 nm, about 10 to about 70 nm, about 10 to about 80 nm, about 10 to about 90 nm, about 20 to about 30 nm, about 20 to about 40 nm, about 20 to about 50 nm, about 20 to about 60 nm, about 20 to about 70 nm, about 20 to about 80 nm, about 20 to about 90 nm, about 20 to about 100 nm, about 30 to about 40 nm, about 30 to about 50 nm, about 30 to about 60 nm, about 30 to about 70 nm, about 30 to about 80 nm, about 30 to about 90 nm, about 30 to about 100 nm, about 40 to about 50 nm, about 40 to about 60 nm, about 40 to about 70 nm, about 40 to about 80 nm, about 40 to about 90 nm, about 40 to about 100 nm, about 50 to about 60 nm, about 50 to about 70 nm about 50 to about 80 nm, about 50 to about 90 nm, about 50 to about 100 nm, about 60 to about 70 nm, about 60 to about 80 nm, about 60 to about 90 nm, about 60 to about 100 nm, about 70 to about 80 nm, about 70 to about 90 nm, about 70 to about 100 nm, about 80 to about 90 nm, about 80 to about 100 nm, and/or about 90 to about 100 nm. In one embodiment, an mRNA may be formulated in a lipid nanoparticle having a diameter from about 30 nm to about 300 nm, about 40 nm to about 200 nm, about 50 nm to about 150 nm, about 70 nm to about 110 nm, or about 80 nm to about 120 nm including ranges in between.

In some embodiments, a lipid nanoparticle may have a diameter greater than 100 nm, greater than 150 nm, greater than 200 nm, greater than 250 nm, greater than 300 nm, greater than 350 nm, greater than 400 nm, greater than 450 nm, greater than 500 nm, greater than 550 nm, greater than 600 nm, greater than 650 nm, greater than 700 nm, greater than 750 nm, greater than 800 nm, greater than 850 nm, greater than 900 nm, or greater than 950 nm.

In some embodiments, the particle size of the lipid nanoparticle may be increased and/or decreased. The change in particle size may be able to help counter a biological reaction such as, but not limited to, inflammation, or may increase the biological effect of the mRNA delivered to a patient or subject.

In certain embodiments, it is desirable to target a nanoparticle, e.g., a lipid nanoparticle, of the invention using a targeting moiety that is specific to a cell type and/or tissue type. In some embodiments, a nanoparticle may be targeted to a particular cell, tissue, and/or organ using a targeting moiety. In particular embodiments, a nanoparticle comprises one or more mRNA described herein and a targeting moiety. Exemplary non-limiting targeting moieties include ligands, cell surface receptors, glycoproteins, vitamins (e.g., riboflavin) and antibodies (e.g., full-length antibodies, antibody fragments (e.g., Fv fragments, single chain Fv (scFv) fragments, Fab' fragments, or F(ab')2 fragments), single domain antibodies, camelid antibodies and fragments thereof, human antibodies and fragments thereof, monoclonal antibodies, and multispecific antibodies (e.g,. bispecific antibodies)). In some embodiments, the targeting moiety may be a polypeptide. The targeting moiety may include the entire polypeptide (e.g., peptide or protein) or fragments thereof. A targeting moiety is typically positioned on the outer surface of the nanoparticle in such a manner that the targeting moiety is available for interaction with the target, for example, a cell surface receptor. A variety of different targeting moieties and methods are known and available in the art, including those described, e.g., in Sapra et al., Prog. Lipid Res. 42(5):439-62, 2003 and Abra et al., J. Liposome Res. 12:1-3, 2002.

In some embodiments, a lipid nanoparticle (e.g., a liposome) may include a surface coating of hydrophilic polymer chains, such as polyethylene glycol (PEG) chains (see, e.g., Allen et al., Biochimica et Biophysica Acta 1237: 99-108, 1995; DeFrees et al., Journal of the American Chemistry Society 118: 6101-6104, 1996; Blume et al., Biochimica et Biophysica Acta 1149: 180-184,1993; Klibanov et al., Journal of Liposome Research 2: 321-334, 1992; U.S. Pat. No. 5,013,556; Zalipsky, Bioconjugate Chemistry 4: 296-299, 1993; Zalipsky, FEBS Letters 353: 71-74, 1994; Zalipsky, in Stealth Liposomes Chapter 9 (Lasic and Martin, Eds) CRC Press, Boca Raton Fla., 1995. In one approach, a targeting moiety for targeting the lipid nanoparticle is linked to the polar head group of lipids forming the nanoparticle. In another approach, the targeting moiety is attached to the distal ends of the PEG chains forming the hydrophilic polymer coating (see, e.g., Klibanov et al., Journal of Liposome Research 2: 321-334, 1992; Kirpotin et al., FEBS Letters 388: 115-118, 1996).

Standard methods for coupling the targeting moiety or moieties may be used. For example, phosphatidylethanolamine, which can be activated for attachment of targeting moieties, or derivatized lipophilic compounds, such as lipid-derivatized bleomycin, can be used. Antibody-targeted liposomes can be constructed using, for instance, liposomes that incorporate protein A (see, e.g., Renneisen et al., J. Bio. Chem., 265:16337-16342, 1990 and Leonetti et al., Proc. Natl. Acad. Sci. (USA), 87:2448-2451, 1990). Other examples of antibody conjugation are disclosed in U.S. Pat. No. 6,027,726. Examples of targeting moieties can also include other polypeptides that are specific to cellular components, including antigens associated with neoplasms or tumors. Polypeptides used as targeting moieties can be attached to the liposomes via covalent bonds (see, for example Heath, Covalent Attachment of Proteins to Liposomes, 149 Methods in Enzymology 111-119 (Academic Press, Inc. 1987)). Other targeting methods include the biotin-avidin system.

In some embodiments, a lipid nanoparticle of the invention includes a targeting moiety that targets the lipid nanoparticle to a cell including, but not limited to, hepatocytes, colon cells, epithelial cells, hematopoietic cells, epithelial cells, endothelial cells, lung cells, bone cells, stem cells, mesenchymal cells, neural cells, cardiac cells, adipocytes, vascular smooth muscle cells, cardiomyocytes, skeletal muscle cells, beta cells, pituitary cells, synovial lining cells, ovarian cells, testicular cells, fibroblasts, B cells, T cells, reticulocytes, leukocytes, granulocytes, and tumor cells (including primary tumor cells and metastatic tumor cells). In particular embodiments, the targeting moiety targets the lipid nanoparticle to a hepatocyte. In other embodiments, the targeting moiety targets the lipid nanoparticle to a colon cell. In some embodiments, the targeting moiety targets the lipid nanoparticle to a liver cancer cell (e.g., a hepatocellular carcinoma cell) or a colorectal cancer cell (e.g., a primary tumor or a metastasis).

### Pharmaceutical Compositions

The present invention includes pharmaceutical compositions comprising an mRNA or a nanoparticle (e.g., a lipid nanoparticle) described herein, in combination with one or more pharmaceutically acceptable excipient, carrier or diluent. In some embodiments, a pharmaceutical composition of the invention includes an mRNA encoding a fusion polypeptide comprising a SteA scaffold polypeptide and one or more binding polypeptides, such as one or more BH3 domains, one or more anti-Ras peptides, one or more SALL4-inhibitory peptides, one or more TOPK-inhibitory peptides or one or more p53-inhibitory peptides. In certain embodiments, a pharmaceutical composition of the invention includes an mRNA encoding a Bcl-2-like polypeptide or fragment or variant thereof. In certain embodiments, a pharmaceutical composition includes both a first mRNA encoding a fusion polypeptide comprising a SteA scaffold polypeptide and one or more BH3 domains, and a second mRNA encoding a Bcl-2-like polypeptide or fragment or variant thereof. In particular embodiments, the mRNA is present in a nanoparticle, e.g., a lipid nanoparticle. In particular embodiments, the mRNA or nanoparticle is present in a pharmaceutical composition. In various embodiments, the one or more mRNA present in the pharmaceutical composition is encapsulated in a nanoparticle, e.g., a lipid nanoparticle. In particular embodiments, the molar ratio of the first mRNA to the second mRNA is about 1:50, about 1:25, about 1:10, about 1:5, about 1:4, about 1:3, about 1:2, about 1:1, about 2:1, about 3:1, about 4:1, or about 5:1, about 10:1, about 25:1 or about 50:1. In particular embodiments, the molar ratio of the first mRNA to the second mRNA is greater than 1:1.

Pharmaceutical compositions may optionally include one or more additional active substances, for example, therapeutically and/or prophylactically active substances. Pharmaceutical compositions of the present invention may be sterile and/or pyrogen-free. General considerations in the formulation and/or manufacture of pharmaceutical agents may be found, for example, in Remington: The Science and Practice of Pharmacy 21st ed., Lippincott Williams & Wilkins, 2005. In particular embodiments, a pharmaceutical composition comprises an mRNA and a lipid nanoparticle, or complexes thereof. In particular embodiments, the pharmaceutical compositions or formulations described herein may contain at least one mRNA encoding a fusion polypeptide comprising a SteA scaffold polypeptide and one or more BH3 domains and/or at least one mRNA encoding a BH3-trap polypeptide (e.g., a Bcl-2-like polypeptide).

Formulations of the pharmaceutical compositions described herein may be prepared by any method known or hereafter developed in the art of pharmacology. In general, such preparatory methods include the step of bringing the active ingredient into association with an excipient and/or one or more other accessory ingredients, and then, if necessary and/or desirable, dividing, shaping and/or packaging the product into a desired single- or multi-dose unit.

Relative amounts of the active ingredient, the pharmaceutically acceptable excipient, and/or any additional ingredients in a pharmaceutical composition in accordance with the invention will vary, depending upon the identity, size, and/or condition of the subject treated and further depending upon the route by which the composition is to be administered. By way of example, the composition may include between 0.1% and 100%, e.g., between 0.5% and 70%, between 1% and 30%, between 5% and 80%, or at least 80% (w/w) active ingredient. In some embodiments, the active agent is an mRNA encoding a fusion polypeptide comprising a SteA scaffold polypeptide and one or more BH3 domains.

The mRNAs of the invention (e.g., mRNA encoding a fusion polypeptide comprising a SteA scaffold polypeptide and one or more BH3 domains and/or a BH3-trap polypeptide, or an mRNA encoding a fusion polypeptide comprising a SteA scaffold polypeptide and one or more anti-Ras peptides, one or more SALL4-inhibitory peptides, one or more TOPK-inhibitory peptides or one or more p53-inhibitory peptides) can be formulated using one or more excipients to: (1) increase stability; (2) increase cell transfection; (3) permit the sustained or delayed release (e.g., from a depot formulation of the mRNA); (4) alter the biodistribution (e.g., target the mRNA to specific tissues or cell types); (5) increase the translation of a polypeptide encoded by the mRNA *in vivo;* and/or (6) alter the release profile of a polypeptide encoded by the mRNA *in vivo.* In addition to traditional excipients such as any and all solvents, dispersion media, diluents, or other liquid vehicles, dispersion or suspension aids, surface active agents, isotonic agents, thickening or emulsifying agents, preservatives, excipients of the present invention can include, without limitation, lipidoids, liposomes, lipid nanoparticles (e.g., liposomes and micelles), polymers, lipoplexes, core-shell nanoparticles, peptides, proteins, carbohydrates, cells transfected with mRNAs (e.g., for transplantation into a subject), hyaluronidase, nanoparticle mimics and combinations thereof. Accordingly, the formulations of the invention can include one or more excipients, each in an amount that together increases the stability of the mRNA, increases cell transfection by the mRNA, increases the expression of a polypeptide encoded by the mRNA, and/or alters the release profile of a mRNA-encoded polypeptide. Further, the mRNAs of the present invention may be formulated using self-assembled nucleic acid nanoparticles.

Various excipients for formulating pharmaceutical compositions and techniques for preparing the composition are known in the art (see Remington: The Science and Practice of Pharmacy, 21st Edition, A. R. Gennaro, Lippincott, Williams & Wilkins, Baltimore, MD, 2006. The use of a conventional excipient medium may be contemplated in the present disclosure, except insofar as any conventional excipient medium may be incompatible with a substance or its derivatives, such as by producing any undesirable biological effect or otherwise interacting in a deleterious manner with any other component(s) of the pharmaceutical composition. Excipients may include, for example: antiadherents, antioxidants, binders, coatings, compression aids, disintegrants, dyes (colors), emollients, emulsifiers, fillers (diluents), film formers or coatings, glidants (flow enhancers), lubricants, preservatives, printing inks, sorbents, suspensing or dispersing agents, sweeteners, and waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

In some embodiments, the formulations described herein may include at least one pharmaceutically acceptable salt. Examples of pharmaceutically acceptable salts that may be included in a formulation of the disclosure include, but are not limited to, acid addition salts, alkali or alkaline earth metal salts, mineral or organic acid salts of basic residues such as amines; alkali or organic salts of acidic residues such as carboxylic acids; and the like. Representative acid addition salts include acetate, acetic acid, adipate, alginate, ascorbate, aspartate, benzenesulfonate, benzene sulfonic acid, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

In some embodiments, the formulations described herein may contain at least one type of polynucleotide. As a non-limiting example, the formulations may contain 1, 2, 3, 4, 5 or more than 5 mRNAs described herein. In some embodiments, the formulations described herein may contain at least one mRNA encoding a fusion polypeptide comprising a SteA scaffold polypeptide and one or more BH3 domains and/or a BH3-trap polypeptide (e.g., a Bcl-2-like polypeptide or variant or fragment thereof), and at least one nucleic acid sequence such as, but not limited to, an siRNA, an shRNA, a snoRNA, and an miRNA. In some embodiments, the formulations described herein may contain at least one mRNA encoding a fusion polypeptide comprising a SteA scaffold polypeptide and one or more anti-Ras peptides, one or more SALL4-inhibitory peptides, one or more TOPK-inhibitory peptides or one or more p53-inhibitory peptides, and at least one nucleic acid sequence such as, but not limited to, an siRNA, an shRNA, a snoRNA, and an miRNA.

Liquid dosage forms for e.g., parenteral administration include, but are not limited to, pharmaceutically acceptable emulsions, microemulsions, nanoemulsions, solutions, suspensions, syrups, and/or elixirs. In addition to active ingredients, liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and/or suspending agents. In certain embodiments for parenteral administration, compositions are mixed with solubilizing agents such as CREMAPHOR®, alcohols, oils, modified oils, glycols, polysorbates, cyclodextrins, polymers, and/or combinations thereof.

Injectable preparations, for example, sterile injectable aqueous or oleaginous suspensions may be formulated according to the known art using suitable dispersing agents, wetting agents, and/or suspending agents. Sterile injectable preparations may be sterile injectable solutions, suspensions, and/or emulsions in nontoxic parenterally acceptable diluents and/or solvents, for example, as a solution in 1,3-butanediol. Among the acceptable vehicles and solvents that may be employed are water, Ringer's solution, U.S.P., and isotonic sodium chloride solution. Sterile, fixed oils are conventionally employed as a solvent or suspending medium. For this purpose any bland fixed oil can be employed including synthetic mono- or diglycerides. Fatty acids such as oleic acid can be used in the preparation of injectables. Injectable formulations can be sterilized, for example, by filtration through a bacterial-retaining filter, and/or by incorporating sterilizing agents in the form of sterile solid compositions which can be dissolved or dispersed in sterile water or other sterile injectable medium prior to use.

In some embodiments, pharmaceutical compositions including at least one mRNA described herein are administered to mammals (e.g., humans). Although the descriptions of pharmaceutical compositions provided herein are principally directed to pharmaceutical compositions which are suitable for administration to humans, it will be understood by the skilled artisan that such compositions are generally suitable for administration to any other animal, e.g., to a non-human mammal. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as poultry, chickens, ducks, geese, and/or turkeys. In particular embodiments, a subject is provided with two or more mRNAs described herein, e.g., a first mRNA encoding a SteA scaffold polypeptide including one or more BH3 domains and a second mRNA encoding a Bcl-2-like polypeptide, or two or more mRNAs that encode fusion proteins selected from the group consisting of SteA scaffold-BH3 domain, Ste A scaffold-anti-Ras peptide, Ste A scaffold-SALL4-inhibitory peptide, Ste A scaffold-TOPK-inhibitory peptide and Ste A scaffold-p53-inhibitory peptide (i.e., any of the Ste A scaffold-binding polypeptide constructs described herein can be used in combination). In particular embodiments, the first and second mRNAs are provided to the subject at the same time or at different times, e.g., sequentially. In particular embodiments, the first and second mRNAs are provided to the subject in the same pharmaceutical composition or formulation, e.g., to facilitate uptake of both mRNAs by the same cells.

The present disclosure also includes kits comprising a container comprising a first mRNA encoding a fusion polypeptide comprising a SteA scaffold polypeptide and one or more BH3 domains and a second mRNA encoding a Bcl-2-like polypeptide or variant or fragment thereof, e.g., a prosurvival Bcl-2-like polypeptide. In other embodiments, the kit comprises a first container comprising the mRNA encoding the SteA scaffold and a second container comprising the mRNA encoding the Bcl-2-like polypeptide or variant or fragment thereof. In particular embodiments, the mRNAs are present in the same or different nanoparticles and/or pharmaceutical compositions. In particular embodiments, the mRNAs are lyophilized, dried, or freeze-dried.

### Pharmacokinetics

Pharmacokinetics of SteA scaffold polypeptides are described in further detail in Examples 7-8. The results demonstrate that introduction of the mmRNA constructs into cells leads to expression of the encoded protein (e.g., SteA scaffold) within about 1 hour, within a steady increase in protein expression such that maximum intracellular concentration for the protein can be reached, for example, within about 18-24 hours, or about 20 hours. The half life of the encoded SteA protein can be, for example, about 30-35 hours, or about 33 hours. The encoded SteA protein is cleared as well over time, for example being no longer detectable about 7 days after the mmRNA construct is introduced into the cells. Alternatively, the minimum intracellular concentration of the encoded SteA protein can be reached within about 7 days after the mmRNA construct is introduced into the cells.

Accordingly, in one aspect, the invention pertains to a modified messenger RNA (mmRNA) encoding a polypeptide comprising: (i) a Stefin A (SteA) scaffold polypeptide comprising an N-terminal insertion site, a loop 1 insertion site, and a loop 2 insertion site; and (ii) a first binding polypeptide, wherein the first binding polypeptide is located within the SteA scaffold polypeptide at the N-terminal insertion site, the loop 1 insertion site, or the loop 2 insertion site, and wherein said mmRNA comprises one or more modified nucleobases, wherein the polypeptide encoded by the mmRNA reaches a maximum intracellular concentration within about 18-24 hours after the mmRNA is introduced into a mammalian cell.

In one embodiment, the polypeptide reaches a maximum intracellular concentration within about 20 hours after the mmRNA is introduced into a mammalian cell. In one embodiment, the polypeptide is detectable within about 1 hour after the mmRNA is introduced into a mammalian cell. In one embodiment, the polypeptide is no longer detectable about 7 days after the mmRNA is introduced into a mammalian cell. In one embodiment, the minimum intracellular concentration of the encoded SteA protein is reached within about 7 days after the mmRNA construct is introduced into the cells. In one embodiment, the polypeptide has a half-life of about 30-35 hours, or about 33 hours.

In another aspect, the disclosure pertains to a method of reaching a maximum intracellular concentration of a polypeptide of interest in a minimum time following administration of a modified mRNA (mmRNA) encoding the polypeptide of interest to a subject comprising administering to the subject an mmRNA encoding a polypeptide comprising: (i) a Stefin A (SteA) scaffold polypeptide comprising an N-terminal insertion site, a loop 1 insertion site, and a loop 2 insertion site; and (ii) a first binding polypeptide, wherein the first binding polypeptide is located within the SteA scaffold polypeptide at the N-terminal insertion site, the loop 1 insertion site, or the loop 2 insertion site, and wherein said mmRNA comprises one or more modified nucleobases, wherein the polypeptide encoded by the mmRNA reaches a maximum intracellular concentration within about 18-24 hours after the mmRNA is introduced into a mammalian cell.

In one embodiment, the polypeptide reaches a maximum intracellular concentration within about 20 hours after the mmRNA is administered to the subject. In one embodiment, the mmRNA is administered intravenously encapsulated in an LNP. In one embodiment, the polypeptide is detectable within about 1 hour after the mmRNA is administered to the subject. In one embodiment, the polypeptide is no longer detectable about 7 days after the polynucleotide is administered to the subject. In one embodiment, the minimum intracellular concentration of the polypeptide is reached within about 7 days after the mmRNA construct is introduced into the cells. In one embodiment, the polypeptide has a half-life of about 30-35 hours.

In another aspect, the disclosure pertains to a method for transiently expressing an intracellular polypeptide of interest comprising administering to the subject a modified mRNA (mmRNA) encoding the polypeptide of interest comprising: (i) a Stefin A (SteA) scaffold polypeptide comprising an N-terminal insertion site, a loop 1 insertion site, and a loop 2 insertion site; and (ii) a first binding polypeptide, wherein the first binding polypeptide is located within the SteA scaffold polypeptide at the N-terminal insertion site, the loop 1 insertion site, or the loop 2 insertion site, and wherein said mmRNA comprises one or more modified nucleobases, wherein the polypeptide of interest is detectable 1-3 hours after the mmRNA is administered to the subject and is no longer detectable about 7 days after the mmRNA is administered to the subject.

In one embodiment, the polypeptide of interest has a half-life of about 30-35 hours, or about 33 hours.

In another aspect, the disclosure pertains to a method for providing a polypeptide of interest which is expressed intracellularly to a subject, comprising administering to the subject intravenously a first dose of a modified mRNA (mmRNA) encapsulated in an LNP, wherein the mmRNA encodes the polypeptide of interest and comprises: (i) a Stefin A (SteA) scaffold polypeptide comprising an N-terminal insertion site, a loop 1 insertion site, and a loop 2 insertion site; and (ii) a first binding polypeptide, wherein the first binding polypeptide is located within the SteA scaffold polypeptide at the N-terminal insertion site, the loop 1 insertion site, or the loop 2 insertion site, and wherein said mmRNA comprises one or more modified nucleobases; and administering to the subject intravenously a second dose of the mmRNA encapsulated in an LNP about 7 days following administration of the first dose.

In one embodiment, the polypeptide of interest has a half-life of about 30-35 hours, or about 33 hours.

### Methods of Modulating Target Activity

The disclosure also provides methods of modulating the activity of a target to which a binding polypeptide as described binds. Accordingly, in one embodiment, the disclosure provides a method for modulating the activity of a target to which a binding polypeptide binds in a cell, the method comprising contacting the cell with an mRNA encoding a fusion polypeptide comprising: (i) a Stefin A (SteA) scaffold polypeptide comprising an N-terminal insertion site, a loop 1 insertion site, and a loop 2 insertion site; and (ii) a first binding polypeptide, wherein the first binding polypeptide is located within the SteA scaffold polypeptide at the N-terminal insertion site, the loop 1 insertion site, or the loop 2 insertion site, and wherein said mmRNA comprises one or more modified nucleobases, such that the activity of the target is modulated. The fusion polypeptide can further comprise a second binding polypeptide, wherein the first and the second binding polypeptides are located at different insertion sites selected from the N-terminal insertion site, the loop 1 insertion site, or the loop 2 insertion site. In various embodiments, the first and the second binding polypeptides comprise the same amino acid sequence or comprise different amino acid sequences. In one embodiment, the fusion polypeptide comprises a binding polypeptide located at the N-terminal insertion site. In another embodiment, the fusion polypeptide comprises a binding polypeptide located at the loop 1 insertion site. In another embodiment, the fusion polypeptide comprises a binding polypeptide located at the loop 2 insertion site. In another embodiment, the fusion polypeptide further comprises a third binding polypeptide, wherein the fusion polypeptide comprises a binding polypeptide located at each of the N-terminal insertion site, the loop 1 insertion site, and the loop 2 insertion site. In one embodiment, the first, second, and third binding polypeptides comprise the same amino acid sequence. In another embodiment, the first, second, and third binding polypeptides comprise at least two different amino acid sequences. In still another embodiment, each of the first, second and third binding polypeptides has a different amino acid sequence.

Binding polypeptides and their targets suitable for use in the method of modulating the activity of a target are as described herein before, including fusion polypeptides comprising a SteA scaffold polypeptide and one or more BH3 domains, one or more anti-Ras peptides, one or more SALL4-inhibitory peptides, one or more TOPK-inhibitory peptides or one or more p53-inhibitory peptides.

In another embodiment, the cells are contacted with a lipid nanoparticle comprising the modified mRNA. In another embodiment, the cells are contacted with a pharmaceutical composition comprising the modified mRNA, wherein the modified mRNA in the pharmaceutical composition is optionally in a lipid nanoparticle.

In one embodiment, the contacting occurs *in vitro.* In another embodiment, the contacting occurs *in vivo.*

### Therapeutic Methods

Also disclosed are methods of treating or preventing a cancer in a subject in need thereof that involve providing or administering to the subject an mRNA encoding a fusion polypeptide described herein, such as an mRNA encoding a fusion polypeptides comprising a SteA scaffold polypeptide and one or more BH3 domains, one or more anti-Ras peptides, one or more SALL4-inhibitory peptides, one or more TOPK-inhibitory peptides or one or more p53-inhibitory peptides.. In related embodiments, the subject is provided with or administered a nanoparticle (e.g., a lipid nanoparticle) comprising the mRNA. In further related embodiments, the subject is provided with or administered a pharmaceutical composition of the invention to the subject. In particular embodiments, the pharmaceutical composition comprises an mRNA encoding a fusion polypeptide described herein, or it comprises a nanoparticle comprising the mRNA. In particular embodiments, the mRNA is present in a nanoparticle, e.g., a lipid nanoparticle. In particular embodiments, the mRNA or nanoparticle is present in a pharmaceutical composition. In certain embodiments, the subject in need thereof has been diagnosed with a cancer, or is considered to be at risk of developing a cancer. In some embodiments, the cancer is liver cancer or colorectal cancer. In particular embodiments, the liver cancer is hepatocellular carcinoma. In some embodiments, the colorectal cancer is a primary tumor or a metastasis. In some embodiments, the cancer is a hematopoietic cancer. In some embodiments, the cancer is an acute myeloid leukemia, a chronic myeloid leukemia, a chronic myelomonocytic leukemia, a myelodystrophic syndrome (including refractory anemias and refractory cytopenias) or a myeloproliferative neoplasm or disease (including polycythemia vera, essential thrombocytosis and primary myelofibrosis).

In other embodiments, the cancer is a blood-based cancer or a hematopoietic cancer. Selectivity for a particular cancer type can be achieved through the combination of use of an appropriate LNP formulation (e.g., targeting specific cell types) in combination with appropriate regulatory site(s) (e.g., microRNAs) engineered into the mRNA constructs.

In some embodiments, the mRNA, nanoparticle, or pharmaceutical composition is administered to the patient parenterally. In particular embodiments, the subject is a mammal, e.g., a human. In particular embodiments, the pharmaceutical composition comprises an mRNA encoding a fusion polypeptide comprising a SteA scaffold polypeptide and one or more BH3 domains, as described herein. In other embodiments, the pharmaceutical composition comprises an mRNA encoding a fusion polypeptide comprising a SteA scaffold polypeptide and one or more anti-Ras peptides, one or more SALL4-inhibitory peptides, one or more TOPK-inhibitory peptides or one or more p53-inhibitory peptides, as described herein. In various embodiments, the subject is provided with an effective amount of the mRNA.

Also disclosed are methods of treating or preventing cancer in a subject in need thereof, comprising providing the subject with an effective amount of an mRNA described herein, e.g., an mRNA encoding a fusion polypeptide comprising a SteA scaffold polypeptide and one or more BH3 domains, one or more anti-Ras peptides, one or more SALL4-inhibitory peptides, one or more TOPK-inhibitory peptides or one or more p53-inhibitory peptides, wherein the mRNA further comprises a regulatory element that enhances expression of the SteA-BH3 polypeptide, SteA-anti-Ras peptide, SteA-SALL4-inhibitory peptide, SteA-TOPK-inhibitory peptide or SteA-p53-inhibitory peptide constuct in cancer cells as compared to normal cells. In particular embodiments, the regulatory element is a binding site for a microRNA that has greater expression in normal cells than cancer cells (e.g., a miR-122 binding site), wherein binding of the microRNA to the binding site inhibits expression of the SteA-binding polypeptide construct. In particular embodiments, the mRNA is present in a nanoparticle, e.g., a lipid nanoparticle. In particular embodiments, the mRNA or nanoparticle is present in a pharmaceutical composition. The nanoparticle or the isolated mRNA may be taken up and translated in the subject's cells to produce the fusion polypeptide comprising a SteA scaffold polypeptide and one or more binding polypeptides. In particular embodiments, expression of the fusion polypeptide is greater in cancer cells than normal cells, resulting in greater apoptosis of cancer cells than normal cells.

In particular embodiments, the subject is provided or administered both a first mRNA encoding a SteA scaffold polypeptide and one or more BH3 domains and a second mRNA encoding a Bcl-2-like polypeptide or variant or fragment thereof. In particular embodiments, the first mRNA comprises a regulatory element to enhance expression of the SteA scaffold polypeptide in cancer cells as compared to normal cells and/or the second mRNA comprises a regulatory element to enhance expression in normal cells as compared to cancer cells. In particular embodiments, the first mRNA comprises a miR-122 binding sequence. In particular embodiments, the second mRNA comprises a miR-21 binding sequence. In certain embodiments, the first mRNA and second mRNA are both present in the same nanoparticle or pharmaceutical composition. In certain embodiments, the first mRNA and the second mRNA are present in different nanoparticles or pharmaceutical compositions. In particular embodiments, the molar ratio of the first mRNA to the second mRNA is about 1:50, about 1:25, about 1:10, about 1:5, about 1:4, about 1:3, about 1:2, about 1:1, about 2:1, about 3:1, about 4:1, or about 5:1, about 10:1, about 25:1 or about 50:1. In particular embodiments, the molar ratio of the first mRNA to the second mRNA is greater than 1:1.

Thus, in some embodiments, also disclosed is a method of treating or preventing cancer in a subject in need thereof, comprising providing to the subject a first mRNA described herein, e.g., an mRNA encoding a fusion polypeptide comprising a SteA scaffold polypeptide and one or more BH3 domains, one or more anti-Ras peptides, one or more SALL4-inhibitory peptides, one or more TOPK-inhibitory peptides or one or more p53-inhibitory peptides, optionally wherein the mRNA comprises a regulatory element that decreases expression of the fusion polypeptide in normal cells as compared to cancer cells, and a second mRNA described herein, e.g., an mRNA encoding a prosurvival Bcl-2-like polypeptide or variant or fragment thereof, optionally wherein the second mRNA comprises a regulatory element that decreases expression of the Bcl-2-like polypeptide or variant or fragment thereof in cancer cells as compared to normal cells. In particular embodiments, the regulatory element present in the first mRNA is a binding site for a microRNA that has greater expression in normal cells than cancer cells (e.g., a miR-122 binding site). In particular embodiments, the regulatory element present in the second mRNA is a binding site for a microRNA that has greater expression in cancer cells than normal cells (e.g., a miR-21 binding site), wherein binding of the microRNA to the binding site inhibits expression of the Bcl-2-like polypeptide or variant thereof. In particular embodiments, the subject is provided with a pharmaceutical composition or nanoparticle comprising the first RNA and/or the second RNA. The mRNAs may be taken up and translated in the subject's cells to produce the fusion polypeptide and the Bcl-2-like polypeptide or variant or fragment thereof. Expression of the fusion polypeptide that includes one or more BH3 domains is greater in cancer cells than normal cells, resulting in greater apoptosis of cancer cells than normal cells. Expression of the Bcl-2-like polypeptide inhibits apoptosis induced by the fusion polypeptide. Since the Bcl-2-like polypeptide is expressed in higher amounts in normal cells, it inhibits a greater amount of apoptosis induced by the fusion polypeptide in normal cells as compared to cancer cells.

In certain embodiments, also disclosed are a method of treating or preventing cancer in a subject in need thereof, comprising providing to the subject a first mRNA described herein, e.g., an mRNA encoding a fusion polypeptide comprising a SteA scaffold polypeptide and one or more BH3 domains, one or more anti-Ras peptides, one or more SALL4-inhibitory peptides, one or more TOPK-inhibitory peptides or one or more p53-inhibitory peptides, in combination with a therapeutic agent, such as a chemotherapeutic drug or other anti-cancer agent. In certain embodiments, a second mRNA as described herein, e.g., an mRNA encoding a prosurvival Bcl-2-like polypeptide or variant or fragment thereof, is also included in the treatment regimen, together with the first mRNA and the therapeutic agent. Optionally, the second mRNA comprises a regulatory element that decreases expression of the Bcl-2-like polypeptide or variant or fragment thereof in cancer cells as compared to normal cells. Suitable therapeutic agents for use in combination therapy include small molecule chemotherapeutic agents, including protein tyrosine kinase inhibitors, as well as biological anti-cancer agents, such as anti-cancer antibodies. A preferred therapeutic agent for combination therapy is sorafenib. Other suitable therapeutic agents for combination therapy are described further below.

A pharmaceutical composition including one or more mRNAs of the invention may be administered to a subject by any suitable route. In some embodiments, compositions of the invention are administered by one or more of a variety of routes, including parenteral (e.g., subcutaneous, intracutaneous, intravenous, intraperitoneal, intramuscular, intraarticular, intraarterial, intrasynovial, intrasternal, intrathecal, intralesional, or intracranial injection, as well as any suitable infusion technique), oral, trans- or intra-dermal, interdermal, rectal, intravaginal, topical (e.g.. by powders, ointments, creams, gels, lotions, and/or drops), mucosal, nasal, buccal, enteral, vitreal, intratumoral, sublingual, intranasal; by intratracheal instillation, bronchial instillation, and/or inhalation; as an oral spray and/or powder, nasal spray, and/or aerosol, and/or through a portal vein catheter. In some embodiments, a composition may be administered intravenously, intramuscularly, intradermally, intra-arterially, intratumorally, subcutaneously, or by inhalation. However, the present disclosure encompasses the delivery of compositions of the invention by any appropriate route taking into consideration likely advances in the sciences of drug delivery. In general, the most appropriate route of administration will depend upon a variety of factors including the nature of the pharmaceutical composition including one or more mRNAs (e.g., its stability in various bodily environments such as the bloodstream and gastrointestinal tract), and the condition of the patient (e.g., whether the patient is able to tolerate particular routes of administration).

In certain embodiments, compositions of the invention may be administered at dosage levels sufficient to deliver from about 0.0001 mg/kg to about 10 mg/kg, from about 0.001 mg/kg to about 10 mg/kg, from about 0.005 mg/kg to about 10 mg/kg, from about 0.01 mg/kg to about 10 mg/kg, from about 0.1 mg/kg to about 10 mg/kg, from about 1 mg/kg to about 10 mg/kg, from about 2 mg/kg to about 10 mg/kg, from about 5 mg/kg to about 10 mg/kg, from about 0.0001 mg/kg to about 5 mg/kg, from about 0.001 mg/kg to about 5 mg/kg, from about 0.005 mg/kg to about 5 mg/kg, from about 0.01 mg/kg to about 5 mg/kg, from about 0.1 mg/kg to about 10 mg/kg, from about 1 mg/kg to about 5 mg/kg, from about 2 mg/kg to about 5 mg/kg, from about 0.0001 mg/kg to about 1 mg/kg, from about 0.001 mg/kg to about 1 mg/kg, from about 0.005 mg/kg to about 1 mg/kg, from about 0.01 mg/kg to about 1 mg/kg, or from about 0.1 mg/kg to about 1 mg/kg in a given dose, where a dose of 1 mg/kg provides 1 mg of mRNA or nanoparticle per 1 kg of subject body weight. In particular embodiments, a dose of about 0.005 mg/kg to about 5 mg/kg of mRNA or nanoparticle of the invention may be administrated.

A dose may be administered one or more times per day, in the same or a different amount, to obtain a desired level of mRNA expression and/or effect (e.g., a therapeutic effect). The desired dosage may be delivered, for example, three times a day, two times a day, once a day, every other day, every third day, every week, every two weeks, every three weeks, or every four weeks. In certain embodiments, the desired dosage may be delivered using multiple administrations (e.g., two, three, four, five, six, seven, eight, nine, ten, eleven, twelve, thirteen, fourteen, or more administrations). In some embodiments, a single dose may be administered, for example, prior to or after a surgical procedure or in the instance of an acute disease, disorder, or condition. The specific therapeutically effective, prophylactically effective, or otherwise appropriate dose level for any particular patient will depend upon a variety of factors including the severity and identify of a disorder being treated, if any; the one or more mRNAs employed; the specific composition employed; the age, body weight, general health, sex, and diet of the patient; the time of administration, route of administration, and rate of excretion of the specific pharmaceutical composition employed; the duration of the treatment; drugs used in combination or coincidental with the specific pharmaceutical composition employed; and like factors well known in the medical arts.

In some embodiments, a pharmaceutical composition of the invention may be administered in combination with another agent, for example, another therapeutic agent, a prophylactic agent, and/or a diagnostic agent. By "in combination with," it is not intended to imply that the agents must be administered at the same time and/or formulated for delivery together, although these methods of delivery are consistent with the present disclosure. For example, one or more compositions including one or more different mRNAs may be administered in combination. Compositions can be administered concurrently with, prior to, or subsequent to, one or more other desired therapeutics or medical procedures. In general, each agent will be administered at a dose and/or on a time schedule determined for that agent. In some embodiments, the present disclosure encompasses the delivery of compositions of the invention, or imaging, diagnostic, or prophylactic compositions thereof in combination with agents that improve their bioavailability, reduce and/or modify their metabolism, inhibit their excretion, and/or modify their distribution within the body.

Exemplary therapeutic agents that may be administered in combination with the compositions of the invention include, but are not limited to, cytotoxic, chemotherapeutic, and other therapeutic agents. Cytotoxic agents may include, for example, taxol, cytochalasin B, gramicidin D, ethidium bromide, emetine, mitomycin, etoposide, teniposide, vincristine, vinblastine, colchicine, doxorubicin, daunorubicin, dihydroxyanthracinedione, mitoxantrone, mithramycin, actinomycin D, 1-dehydrotestosterone, glucocorticoids, procaine, tetracaine, lidocaine, propranolol, puromycin, maytansinoids, rachelmycin, and analogs thereof. Radioactive ions may also be used as therapeutic agents and may include, for example, radioactive iodine, strontium, phosphorous, palladium, cesium, iridium, cobalt, yttrium, samarium, and praseodymium. Other therapeutic agents may include, for example, antimetabolites (e.g., methotrexate, 6-mercaptopurine, 6-thioguanine, cytarabine, and 5-fluorouracil, and decarbazine), alkylating agents (e.g., mechlorethamine, thiotepa, chlorambucil, rachelmycin, melphalan, carmustine, lomustine, cyclophosphamide, busulfan, dibromomannitol, streptozotocin, mitomycin C, and cis-dichlorodiamine platinum (II) (DDP), and cisplatin), anthracyclines (e.g., daunorubicin and doxorubicin), antibiotics (e.g., dactinomycin, bleomycin, mithramycin, and anthramycin), and anti-mitotic agents (e.g., vincristine, vinblastine, taxol, and maytansinoids).

The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, a composition useful for treating cancer may be administered concurrently with a chemotherapeutic agent), or they may achieve different effects (e.g., control of any adverse effects).

### Methods of Inducing Apoptosis

The disclorure provides methods of inducing apoptosis in a cell, e.g., a mammalian cell. In some embodiments, a method of inducing apoptosis in a cell involves contacting a cell with an mRNA described herein, e.g., an mRNA encoding a SQT-BH3 polypeptide. In certain embodiments, such a method involves contacting a cell with an isolated mRNA encoding a SteA scaffold polypeptide that includes one or more BH3 domains. In particular embodiments, the cell is contacted with a lipid nanoparticle composition including an mRNA encoding a SteA scaffold polypeptide that includes one or more BH3 domains. Upon contacting the cell with the lipid nanoparticle composition or the isolated mRNA, the mRNA may be taken up and translated in the cell to produce the SteA scaffold polypeptide that includes one or more BH3 domains.

The disclosure further provides methods of selectively inducing apoptosis in a cancer cell as compared to a normal cell. In some embodiments, a method of selectively inducing apoptosis in a cancer cell involves contacting a cell with an mRNA described herein, e.g., an mRNA encoding a fusion polypeptide comprising a SteA scaffold polypeptide and a BH3 domain, wherein the mRNA further comprises a regulatory element that reduces expression of the fusion polypeptide in normal cells as compared to cancer cells. In particular embodiments, the regulatory element is a binding site for a microRNA that has greater expression in normal cells than cancer cells (e.g., a miR-122 binding site), wherein binding of the microRNA to the binding site inhibits expression of the fusion polypeptide. In particular embodiments, the cell is contacted with a nanoparticle composition comprising an mRNA comprising a region encoding the fusion polypeptide and a microRNA binding site. Upon contacting the cell with the nanoparticle composition or the isolated mRNA, the mRNA may be taken up and translated in the cell to produce the fusion polypeptide. Expression of the fusion polypeptide is greater in cancer cells than normal cells, resulting in greater apoptosis of cancer cells than normal cells.

In some embodiments, a method of selectively inducing apoptosis in a cancer cell involves contacting a cell with a first mRNA described herein, e.g., an mRNA encoding a fusion polypeptide comprising a SteA scaffold polypeptide and one or more BH3 domains, optionally wherein the mRNA comprises a regulatory element that reduces expression of the SteA scaffold polypeptide in normal cells as compared to cancer cells, and a second mRNA described herein, e.g., an mRNA encoding a prosurvival Bcl-2-like polypeptide or variant or fragment thereof, optionally wherein the second mRNA comprises a regulatory element that reduces expression of the Bcl-2-like polypeptide or variant thereof in cancer cells as compared to normal cells. In particular embodiments, the regulatory element in the first mRNA is a binding site for a microRNA that has greater expression in normal cells than cancer cells (e.g., a miR-122 binding site), wherein binding of the microRNA to the binding site inhibits expression of the fusion polypeptide. In particular embodiments, the regulatory element in the second mRNA is a binding site for a microRNA that has greater expression in cancer cells than normal cells (e.g., a miR-21 binding site), wherein binding of the microRNA to the binding site inhibits expression of the Bcl-2-like polypeptide or variant thereof. In particular embodiments, the cell is contacted with a nanoparticle composition comprising the first RNA and the second RNA. Upon contacting the cell with the lipid nanoparticle composition or the isolated mRNAs, the mRNAs may be taken up and translated in the cell to produce the fusion polypeptide and the Bcl-2-like polypeptide or variant thereof. Expression of the fusion polypeptide is greater in cancer cells than normal cells, resulting in greater apoptosis of cancer cells than normal cells. Expression of the Bcl-2-like polypeptide inhibits apoptosis induced by the SteA scaffold polypeptide. Since the Bcl-2-like polypeptide is expressed in higher amounts in normal cells, it inhibits a greater amount of apoptosis induced by the SteA scaffold polypeptide in normal cells as compared to cancer cells. In particular embodiments, a normal cell is a non-cancerous cell of the same cell type, e.g., as the cancer cell.

In general, the step of contacting a mammalian cell with a composition (e.g., an isolated mRNA, nanoparticle, or pharmaceutical composition of the invention) may be performed *in vivo, ex vivo,* in culture, or *in vitro.* In exemplary embodiments of the disclosure, the step of contacting a mammalian cell with a composition (e.g., an isolated mRNA, nanoparticle, or pharmaceutical composition of the invention) is performed *in vivo* or *ex vivo.* The amount of the composition contacted with a cell, and/or the amount of mRNA therein, may depend on the type of cell or tissue being contacted, the means of administration, the physiochemical characteristics of the composition and the mRNA (e.g., size, charge, and chemical composition) therein, and other factors. In general, an effective amount of the composition will allow for efficient production of the encoded polypeptide in the cell. Metrics for efficiency may include polypeptide translation (indicated by polypeptide expression), level of mRNA degradation, and immune response indicators.

The step of contacting a composition including an mRNA, or an isolated mRNA, with a cell may involve or cause transfection. In some embodiments, a phospholipid included in a lipid nanoparticle may facilitate transfection and/or increase transfection efficiency, for example, by interacting and/or fusing with a cellular or intracellular membrane. Transfection may allow for the translation of the mRNA within the cell. Translation of a SteA scaffold polypeptide that includes one or more BH3 domains may induce apoptosis in the cell.

The ability of a composition of the invention (e.g., a lipid nanoparticle or isolated mRNA) to induce apoptosis may be readily determined, for example by comparing the ability of the composition to induce apoptosis as compared to known agents or manipulations that may induce apoptosis, including but not limited to: anti-Fas antibody or Fas ligand, staurosporin, interleukins, Apo3 ligand, and TRAIL. A wide variety of methods of determining whether an agent is capable of inducing apoptosis are known in the art, for example, caspase activation assays (e.g., caspase-3/7 activation assays), stains and dyes (e.g., CELLTOX™, MITOTRACKER® Red, propidium iodide, and YOYO3), cell viability assays, cell morphology, and detection of PARP-1 cleavage, for example, by Western blotting with an anti-PARP-1 antibody.

### Definitions

*Administering:* As used herein, "administering" refers to a method of delivering a composition to a subject or patient. A method of administration may be selected to target delivery (e.g., to specifically deliver) to a specific region or system of a body. For example, an administration may be parenteral (e.g., subcutaneous, intracutaneous, intravenous, intraperitoneal, intramuscular, intraarticular, intraarterial, intrasynovial, intrasternal, intrathecal, intralesional, or intracranial injection, as well as any suitable infusion technique), oral, trans- or intra-dermal, interdermal, rectal, intravaginal, topical (e.g.. by powders, ointments, creams, gels, lotions, and/or drops), mucosal, nasal, buccal, enteral, vitreal, intratumoral, sublingual, intranasal; by intratracheal instillation, bronchial instillation, and/or inhalation; as an oral spray and/or powder, nasal spray, and/or aerosol, and/or through a portal vein catheter.
*Apoptosis:* As used herein, "apoptosis" refers to a form of cell death in which a programmed sequence of events leads to the death of a cell. Hallmarks of apoptosis include morphological changes, cell shrinkage, caspase activation, nuclear and cytoplasmic condensation, and alterations in plasma membrane topology. Biochemically, apoptotic cells are characterized by increased intracellular calcium concentration, fragmentation of chromosomal DNA, and expression of novel cell surface components. In particular embodiments, a cell undergoing apoptosis may undergo mitochondrial outer membrane permeabilization (MOMP).
*Approximately, about:* As used herein, the terms "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).
*Bcl-2 homology 3 (BH3) domain:* As used herein, a Bcl-2 homology 3 (BH3) domain is a polypeptide or fragment thereof derived from or having homology to a conserved amino acid sequence from a Bcl-2 family protein. In some embodiments, a BH3 domain is capable of inhibiting an anti-apoptotic or prosurvival Bcl-2 family member (including, for example, BCL-2, BCL-X_{L}, BCL-w, MCL-1 and/or BCL2A1 (also referred to as A1)). Bcl-2 family members typically include at least one of four Bcl-2 homology domains (BH1 domain, BH2 domain, BH3 domain, and BH4 domain), which are highly conserved (see, for example, Ankevar et al. Front.Oncol. 1: 34, 2011). There are two main categories of Bcl-2 family proteins: pro-apoptotic Bcl-2 family members and anti-apoptotic Bcl-2 family members. Pro-apoptotic BCL family proteins include "effector" BCL-2 proteins (e.g., BAK and BAX) and "BH3-only" proteins (e.g., BID, BIM, BAD, BAK, BMF, bNIP3, HRK, Noxa, and PUMA). In particular embodiments, the BH3 domain is derived from a BH3-only protein.
*Bcl-2-like polypeptide:* As used herein, a "Bcl-2-like polypeptide" is a member of the Bcl-2 family of proteins that binds a BH3 peptide and, in certain instances, promotes cell survival. Members of the Bcl-2 protein family that promote cell survival include: Bcl-2, Bcl-X_{L}, Bcl-w, Mcl-1 and A-1. Bcl-2-like polypeptides also include variants of these prosurvival Bcl-2 family members that retain the ability to bind a BH3 domain and/or promote cell survival, which may be termed "functional variants." "Variants" are polypeptides that include one or more amino acid modifications, e.g., deletions, substitutions or insertions, as compared to a wild-type Bcl-2-like polypeptide. Variants include both fragments of Bcl-2-like polypeptides.
*Cancer:* As used herein, "cancer" is a condition involving abnormal and/or unregulated cell growth. The term cancer encompasses benign and malignant cancers. Exemplary non-limiting cancers include adrenal cortical cancer, advanced cancer, anal cancer, aplastic anemia, bileduct cancer, bladder cancer, bone cancer, bone metastasis, brain tumors, brain cancer, breast cancer, childhood cancer, cancer of unknown primary origin, Castleman disease, cervical cancer, colorectal cancer, endometrial cancer, esophagus cancer, Ewing family of tumors, eye cancer, gallbladder cancer, gastrointestinal carcinoid tumors, gastrointestinal stromal tumors, gestational trophoblastic disease, Hodgkin disease, Kaposi sarcoma, renal cell carcinoma, laryngeal and hypopharyngeal cancer, acute lymphocytic leukemia, acute myeloid leukemia, chronic lymphocytic leukemia, chronic myeloid leukemia, chronic myelomonocytic leukemia, myelodysplastic syndrome (including refractory anemias and refractory cytopenias), myeloproliferative neoplasms or diseases (including polycythemia vera, essential thrombocytosis and primary myelofibrosis), liver cancer (e.g., hepatocellular carcinoma), non-small cell lung cancer, small cell lung cancer, lung carcinoid tumor, lymphoma of the skin, malignant mesothelioma, multiple myeloma, myelodysplasia syndrome, nasal cavity and paranasal sinus cancer, nasopharyngeal cancer, neuroblastoma, non-Hodgkin lymphoma, oral cavity and oropharyngeal cancer, osteosarcoma, ovarian cancer, pancreatic cancer, penile cancer, pituitary tumors, prostate cancer, retinoblastoma, rhabdomyosarcoma, salivary gland cancer, sarcoma in adult soft tissue, basal and squamous cell skin cancer, melanoma, small intestine cancer, stomach cancer, testicular cancer, throat cancer, thymus cancer, thyroid cancer, uterine sarcoma, vaginal cancer, vulvar cancer, Waldenstrom macroglobulinemia, Wilms tumor and secondary cancers caused by cancer treatment. In particular embodiments, the cancer is liver cancer (e.g., hepatocellular carcinoma) or colorectal cancer. In other embodiments, the cancer is a blood-based cancer or a hematopoietic cancer.
*Cleavable Linker:* As used herein, the term "cleavable linker" refers to a linker, typically a peptide linker (e.g., about 5-30 amino acids in length, typically about 10-20 amino acids in length) that can be incorporated into multicistronic mRNA constructs such that equimolar levels of multiple genes can be produced from the same mRNA. Non-limiting examples of cleavable linkers include the 2A family of peptides, including F2A, P2A, T2A and E2A, first discovered in picornaviruses, that when incorporated into an mRNA construct (e.g., between two polypeptide domains) function by making the ribosome skip the synthesis of a peptide bond at C-terminus of the 2A element, thereby leading to separation between the end of the 2A sequence and the next peptide downstream.
*Conjugated:* As used herein, the term "conjugated," when used with respect to two or more moieties, means that the moieties are physically associated or connected with one another, either directly or via one or more additional moieties that serves as a linking agent, to form a structure that is sufficiently stable so that the moieties remain physically associated under the conditions in which the structure is used, e.g., physiological conditions. In some embodiments, two or more moieties may be conjugated by direct covalent chemical bonding. In other embodiments, two or more moieties may be conjugated by ionic bonding or hydrogen bonding.
*Contacting:* As used herein, the term "contacting" means establishing a physical connection between two or more entities. For example, contacting a cell with an mRNA or a lipid nanoparticle composition means that the cell and mRNA or lipid nanoparticle are made to share a physical connection. Methods of contacting cells with external entities both *in vivo, in vitro,* and *ex vivo* are well known in the biological arts. In exemplary embodiments of the disclosure, the step of contacting a mammalian cell with a composition (e.g., an isolated mRNA, nanoparticle, or pharmaceutical composition of the invention) is performed *in vivo.* For example, contacting a lipid nanoparticle composition and a cell (for example, a mammalian cell) which may be disposed within an organism (e.g., a mammal) may be performed by any suitable administration route (e.g., parenteral administration to the organism, including intravenous, intramuscular, intradermal, and subcutaneous administration). For a cell present *in vitro,* a composition (e.g., a lipid nanoparticle or an isolated mRNA) and a cell may be contacted, for example, by adding the composition to the culture medium of the cell and may involve or result in transfection. Moreover, more than one cell may be contacted by a nanoparticle composition.
*Encapsulate:* As used herein, the term "encapsulate" means to enclose, surround, or encase. In some embodiments, a compound, polynucleotide (e.g., an mRNA), or other composition may be fully encapsulated, partially encapsulated, or substantially encapsulated. For example, in some embodiments, an mRNA of the invention may be encapsulated in a lipid nanoparticle, e.g., a liposome.
*Effective amount:* As used herein, the term "effective amount" of an agent is that amount sufficient to effect beneficial or desired results, for example, clinical results, and, as such, an "effective amount" depends upon the context in which it is being applied. For example, in the context of administering an agent that treats cancer, an effective amount of an agent is, for example, an amount sufficient to achieve treatment, as defined herein, of cancer, as compared to the response obtained without administration of the agent. In some embodiments, a therapeutically effective amount is an amount of an agent to be delivered (e.g., nucleic acid, drug, therapeutic agent, diagnostic agentor prophylactic agent) that is sufficient, when administered to a subject suffering from or susceptible to an infection, disease, disorder, and/or condition, to treat, improve symptoms of, diagnose, prevent, and/or delay the onset of the infection, disease, disorder, and/or condition.
*Expression:* As used herein, "expression" of a nucleic acid sequence refers to one or more of the following events: (1) production of an RNA template from a DNA sequence (e.g., by transcription); (2) processing of an RNA transcript (e.g., by splicing, editing, 5' cap formation, and/or 3' end processing); (3) translation of an RNA into a polypeptide or protein; and (4) post-translational modification of a polypeptide or protein.
*Fragment:* A "fragment," as used herein, refers to a portion. For example, fragments of proteins may include polypeptides obtained by digesting full-length protein isolated from cultured cells or obtained through recombinant DNA techniques.
*Heterologous:* As used herein, "heterologous" indicates that a sequence (e.g., an amino acid sequence or the polynucleotide that encodes an amino acid sequence) is not normally present in a given polypeptide or polynucleotide. For example, an amino acid sequence that corresponds to a domain or motif of one protein may be heterologous to a second protein.
*Hydrophobic amino acid:* As used herein, a "hydrophobic amino acid" is an amino acid having an uncharged, nonpolar side chain. Examples of naturally occurring hydrophobic amino acids are alanine (Ala), valine (Val), leucine (Leu), isoleucine (Ile), proline (Pro), phenylalanine (Phe), methionine (Met), and tryptophan (Trp).
*Identity:* As used herein, the term "identity" refers to the overall relatedness between polymeric molecules, e.g., between polynucleotide molecules (e.g., DNA molecules and/or RNA molecules) and/or between polypeptide molecules. Calculation of the percent identity of two polynucleotide sequences, for example, can be performed by aligning the two sequences for optimal comparison purposes (e.g., gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap which needs to be introduced for optimal alignment of the two sequences. The comparison of sequences and determination of percent identity between two sequences can be accomplished using a mathematical algorithm. For example, the percent identity between two nucleotide sequences can be determined using methods such as those described in Computational Molecular Biology, Lesk, A. M., ed., Oxford University Press, New York, 1988; Biocomputing: Informatics and Genome Projects, Smith, D. W., ed., Academic Press, New York, 1993; Sequence Analysis in Molecular Biology, von Heinje, G., Academic Press, 1987; Computer Analysis of Sequence Data, Part I, Griffin, A. M., and Griffin, H. G., eds., Humana Press, New Jersey, 1994; and Sequence Analysis Primer, Gribskov, M. and Devereux, J., eds., M Stockton Press, New York, 1991. For example, the percent identity between two nucleotide sequences can be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix. Methods commonly employed to determine percent identity between sequences include, but are not limited to those disclosed in Carillo, H., and Lipman, D., SIAM J Applied Math., 48:1073 (1988). Techniques for determining identity are codified in publicly available computer programs. Exemplary computer software to determine homology between two sequences include, but are not limited to, GCG program package, Devereux et al., Nucleic Acids Research, 12(1): 387,1984, BLASTP, BLASTN, and FASTA, Altschul, S. F. et al., J. Molec. Biol., 215, 403, 1990.
*Inducing Apoptosis:* As used herein, "inducing apoptosis" is meant to indicate a process or method of triggering changes in a cell that, once initiated, will lead to apoptosis. The ability of a substance to induce apoptosis may be readily determined, for example by comparing the ability of a substance to induce apoptosis as compared to known agents or manipulations that may induce apoptosis, including but not limited to: anti-Fas antibody or Fas ligand, staurosporin, interleukins, Apo3 ligand, and TRAIL. A variety of methods of determining whether an agent can induce apoptosis are known in the art, for example, caspase activation assays (e.g., caspase-3/7 activation assays), stains and dyes (e.g., CELLTOX™, MITOTRACKER® Red, propidium iodide, and YOYO3), cell viability assays, cell morphology, and PARP-1 cleavage.
*Insertion:* As used herein, an "insertion" or an "addition" refers to a change in an amino acid or nucleotide sequence resulting in the addition of one or more amino acid residues or nucleotides, respectively, to a molecule as compared to a reference sequence, for example, the sequence found in a naturally-occurring molecule. For example, an amino acid sequence of a heterologous polypeptide (e.g., a BH3 domain) may be inserted into a scaffold polypeptide (e.g. a SteA scaffold polypeptide) at a site that is amenable to insertion. In some embodiments, an insertion may be a replacement, for example, if an amino acid sequence that forms a loop of a scaffold polypeptide (e.g., loop 1 or loop 2 of SteA or a SteA derivative) is replaced by an amino acid sequence of a heterologous polypeptide.
*Insertion Site:* As used herein, an "insertion site" is a position or region of a scaffold polypeptide that is amenable to insertion of an amino acid sequence of a heterologous polypeptide. It is to be understood that an insertion site also may refer to the position or region of the polynucleotide that encodes the polypeptide (e.g., a codon of a polynucleotide that codes for a given amino acid in the scaffold polypeptide). In some embodiments, insertion of an amino acid sequence of a heterologous polypeptide into a scaffold polypeptide has little to no effect on the stability (e.g., conformational stability), expression level, or overall secondary structure of the scaffold polypeptide.
*Isolated:* As used herein, the term "isolated" refers to a substance or entity that has been separated from at least some of the components with which it was associated (whether in nature or in an experimental setting). Isolated substances may have varying levels of purity in reference to the substances from which they have been associated. Isolated substances and/or entities may be separated from at least about 10%, about 20%, about 30%, about 40%, about 50%, about 60%, about 70%, about 80%, about 90%, or more of the other components with which they were initially associated. In some embodiments, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components.
*Liposome:* As used herein, by "liposome" is meant a structure including a lipid-containing membrane enclosing an aqueous interior. Liposomes may have one or more lipid membranes. Liposomes include single-layered liposomes (also known in the art as unilamellar liposomes) and multi-layered liposomes (also known in the art as multilamellar liposomes).
*Loop 1 insertion site:* As used herein, a "loop 1 insertion site" is an insertion site of a SteA scaffold polypeptide in or near loop 1. In some embodiments, a loop 1 insertion site includes one or more of codons 46 to 54 inclusive of a SteA scaffold polypeptide that encode amino acids within or adjacent to loop 1 of a SteA scaffold polypeptide.
*Loop 2 insertion site:* As used herein, a "loop 2 insertion site" is an insertion site of a SteA scaffold polypeptide in or near loop 2. In some embodiments, a loop 2 insertion site includes one or more of codons 67 to 84 inclusive of a SteA scaffold polypeptide that encode amino acids within or adjacent to loop 2 of a SteA scaffold polypeptide
*Metastasis:* As used herein, the term "metastasis" means the process by which cancer spreads from the place at which it first arose as a primary tumor to distant locations in the body. A secondary tumor that arose as a result of this process may be referred to as "a metastasis."
*mRNA:* As used herein, an "mRNA" refers to a messenger ribonucleic acid. An mRNA may be naturally or non-naturally occurring. For example, an mRNA may include modified and/or non-naturally occurring components such as one or more nucleobases, nucleosides, nucleotides, or linkers. An mRNA may include a cap structure, a chain terminating nucleoside, a stem loop, a polyA sequence, and/or a polyadenylation signal. An mRNA may have a nucleotide sequence encoding a polypeptide. Translation of an mRNA, for example, *in vivo* translation of an mRNA inside a mammalian cell, may produce a polypeptide. Traditionally, the basic components of an mRNA molecule include at least a coding region, a 5'-untranslated region (5'-UTR), a 3'UTR, a 5' cap and a polyA sequence.
*microRNA (miRNA):* As used herein, a "microRNA (miRNA)" is a small noncoding RNA molecule which may function in post-transcriptional regulation of gene expression (e.g., by RNA silencing, such as by cleavage of the mRNA, destabilization of the mRNA by shortening its polyA tail, and/or by interfering with the efficiency of translation of the mRNA into a polypeptide by a ribosome). A mature miRNA is typically about 22 nucleotides long.
*microRNA-122 (miR-122):* As used herein, "microRNA-122 (miR-122)" refers to any native miR-122 from any vertebrate source, including, for example, humans, unless otherwise indicated. miR-122 is typically highly expressed in the liver, where it may regulate fatty-acid metabolism. miR-122 levels are reduced in liver cancer, for example, hepatocellular carcinoma. miR-122 is one of the most highly-expressed miRNAs in the liver, where it regulates targets including but not limited to CAT-1, CD320, AldoA, Hjv, Hfe, ADAM10, IGFR1, CCNG1, and ADAM17. Mature human miR-122 may have a sequence of AACGCCAUUAUCACACUAAAUA (SEQ ID NO: 31, corresponding to hsa-miR-122-3p) or UGGAGUGUGACAAUGGUGUUUG (SEQ ID NO: 32, corresponding to hsa-miR-122-5p).
*microRNA-21 (miR-21):* As used herein, "microRNA-21 (miR-21)" refers to any native miR-21 from any vertebrate source, including, for example, humans, unless otherwise indicated. miR-21 levels are increased in liver cancer, for example, hepatocellular carcinoma, as compared to normal liver. Mature human miR-21 may have a sequence of UAGCUUAUCAGACUGAUGUUGA (SEQ ID NO: 33, corresponding to has-miR-21-5p) or 5' - CAACACCAGUCGAUGGGCUGU - 3' (SEQ ID NO: 34, corresponding to has-miR-21-3p). An additional miR-21 sequence is shown in SEQ ID NO: 106.
*microRNA-142 (miR-142):* As used herein, "microRNA-142 (miR-142)" refers to any native miR-142 from any vertebrate source, including, for example, humans, unless otherwise indicated. miR-142 is typically highly expressed in myeloid cells. Mature human miR-142 may have a sequence of UGUAGUGUUUCCUACUUUAUGGA (SEQ ID NO: 113, corresponding to hsa-miR-142-3p) or CAUAAAGUAGAAAGCACUACU (SEQ ID NO: 114, corresponding to hsa-miR-142-5p).
*microRNA (miRNA) binding site:* As used herein, a "microRNA (miRNA) binding site" refers to a miRNA target site or a miRNA recognition site, or any nucleotide sequence to which a miRNA binds or associates. In some embodiments, a miRNA binding site represents a nucleotide location or region of a polynucleotide (e.g., an mRNA) to which at least the "seed" region of a miRNA binds. It should be understood that "binding" may follow traditional Watson-Crick hybridization rules or may reflect any stable association of the miRNA with the target sequence at or adjacent to the microRNA site.
*miRNA seed:* As used herein, a "seed" region of a miRNA refers to a sequence in the region of positions 2-8 of a mature miRNA, which typically has perfect Watson-Crick complementarity to the miRNA binding site. A miRNA seed may include positions 2-8 or 2-7 of a mature miRNA. In some embodiments, a miRNA seed may comprise 7 nucleotides (e.g., nucleotides 2-8 of a mature miRNA), wherein the seed-complementary site in the corresponding miRNA binding site is flanked by an adenine (A) opposed to miRNA position 1. In some embodiments, a miRNA seed may comprise 6 nucleotides (e.g., nucleotides 2-7 of a mature miRNA), wherein the seed-complementary site in the corresponding miRNA binding site is flanked by an adenine (A) opposed to miRNA position 1. When referring to a miRNA binding site, an miRNA seed sequence is to be understood as having complementarity (e.g., partial, substantial, or complete complementarity) with the seed sequence of the miRNA that binds to the miRNA binding site.
*Modified:* As used herein "modified" refers to a changed state or structure of a molecule of the invention. Molecules may be modified in many ways including chemically, structurally, and functionally. In one embodiment, the mRNA molecules of the present invention are modified by the introduction of non-natural nucleosides and/or nucleotides, e.g., as it relates to the natural ribonucleotides A, U, G, and C. Noncanonical nucleotides such as the cap structures are not considered "modified" although they differ from the chemical structure of the A, C, G, U ribonucleotides.
*Nanoparticle:* As used herein, "nanoparticle" refers to a particle having any one structural feature on a scale of less than about 1000 nm that exhibits novel properties as compared to a bulk sample of the same material. Routinely, nanoparticles have any one structural feature on a scale of less than about 500 nm, less than about 200 nm, or about 100 nm. Also routinely, nanoparticles have any one structural feature on a scale of from about 50 nm to about 500 nm, from about 50 nm to about 200 nm or from about 70 nm to about 120 nm. In exemplary embodiments, a nanoparticle is a particle having one or more dimensions of the order of about 1 - 1000 nm. In other exemplary embodiments, a nanoparticle is a particle having one or more dimensions of the order of about 10- 500 nm. In other exemplary embodiments, a nanoparticle is a particle having one or more dimensions of the order of about 50- 200 nm. A spherical nanoparticle would have a diameter, for example, of between about 50-100 or 70-120 nanometers. A nanoparticle most often behaves as a unit in terms of its transport and properties. It is noted that novel properties that differentiate nanoparticles from the corresponding bulk material typically develop at a size scale of under 1000 nm, or at a size of about 100 nm, but nanoparticles can be of a larger size, for example, for particles that are oblong, tubular, and the like. Although the size of most molecules would fit into the above outline, individual molecules are usually not referred to as nanoparticles.
*N-terminal insertion site:* As used herein, an "N-terminal insertion site" is an insertion site of a SteA scaffold polypeptide in the N-terminus. In some embodiments, an N-terminal insertion site includes one or more of the first 8 codons encoding the first 8 amino acids of a SteA scaffold polypeptide. In particular embodiments, an N-terminal insertion site is position 4 of a SteA scaffold polypeptide.
*Nucleic acid:* As used herein, the term "nucleic acid" is used in its broadest sense and encompasses any compound and/or substance that includes a polymer of nucleotides. These polymers are often referred to as polynucleotides. Exemplary nucleic acids or polynucleotides of the invention include, but are not limited to, ribonucleic acids (RNAs), deoxyribonucleic acids (DNAs), DNA-RNA hybrids, RNAi-inducing agents, RNAi agents, siRNAs, shRNAs, miRNAs, antisense RNAs, ribozymes, catalytic DNA, RNAs that induce triple helix formation, threose nucleic acids (TNAs), glycol nucleic acids (GNAs), peptide nucleic acids (PNAs), locked nucleic acids (LNAs, including LNA having a β-D-ribo configuration, α-LNA having an α-L-ribo configuration (a diastereomer of LNA), 2'-amino-LNA having a 2'-amino functionalization, and 2'-amino-α-LNA having a 2'-amino functionalization) or hybrids thereof.
*Patient:* As used herein, "patient" refers to a subject who may seek or be in need of treatment, requires treatment, is receiving treatment, will receive treatment, or a subject who is under care by a trained professional for a particular disease or condition. In particular embodiments, a patient is a human patient. In some embodiments, a patient is a patient suffering from cancer (e.g., liver cancer or colorectal cancer).
*Pharmaceutically acceptable:* The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.
*Pharmaceutically acceptable excipient:* The phrase "pharmaceutically acceptable excipient," as used herein, refers any ingredient other than the compounds described herein (for example, a vehicle capable of suspending or dissolving the active compound) and having the properties of being substantially nontoxic and non-inflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspensing or dispersing agents, sweeteners, and waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.
*Pharmaceutically acceptable salts:* As used herein, "pharmaceutically acceptable salts" refers to derivatives of the disclosed compounds wherein the parent compound is modified by converting an existing acid or base moiety to its salt form (e.g., by reacting the free base group with a suitable organic acid). Examples of pharmaceutically acceptable salts include, but are not limited to, mineral or organic acid salts of basic residues such as amines; alkali or organic salts of acidic residues such as carboxylic acids; and the like. Representative acid addition salts include acetate, acetic acid, adipate, alginate, ascorbate, aspartate, benzenesulfonate, benzene sulfonic acid, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. The pharmaceutically acceptable salts of the present disclosure include the conventional non-toxic salts of the parent compound formed, for example, from non-toxic inorganic or organic acids. The pharmaceutically acceptable salts of the present disclosure can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, Pharmaceutical Salts: Properties, Selection, and Use, P.H. Stahl and C.G. Wermuth (eds.), Wiley-VCH, 2008, and Berge et al., Journal of Pharmaceutical Science, 66, 1-19 (1977).
*Polypeptide:* As used herein, the term "polypeptide" or "polypeptide of interest" refers to a polymer of amino acid residues typically joined by peptide bonds that can be produced naturally (e.g., isolated or purified) or synthetically.
*Preventing:* As used herein, the term "preventing" refers to partially or completely inhibiting the onset of one or more symptoms or features of a particular infection, disease, disorder, and/or condition.
*Stefin A (SteA) scaffold polypeptide:* By "Stefin A (SteA) scaffold polypeptide" is meant a SteA polypeptide or any derivative thereof able to accept an insertion of a heterologous polypeptide amino acid sequence. In some embodiments, a SteA scaffold polypeptide includes an N-terminal insertion site, a loop 1 insertion site, and/or a loop 2 insertion site. Exemplary SteA scaffold polypeptides include wild-type Stefin A (for example, human Stefin A, also known as cystatin A), STM (Stefin A Triple Mutant, as described, e.g., in U.S. Patent No. 8,063,019 and in Woodman et al., J. Mol. Biol. 352: 1118-1133, 2005), SQM (Stefin A Quadruple Mutant, as described, e.g., in U.S. Patent No. 8,853,131, and SQT (Stefin A Quadruple Mutant, Tracy, as described, e.g., in U.S. Patent No. 8,853,131 and Stadler et al., Protein Eng. Des. Sel. 24(9): 751-763, 2011). Additional exemplary SteA scaffold proteins are described in U.S. Patent No. 8,853,131 including SDM, SUC, SUM, SUN, SDM-, SDM--, SQM-, SQM--, SUC-, SUC--, SUM-, SUM--, SUN-, SUN--, and SQL.
*Subject:* As used herein, the term "subject" refers to any organism to which a composition in accordance with the invention may be administered, e.g., for experimental, diagnostic, prophylactic, and/or therapeutic purposes. Typical subjects include animals (e.g., mammals such as mice, rats, rabbits, non-human primates, and humans) and/or plants. In some embodiments, a subject may be a patient.
*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.
*Suffering from:* An individual who is "suffering from" a disease, disorder, and/or condition has been diagnosed with or displays one or more symptoms of a disease, disorder, and/or condition.
*Targeting moiety:* As used herein, a "targeting moiety" is a compound or agent that may target a nanoparticle to a particular cell, tissue, and/or organ type.
*Therapeutic Agent:* The term "therapeutic agent" refers to any agent that, when administered to a subject, has a therapeutic, diagnostic, and/or prophylactic effect and/or elicits a desired biological and/or pharmacological effect.
*Transfection:* As used herein, the term "transfection" refers to methods to introduce a species (e.g., a polynucleotide, such as a mRNA) into a cell.
*Treating:* As used herein, the term "treating" refers to partially or completely alleviating, ameliorating, improving, relieving, delaying onset of, inhibiting progression of, reducing severity of, and/or reducing incidence of one or more symptoms or features of a particular infection, disease, disorder, and/or condition. For example, "treating" cancer may refer to inhibiting survival, growth, and/or spread of a tumor. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.
*Tumor:* As used herein, a "tumor" is an abnormal growth of tissue, whether benign or malignant.
*Unmodified:* As used herein, "unmodified" refers to any substance, compound or molecule prior to being changed in any way. Unmodified may, but does not always, refer to the wild type or native form of a biomolecule. Molecules may undergo a series of modifications whereby each modified molecule may serve as the "unmodified" starting molecule for a subsequent modification.

### Equivalents and Scope

Those skilled in the art will recognize, or be able to ascertain using no more than routine experimentation, many equivalents to the specific embodiments. The scope of the present invention is set forth in the appended claims.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. Such disclosure includes embodiments in which exactly one member of the group is present in, employed in, or otherwise relevant to a given product or process. Such disclosure includes embodiments in which more than one, or all of the group members are present in, employed in, or otherwise relevant to a given product or process.

It is also noted that the term "comprising" is intended to be open and permits but does not require the inclusion of additional elements or steps. When the term "comprising" is used herein, the term "consisting of' is thus also encompassed and disclosed.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

In case of conflicting statements of a cited source and the instant application, the statement in the instant application shall control.

### Examples

### EXAMPLE 1

### RAPID INDUCTION OF APOPTOSIS AND CELL KILLING BY TRANSFECTION OF MRNA ENCODING SQT-PUMA-BH3 OR SQT-BIM-BH3

Selected BH3 domains from the "BH3 only" family of pro-apoptotic BCL-2 family proteins were inserted into the N-terminus of the Stefin A Quadruple Mutant-Tracy (SQT) variant of Stefin A, resulting in SQT-PUMA-BH3, SQT-Bim-BH3, SQT-Bad-BH3, and SQT-Noxa-BH3 constructs. These constructs also contained a FLAG epitope tag to facilitate detection. The constructs had the structures as shown below in Table 10:

**Table 10:**

| **Name** | **5'UTR SEQ ID** | **mRNA SEQ ID** | **3'UTR SEQ ID** |
|---|---|---|---|
| SQT-PUMA-BH3 | 72 | 49 | 73 |
| SQT-Bim-BH3 | 72 | 50 | 73 |
| SQT-BAD-BH3 | 72 | 51 | 73 |
| SQT-Noxa-BH3 | 72 | 52 | 73 |

Additionally, all constructs contained a Cap 1 5' Cap (7mG(5')ppp(5')NlmpNp), a poly A tail of 100 nucleotides and were fully modified with 1-methyl-pseudouridine (m1ψ). The mRNA sequences used in the constructs shown in Table 10 include an epitope tag coding sequence (FLAG) incorporated into the sequence. Sequences corresponding to SEQ ID NOs: 49-52 but without the FLAG tag are shown in SEQ ID NOs: 74-77.

To determine whether SQT-BH3 constructs could induce apoptosis, HeLa cells were co-transfected with the SQT-BH3 constructs and a plasmid expressing green fluorescent protein (GFP) as a marker of transfected cells. Transfection of mRNAs encoding SQT-PUMA-BH3 or SQT-Bim-BH3 induced cleavage of poly(ADP-ribose) polymerase 1 (PARP-1), a well-established target of caspases including caspase-3, within 4 hours after transfection. Transfection of mRNAs encoding SQT-Bad-BH3 or SQT-Noxa-BH3 also induced PARP-1 cleavage, although to a lesser extent than SQT-PUMA-BH3 or SQT-Bim-BH3 at this time point. The cleavage of PARP-1 was detected by the appearance of a faster-migrating band and concomitant loss of the full-length band by SDS-PAGE and Western blotting. Western blotting with anti-actin antibodies served as a loading control. Expression of SQT-PUMA-BH3 and SQT-Bim-BH3 was difficult to detect by anti-FLAG Western blotting, suggesting that expression of these constructs was causing cell death in the transfected cells. Consistently, it was also difficult to detect GFP by anti-GFP Western blotting, further indicating that cells transfected with SQT-PUMA-BH3 or SQT-BIM-BH3 underwent cell death.

SQT-PUMA-BH3 and SQT-Bim-BH3 that were introduced into cells by mRNA transfection were able interact with the anti-apoptotic BCL-2 family members BCL-XL, and, to a lesser extent, BCL-2, within two hours after transfection, as determined by coimmunoprecipitation, indicating that the BH3 domains were displayed by the SQT scaffold in a manner that allowed interaction with endogenous binding partners. SQT-Bad-BH3 was also able to co-immunoprecipitate BCL-XL and BCL-2, although to a lesser extent than SQT-PUMA-BH3 and SQT-Bim-BH3. In similar experiments performed in lysates of HeLa cells transfected with mRNAs encoding SQT-BH3 constructs, SQT-PUMA-BH3, SQT-Bim-BH3, and SQT-Noxa-BH3 were able to co-immunoprecipitate the anti-apoptotic BCL-2 family member MCL-1. These results show that SQT-displayed BH3 domains retain the ability of the full-length BH3-only protein to specifically interact to a set of anti-apoptotic BCL-2 family members, because unlike Bim and PUMA, which can typically interact with all of the known anti-apoptotic BCL-2 family members, Noxa is thought to interact solely with MCL-1 and A2 (see, e.g., Chen et al. Mol. Cell 17(3): 393-403, 2005).

Consistent with the appearance of cleaved PARP-1, HeLa cells that were co-transfected with mRNA encoding SQT-PUMA-BH3 or SQT-Bim-BH3 and a plasmid expressing GFP underwent cell death by 4 hours following transfection, as indicated by the decreased growth rate of GFP-positive cells in a long-term live cell imaging experiment. In contrast, cells transfected with SQT-Bad-BH3 or SQT-Noxa did not show cell-killing activity. Transfection of a human melanoma cells (A375) and human hepatocellular carcinoma (Hep G2) cells with mRNAs encoding SQT-PUMA-BH3 or SQT-Bim-BH3 also caused rapid cell death almost immediately following transfection.

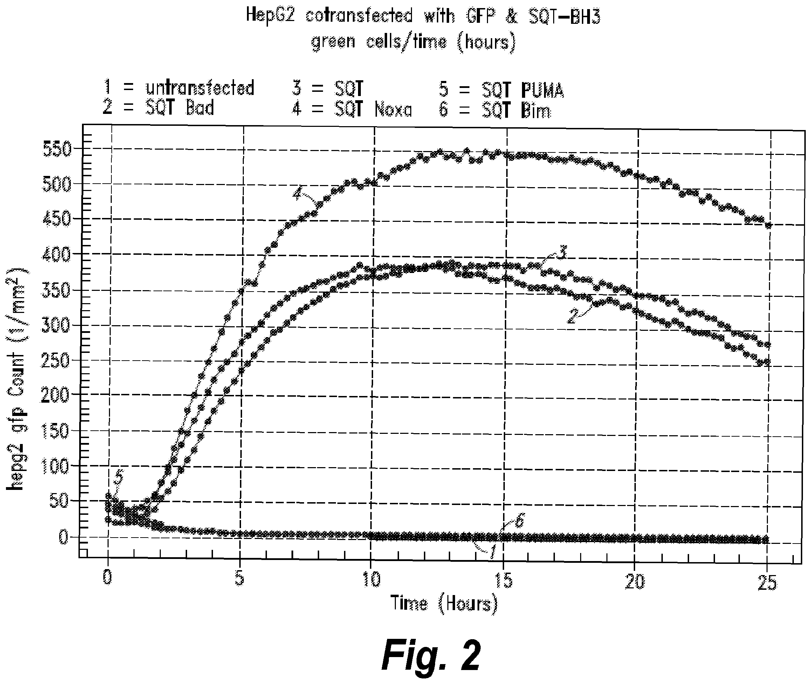

A representative graph of the apoptotic ability of the SQT-BH3 constructs is shown in Figure 2, showing the cell killing capacity of the SQT-BH3 constructs in Hep G2 cells. Nearly all of the cells transfected with mRNA encoding SQT-PUMA-BH3 or SQT-Bim-BH3 had a rounded-up cell morphology that is characteristic of apoptotic cells, compared to the normal spread morphology of cells that were transfected with mRNA encoding the control SQT protein.

To validate that cells transfected with mRNA encoding SQT-PUMA-BH3 and SQT-Bim-BH3 were undergoing cell death via apoptosis, the status of the mitochondria was assessed using MITOTRACKER® Red staining (CM-H2XRos). HeLa cells transfected with mRNA encoding SQT-PUMA-BH3 or SQT-Bim-BH3 and a plasmid expressing GFP displayed mitochondrial outer membrane permeabilization (MOMP), which is considered an irreversible step that commits the cell to apoptosis, within 4 hours following transfection. Staurosporine treatment served as a positive control. As a control, transfection of mRNA encoding SQT does not cause apoptosis in MITOTRACKER®-stained HeLa cells, as assessed by the lack of cell death and MOMP. In contrast, transfection of mRNA encoding SQT-PUMA-BH3 or SQT-Bim-BH3 caused MOMP and cell death in HeLa cells within 2 hours following transfection, as assessed by MITOTRACKER® Red staining, thereby validating that the cells were undergoing cell death via apoptosis.

### EXAMPLE 2

### LIPID NANOPARTICLE-FORMULATED SQT-PUMA-BH3 AND SQT-BIM-BH3 KILL HEPATOCELLULAR CARCINOMA CELLS AND PRIMARY HEPATOCYTES

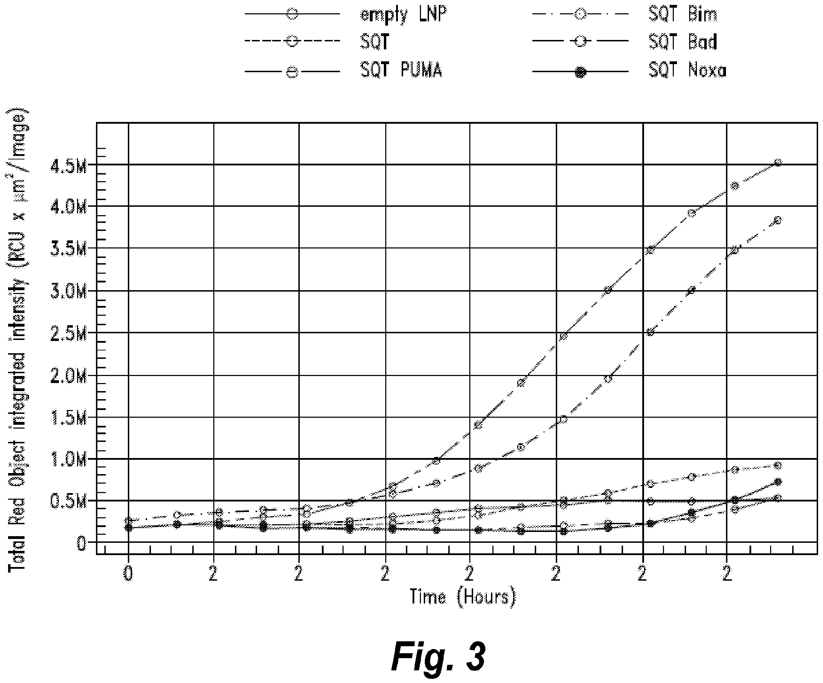

The efficacy of mRNAs encoding SQT-BH3 constructs in inducing cell death was tested both in hepatocellular carcinoma cell lines as well as in primary hepatocytes using lipid nanoparticle (LNP)-formulated constructs. Hep3B cells were treated with varying doses (0.78 ng to 50 ng) of MC3-formulated LNPs in the presence of YOYO-3 (Life Technologies), a DNA dye that is taken up preferentially by dead cells that is used to measure the extent of cell death. MC3 lipid nanoparticles included MC3 50%, DSPC 10%, Cholesterol 38.5%, PEG-DMG 1.5%, N:P -5.5. (Values are based on mol. %.) The results are shown in Figure 3. When administered at a relatively high concentration to Hep3B hepatocellular carcinoma cells in MC3-formulated LNPs, mRNAs encoding SQT-PUMA-BH3 or SQT-Bim-BH3 both induced apoptosis and cell death, although SQT-PUMA-BH3 showed a modest increase in both the efficacy and timing of cell killing compared to SQT-Bim-BH3. In contrast, mRNAs encoding SQT-Bad-BH3 and SQT-Noxa-BH3 behaved similar to the empty MC3 LNP control and mRNAs encoding SQT. However, when administered at a four-fold lower concentration in MC3-fomulated LNPs, mRNAs encoding SQT-Bim-BH3 induced cell killing in Hep3B cells more efficiently than SQT-PUMA-BH3.

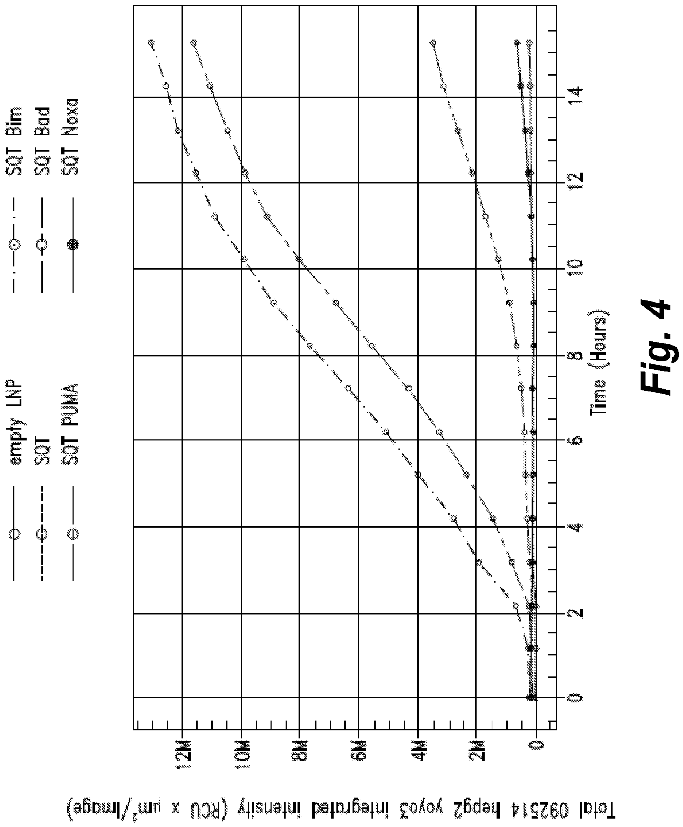

These results were confirmed and extended using a different hepatocellular carcinoma cell line (HepG2 cells). Administration of mRNAs encoding SQT-PUMA-BH3 or SQT-Bim-BH3 formulated in MC3 LNPs to HepG2 cells lead to cell death, as assessed by an increase in staining by the YOYO3 dye, a membrane-impermeant nucleic acid dye that brightly stains dead cells with compromised membranes. The results are shown in Figure 4. In this experiment, SQT-Bim-BH3 induced cell death with an increase in both efficacy and timing compared to SQT-PUMA-BH3.

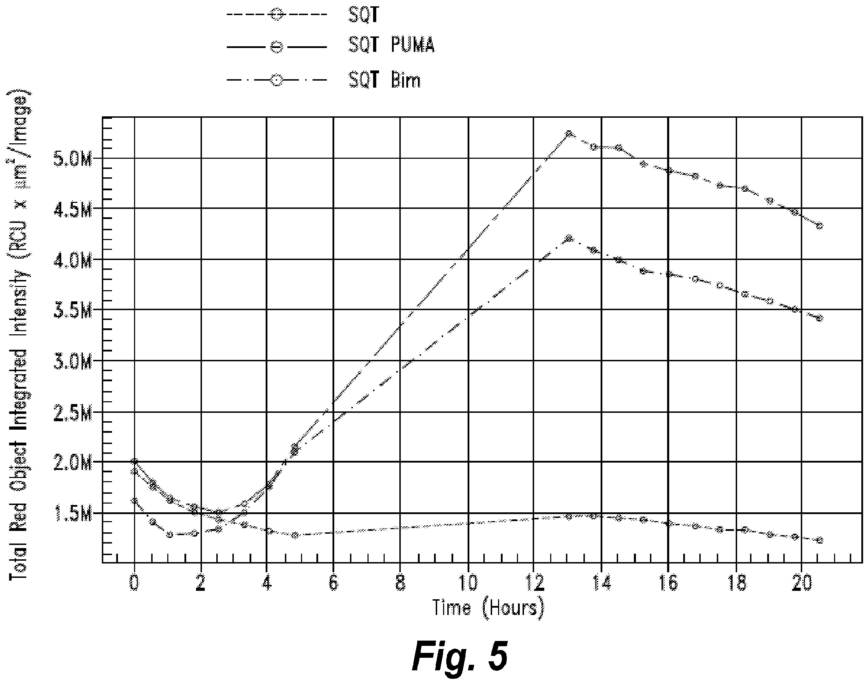

Next, the efficacy of apoptosis induction in primary hepatocytes was tested by administering LNP formulations containing mRNAs encoding SQT, SQT-PUMA-BH3, or SQT-Bim-BH3 formulated in MC3 LNPs to primary hepatocytes cultured *in vitro.* Administration of LNPs containing mRNAs encoding SQT-PUMA-BH3 or SQT-Bim-BH3 induced apoptosis in primary hepatocytes within approximately 4-6 hours. The results are shown in Figure 5. Consistent with these results, administration of LNPs containing mRNAs encoding SQT-PUMA-BH3 or SQT-Bim-BH3 also induced PARP-1 cleavage in primary hepatocytes, whereas SQT, SQT-Bad-BH3, or SQT-Noxa-BH3 did not.

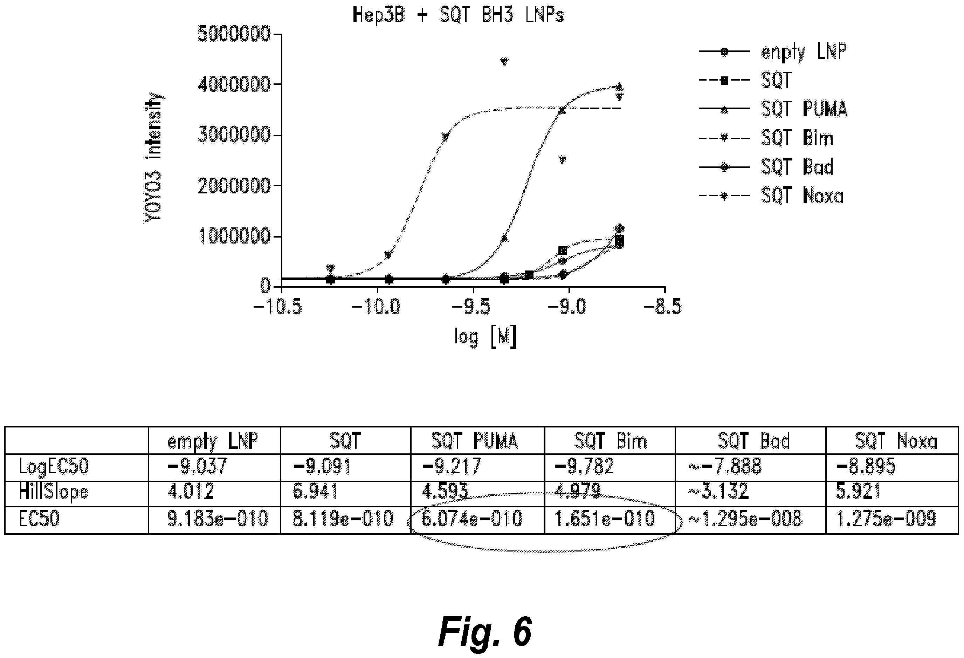

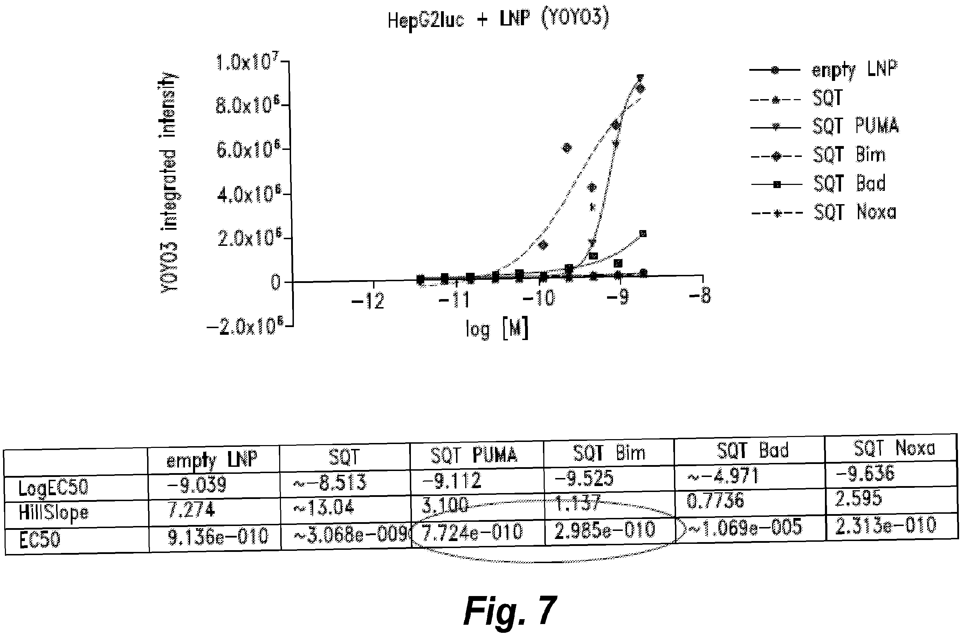

Next, the efficiency of cell killing by mRNA encoding SQT-PUMA-BH3 or SQT-Bim-BH3 was compared. At a dose of approximately 0.78 ng mRNA, which is approximately 100 fold lower than standard transfection conditions, SQT-Bim-BH3 killed both HeLa cells and Hep3B cells with greater efficiency than SQT-PUMA-BH3, indicating that at lower doses of mRNA, SQT-Bim-BH3 was more efficient at inducing apoptosis than SQT-PUMA-BH3. In Hep3B cells, a dose-response curve 12 hours after transfection using L2K lipid nanoparticles containing mRNA encoding control SQT, SQT-PUMA-BH3, or SQT-Bim-BH3 confirmed the more efficient cell killing by SQT-Bim-BH3 at low doses. Calculations of the IC50s for SQT-PUMA-BH3 and SQT-Bim-BH3 in Hep3B and HepG21uc cells are shown in Figures 6 and 7, respectively.

### EXAMPLE 3

### HEPATOCELLULAR CARCINOMA-TARGETED MRNAS ENCODING SQT-BH3 CONSTRUCTS

As described in the preceding examples, Stefin A-BH3 domain constructs (e.g., SQT-PUMA-BH3 or SQT-Bim-BH3) can induce apoptosis and cell death in a wide variety of cell types, including cervical cancer (HeLa), melanoma (A375), primary hepatocytes, and hepatocellular carcinoma cells. To increase the specificity of apoptosis induction and cell death to cancer cells as opposed to normal cells, mRNAs encoding a Stefin A-BH3 construct were engineered to contain a miR-122 binding site, as described in WO 2014/113089. miR-122 is a regulator of fatty acid metabolism and is one of the most abundant microRNAs in normal liver cells (hepatocytes). Compared to normal hepatocytes, hepatocellular carcinoma cells typically express low levels of miR-122. Without wishing to be bound by theory, in normal hepatocytes, mRNAs encoding a Stefin A-BH3 construct that further include one or more miR-122 binding elements will be largely repressed, but in hepatocellular carcinoma cells, the absence of miR-122 will allow translation of the Stefin A-BH3 construct, followed by induction of apoptosis and cell death.

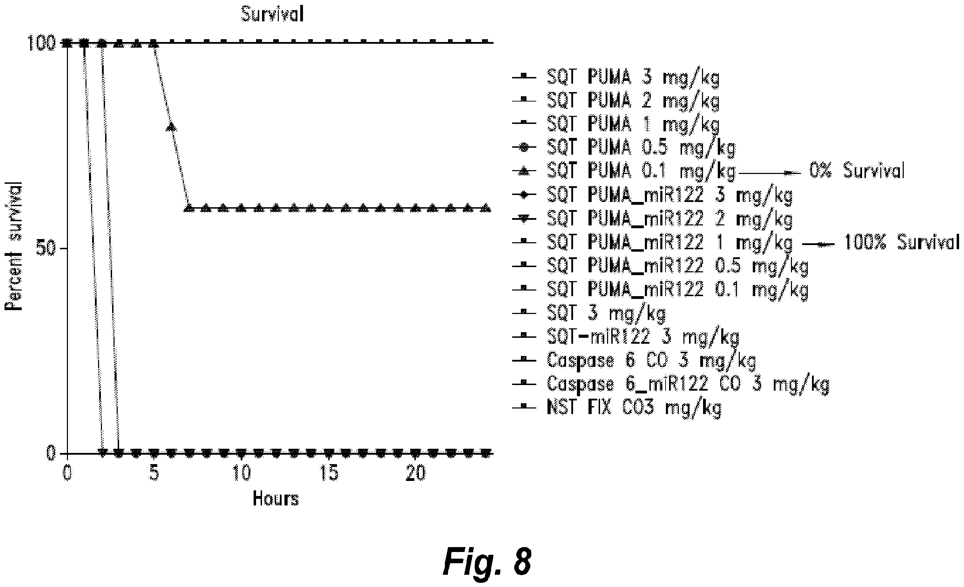

To test whether miR-122 binding sites could be used to widen the therapeutic window for Stefin A-BH3 proteins, enabling targeted killing of hepatocellular carcinoma cells but sparing normal liver cells, mice were treated with MC3 LNP formulations including 0.1 mg/kg, 0.5 mg/kg, 1 mg/kg, 2 mg/kg, or 3 mg/kg of mRNA encoding SQT-PUMA-BH3 that either did or did not include a miR-122 binding site. Negative controls included mRNA encoding SQT alone or SQT with a miR-122 binding site. Constitutively active caspase 6 was used as a positive control. An LNP containing a non-translatable factor IX cap 0 mRNA was used to control for mRNA and lipid effects that might be independent of the proteins encoded by the modified mRNAs. The results are shown in Figure 8. Administration of MC3 LNP formulations that included mRNAs encoding SQT-PUMA-BH3 without miR-122 binding sites was very toxic over a range of doses, resulting in the death of the treated animals within 5 hours; only animals treated with the lowest dose of 0.1 mg/kg survived at a rate of approximately 60%. In contrast, addition of a miR-122 binding site to the mRNA encoding SQT-PUMA-BH3 markedly increased the tolerable dose of SQT-PUMA-BH3; for instance, mice treated with 1 mg/kg of mRNA encoding SQT-PUMA-BH3 that included a miR-122 binding element had a 100% survival rate. This dose was 10 times greater than a dose of mRNA encoding SQT-PUMA-BH3 that killed 40% of mice.

### EXAMPLE 4

### INHIBITION OF SQT-BH3-INDUCED APOPTOSIS BY A BCL-2-LIKE POLYPEPTIDE

As described in the preceding examples, Stefin A-BH3 domain constructs (e.g., SQT-PUMA-BH3 or SQT-Bim-BH3) can induce apoptosis and cell death in a wide variety of cell types, including cervical cancer (HeLa), melanoma (A375), primary hepatocytes, and hepatocellular carcinoma cells. To determine whether SQT-BH3-induced apoptosis could be regulated by coexpression of a prosurvival Bcl-2-like protein, mRNAs encoding various Bcl-2-like prosurvival polypeptides (i.e., Trap constructs) were introduced into Hep3B cells together with an SQT-BH3 mRNA.

The Bcl-2-like constructs had the structures as shown below in Table 11:

**Table 11:**

| **Name** | **5'UTR SEQ ID** | **mRNA SEQ ID** | **3'UTR SEQ ID** |
|---|---|---|---|
| Bcl-w wild-type | 72 | 78 | 73 |
| Bcl-xL wild-type | 72 | 79 | 73 |
| Mcl-1 del.N/C | 72 | 80 | 73 |
| Bcl-w(C29S/A128E) | 72 | 81 | 73 |
| Bcl-2(D34A) del.C32 | 72 | 82 | 73 |
| Bcl-xL del.C24 | 72 | 83 | 73 |
| Mcl-1 del.N/C(2010) | 72 | 84 | 73 |
| Bcl-xL(D61A) del. C24 | 72 | 85 | 73 |

Additionally, all constructs contained a Cap 1 5' Cap (7mG(5')ppp(5')NlmpNp), a poly A tail of 100 nucleotides and were fully modified with 1-methyl-pseudouridine (m1ψ). The mRNA sequences used in the constructs shown in Table 11 include an epitope tag coding sequence (FLAG or V5) incorporated into the sequence. Sequences corresponding to SEQ ID NOs: 78-85 but without the epitope tag are shown in SEQ ID NOs: 92-99.

For the cotransfection experiments, Hep3B cells were co-transfected with an SQT-Bim-BH3 mRNA in combination with a wildtype or mutant Mcl-1, Bcl-2, Bcl-xL, or Bcl-w mRNA, including the following mutant Bcl-2-like mutants: Mcl-1 del.N/C, Bcl-w (C29S/A128E), Bcl-2 (D34A) del. C32, Bcl-xL del.C24, Mcl-1 del.N/C(2010), and Bcl-xL (D61A) del.C24. The SQT-Bim-BH3 mRNAs and the Bcl-2-like mRNAs were tested at various ratios and in serial dilution. % phase confluence of the Hep3B cells treated with the indicated constructs over a 24 hour time period was determined at various time points.

In a first set of experiments, the most potent inhibitor of SQT-BH3-induced cell death was Bcl-w (C29S/A128E) BH3 Trap.n9V5 at a ratio of Trap construct:SQT-BH3 construct of 1:2. This construct inhibited SQT-Bim-BH3-induced cell death in Hep3B cells to a substantially greater degree than Bcl-xL (D61A) del.C24 BH3 Trap.cFLAG. While SQT-BimBH3-transfected cells co-transfected with Bcl-xL del.C24 BH3 Trap.cFLAG exhibited substantial apoptosis and cell death, those co-transfected with Bcl-w (C29S/A128E) BH3 Trap.n9V5 remained alive.

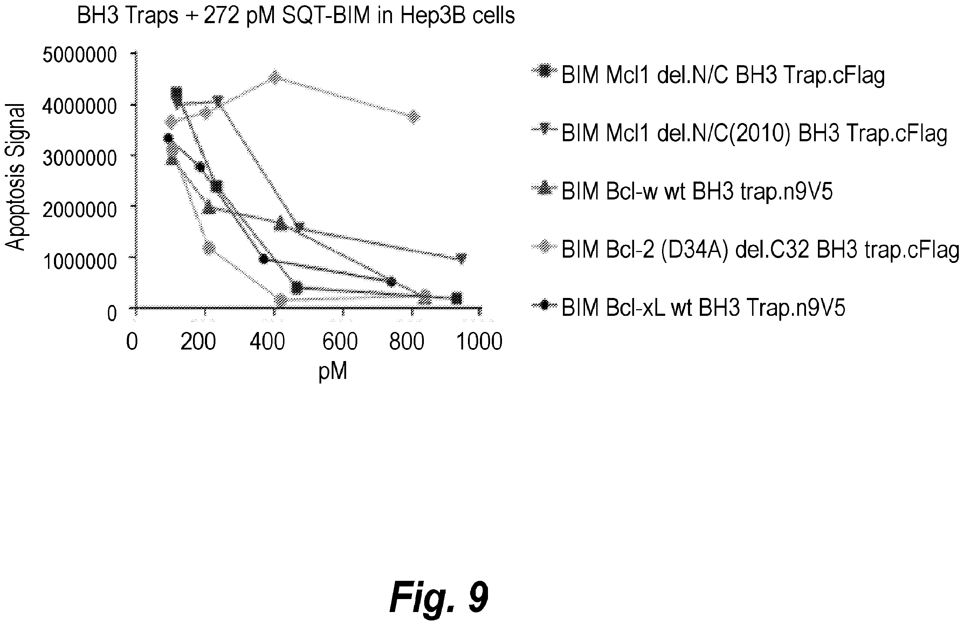

In a second study, 20,000 Hep3b cells/well in a 96-well plate were co-transfected with 272 picomolar SQT-Bim-BH3 construct in MC3 LNPs together with increasing amounts of one of the following Bcl-2-like trap mRNA constructs: Mcl-1 del.N/C, Mcl-1 del N/C (2010), Bcl-w wild-type, Bcl-2 (D34A) del.C32 or Bcl-xL wild type. YOYO-3 viability dye was used to assess apoptosis of cells. The results are shown in Figure 9. The results showed that all of the tested trap constructs except Bcl-2 (D34A) del.C32 were able to protect the Hep3B cells from apoptosis by the SQT-Bim-BH3 construct, with their effectiveness increasing in a dose dependent manner.

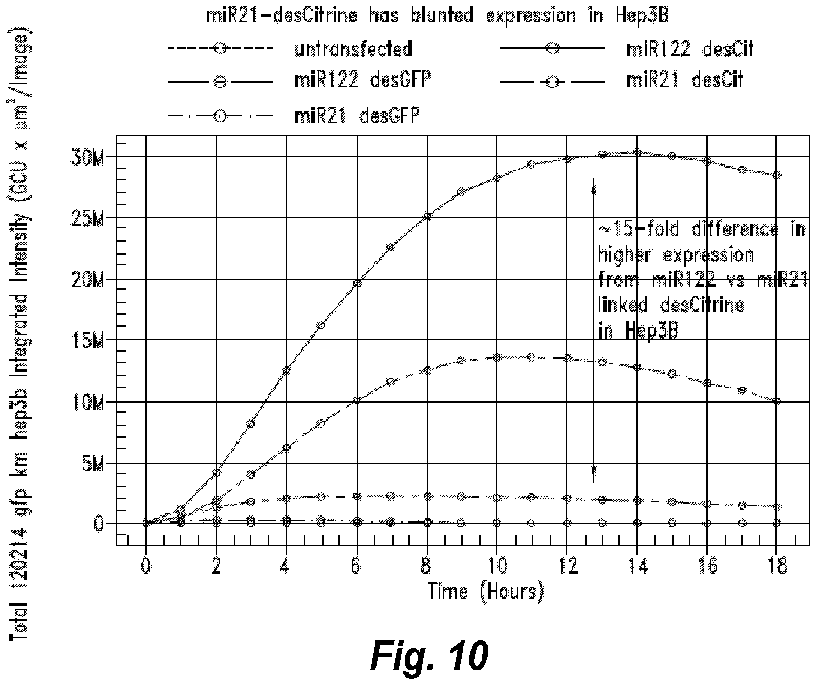

miR-21 is a regulator of fatty acid metabolism and is one of the most abundant microRNAs in normal liver cells (hepatocytes). High levels of circulating miR-21 have been linked to cancer, and compared to normal hepatocytes, hepatocellular carcinoma cells typically express high levels of miR-21. To determine in the presence of a miR-21 binding element reduced expression of an mRNA encoding desCitrine in Hep3B cells, Hep3B cells were transfected with mRNA constructs encoding either desGFP or desCit and containing either a miR-21 or miR-122 binding element. The miR-21 binding element had the sequence shown in SEQ ID NO:106. Expression of either desGFP or desCit was measured over an 18 our time period following transfection. Representative results are shown in Figure 10. Constructs containing a miR-122 binding element showed substantially higher expression than those containing a miR-21 binding element, with an approximately 15-fold difference in higher expression from miR-122 vs. miR-21 linked desCitrine in Hep3B cells.

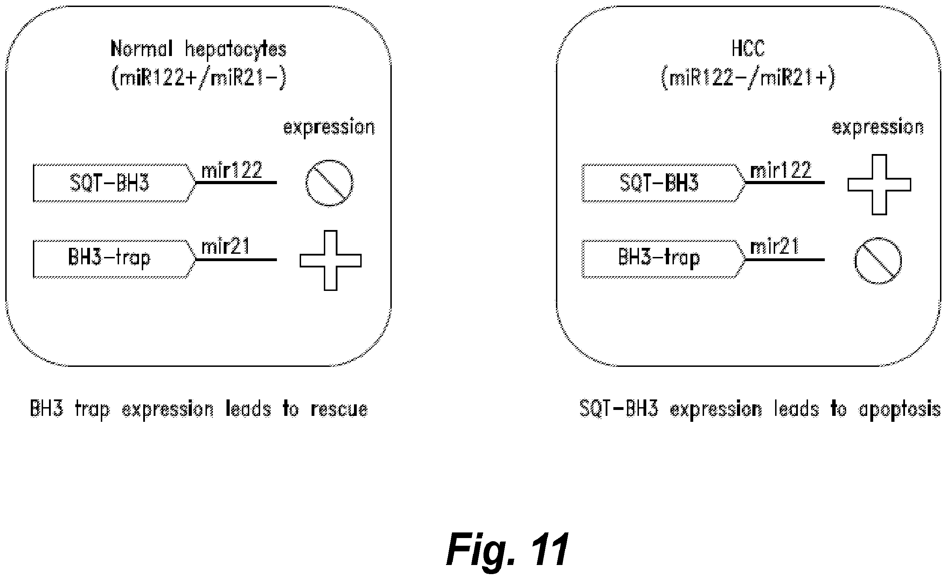

Without wishing to be bound by theory, in normal hepatocytes, BH3-Trap constructs that further include one or more miR-21 binding elements will be expressed, but in hepatocellular carcinoma cells, the presence of miR-21 will inhibit the expression of BH3-Trap constructs that include a miR-21 binding element. Therefore, the expression of SQT-BH3 can be limited to cancer cells by co-introducing a SQT-BH3 construct comprising a mirR-122 binding element in combination with a BH3-Trap construct comprising a miR-21 biding element, as diagrammed in Figure 11, thus providing tight regulation of unopposed pro-apoptotic SQT-BH3 polypeptide to cancer cells.

### EXAMPLE 5

### EFFECT OF INSERTION SITE POSITION ON APOPTOTIC POTENCY IN SQT-BH3 CONSTRUCTS

In this example, SQT-BID-BH3 constructs were made in which a single BID BH3 domain was inserted into either the N-terminal insertion site, the loop 1 insertion site or the loop 2 insertion site of the SQT scaffold polypeptide. The mRNA sequences of these SQT-BID-BH3 constructs, with a FLAG epitope tag, are shown in SEQ ID NOs: 43-45, respectively. Corresponding mRNA sequences without the FLAG tag are shown in SEQ ID NOs: 46-48. Hep3B cells were transfected with the constructs and the apoptotic ability of the constructs was assessed using YOYO3 to determine cell death, as described above. SQT-PUMA-BH3 and SQT-Bim-BH3 constructs were also tested for comparison purposes, as was the SQT scaffold alone. The results showed that the SQT-BID-BH3 constructs were able to induce apoptosis of Hep3B cells with similar potency to the SQT-PUMA-BH3 and SQT-Bim-BH3 constructs. Moreover, the SQT-BID-BH3 construct with the BH3 domain inserted in the N-terminal insertion site was more potent than the SQT-BID-BH3 constructs with the BH3 domain inserted at either the loop 1 insertion site or the loop 2 insertion site, thereby demonstrating that locating a single BH3 domain at the N-terminal insertion site is most effective for achieving the most potent apoptotic ability of a single BH3 domain presented by the SQT scaffold.

### EXAMPLE 6

### ADDITIONAL SQT CONSTRUCTS

Additional mRNA constructs utilizing the SQT scaffold polypeptide were prepared. The SQT scaffold constructs had the structures as shown below in Table 12:

**Table 12:**

| **Name** | **5'UTR SEQ ID** | **mRNA SEQ ID** | **3'UTR SEQ ID** |
|---|---|---|---|
| SQT anti-Mcll.(Ll) BimBH3.2A | 72 | 86 | 73 |
| SQT anti-Mcll.ntBim BH3.2A | 72 | 87 | 73 |
| SQT.(Ll)Mcll.BH3 | 72 | 88 | 73 |
| SQT.(L2)Mcll.BH3 | 72 | 89 | 73 |
| biSQT anti-Bcl/Mcll.(nt) BimBH2.2A.(L2)Bad | 72 | 90 | 73 |
| SQT anti-Mcl1.(L2)BimBH3.2A | 72 | 91 | 73 |

Additionally, all constructs contained a Cap 1 5' Cap (7mG(5')ppp(5')NlmpNp), a poly A tail of 100 nucleotides and were fully modified with 1-methyl-pseudouridine (m1ψ). The mRNA sequences used in the constructs shown in Table 12 include an epitope tag coding sequence (FLAG) incorporated into the sequence. Sequences corresponding to SEQ ID NOs: 86-91 but without the epitope tag are shown in SEQ ID NOs: 100-105.

Expression of these constructs in cells by transfection was confirmed by Western blot analysis using an anti-Flag antibody directed against the Flag epitope tag incorporated into each construct.

### EXAMPLE 7

### HALF-LIFE DETERMINATION FOR SQT SCAFFOLD POLYPEPTIDE

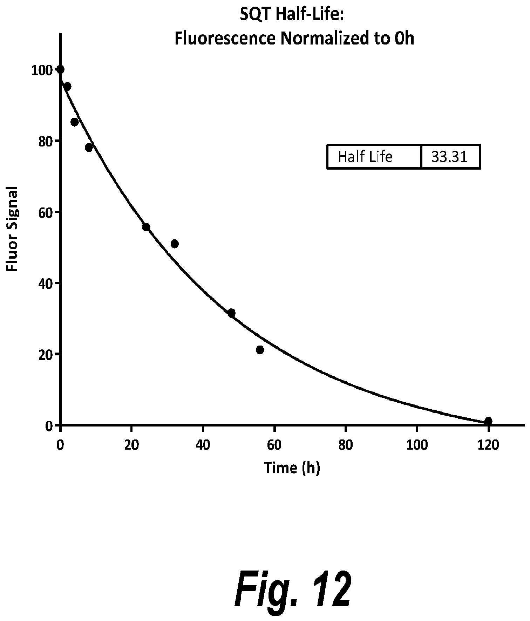

The half-life of the SQT protein scaffold in cells was determined by Bio-orthogonal non-canonical amino acid tagging (BONCAT). An mmRNA construct encoding the SQT scaffold polypeptide that did not contain any presenting polypeptides (i.e., a "dummy" construct), was transfected into cells, substituting an unnatural amino acid (azidohomoalanine; AHA) for a natural amino acid (methionine). AHA contains a reactive group (a terminal azide), which can be conjugated to a fluorophore via click chemistry. Newly synthesized proteins in the cells were separated by molecular weight (SDS-PAGE) at various time points post-transfection and visualized via in-gel fluorescence. The fluorescent signal was quantified using Li-cor's Image Studio (Version 3.1). One-phase decay nonlinear regression fit was by GraphPad Prism (Version 6). The results are shown in Figure 12. A half-life of 33.31 hours post-transfection was determined based on the data for the SQT scaffold.

### EXAMPLE 8

### PHARMACOKINETIC ANALYSIS OF INTRACELLULAR EXPRESSION OF SQT SCAFFOLD POLYPEPTIDE IN MOUSE LIVERS IN VIVO

Studies were performed to assess the pharmacokinetics of intracellular expression of the SQT scaffold polypeptide in mouse livers *in vivo* following multiple dosing. Mice were dosed on day 0 and on day 7 with an LNP carrying an mmRNA construct encoding the SQT scaffold polypeptide. The construct lacked any presenting polypeptides at any of the insertion sites (i.e., the construct was a "dummy" construct of only the scaffold), and was labeled with a FLAG epitope tag. Following dosing with the LNPs, liver protein was recovered and analyzed for expression of the SQT scaffold using the FLAG epitope tag for detection and quantification by quantitative Western blot analysis (LICOR/Western). Protein amounts were normalized to vinculin.

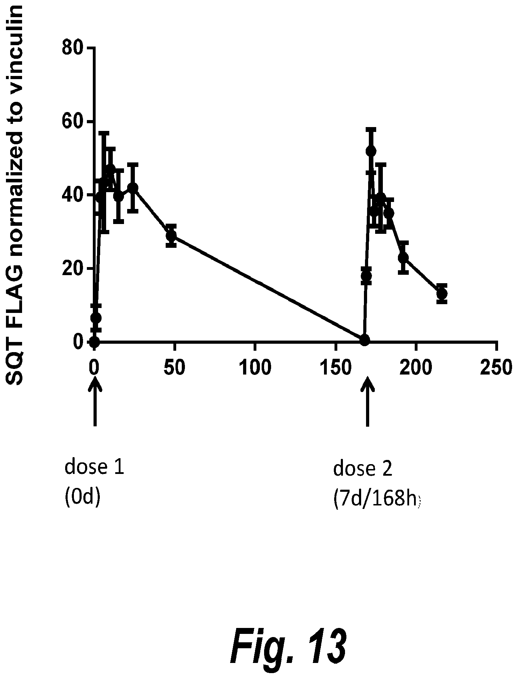

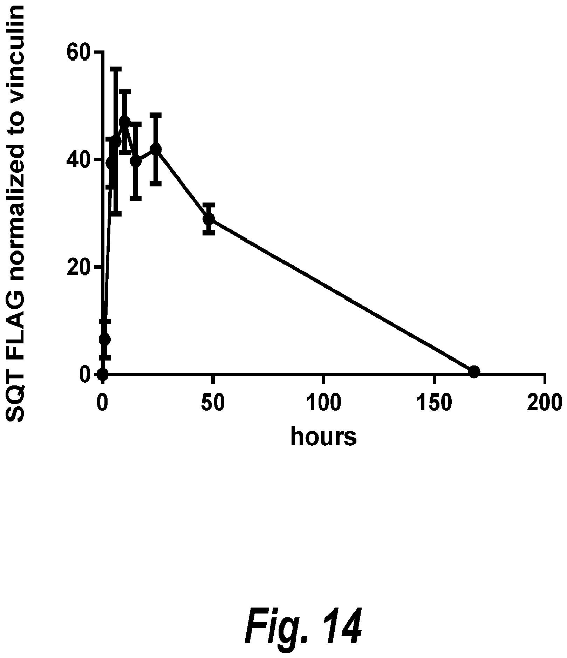

The results are shown in Figures 13 and 14. Figure 14 shows intracellular SQT scaffold expression over 250 hours, following administration of the mmRNA at day 0 and day 7 (168 hours). Figure 13 is a more detailed graph of the first 150 hours post-injection of the first dose at day 0. Consistent with the half-life results reported in Example 7, the results in Figure 13 demonstrate that there was rapid onset of SQT protein production, followed by increased protein production, with peak protein levels being reached within approximately the first 20 hours. Protein levels then began to subside gradually and were essentially finished by approximately 160 hours post-injection. The results in Figure 14 demonstrate that after the initial peak and subsiding of protein expression following administration of the first dose, intracellular SQT production is re-instated in the cells following administration of a second dose on day 7. The time course of protein production for the second dose mimicked that of the first dose, with rapid onset of protein production and peak levels being reached within the first approximately 20 hours post-injection, followed by gradual subsiding of protein expression.

These results demonstrate the ability of the mmRNA construct encoding the SQT scaffold to allow both for controlled production of the scaffold intracellularly, as well as clearance of the scaffold from the cells. Furthermore, following such clearance, SQT scaffold expression can be re-instated in the cells by injection of another dose of the mmRNA construct.

### EXAMPLE 9

### SQT-BH3 AND SORAFENIB COMBINATIONS ARE SYNERGISTIC

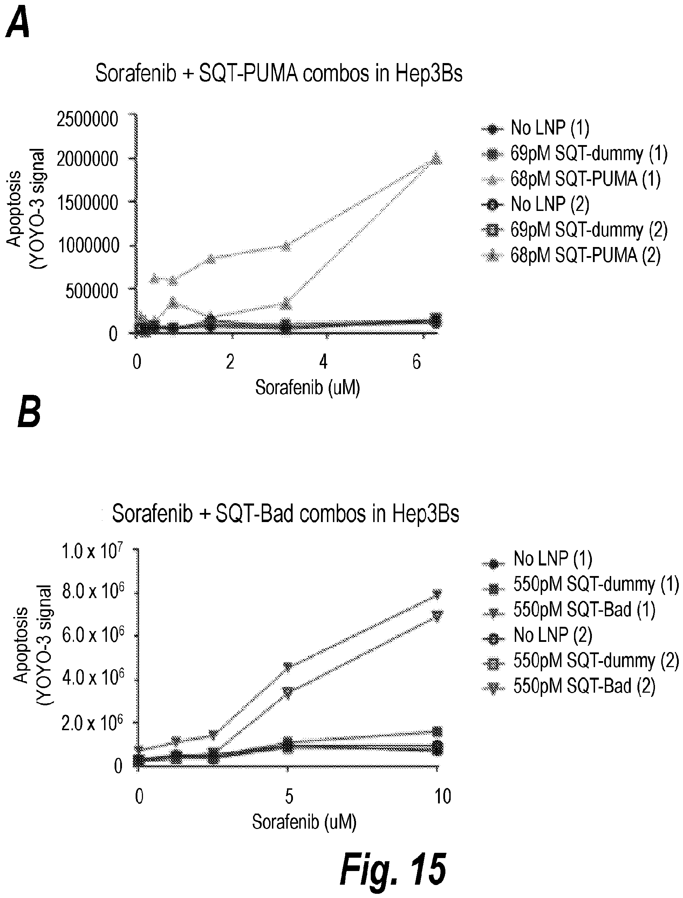

To determine the effect of SQT-BH3 constructs in combination with the protein tyrosine kinase inhibitor Sorafenib on liver cells, 20,000 Hep3B cells/well in a 96 well plate were treated with increasing amounts of sorafenib tosylate (Selleck Chemicals) and transfected with LNPs containing SQT-PUMA-BH3 (68 pM) or SQT-Bad-BH3 (550pM). Controls were transfection with LNP containing an SQT-dummy construct (that did not contain a BH3 domain) or leaving the cells untransfected (no LNP). YOYO-3 iodide (Life Technologies) viability dye was added to each well to allow for assessment of apoptosis. Cell death was measured on an IncuCyte ZOOM using YOYO-3 fluorescence as a read-out for apoptosis. The results, summarized in the graphs of Figure 15A and B, demonstrate that combination treatment with sorafenib and either SQT-PUMA-BH3 (Figure 15A) or SQT-Bad-BH3 (Figure 15B) increased apoptosis of the liver cells as compared to treatment with sorafenib alone. Moreover, this enhanced effect of the combination therapy was not merely an additive effect, but rather was a synergistic effect.

Similar combination experiments were performed with primary mouse hepatocytes, treated with SQT-PUMA-BH3 (205pM or 1100pM) or SQT-Bad-BH3 (549pM or 1100pM) in combination with sorafenib (0-10µM). The results showed that neither SQT-BH3 construct, in combination with sorafenib, caused apoptosis of the primary mouse hepatocytes. Thus, normal primary liver cells are spared by the combination treatment, indicating the combination treatment can selectively kill liver cancer cells over normal liver cells.

### EXAMPLE 10

### EVALUATION OF PEG-LIPID RATIOS IN LIPID NANOPARTICLES

Optimal molar percentages of PEG-Lipids for *in vivo* targeted delivery of lipid nanoparticles (LNPs) to tumors were evaluated by varying the molar percentages of different PEG-Lipids in LNPs and testing their physicochemical properties in the CT-26 and Hep3B mouse tumor models.

### Preparation and Characterization of PEG-Lipid LNPs

Lipid nanoparticle formulations were prepared that were composed of: (i) 50 mol% DLin-KC2-DMA (cationic lipid or ionizable amino lipid); (ii) 10 mol% DODMA-DSPC (non-cationic lipid); (iii) 30-39.5 mol% Cholesterol; and (iv) 0.5-10 mol% PEG-Lipid. The total percentage of Cholesterol + PEG-Lipid = 40 mol%. Thus, when the PEG-Lipid was present at 0.5 mol%, the Cholesterol was present at 39.5 mol%, when the PEG-Lipid was present at 1.5 mol%, the Cholesterol was present at 38.5 mol%, when the PEG-Lipid was present at 5 mol%, the Cholesterol was present at 35 mol% and when the PEG-Lipid was present at 10 mol%, the Cholesterol was present at 30 mol%.

Three forms of PEG-Lipid were tested: PEG-DMG, PEG-DSG and PEG-DSPE. PEG-DMG and PEG-DSG have the following structure: 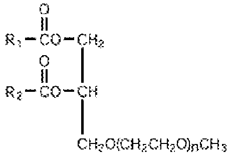 wherein for DMG: R1, R2 = C14 and for DSG: R1, R2 = C18.
PEG-DSPE has the following structure: 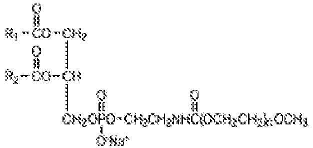 wherein for DSPE: R1, R2 = C18.

The PEG-Lipids having longer R1, R2 lipid chains (e.g., DSG and DSPE) are less diffusible than PEG-Lipids having shorter R1, R2 lipid chains (e.g., DMG).

The physiochemical properties of the LNPs containing different percentages of the three different PEG-Lipids was examined, the results of which are shown below in Tables 13-16 below.

**Table 13**

| | Z-Average (diameter in nm) | | | |
|---|---|---|---|---|
| | 0.5 mol% | 1.5 mol% | 5 mol% | 10 mol% |
| PEG-DMG | 184.3 | 77.03 | 62.2 | 53.49 |
| PEG-DSG | 190.9 | 82.52 | 65.64 | 49.81 |
| PEG-DSPE | 179.9 | 84.58 | 58.74 | 47.4 |

These results demonstrate that the 0.5 mol% PEG-containing LNPs were larger than those with a higher mol% PEG-Lipid concentration.

**Table 14**

| | Polydispersity Index (PDI) | | | |
|---|---|---|---|---|
| | 0.5 mol% | 1.5 mol% | 5 mol% | 10 mol% |
| PEG-DMG | 0.069 | 0.149 | 0.205 | 0.213 |
| PEG-DSG | 0.065 | 0.145 | 0.172 | 0.196 |
| PEG-DSPE | 0.077 | 0.079 | 0.255 | 0.287 |

**Table 15**

| | Encapsulation Efficiency (%) | | | |
|---|---|---|---|---|
| | 0.5 mol% | 1.5 mol% | 5 mol% | 10 mol% |
| PEG-DMG | 92 | 97 | 92 | 48 |
| PEG-DSG | 91 | 98 | 97 | 39 |
| PEG-DSPE | 92 | 97 | 97 | 58 |

These results demonstrate there was a lower encapsulation efficiency for LNPs containing 10 mol% PEG-Lipid than for LNPs containing a lower mol% PEG-Lipid concentration.

**Table 16**

| | Zeta Potential (mV) | | | |
|---|---|---|---|---|
| | 0.5 mol% | 1.5 mol% | 5 mol% | 10 mol% |
| PEG-DMG | 0.422 | 2.72 | -4.52 | -1.48 |
| PEG-DSG | 0.477 | -7.05 | -5.36 | -2.2 |
| PEG-DSPE | -2.93 | -1.87 | -2.53 | -2.15 |

### Intratumoral Administration of PEG-Lipid LNPs

The LNP formulations containing varying mol% of different PEG-Lipids were used to administer a reporter mRNA construct into CT-26 tumors in mice. The reporter mRNA construct encoded a luciferase reporter protein to allow for evaluation of the biodistribution of the mRNA encapsulated by the LNPs. The mRNA construct included a Cap 15' cap, and a 140 polyA tail and was fully modified with 1-methylpseudouridine and 5-methyl-cytosine.

LNPs formulations as described above, containing either 0.5 mol% or 1.5 mol% PEG-DMG, PEG-DSG or PEG-DSPE were administered intratumorally into BALB/cAnNCrl mice bearing CT-26 tumors (n = 4 per group; average tumor size of 290 mm³) at a single dose of 0.05 mg/kg luciferase mRNA. Controls were PBS alone and naked mRNA luciferase in 8.5% sucrose.

Whole body BLI was performed 2 hours post-treatment. Twenty minutes prior to imaging, mice were injected intraperitoneally with a D-luciferin solution at 150 mg/kg. Animals were then anesthetized and images were acquired with an IVIS Lumina II imaging system (Perkin Elmer). Bioluminescence was measured as total flux (photons/second) of the entire mouse.

Animals were sacrificed and tumors and livers were harvested at 4 hours post-treatment for *ex vivo* luciferase protein quantification. The tumor:liver expression ratio for the luciferase reporter protein was determined for each of the PEG-Lipids tested and different mol% concentrations tested.

The results from both the BLI analysis and the *ex vivo* protein quantitation showed that, overall, the lower molar percentages of PEG-Lipid improved the tumor:liver ratio post intra-tumor administration and the LNPs containing 0.5 mol% of any of the three PEGs exhibited a higher tumor:liver expression profile at the time points examined compared to those with 1.5 mol%.

### Systemic Administration of PEG-Lipid LNPs

The LNP formulations containing varying mol% of different PEG-Lipids were used to administer a reporter mRNA construct systemically into mice bearing either a CT-26 subcutaneous tumor or a Hep3B subcutaneous tumor. The same luciferase reporter mRNA construct described above for the intra-tumor studies was used for the systemic administration studies.

LNPs formulations as described above, containing either 1.5 mol%, 5 mol% or 10 mol% PEG-DMG, PEG-DSG or PEG-DSPE were administered intravenously into BALB/c nude mice bearing Hep3B SC tumors (tumor size range = 254-876 mm³; average = 548 mm³) or CT-26 SC tumors (tumor size range = 278-664 mm³; average = 405 mm³) (n = 4 per group). Mice were injected IV with 100 µl of 0.04 mg/ml RNA, which is a dose of approximately 0.2 mg/kg luciferase mRNA. Control was PBS alone.

Whole body BLI was performed 2, 6, 24 and 48 hours post-treatment. Twenty minutes prior to imaging, mice were injected intraperitoneally with a D-luciferin solution at 150 mg/kg. Animals were then anesthetized and images were acquired with an IVIS Lumina II imaging system (Perkin Elmer). The ventral view was imaged at 15 minutes post D-luciferin administration and the tumor side view was imaged at 20 minutes post D-luciferin administration. Bioluminescence was measured as total flux (photons/second) of the entire mouse.

Animals were sacrificed and tumors and livers were harvested at 4 days post-treatment for *ex vivo* luciferase protein quantification. The tumor:liver expression ratio for the luciferase reporter protein was determined for each of the PEG-Lipids tested and different mol% concentrations tested.

The results from both the BLI analysis and the *ex vivo* protein quantitation showed that the expression profiles of the systemically administered LNPs was better in the Hep3B tumor than in the CT-26 tumors. The BLI analysis showed that 1.5 mol% PEG-DSPE and 5 mol% DSG exhibited the most promising Hep3B tumor expression profile (i.e., higher relative liver signal) within the first 2-48 hours post-treatment. The *ex vivo* protein quantification analysis demonstrated that the following LNPs exhibited the highest average luciferase protein levels in tumors 52 hours post-treatment: 1.5 mol% PEG-DSG, 5 mol% PEG-DSG and 1.5 mol% DSPE. Since both PEG-DSG and PEG-DSPE utilize C18 lipid side chains, these studies demonstrated that the C18 PEG-Lipids in the LNPs displayed improved tumor:liver expression profiles post systemic administration of the LNPs.

### EXAMPLE 11

### EFFICACY OF PRO-APOPTOTIC MMRNAS IN SCID MICE BEARING SUBCUTANEOUS HEP3B XENOGRAFTS

LNPs comprising the cationic lipid MC3 were tested using the optimal molar percentage of PEG-lipid and optimal PEG length (1.5% DSPE, C18, as described in Example 10), for *in vivo* targeted delivery of SQT-PUMA-BH3.

SCID mice were dosed weekly (Day 1 and Day 8) over a two week period with mmRNA encoding SQT-PUMA-BH3 (comprising a miR-122 binding site), as described above. Mice were dosed at two doses as follows:
Group 1_PBS
Group 3_PUMA miR122 (3mpk)
Group 5_PUMA miR122 (2mpk)

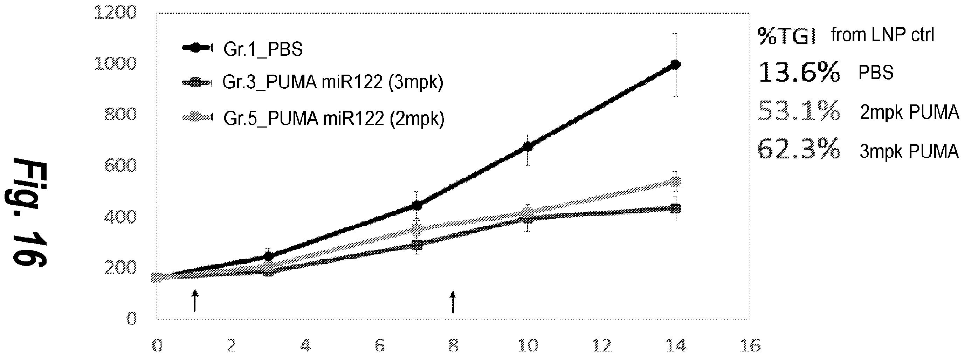

Multiple weekly dosing of SCID mice over a 2 week timeframe allowed for a robust response in Hep3b xenografts with both doses of SQT-BH3-PUMA-miR122 tested. Tumor growth inhibition (TGI) was calculated as compared to control (LNP with SQT alone). The results are shown in Figure 16. The results show that the mice treated with the SQT-PUMA-BH3 construct at either dose showed significantly greater TGI than the mice treated with PBS alone. These data evidence efficacy of the SQT-BH3 constructs of the invention in reducing tumor size in a relevant animal model.

### EXAMPLE 12

### SYSTEMIC ADMINISTRATION OF PEG-LIPID LNPS IN COMBINATION WITH THE TYROSINE KINASE INHIBITOR SORAFENIB

LNPs comprising the cationic lipid MC3 were tested using the optimal percentage of PEG-lipid and optimal PEG length (1.5% DSPE, C18, as described in Example 10), for *in vivo* targeted delivery of SQT-PUMA.

SCID mice bearing Hep3B SC tumors (tumor size range 140 - 227 mm³; average = 183 mm³) were dosed over a three week period with a combination of 0.5% methyl cellulose daily and SQT IV at 1.5 mg/kg alone weekly (Days 1, 8 and 15; Group 1), Sorafenib (in 0.5% methyl cellulose) at 10 mg/kg orally daily alone (Group 2), mmRNA encoding SQT-PUMA-BH3 (comprising a miR122 binding site) weekly at 1.5 mg/kg IV alone (Group 3) or a combination of mmRNA encoding SQT-PUMA-BH3 (with miR122 binding site) at 1.5 mg/kg IV weekly with sorafenib at 10 mg/kg orally daily (Group 4; 10 animals per group).

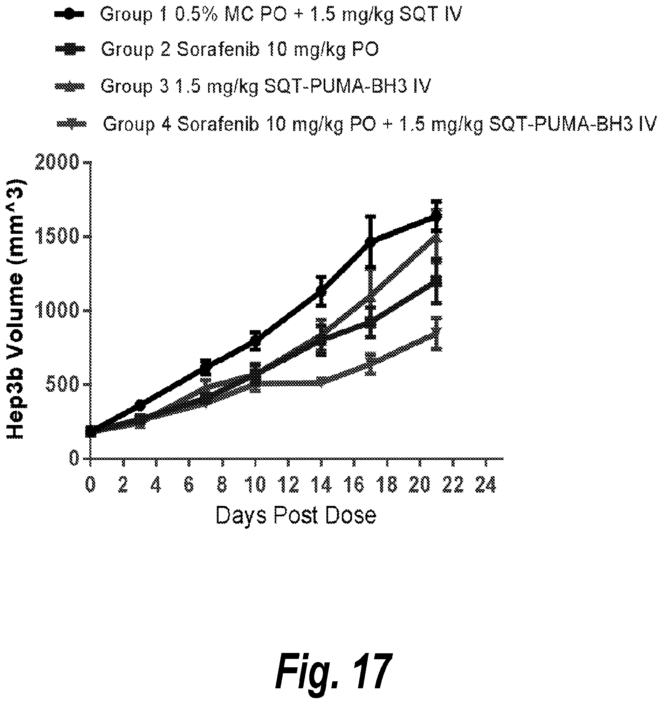

Treatment with Sorafenib and the SQT-PUMA-BH3 construct alone resulted in tumor growth inhibition (TGI) of 38% and 28%, respectively, at the end of the study, compared to the PBS treatment group, as shown in Figure 17. The combination treatment of sorafenib and SQT-PUMA-BH3 resulted in a significantly greater effect on tumor growth than each agent alone, resulting in a TGI of 59%. These data provide evidence for a synergistic effect of SQT-PUMA-BH3 constructs in combination with the standard of care treatment for HCC, the tyrosine kinase inhibitor sorafenib.

### EXAMPLE 13

### EFFICACY OF PRO-APOPTOTIC MMRNAS IN SCID MICE BEARING ORTHOTOPIC HEP3B XENOGRAFTS

LNPs comprising the cationic lipid MC3 were tested using the optimal percentage of PEG-lipid and optimal PEG length (1.5% DSPE, C18, as described in Example 10), for *in vivo* targeted delivery of SQT-PUMA-BH3 in a model of Orthotopic Hep3B xenografts.

SCID mice were anesthetized, a transverse incision was made below the xiphoid and Hep3B cells (2 x 10⁶) were injected into the upper left lobe of the liver, followed by surgical closure of the abdomen and skin. Tumor growth was measured by whole body bioluminescent imaging (BLI) in anaesthetized animals following SC injection of 150 mg/kg D-Luciferin, in an imaging chamber (Spectrum CT). Bioluminescence was measured as total flux (photons/second) of the entire animals. On Day 8 following tumor implantation when tumor BLI reached an average of 3.17 x 10⁸ photons/sec (range 12.37 x 10⁷ - 1.38 x 10⁹ photons/sec), animals were randomized into study groups and treated weekly over a three week period (Days 9, 16 and 23) with PBS IV (Group 1), SQT 3 mg/kg IV (Group 2), mmRNA encoding SQT-PUMA-BH3 (comprising a miR122 binding site) weekly at 0.75 mg/kg (Group 3), 1.5 mg/kg (Group 4) or 3 mg/kg IV (Group 5; 12 animals per group). One week following the final dose, animals were euthanized and tumors excised and weighed.

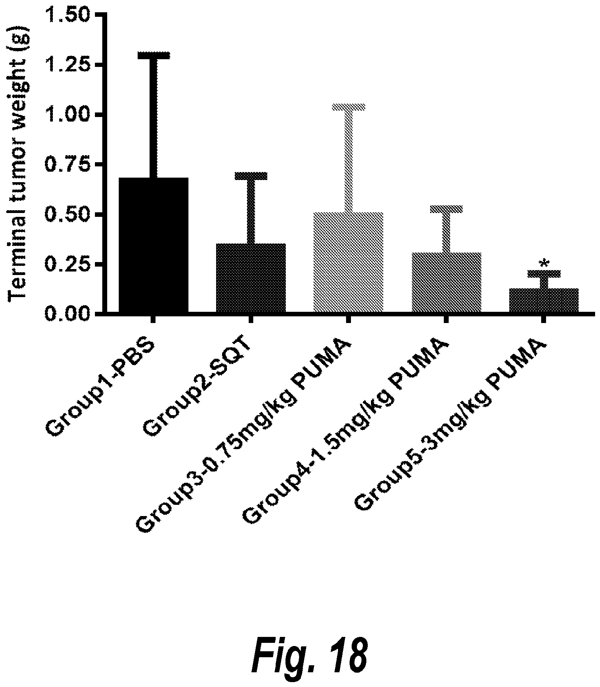

SQT at 3 mg/kg IV alone had an inhibitory effect on orthotopic Hep3B tumor cell growth, with a 49% TGI compared to the PBS treatment group at the end of the study. mmRNA encoding SQT-PUMA-BH3 (with the miR122 binding site) evoked a dose-dependent inhibitory effect on tumor cell growth, attaining a TGI inhibition of 25, 56 and 82%, respectively, at 0.75, 1.5 and 3 mg/kg IV relative to the PBS treatment group, reaching a significantly statistical difference at the highest dose tested (Figure 18).

Percent body weight change was also measured over the course of the study, the results of which showed that treatment with SQT-PUMA-BH3 did not significantly impact body weight, thus indicating that the formulation was well tolerated.

These data provide evidence of efficacy of the SQT-PUMA-BH3 constructs in reducing tumor size in a relevant animal model of HCC.

### EXAMPLE 14

### EFFICACY OF SQT-PUMA-BH3 IN ANIMAL MODELS OF ACUTE MYELOID LEUKEMIA (AML)

LNPs comprising the cationic lipid MC3 were tested using the optimal percentage of PEG-lipid and optimal PEG length (1.5% DSPE, C18, as described in Example 8), for *in vivo* targeted delivery of SQT-PUMA-BH3 in two different *in vivo* models of acute myeloid leukemia (AML). One model used systemic TF1a xenografts in SCID mice. The other model used systemic HEL xenografts in SCID mice. Each of these models uses a human erythroleukemia cell line. The HEL erythroleukia cell line is described in, for example, Martin, P. and Papayannopoulou, T. (1982) Science 216:1233-1235. The TF1 erythroleukmia cell line (from which TF1a is derived) is described in, for example, Kitamura, T. et al. (1989) J. Cell. Physiol. 140:323-334. Unless otherwise noted, mRNAs were fully modified with N1-methlypseudouridine to avoid stimulating an innate immune response to said mRNAs when administered *in vivo.*

In a first set of experiments, TF1a cells stably expressing luciferase (TF1a-luc cells) were implanted systemically in SCID mice. Tumor growth was measured by whole body bioluminescent imaging (BLI) in anaesthetized animals following SC injection of 150 mg/kg D-Luciferin, in an imaging chamber (Spectrum CT). Animals showing an increasing tumor burden at day 12 post-cell implantation were recruited for treatment. Animals were randomized into four study groups, as follows: PBS IV (Group 1), cytarabine at 2 mg/kg three times per week (Group 2), LNPs containing mRNA encoding Green Fluorescent Protein (GFP) at 3 mg/kg one time per week (Group 3) and LNPs containing mRNA encoding SQT-PUMA-BH3 (comprising a miR122 binding site) at 3 mg/kg one time per week. Mice were dosed intravenously for four weeks.

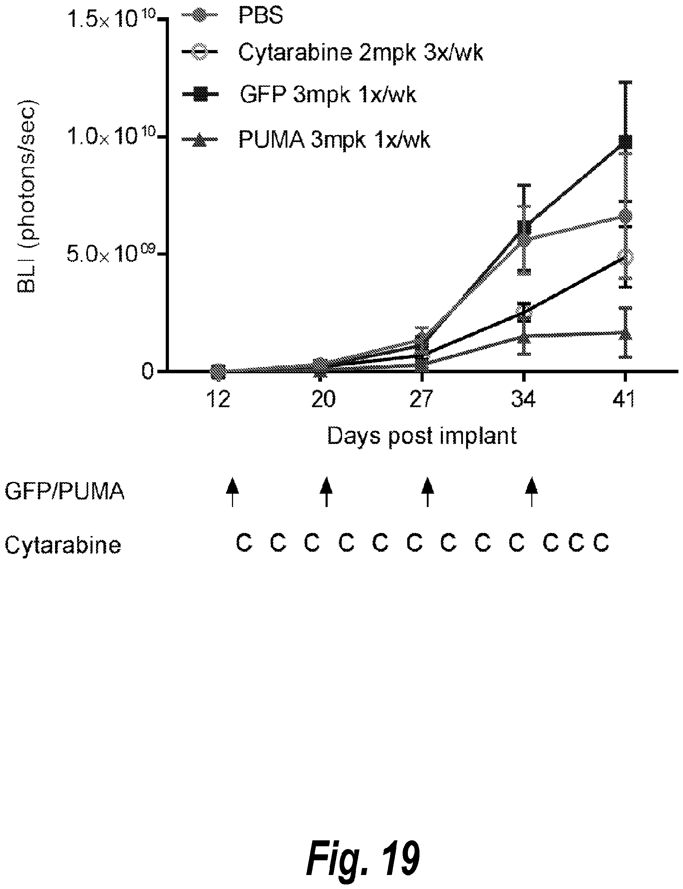

The results for the TF1a-SCID studies are shown in the graph of Figure 19, which demonstrates that treatment with PBS or mRNA encoding GFP alone did not significantly impact tumor growth, treatment with the chemotherapeutic agent cytarabine alone did inhibit systemic tumor growth and treatment with mRNA encoding SQT-PUMA-BH3 led to greater tumor growth inhibition than treatment with cytarabine. Body weight of the mice was also monitored, which showed that treatment with mRNA encoding SQT-PUMA-BH3 did not cause significant body weight changes in the TF1a-SCID mice. Furthermore, treatment with mRNA encoding SQT-PUMA-BH3 LNPs was well tolerated, resulting in no treatment-related mortality. These data provide evidence of efficacy of the SQT-PUMA-BH3 constructs in inhibiting tumor growth in a relevant animal model of acute myeloid leukemia (AML).

In a second set of experiments, HEL cells stably expressing luciferase (HEL-luc cells) were implanted systemically in SCID mice. Tumor growth was measured by whole body bioluminescent imaging (BLI) in anaesthetized animals following SC injection of 150 mg/kg D-Luciferin, in an imaging chamber (Spectrum CT). Animals showing an increasing tumor burden at day 27 post-cell implantation were recruited for treatment. Animals were randomized into five study groups, as follows: PBS IV (Group 1), LNPs containing mRNA encoding Green Fluorescent Protein (GFP) at 1.5 mg/kg (Group 2), LNPs containing mRNA encoding Green Fluorescent Protein (GFP) at 3 mg/kg (Group 3), LNPs containing mRNA encoding SQT-PUMA-BH3 (comprising a miR122 binding site) at 1.5 mg/kg, and LNPs containing mRNA encoding SQT-PUMA-BH3 (comprising a miR122 binding site) at 3 mg/kg. Mice were dosed intravenously at days 28 and 36 and tumor burden was assessed by BLI on days 33, 36, 40 and 43.

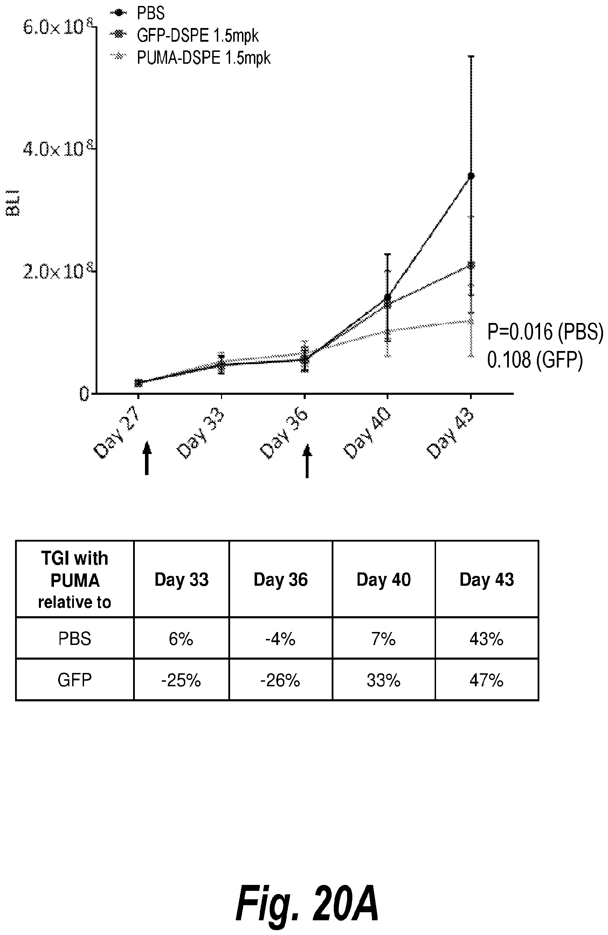

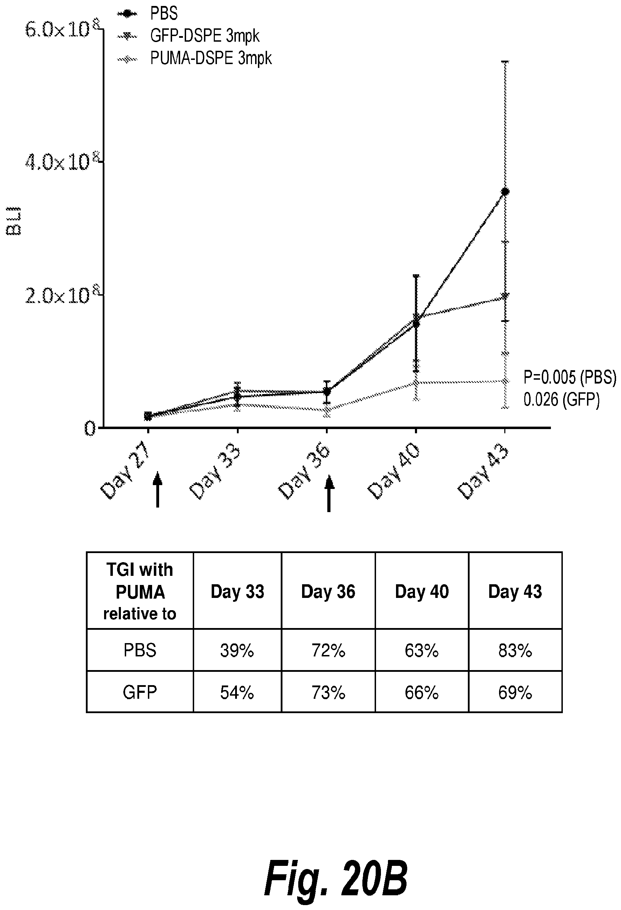

The results for the HEL-SCID studies are shown in the graphs of Figures 20A (1.5 mg/kg dose) and 20B (3 mg/kg dose). At the lower dose (1.5 mg/kg), by days 40 and 43, the mice treated with mRNA encoding SQT-PUMA-BH3 exhibited greater tumor growth inhibition (TGI) as compared to the mice treated with the PBS or GFP controls. At the higher dose (3 mg/kg), at day 33 the mice treated with mRNA encoding SQT-PUMA-BH3 already exhibited significantly greater tumor growth inhibition than the mice treated with the PBS or GFP controls, which TGI increased during the course of the experiment. These data provide evidence of efficacy of the SQT-PUMA-BH3 constructs in inhibiting tumor growth in a second relevant animal model of acute myeloid leukemia (AML).

In a repeat study using the TF1a-SCID model, a study was designed in which animals on the study that remained alive after the first 4 weeks of treatment continued to be dosed up to eight weeks in order to assess survival advantage. In this repeat study, tumor growth inhibition (TGI) was the primary efficacy over the first 4 weeks of the study and survival was the primary efficacy endpoint over the second 4 weeks of the study. The repeat study had a similar design compared to the first set of studies with the TF1a-SCID model (described above), with slight variation in treatment regimens. Animals were dosed as follows: PBS (0.1ml/20g one time per week) IV (Group 1), cytarabine IV at 2 mg/kg three times per week (Group 2), LNPs containing mRNA encoding Green Fluorescent Protein (GFP) at 3 mg/kg one time per week (Group 3) and LNPs containing mRNA encoding SQT-PUMA-BH3 (comprising a miR122 binding site) at 3 mg/kg one time per week (Group 4). Mice were dosed intravenously for four weeks, with animals in Group 4 being dosed for eight weeks (i.e., weekly dosing up to eight weeks).

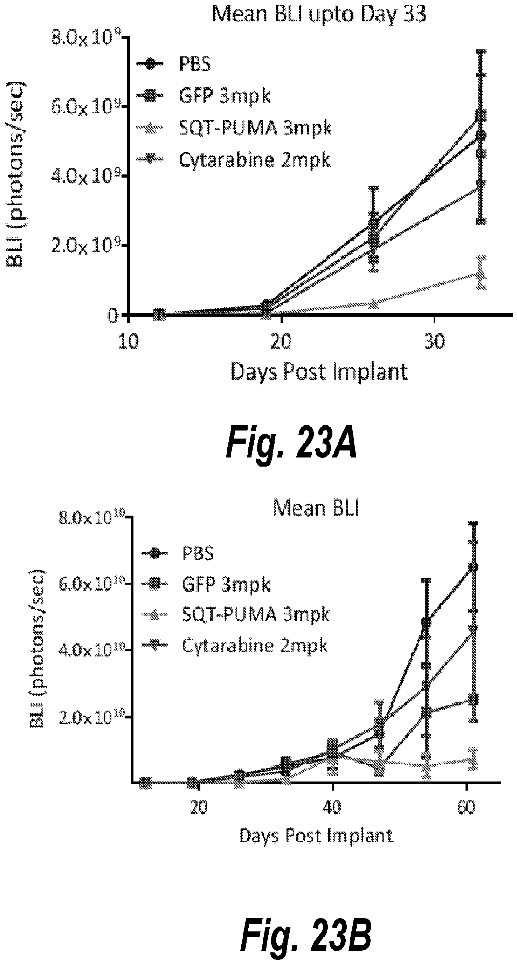

In this repeat study, tumor growth inhibition (TGI) was again used as a primary endpoint to quantify efficacy with BLI being used as a surrogate for tumor burden. Bioluminescence Imaging (BLI) was measured weekly out to at least Day 61 post disease induction. The mean BLI results at Day 33 and Day 61 are shown in Figures 23A and 23B, respectively. As was observed in the original study, in this repeat study animals treated with LNP-encapsulated mRNA encoding SQT-PUMA-BH3 showed marked tumor growth regression as compared to both PBS and LNP controls. Treatment with LNP-encapsulated mRNA encoding SQT-PUMA-BH3 was again the most effective treatment for inhibiting tumor growth, showing greater efficacy than the cytarabine SOC control.

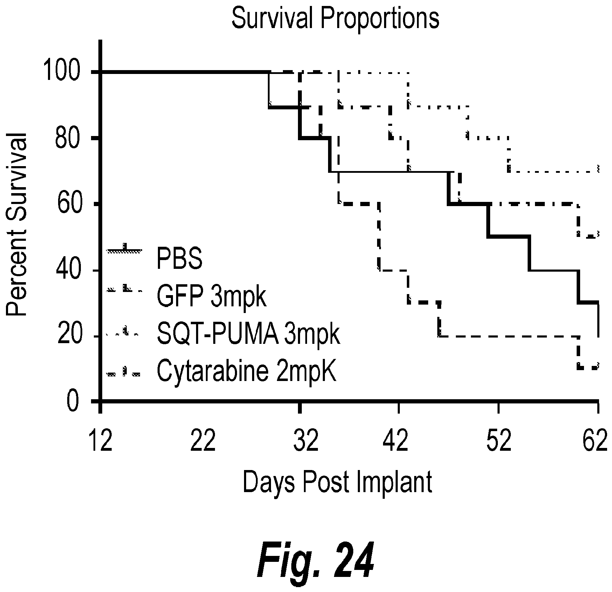

The survival of the mice over eight weeks was monitored and the percent survival of the mice in the study was assessed. The results are presented in the Kaplan-Meier curve shown in Figure 24. Notably, median survival was reached for GFP treated control animals within 4-5 weeks post-implant, whereas median survival had not yet been reached in animals treated with LNP-encapsulated mRNA encoding SQT-PUMA-BH3.

Collectively, the data from the repeat study clearly demonstrated that systemic administration of LNP-encapsulated mRNA encoding SQT-PUMA-BH3 was efficacious both in promoting tumor growth inhibition and in extending survival in the TF1a tumor-bearing animals. These data further evidence that systemic delivery using the tumor-optimized MC3-DSPE LNPs (C18-PEG optimized LNPs for enhanced tumor:liver expression profiles) successfully deliver the SQT-PUMA-BH3 mRNA to tumors where the apoptotic PUMA-BH3 domain inhibits TF1a-derived tumor growth in this disseminated cancer model.

Percent body weight change was also measured over the course of the study, the results of which showed that treatment with SQT-PUMA-BH3 did not significantly impact body weight, thus indicating that the formulations were well-tolerated. Furthermore, additional clinical signs and necropsy findings also indicated that the formulations were well-tolerated.

### EXAMPLE 15

### SYNERGISTIC PRO-APOPTOTIC EFFECT OF TARGETING MCL1 IN COMBINATION WITH SQT-BH3 CONSTRUCTS

In this example, the effect on apoptosis of combining SQT-BH3 mRNA constructs with mRNA constructs specifically targeting MCL1 was examined further. As described in Examples 9 and 12, combining SQT-BH3 with sorafenib led to synergistic pro-apoptotic effects. Sorafenib can cause downregulation of MCL1 but also affects other pathways and thus unwanted side effects could occur through the use of sorafenib to target MCL1. Furthermore, sorafenib does not cause downregulation of MCL1 in all cell types (e.g., primary hepatocytes). Accordingly, specific anti-MCL1 inhibitors were desired. While not intending to be limited by mechanism, it is thought that by neutralizing MCL1 in tumor cells, the tumor then reverts to sole reliance on BCLXL, BCL2 and/or other prosurvival members of the family as the pro-survival mechanism/pathway. Accordingly, use of SQT-BH3, which specifically destroys BCLXL, BCL2 and/or other prosurvival members of the family then leads to better tumor killing when used in combination with an anti-MCL1 agent.

A first series of experiments was conducted to determine whether specific targeting of MCL1 recapitulates sorafenib synergy with SQT-BH3 in cells *in vitro.* In these experiments, MCL1 was targeted using a commercially available siRNA (Dharmacon, ON-TARGETplus Human MCL1 (4170) siRNA - SMARTpool, # L-004501-00-0005). Western blotting experiments confirmed that treatment of Hep3B cells with this siRNA or with 10uM sorafenib for 24 hours downregulates MCL1, whereas treatment with a non-targeting control siRNA or with PBS did not downregulate MCL1.

Next, Hep3B cells and primary hepatocytes were treated with either SQT-PUMA-BH3 or SQT-Bad-BH3 in combination with either siMCL1 or sorafenib. Controls were an empty SQT construct ("SQT-dummy"), a non-targeting siRNA and PBS. For treatment of Hep3B cells, 20,000 Hep3B cells/well per plated in 96-well plates. At Day 1, some wells were transfected with siMCL1 (Dharmacon, ON-TARGETplus Human MCL1 (4170) siRNA - SMARTpool, # L-004501-00-0005) or non-targeting siRNA (ON-TARGETplus Non-targeting Control Pool, # D-001810-10-05). At Day 3, cells were treated with the 10uM sorafenib tosylate (Selleck Chemicals) or PBS, and transfected with SQT-dummy or SQT-PUMA or SQT-Bad. 24 hours after treatment, YOYO®-3 iodide (Life Technologies) viability dye was added to each well and cell death was measured on an IncuCyte ZOOM® using YOYO-3 fluorescence as a readout (="Apoptosis"). For treatment of primary hepatocytes, 18,000 mouse hepatocytes (Gibco) were seeded in each well of a 96-well plate. At Day 1, some wells were transfected with siMCL1 (Dharmacon, SMARTpool: ON-TARGETplus Mcl1 siRNA, # L-062229-00-0005) or non-targeting siRNA (ON-TARGETplus Non-targeting Control Pool, # D-001810-10-05). At Day 3, cells were treated with the 10uM sorafenib tosylate (Selleck Chemicals) or PBS, and transfected with SQT-dummy or SQT-PUMA or SQT-Bad. 24 hours after treatment, Caspase 3/7 reagent (EssenBio) apoptosis dye was added to each well and cell death was measured on an IncuCyte ZOOM® using the caspase 3/7 reagent fluorescence as a readout (="Apoptosis").

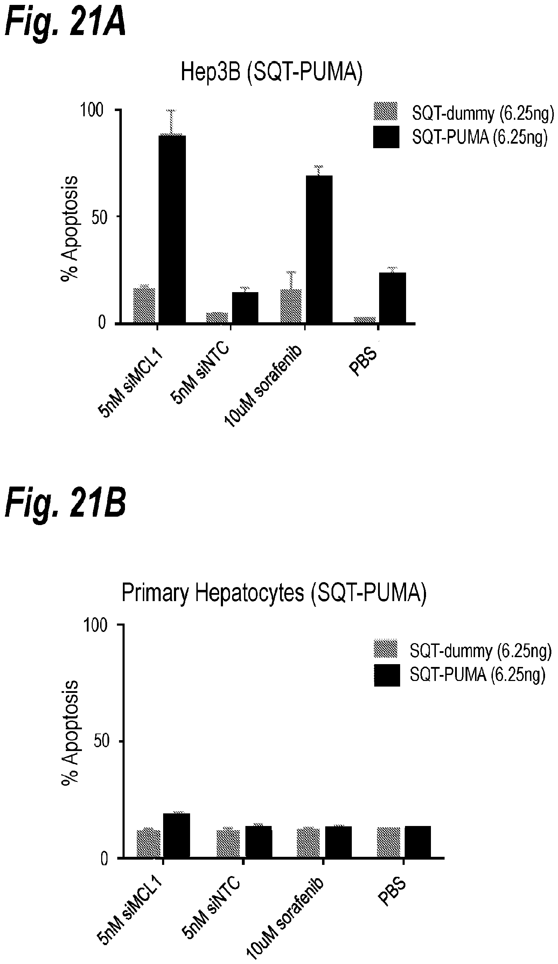

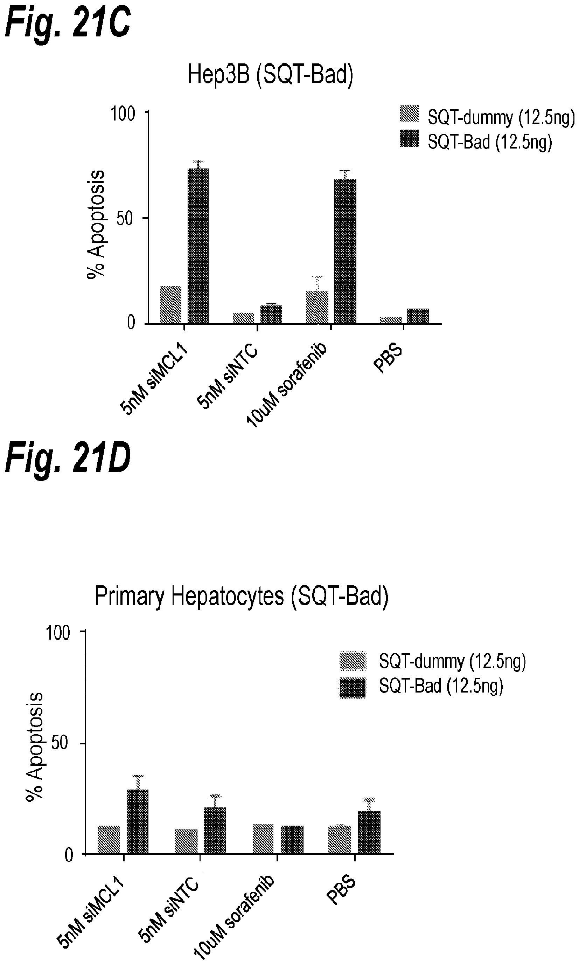

The results are shown in Figures 21A-D, wherein Figures 21A and 21C show the results for the Hep3B cells and Figures 21B and 21D show the results for the primary hepatocytes. Figures 21A and 21B show the results for treatment with SQT-PUMA-BH3. Figures 21C and 21D show the results for treatment with SQT-Bad-BH3. The results demonstrate that in primary hepatocytes, treatment with either SQT-PUMA-BH3 or SQT-Bad-BH3, in combination with either siMCL1, sorafenib or controls, did not lead to increased apoptosis as compared to treatment with the control SQT-dummy construct. In contrast, treatment of the Hep3B cells with either SQT-PUMA-BH3 or SQT-Bad-BH3, in combination with either siMCL1 or sorafenib, led to a marked increase in apoptosis as compared to treatment with the SQT-BH3 construct in combination with non-targeting siRNA or PBS. These results demonstrate that in the hepatocellular carcinoma cells (but not primary hepatocytes) specific targeting of MCL1 recapitulates the pro-apoptotic synergy previously observed with SQT-BH3 in combination with sorafenib.

Therefore, an anti-MCL1 mmRNA construct was designed. The amino acid sequence of the open reading frame (ORF) of the construct, and the corresponding nucleotide sequence encoding the amino acid sequence, is shown below in Table 17:

**Table 17:**

| **Name** | **ORF Amino Acid SEQ ID NO:** | **Nucleotide SEQ ID NO:** |
|---|---|---|
| SQT.(nt)Mcl1.BH3.cv5 | 108 | 112 |

Additionally, the construct contained 5' UTR having the sequence shown in SEQ ID NO: 72, a 3' UTR having the sequence shown in SEQ ID NO: 73, a Cap 15' Cap (7mG(5')ppp(5')NlmpNp), a poly A tail of 100 nucleotides and were fully modified with 1-methyl-pseudouridine (m1ψ). The mRNA sequence used in the constructs shown in Table 17 include an epitope tag coding sequence (v5) incorporated into the sequence. The open reading frame (ORF) amino acid sequence corresponding to SEQ ID NO: 108 but without the epitope tag is shown in SEQ ID NO: 110.

A naturally occurring isoform of the Mcl1 protein (i.e., MCL1-ES) with a v5 tag (MCL1-ES.cV5) was used as a control. The amino acid sequence corresponding to MCL1-ES.cV5 is shown in SEQ ID NO: 164, whereas the nucleotide sequence is shown in SEQ ID NO: 165.

The anti-MCL1 mmRNA construct was tested for synergistic killing when combined with SQT-PUMA-BH3 in Hep3B cells. 20,000 Hep3B cells were seeded per well in 96-well plates. Each well was transfected with (a) 1.6ng SQT-empty ("SQT-dummy") or SQT-PUMA-BH3; and (b) 50ng or 12.5ng anti-MCLl mRNA. Transfections of mRNA were done with Lipofectamine 2000 (L2K). After 48 hours, apoptosis measurements performed using YOYO-3 viability dye in an Incucyte instrument.

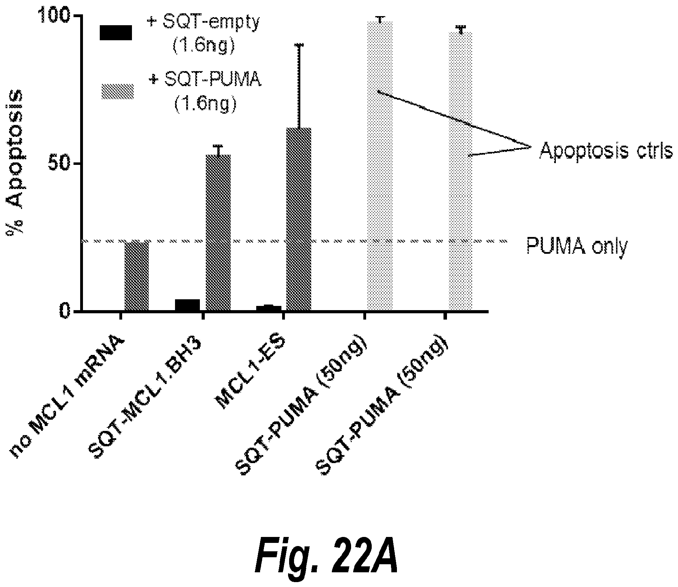

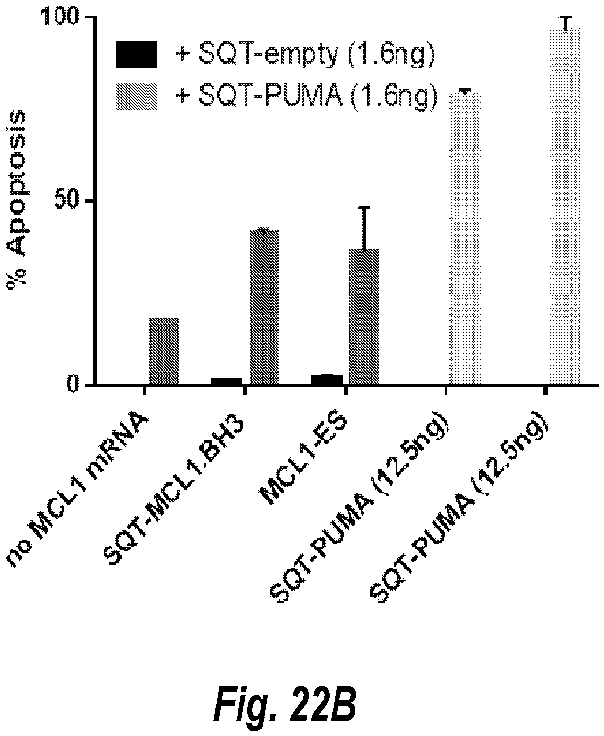

The results are shown in Figures 22A-B. Figure 22A shows the results for treatment with 50 ng anti-MCL1 mRNA; Figure 22B shows the results for treatment with 12.5 ng anti-MCL1 mRNA. The results demonstrate that at the 50 ng dosage, the anti-MCL1 construct tested demonstrated a synergistic anti-apoptotic effect in combination with SQT-PUMA-BH3, as compared to treatment with SQT-PUMA-BH3 alone.

### EXAMPLE 16

### SQT-p53-INHIBITORY PEPTIDE CONSTRUCTS

In this example, SQT scaffold constructs were made that contained a p53-inhibitory peptide inserted in the N-terminus, Loop 1 or Loop 2. The p53-inhibitory peptide constructs used peptides that inhibit the interaction of p53 with MDM2. Thus, these constructs are also called SQT-MDM2 constructs herein. Inhibition of the p53/MDM2 interaction activates the tumor suppressor activity of p53 and decreases p53 degradation. Accordingly, the SQT-MDM2 constructs described herein can be used for tumor suppression (e.g., in the treatment of cancer).

The amino acid sequences of the p53 inhibitory peptides used in the SQT-MDM2 constructs are shown in Table 8 and in SEQ ID NOs: 130-141. The open reading frame (ORF) amino acid sequences and ORF nucleotide sequences of representative SQT-p53-inhibitory peptide fusion proteins are shown the Sequence Listing in the SEQ ID NOs summarized in Table 18 below.

**Table 18:**

| **#** | **Construct Name and Description** | **ORF a.a. SEQ ID NO:** | **ORF nt. SEQ ID NO:** |
|---|---|---|---|
| 1 | SQT(MDM2)(nt).P3848.cV5 (P3848 peptide in N-terminus of SQT) | 142 | 149 |
| 2 | SQT(MDM2)(nt).P7041.cV5 (P7041 peptide in N-terminus of SQT) | 143 | 150 |
| 3 | SQT(MDM2)(nt).pDIQ.cV5 (pDIQ peptide in N-terminus of SQT) | 144 | 151 |
| 4 | SQT(MDM2)(nt).pMI.cV5 (pMI peptide in N-terminus of SQT) | 145 | 152 |
| 5 | SQT(MDM2)(L2).P3848.cV5 (P3848 peptide in Loop 2 of SQT) | 146 | 153 |
| 6 | SQT(MDM2)(L2).P1E6W.cV5 (P1E6W peptide in Loop 2 of SQT) | 147 | 154 |
| 7 | SQT(MDM2)(nt).P6W.cV5 (P6W peptide in N-terminus of SQT) | 148 | 155 |

The open reading frame for each construct also included a V5 tag at the C-terminus, the sequence of which is not shown in the SEQ ID NOs summarized above in Table 18. The amino acid sequence of the V5 tag is GKPIPNPLLGLDST (SEQ ID NO: 156). Additionally, all mRNA constructs contained a 5'UTR, a 3' UTR, a Cap 1 5' Cap (7mG(5')ppp(5')NlmpNp), a poly A tail of about 100 nucleotides and were fully modified with 1-methyl-pseudouridine (m1ψ). The full mRNA sequences for constructs 1-7 shown in Table 18 (including the V5 tag) are shown in SEQ ID NOs: 157-163.

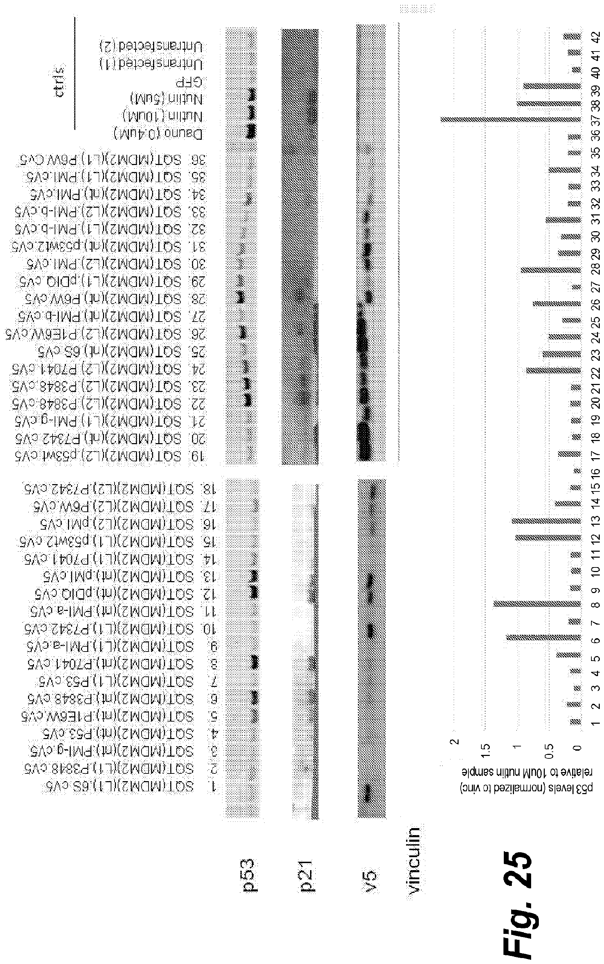

To examine the ability of the fusion proteins encoded by the SQT-MDM2 constructs to activate p53, the mRNA constructs were transfected into HCT116 cells and the levels of p53 and p21 in the cells were assayed (as a measure of the disruption of p53/MDM2 interaction by the SQT-p53-inhibitory peptide fusion proteins). A known activator of p53, nutlin-3a, was used as positive controls. The results are shown in Figure 25, which shows photographs of Western blots showing levels of p53 and p21 in the treated cells, as well as levels of the V5 tag (as an indicator of construct levels) and vinculin (as a loading control). The results are summarized in the bar graph in Figure 25, which shows p53 levels (normalized to vinculin) relative to the 10 µM nutlin-3a sample. The results demonstrate that the SQT-MDM2 constructs shown in Table 18 (samples 6, 8, 12, 13, 22, 26 and 28 in Figure 25) activated p53 at levels similar to the nutlin-3a positive control.

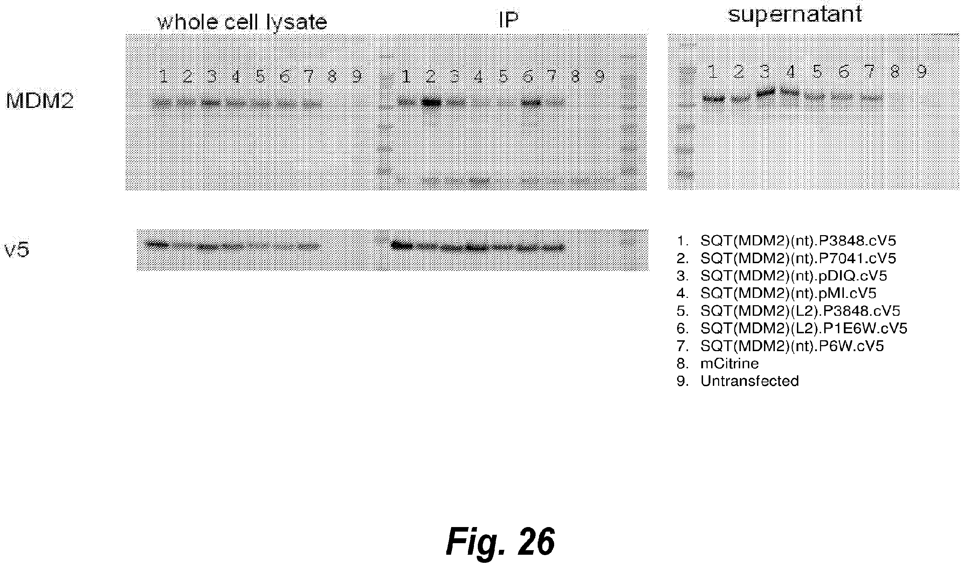

To determine whether the fusion protein encoded by the SQT-MDM2 constructs bound MDM2, an immunoprecipitation experiment was conducted in which HCT116 cells were transfected with the SQT-MDM2 mRNA constructs, lysates were prepared after 6 hours, the lysates were immunoprecipitated using a anti-V5 tag antibody and the immunoprecipation membranes were probed with anti-MDM2 antibody (as well as anti-V5 antibody as a control). The initial results, shown in Figure 26, indicate that the fusion proteins encoded by the SQT-MDM2 mRNA constructs (each of which contains an MDM2-binding peptide) does bind MDM2.

### SEQUENCE LISTING SUMMARY

| SEQ ID NO: | SEQUENCE |
|---|---|
| 1 | LALRLACIGDEMDVS (BH3 domain amino acid sequence) |
| 2 | EKAELLQGGDLLRQR (BH3 domain amino acid sequence) |
| 3 | VESILKKNSDWIWDW (BH3 domain amino acid sequence) |
| 4 | EVEALKKSADWVSDW (BH3 domain amino acid sequence) |
| 5 | TAARLKALGDELHQR (BH3 domain amino acid sequence) |
| 6 | IAQELRRIGDEFNAY (BH3 domain amino acid sequence) |
| 7 | YGRELRRMSDEFVDS (BH3 domain amino acid sequence) |
| 8 | IARHLAQVGDSMDRS (BH3 domain amino acid sequence) |
| 9 | IARKLQCIADQFHRL (BH3 domain amino acid sequence) |
| 10 | CATQLRRFGDKLNFR (BH3 domain amino acid sequence) |
| 11 | IGAQLRRMADDLNAQ (BH3 domain amino acid sequence) |
| 12 | LSRRLKVTGDLFDIM (BH3 domain amino acid sequence) |
| 13 | IVELLKYSGDQLERK (BH3 domain amino acid sequence) |
| 14 | ALETLRRVGDGVQRN (BH3 domain amino acid sequence) |
| 15 | IGSKLAAMCDDFDAQ (BH3 domain amino acid sequence) |
| 16 | IGRKLTVMCDEFDSE (BH3 domain amino acid sequence) |
| 17 | NIRRLRALADGVQKV (BH3 domain amino acid sequence) |
| 18 | NIDKLRALADDIDKT (BH3 domain amino acid sequence) |
| 19 | MVTLLPIEGQEIHFF (BH3 domain amino acid sequence) |
| 20 | PTVPLPSETDGYVAP (BH3 domain amino acid sequence) |
| 21 | PQRYLVIQGDDRMKL (BH3 domain amino acid sequence) |
| 22 | TVGELSRALGHENGS (BH3 domain amino acid sequence) |
| 23 | VGQLLQDMGDDVYQQ (BH3 domain amino acid sequence) |
| 24 | LHEVLNGLLDRPDWE (BH3 domain amino acid sequence) |
| 25 | AVHSLSRIGDELYLE (BH3 domain amino acid sequence) |
| 26 | NPKFLKNAGRDCSRR (BH3 domain amino acid sequence) |
| 27 | EEQWAREIGAQLRRMADDLNAQYERR (BH3 domain of PUMA amino acid sequence) |
| 28 | DMRPEIWIAQELRRIGDEFNAYYARR (BH3 domain of BIM amino acid sequence) |
| 29 | NLWAAQRYGRELRRMSDEFVDSFKKG (BH3 domain of Bad amino acid sequence) |
| 30 | PAELEVECATQLRRFGKLNFRQKLL (BH3 domain of Noxa amino acid sequence) |
| 31 | UAUUUAGUGUGAUAAUGGCGUU (miR-122-3p sequence) |
| 32 | CAAACACCAUUGUCACACUCCA (miR-122-5p sequence) |
| 33 | UAGCUUAUCAGACUGAUGUUGA (miR-21-5p sequence) |
| 34 | CAACACCAGUCGAUGGGCUGU (miR-21-3p sequence) |
| 35 | GSGATNFSLLKQAGDVEENPGP (2A peptide amino acid sequence) |
| 36 | GGAAGCGGAGCTACTAACTTCAGCCTGCTGAAGCAGGCTGGAGACGTGGAGGAG AACCCTGGACCT (2A peptide encoding sequence) |
| 37 | TCCGGACTCAGATCCGGGGATCTCAAAATTGTCGCTCCTGTCAAACAAACTCTTAA CTTTGATTTACTCAAACTGGCTGGGGATGTAGAAAGCAATCCAGGTCCACTC |
| | (2A peptide encoding sequence) |
| 38 | MAHAGRTGYDNREIVMKYIHYKLSQRGYEWDAGDVGAAPPGAAPAP GIFSSQPGHTPHPAASRDPVARTSPLQTPAAPGAAAGPALSPVPPVVHL TLRQAGDDFSRRYRRDFAEMSSQLHLTPFTARGRFATVVEELFRDGVN WGRIVAFFEFGGVMCVESVNREMSPLVDNIALWMTEYLNRHLHTWIQ DNGGWDAFVELYGPSMRPLFDFSWLSLKTLLSLALVGACITLGAYLGHK |
| | (Human Bcl-2 amino acid sequence, Genbank NP_000624) |
| 39 | MSQSNRELVVDFLSYKLSQKGYSWSQFSDVEENRTEAPEGTESEMETPSA INGNPSWHLADSPAVNGATGHSSSLDAREVIPMAAVKQALREAGDEFELR YRRAFSDLTSQLHITPGTAYQSFEQVVNELFRDGVNWGRIVAFFSFGGAL CVESVDKEMQVLVSRIAAWMATYLNDHLEPWIQENGGWDTFVELYGNN AAAESRKGQERFNRWFLTGMTVAGVVLLGSLFSRK |
| | (Human Bcl-X_{L} amino acid sequence, Genbank NP_612815.1) |
| 40 | MATPASAPDTRALVADFVGYKLRQKGYVCGAGPGEGPAADPLHQAMRA AGDEFETRFRRTFSDLAAQLHVTPGSAQQRFTQVSDELFQGGPNWGRLVA FFVFGAALCAESVNKEMEPLVGQVQEWMVAYLETRLADWIHSSGGWAEF TALYGDGALEEARRLREGNWASVRTVLTGAVALGALVTVGAFFASK |
| | (Human Bcl-w amino acid sequence, Genbank NP_004041.1) |
| 41 | MFGLKRNAVIGLNLYCGGAGLGAGSGGATRPGGRLLATEKEASARREIGG GEAGAVIGGSAGASPPSTLTPDSRRVARPPPIGAEVPDVTATPARLLFFAPTR RAAPLEEMEAPAADAIMSPEEELDGYEPEPLGKRPAVLPLLELVGESGNNT STDGSLPSTPPPAEEEEDELYRQSLEIISRYLREQATGAKDTKPMGRSGATSR KALETLRRVGDGVQRNHETAFQGMLRKLDIKNEDDVKSLSRVMIHVFSDGV TNWGRIVTLISFGAFVAKHLKTINQESCIEPLAESITDVLVRTKRDWLVKQRG WDGFVEFFHVEDLEGGIRNVLLAFAGVAG VGAGLAYLIR |
| | (Human Mcl-1 amino acid sequence, Genbank NP_068779.1) |
| 42 | MTDCEFGYIYRLAQDYLQCVLQIPQPGSGPSKTSRVLQNVAFSVQKEVEK NLKSCLDNVNVVSVDTARTLFNQVMEKEFEDGIINWGRIVTIFAFEGILIKK LLRQQIAPDVDTYKEISYFVAEFIMNNTGEWIRQNGGWENGFVKKFEPKSG WMTFLEVTGKICEMLSLLKQYC |
| | (Human Bcl-related protein A1 amino acid sequence, Genbank NP_004040.1) |
| 43 | ATGATACCCAGACAAGAGGACATCATTCGTAACATCGCGCGACACCTGGCCCAGG TGGGGGACTCCATGGATCGATCAATCCCCCCTGGCTTATCCGAAGCTAAGCCAGCC ACCCCTGAGATCCAAGAAATCGTCGACAAGGTCAAGCCCCAACTTGAAGAGAAAA CTAATGAAACTTATGGTAAGCTCGAAGCCGTGCAATACAAGACTCAGGTCCTGGCG TCTACAAATTATTACATCAAGGTGAGGGCCGGGGACAATAAGTATATGCATCTGAA AGTCTTCAATGGCCCCCCTGGACAAAACGCAGATCGGGTGCTCACAGGATACCAGG TCGATAAAAATAAAGATGACGAACTTACTGGGTTTGATTATAAAGATGATGATGAC AAG |
| | (SQT(nt)BID-BH3 mRNA sequence with FLAG epitope tag) |
| 44 | ATGATTCCTCGGGGTTTGTCAGAGGCAAAGCCAGCCACCCCAGAAATACAGGAAAT CGTAGACAAAGTGAAGCCCCAGCTTGAGGAAAAAACTAACGAGACCTACGGGAAG CTGGAAGCTGTGCAGTACAAGACCCAGGTGCTACAGGAGGACATCATTAGGAATAT TGCGAGACATCTGGCGCAAGTGGGTGACTCTATGGATCGTTCCATTCCACCGGGTGC AAGCACCAATTATTACATCAAAGTGCGTGCTGGAGACAACAAATATATGCACCTCA AGGTTTTTAACGGGCCGCCAGGACAGAATGCAGACCGTGTGCTTACCGGGTATCAG GTAGACAAAAATAAAGATGACGAGCTGACTGGCTTTGACTACAAGGATGACGATGA CAAG |
| | (SQT(L1)BID-BH3 mRNA sequence with FLAG epitope tag) |
| 45 | ATGATCCCCAGGGGACTCTCCGAGGCAAAGCCAGCCACACCTGAAATTCAGGAAATC GTTGATAAAGTAAAGCCACAGCTTGAGGAAAAAACCAATGAGACGTACGGCAAACTC GAAGCCGTGCAGTACAAGACCCAGGTATTAGCCTCCACCAACTACTACATAAAGGTG CGGGCCGGCGACAACAAGTACATGCATCTGAAAGTATTCAACGGTCCCCAGGAAGAC ATTATAAGGAACATTGCCCGCCATCTTGCTCAGGTGGGCGATTCCATGGACCGTAGCA TACCTCCTGGGCAGAACGCCGATCGCGTACTCACGGGCTATCAAGTCGATAAAAACAA GGATGATGAATTGACTGGGTTTGATTACAAAGATGATGACGATAAG |
| | (SQT(L2)BID-BH3 mRNA sequence with FLAG epitope tag) |
| 46 | ATGATACCCAGACAAGAGGACATCATTCGTAACATCGCGACACCTGGCCCAGGTGG GGGACTCCATGGATCGATCAATCCCCCCTGGCTTATCCGAAGCTAAGCCAGCCACCCCT GAGATCCAAGAAATCGTCGACAAGGTCAAGCCCCAACTTGAAGAGAAAACTAATGAAA CTTATGGTAAGCTCGAAGCCGTGCAATACAAGACTCAGGTCCTGGCGTCTACAAATTAT TACATCAAGGTGAGGGCCGGGGACAATAAGTATATGCATCTGAAAGTCTTCAATGGCC CCCCTGGACAAAACGCAGATCGGGTGCTCACAGGATACCAGGTCGATAAAAATAAAGA TGACGAACTTACTGGGTTT |
| | (SQT(nt)BID-BH3 mRNA sequence without epitope tag) |
| 47 | ATGATTCCTCGGGGTTTGTCAGAGGCAAAGCCAGCCACCCCAGAAATACAGGAAATCGT AGACAAAGTGAAGCCCCAGCTTGAGGAAAAAACTAACGAGACCTACGGAAGCTGGAA |
| | GCTGTGCAGTACAAGACCCAGGTGCTACAGGAGGACATCATTAGGAATATTGCGAGACA TCTGGCGCAAGTGGGTGACTCTATGGATCGTTCCATTCCACCGGGTGCAAGCACCAATTA TTACATCAAAGTGCGTGCTGGAGACAACAAATATATGCACCTCAAGGTTTTTAACGGGCC GCCAGGACAGAATGCAGACCGTGTGCTTACCGGGTATCAGGTAGACAAAAATAAAGATG ACGAGCTGACTGGCTTT |
| | (SQT(L1)BID-BH3 mRNA sequence without epitope tag) |
| 48 | ATGATCCCCAGGGGACTCTCCGAGGCAAAGCCAGCCACACCTGAAATTCAGGAAATCGT TGATAAAGTAAAGCCACAGCTTGAGGAAAAAACCAATGAGACGTACGGCAAACTCGAA GCCGTGCAGTACAAGACCCAGGTATTAGCCTCCACCAACTACTACATAAAGGTGCGGGC CGGCGACAACAAGTACATGCATCTGAAAGTATTCAACGGTCCCCAGGAAGACATTATAA GGAACATTGCCCGCCATCTTGCTCAGGTGGGCGATTCCATGGACCGTAGCATACCTCCTG GGCAGAACGCCGATCGCGTACTCACGGGCTATCAAGTCGATAAAAACAAGGATGATGAA TTGACTGGGTTT |
| | (SQT(L2)BID-BH3 mRNA sequence without epitope tag) |
| 49 | ATGATCCCAAGAGAAGAACAGTGGGCACGCGAAATTGGAGCTCAACTCAGACGGATGGCCGACGACCTG AACGCCCAGTACGAGCGGCGGGACTCTCAGAAGCGAAGCCTGCCACCCCCGAAATCCAGGAAATCGTCG ACAAGGTCAAGCCGCAGCTTGAGGAAAAGACCAACGAGACTTACGGAAAACTGGAGGCCGTGCAGTATAA GACCCAAGTGTTGGCCTCCACCAACTACTACATTAAGGTCCGCGCCGGCGATAACAAGTACATGCACCTGAA GGTGTTCAATGGTCCTCCGGGACAGAACGCGGACAGGGTGCTGACTGGCTACCAAGTGGACAAGAACAAG GACGACGAGCTGACGGGCTTCGACTACAAGGATGATGATGACAAA |
| | (SQT-PUMA-BH3 mRNA sequence with FLAG epitope tag) |
| 50 | ATGATCCCAAGAGACATGCGCCCCGAAATTTGGATCGCCCAGGAGCTCAGACGCATCGGAGATGAATTCAA CGCATACTACGCTCGGCGGGGACTCTCAGAAGCGAAGCCTGCCACCCCCGAAATCCAGGAAATCGTCGACA AGGTCAAGCCGCAGCTTGAGGAAAAGACCAACGAGACTTACGGAAAACTGGAGGCCGTGCAGTATAAGAC CCAAGTGTTGGCCTCCACCAACTACTACATTAAGGTCCGCGCCGGCGATAACAAGTACATGCACCTGAAGGT GTTCAATGGTCCTCCGGGACAGAACGCGGACAGGGTGCTGACTGGCTACCAAGTGGACAAGAACAAGGAC GACGAGCTGACGGGCTTCGACTACAAGGATGATGATGACAAA |
| | (SQT-BIM-BH3 mRNA sequence with FLAG epitope tag) |
| 51 | ATGATCCCAAGAAACCTGTGGGCTGCACAACGCTACGGAAGAGAACTGCGCCGGATGAGCGACGAATTTGT GGACTCGTTCAAGAAGGGGGGACTCTCAGAAGCGAAGCCTGCCACCCCCGAAATCCAGGAAATCGTCGACA AGGTCAAGCCGCAGCTTGAGGAAAAGACCAACGAGACTTACGGAAAACTGGAGGCCGTGCAGTATAAGACC CAAGTGTTGGCCTCCACCAACTACTACATTAAGGTCCGCGCCGGCGATAACAAGTACATGCACCTGAAGGTGT TCAATGGTCCTCCGGGACAGAACGCGGACAGGGTGCTGACTGGCTACCAAGTGGACAAGAACAAGGACGAC GAGCTGACGGGCTTCGACTACAAGGATGATGATGACAAA |
| | (SQT-BAD-BH3 mRNA sequence with FLAG epitope tag) |
| 52 | ATGATCCCAAGACCCGCAGAGCTCGAAGTGGAATGCGCTACCCAACTTAGACGGTTCGGAGACAAGCTGAA CTTTCGGCAGAAGCTCCTGGGACTCTCAGAAGCGAAGCCTGCCACCCCCGAAATCCAGGAAATCGTCGACAA GGTCAAGCCGCAGCTTGAGGAAAAGACCAACGAGACTTACGGAAAACTGGAGGCCGTGCAGTATAAGACCC AAGTGTTGGCCTCCACCAACTACTACATTAAGGTCCGCGCCGGCGATAACAAGTACATGCACCTGAAGGTGTT CAATGGTCCTCCGGGACAGAACGCGGACAGGGTGCTGACTGGCTACCAAGTGGACAAGAACAAGGACGA CGAGCTGACGGGCTTCGACTACAAGGATGATGATGACAAA |
| | (SQT-NoxA-BH3 mRNA sequence with FLAG epitope tag) |
| 53 | MIPGGLSEAKPATPEIQEIVDKVKPQLEEKTNETYGKLEAVQYKTQVVAGTNYYIK VRAGDNKYMHLKVFKSLPGQNEDLVLTGYQVDKNKDDELTGF (Wild type SteA scaffold amino acid sequence) |
| | (Wild-type SteA scaffold amino acid sequence) |
| 54 | MIPWGLSEAKPATPEIQEIVDKVKPQLEEKTNETYGKLEAVQYKTQVDAGTNYYIK VRAGDNKYMHLKVFNGPPGQNEDLVLTGYQVDKNKDDELTGF |
| | (SteA Triple Mutant (STM) scaffold amino acid sequence) |
| 55 | MIPGGLSEAKPATPEIQEIVDKVKPQLEEKTNETYGKLEAVQYKTQVLASTNYYIK VRAGDNKYMHLKVFNGPPGQNEDLVRSGYQVDKNKDDELTGF |
| | (SteA Double Mutant (SDM) scaffold amino acid sequence) |
| 56 | MIPRGLSEAKPATPEIQEIVDKVKPQLEEKTNETYGKLEAVQYKTQVLASTNYYIK VRAGDNKYMHLKVFNGPPGQNEDLVRSGYQVDKNKDDELTGF |
| | (SteA Quadruple Mutant (SQM) scaffold amino acid sequence) |
| 57 | MIPGGLSEAKPATPEIQEIVDKVKPQLEEKTNETYGKLEAVQYKTQVVAGTNYYIK VRAGDNKYMHLKVF NGPPGQNEDLVRSGYQVDKNKDDELTGF |
| | (SteA Unique C-Terminus (SUC) scaffold amino acid sequence) |
| 58 | MIPGGLSEAKPATPEIQEIVDKVKPQLEEKTNETYGKLEAVQYKTQVLASTNYYIK |
| | VRAGDNKYMHLKVFKSLPGQNEDLVLTGYQVDKNKDDELTGF |
| | (SteA Unique Middle (SUM) scaffold amino acid sequence) |
| 59 | MIPRGLSEAKPATPEIQEIVDKVKPQLEEKTNETYGKLEAVQYKTQVVAGTNYYIK VRAGDNKYMHLKVFKSLPGQNEDLVLTGYQVDKNKDDELTGF |
| | (SteA Unique N-Terminus (SUN) scaffold amino acid sequence) |
| 60 | MIPGGLSEAKPATPEIQEIVDKVKPQLEEKTNETYGKLEAVQYKTQVLASTNYYIK VRAGDNKYMHLKVFNGPPGQNEDLVRS |
| | (SteA SDM- scaffold amino acid sequence) |
| 61 | MIPGGLSEAKPATPEIQEIVDKVKPQLEEKTNETYGKLEAVQYKTQVLASTNYYIK VRAGDNKYMHLKVFNGP |
| | (SteA SMD-- scaffold amino acid sequence) |
| 62 | MIPRGLSEAKPATPEIQEIVDKVKPQLEEKTNETYGKLEAVQYKTQVLASTNYYIK VRAGDNKYMHLKVFNGPPGQNEDLVRS |
| | (SteA SQM- scaffold amino acid sequence) |
| 63 | MIPRGLSEAKPATPEIQEIVDKVKPQLEEKTNETYGKLEAVQYKTQVLASTNYYIK VRAGDNKYMHLKVFNGP |
| | (SteA SQM-- scaffold amino acid sequence) |
| 64 | MIPGGLSEAKPATPEIQEIVDKVKPQLEEKTNETYGKLEAVQYKTQVVAGTNYYIK VRAGDNKYMHLKVFNGPPGQNEDLVRS |
| | (SteA SUC- scaffold amino acid sequence) |
| 65 | MIPGGLSEAKPATPEIQEIVDKVKPQLEEKTNETYGKLEAVQYKTQVVAGTNYYIK VRAGDNKYMHLKVFNGP |
| | (SteA SUC-- scaffold amino acid sequence) |
| 66 | MIPGGLSEAKPATPEIQEIVDKVKPQLEEKTNETYGKLEAVQYKTQVLASTNYYIK VRAGDNKYMHLKVFKSLPGQNEDLVLT |
| | (SteA SUM- scaffold amino acid sequence) |
| 67 | MIPGGLSEAKPATPEIQEIVDKVKPQLEEKTNETYGKLEAVQYKTQVLASTNYYIK VRAGDNKYMHLKVFKSL |
| | (SteA SUM-- scaffold amino acid sequence) |
| 68 | MIPRGLSEAKPATPEIQEIVDKVKPQLEEKTNETYGKLEAVQYKTQVVAGTNYYIK VRAGDNKYMHLKVFKSLPGQNEDLVLT |
| | (SteA SUN- scaffold amino acid sequence) |
| 69 | MIPRGLSEAKPATPEIQEIVDKVKPQLEEKTNETYGKLEAVQYKTQVVAGTNYYIK VRAGDNKYMHLKVFKSL |
| | (SteA SUN-- scaffold amino acid sequence) |
| 70 | MIPRGLSEAKPATPEIQEIVDKVKPQLEEKTNETYGKLEAVQYKTQVLASTNYYIK VRAGDNKYMHLKVFNGPPGQNADRVLTGYQVDKNKDDELTGF |
| | (SteA SQT scaffold amino acid sequence) |
| 71 | MIPRGLSEAKPATPEIQEIVDKVKPQLEEKTNETYGKLEAVQYKTQVLALASTNYYIK VRAGDNKYMHLKVFNGPPGQNADRVLTGYQVDKNKDDELTGF |
| | (SteA SQL scaffold amino acid sequence) |
| 72 | TCAAGCTTTTGGACCCTCGTACAGAAGCTAATACGACTCACTATAGGGAAATAAGAGA GAAAAGAAGAGTAAGAAGAAATATAAGAGCCACC (5' UTR) |
| 73 | TGATAATAGGCTGGAGCCTCGGTGGCCATGCTTCTTGCCCCTTGGGCCTCCCCCCAGCC CCTCCTCCCCTTCCTGCACCCGTACCCCCGTGGTCTTTGAATAAAGTCTGAGTGGGCGGC (3' UTR) |
| 74 | ATGATCCCAAGAGAAGAACAGTGGGCACGCGAAATTGGAGCTCAACTCAGACGGATG GCCGACGACCTGAACGCCCAGTACGAGCGGCGGGACTCTCAGAAGCGAAGCCTGCC ACCCCCGAAATCCAGGAAATCGTCGACAAGGTCAAGCCGCAGCTTGAGGAAAAGACC AACGAGACTTACGGAAAACTGGAGGCCGTGCAGTATAAGACCCAAGTGTTGGCCTCCA CCAACTACTACATTAAGGTCCGCGCCGGCGATAACAAGTACATGCACCTGAAGGTGTTC AATGGTCCTCCGGGACAGAACGCGGACAGGGTGCTGACTGGCTACCAAGTGGACAAGA ACAAGGACGACGAGCTGACGGGCTTC |
| | (SQT-PUMA-BH3 mRNA sequence without epitope tag) |
| 75 | ATGATCCCAAGAGACATGCGCCCGAAATTTGGATCGCCCAGGAGCTCAGACGCATCGG AGATGAATTCAACGCATACTACGCTCGGCGGGACTCTCAGAAGCGAAGCCTGCCACCC CCGAAATCCAGGAAATCGTCGACAAGGTCAAGCCGCAGCTTGAGGAAAAGACCAACGA |
| | GACTTACGGAAAACTGGAGGCCGTGCAGTATAAGACCCAAGTGTTGGCCTCCACCAACT ACTACATTAAGGTCCGCGCCGGCGATAACAAGTACATGCACCTGAAGGTGTTCAATGGT CCTCCGGGACAGAACGCGGACAGGGTGCTGACTGGCTACCAAGTGGACAAGAACAAGG ACGACGAGCTGACGGGCTTC |
| | (SQT-BIM-BH3 mRNA sequence without epitope tag) |
| 76 | ATGATCCCAAGAAACCTGTGGGCTGCACAACGCTACGGAAGAGAACTGCGCCGGATGAG CGACGAATTTGTGGACTCGTTCAAGAAGGGGGGACTCTCAGAAGCGAAGCCTGCCACCC CCGAAATCCAGGAAATCGTCGACAAGGTCAAGCCGCAGCTTGAGGAAAAGACCAACGA GACTTACGGAAAACTGGAGGCCGTGCAGTATAAGACCCAAGTGTTGGCCTCCACCAACT ACTACATTAAGGTCCGCGCCGGCGATAACAAGTACATGCACCTGAAGGTGTTCAATGGT CCTCCGGGACAGAACGCGGACAGGGTGCTGACTGGCTACCAAGTGGACAAGAACAAGG ACGACGAGCTGACGGGCTTC |
| | (SQT-BAD-BH3 mRNA sequence without epitope tag) |
| 77 | ATGATCCCAAGACCCGCAGAGCTCGAAGTGGAATGCGCTACCCAACTTAGACGGTTCGGAGACAAGCTGAAC TTTCGGCAGAAGCTCCTGGGACTCTCAGAAGCGAAGCCTGCCACCCCCGAAATCCAGGAAATCGTCGACAAG GTCAAGCCGCAGCTTGAGGAAAAGACCAACGAGACTTACGGAAAACTGGAGGCCGTGCAGTATAAGACCCA AGTGTTGGCCTCCACCAACTACTACATTAAGGTCCGCGCCGGCGATAACAAGTACATGCACCTGAAGGTGTT CAATGGTCCTCCGGGACAGAACGCGGACAGGGTGCTGACTGGCTACCAAGTGGACAAGAACAAGGACGAC GAGCTGACGGGCTTC |
| | (SQT-NoxA-BH3 mRNA sequence without epitope tag) |
| 78 | ATGATTCCTAATCCTCTCTTAGGGCTGGACGCAACACCCGCGTCCGCACCCGATACAAG AGCACTGGTTGCAGACTTCGTGGCTACAAACTGAGGCAGAAGGGTACGTCTGTGGT GCGGGCCCCGGTGAAGGGCCTGCCGCCGACCCTCTGCACCAGGCTATGCGAGCTGCAG GGGACGAGTTTGAGACACGCTTTAGACGTACATTTTCTGATTTGGCGGCTCAGCTCCAC GTCACCCCTGGGAGCGCTCAGCAGCGATTTACCCAAGTAAGCGACGAGCTCTTTCAAGG AGGCCCCAATTGGGGCCGGCTGGTCGCGTTCTTTGTGTTTGGGGCCGCATTATGCGCGGA AAGCGTTAACAAAGAGATGGAACCCTTAGTAGGACAAGTCCAGGAATGGATGGTGGCCT ACCTAGAGACACAGCTAGCTGACTGGATTCATTCATCAGGGGGCTGGGCCGAGTTTACTG CCTTATATGGGGATGGGGCACTCGAAGAAGCCAGGAGACTTCGCGAAGGAATTGGGCA TCAGTGAGGACTGTGCTAACTGGAGCCGTCGCCCTGGGTGCCCTGGTTACCGTGGGTGCT TTCTTTGCGTCTAAA |
| | (Bcl-w wild-type mRNA sequence with V5 epitope tag) |
| 79 | ATGATTCCTAACCCCCTACTAGGACTTGACTCTCAGAGCAACAGAGAATTGGTAGTGGAT TTTCTGAGTTATAAACTATCGCAGAAAGGCTACTCTTGGTCACAGTTCTCAGATGTTGAGG AGAACAGGACTGAAGCACCAGAGGGGACAGAGTCTGAGATGGAGACCCCCAGTGCTATA AACGGTAACCCCTCCTGGCACCTAGCCGATAGCCCCGCCGTGAACGGAGCCACCGGACAC AGCAGCTCCTTAGATGCCCGCGAGGTGATTCCGATGGCCGCAGTAAAGCAGGCTCTCCGA GAAGCCGGCGATGAATTTGAGCTTAGGTATAGGAGGGCCTTCTCTGATCTTACTTCACAG CTTCACATCACACCTGGCACAGCTTATCAGAGCTTCGAACAGGTGGTGAATGAACTTTTTC GAGATGGCGTAAACTGGGGCCGCATCGTGGCCTTCTTTAGCTTCGGTGGGGCCCTGTGCGT CGAATCAGTGGACAAAGAAATGCAAGTCCTGGTGAGCCGAATCGCAGCCTGGATGGCCAC GTACTTGAACGATCACCTGGAGCCTTGGATCCAAGAGAACGGCGGATGGGACACTTTCGTC GAGTTGTATGGCAATAATGCCGCTGCCGAAAGTCGGAAAGGCAGGAGCGATTTAACCGC TGGTTCCTTACAGGCATGACTGTTGCTGGGGTGGTGCTCTTGGGTTCTCTCTTCAGCCGCAA A |
| | (Bcl-xL wild-type mRNA sequence with V5 epitope tag) |
| 80 | ATGGGACCACTCGGGTCGGAGGATGACCTGTATCGCCAATCCCTGGAAATCATCTCTAG GTACCTGAGGGAGCAGGCCACCGGCTCGAAAGATAGTAAACCACTTGGCGAAGCCGGA GCTGCTGGCAGAAGGGCTCTCGAGACACTCCGGCGCGTGGGTGATGGAGTGCAACGCA ATCACGAGACTGCCTTCCAGGGCATGCTCAGGAAGCTGGATATCAAAAACGAGGATGA TGTTAAGAGTCTCTCTCGAGTCATGATTCATGTGTTTTCCGACGGTGTCACTAACTGGGG TAGAATTGTTACTCTGATAAGTTTTGGAGCATTCGTTGCCAAGCACTTGAAGACAATTAA CCAGGAGTCGTGCATCGAGCCCCTAGCGGAAAGCATCACAGATGTTCTCGTGCGAACCA AGCGCGATTGGCTGGTCAAGCAGAGGGGATGGGACGGGTTTGTTGAGTTTTTCCACGTC GAGGATCTGGAAGGGGGGGACTACAAGGATGATGACGACAAG |
| | (Mcl-1 del.N/C mRNA sequence with FLAG epitope tag) |
| 81 | ATGATTCCCAATCCGCTCCTCGGGCTTGACGCTACACCAGCCAGTGCACCAGACACGAG GGCGCTGGTTGCCGATTTCGTGGATACAAGCTCAGGCAAAAAGGGTACGTAAGCGGT GCCGGGCCCGGGGAGGGACCGGCTGCCGATCCACTCCACCAGGCTATGAGGGCAGCTG |
| | GGGACGAATTCGAAACACGCTTTCGGCGGACTTTCTCCGACCTTGCCGCACAGCTACAC GTCACTCCCGGGAGCGCTCAGCAGCGCTTCACGCAGGTCTCAGACGAATTGTTCCAAGG CGGCCCTAATTGGGGAAGATTGGTGGCCTTCTTCGTGTTTGGGGCAGCACTGTGCGCTGA GTCCGTGAACAAAGAAATGGAACCCCTCGTTGGCCAAGTCCAAGAATGGATGGTGGAAT ATCTGGAAACACAGTTAGCGGATTGGATTCACTCATCTGGGGGCTGGGCTGAGTTTACAG CACTGTACGGGGACGGTGCCCTAGAGGAGGCCCGAAGACTGAGGGAAGGCAACTGGGCC AGCGTTCGCACGGTGTTGACCGGCGCGGTTGCTTTGGGCGCTTTGGTTACCGTCGGCGCAT TCTTCGCCAGTAAA |
| | (Bcl-w (C29S/A128E)BH3 mRNA sequence with V5 epitope tag) |
| 82 | ATGGCACACGCTGGTAGGACAGGCTACGACAACCGTGAGATTGTGATGAAGTATATTCA CTATAAGCTGTCTCAAAGAGGCTACGAATGGGATGCGGGGGCCGTCGGGGCTGCCCCAC CTGGTGCCGCCCCGGCTCCTGGGATTTTCTCTTCCCAGCCGGGACATACCCCTCATCCTGC CGCTTCTAGGGACCCTGTGGCAAGAACATCCCCTCTACAGACCCCTGCCGCCCCAGGTGC GGCTGCTGGCCCAGCGTTAAGTCCCGTTCCACCAGTCGTGCACCTTACCCTTAGGCAAGC GGGCGACGATTTCTCTAGGCGATACCGGAGGGACTTTGCCGAAATGTCATCACAGCTCCA TCTTACTCCATTTACTGCCCGTGGCAGATTCGCCACCGTAGTGGAAGAACTCTTTCGAGAC GGCGTAAATTGGGGGAGGATCGTGGCATTTTTCGAATTTGGAGGGGTTATGTGTGTGGAG AGCGTGAATCGGGAAATGTCTCCTCTGGTTGACAATATTGCCTTGTGGATGACCGAATAT CTTAACAGGCACCTCCACACCTGGATTCAAGATAATGGTGGATGGGATGCGTTTGTGGAA CTTTATGGGCCTTCAATGCGTGACTATAAGGACGACGACGACAAA |
| | (Bcl-2(D34A)del.C32 BH3 mRNA sequence with FLAG epitope tag) |
| 83 | ATGTCCCAGAGCAACAGGGAACTCGTAGTCGATTTCTTGTCCTACAAGCTCAGTCAG AAGGGCTACTCTTGGTCCCAATTTCCGACGTGGAAGAAAATCGGACAGAAGCCCCT GAGGGAACCGAGAGTGAGATGGAGACCCCGTCTGCAATCAACGGGAACCCTTCTTG GCACTTGGCCGACAGTCCCGCTGTGAATGGCGCCACAGGTCACAGTTCATCACTCGA CGCTAGAGAAGTTATACCCATGGCAGCAGTCAAGCAAGCTCTGCGGGAGGCCGGCG ACGAGTTCGAACTTCGGTACCGCCGAGCTTTTTCTGACCTGACTAGTCAGCTGCATAT TACTCCGGGCACTGCGTACCAGTCGTTCGAACAGGTGGTTAATGAACTATTTCGAGAC GGCGTGAATTGGGGCAGAATTGTAGCCTTTTTCTCCTTTGGAGGTGCACTGTGTGTCGA GAGCGTCGATAAGGAAATGCAGGTGTTGGTGAGCCGTATCGCGGCCTGGATGGCCAC GTATCTGAACGACCATTTGGAACCATGGATCCAAGAAAACGGGGGTTGGGATACTTTC GTGGAGTTATATGGCAACAATGCTGCAGCGGAAAGTCGCAAGGGCCAGGAACGAGAC TATAAAGATGACGACGATAAA |
| | (Bcl-xL del.C24 mRNA sequence with FLAG epitope tag) |
| 84 | ATGGGCTCTGATGAGCTGTACCGGCAGAGCCTCGAGATCATAAGCAGATACCTGAGGG AACAAGCGACGGGGGCCAAGGATACTAAGCCCATGGGACGCTCCGGGGCAACGTCTC GCAAAGCCCTCGAGACCCTCAGAAGGGTCGGCGATGGGGTACAGCGCAACCACGAGA CTGCTTTCCAGGGCATGCTCCGAAAGCTGGACATCAAGAACGAAGACGATGTGAAGTC TCTTTCCCGTGTGATGATACATGTGTTTTCTGATGGGGTTACCAACTGGGGACGCATTG TGACCCTTATTAGCTTCGGGGCGTTCGTCGCTAAGCACCTCAAGACAATCAATCAGGA AAGTTGTATTGAACCTTTAGCTGAGAGCATAACTGACGTCTTGGTCCGTACAAAGAGG GATTGGTTGGTCAAACAACGTGGTTGGGATGGTTTCGTAGAGTTCTTCCATGTCGAAGA TCTTGAGGGAGGGGACTACAAAGATGACGATGATAAA |
| | (Mcl1 del.N/C(2010) mRNA sequence with FLAG epitope tag) |
| 85 | ATGAGCCAGTCTAACCGCGAACTTGTTGTGGACTTCCTTAGTTATAAACTCTCTCAGAA AGGGTATTCTTGGAGTCAGTTTTCTGATGTTGAAGAAAATCGGACTGAAGCTCCCGAG GGGACCGAAAGTGAGATGGAGACCCCAAGCGCAATAAATGGCAACCCATCTTGGCAT CTCGCCGCCTCTCCCGCAGTAAACGGAGCTACCGGTCACTCTTCTAGCCTGGACGCCC GGGAGGTGATACCCATGGCAGCCGTTAAGCAAGCCTTGCGGGAAGCAGGGGATGAGT TCGAACTTCGCTACAGGAGGGCATTTTCTGACCTCACCTCCCAGTTGCATATTACGCCA GGGACGGCATACCAGAGCTTTGAGCAAGTGGTCAATGAACTCTTTCGAGACGGAGTCA ACTGGGGACGCATTGTAGCCTTCTTTTCATTTGGGGGCGCCCTGTGTGTGGAGTCTGTC GACAAGGAGATGCAGGTGCTCGTAAGCCGCATCGCCGCCTGGATGGCGACCTATCTTA ATGATCACCTAGAACCTTGGATACAGGAGAATGGCGGATGGGACACCTTCGTTGAGCT GTACGGCAACAACGCGGCCGCAGAAAGCCGCAAGGGTCAAGAGAGGGATTATAAAGA CGATGACGACAAA |
| | (Bcl-xL(D61A)del.C24 mRNA sequence with FLAG epitope tag) |
| 86 | ATGATTCCTCGAGGGCTATCAGAAGCTAAGCCTGCCACACCAGAGATTCAGGAGATCG TGGACAAGGTGAAGCCACAACTCGAGGAGAAAACCAACGAAACATACGGCAAGCTCG AAGCTGTCCAGTATAAAACTCAGGTCTTAGATATGCGACCAGAAATTTGGATTGCCCA |
| | GGAAGCTCGGCGCATCGGTGACGAGGCTAATGCCTATTATGCCAGAAGGGCAAGCAC TAATTATTATATCAAAGTGCGAGCCGGAGATAATAAATACATGCACCTCAAGGTATTC AACGGACCCCCTGGGCAAAACGCTGACAGAGTCTTAACCGGATATCAGGTTGACAAA AATAAGGACGACGAGCTTACCGGATTCGATTATAAAGATGATGACGACAAA |
| | (SQTanti-Mcl1.(L1)BimBH3.2A mRNA sequence with FLAG epitope tag) |
| 87 | ATGATACCTAGGGATATGAGGCCTGAGATTTGGATAGCACAGGAGGCACGTAGGAT AGGAGATGAAGCCAATGCATACTACGCCAGGCGCGGTCTGTCAGAGGCCAAACCGG CAACACCAGAGATCCAAGAAATTGTCGATAAAGTCAAGCCTCAGTTAGAGGAGAAG ACTAATGAGACGTATGGCAAGCTCGAAGCAGTGCAATACAAGACTCAGGTCCTGGCT TCCACCAATTATTATATCAAAGTGCGCGCCGGCGACAACAAGTACATGCACCTTAAG GTTTTTAACGGCCCTCCAGGCCAGAATGCTGACCGTGTGCTGACAGGTTACCAGGTCG ACAAGAATAAGGACGATGAGTTGACCGGCTTCGACTATAAGGACGATGACGATAAA |
| | (SQTanti-Mcl1.(nt)BimBH3.2A mRNA sequence with FLAG epitope tag) |
| 88 | ATGATTCCTCGTGGCCTCTCTGAGGCCAAGCCTGCCACCCCCGAGATACAGGAAATCG TTGACAAAGTTAAACCTCAGCTCGAGGAAAAGACCAACGAGACTTACGGAAAACTTG AGGCAGTGCAGTATAAGACTCAGGTCCTGAAAGCTCTCGAAACTCTGAGACGCGTGG GGATGGTGTCCAGCGTAATCACGAGACGGCCTTTGCAAGCACAAATTATTACATTA AGGTGCGAGCCGGCGATAACAAATACATGCACCTCAAAGTGTTTAATGGACCGCCCG GCCAGAACGCCGACCGCGTATTGACTGGGTACCAGGTAGACAAAAACAAGGATGACG AACTTACAGGGTTCGACTACAAAGATGATGACGATAAG |
| | (SQT.(L1)Mcl1.BH3 mRNA sequence with FLAG epitope tag) |
| 89 | ATGATACCCCGGGGGCTTTCGGAGGCAAAGCCAGCTACCCCTGAAATCCAAGAAAT CGTGGATAAGGTGAAACCTCAGCTGGAGGAGAAAACCAACGAAACATACGGAAA CTGGAGGCAGTTCAATACAAAACACAGGTTTTGGCCAGCACTAATTATTACATCAAA GTGAGAGCGGGCGACAATAAGTATATGCATTTGAAGGTGTTCAACGGGCCTAAGGCT CTCGAGACTCTACGGCGGGTCGGAGACGGCGTCCAGAGAAACCACGAGACTGCATTC CCTGGCCAGAACGCAGACCGTGTCCTGACTGGCTACCAGGTCGATAAAAACAAGGAC GATGAGCTGACGGGATTCGACTATAAGGACGATGATGACAAA |
| | (SQT.(L2)Mcl1.BH3 mRNA sequence with FLAG epitope tag) |
| 90 | ATGATTCCAAGGGACATGAGACCAGAGATCTGGATTGCACAGGAGGCTAGGCGCAT AGGAGATGAAGCAAATGCGTATTATGCACGACGCGGTCTTTCTGAAGCTAAGCCTGC GACGCCGGAGATTCAGGAGATAGTGGATAAAGTTAAACCTCAATTAGAGGAAAAGA CCAATGAGACCTACGGCAAACTGGAAGCCGTGCAATATAAAACTCAGGTTCTGGCCT CCACTAACTATTATATCAAAGTGAGGGCAGGAGACAACAAGTACATGCACTTAAAGG TGTTCAATGGGCCCAACCTGTGGGCGGCCCAGCGCTACGGTCGCGAGCTACGAAGGA TGTCTGACGAGTTCGTTGACAGCTTCAAGAAGGGGCCTGGACAGAACGCCGATAGAG TGTTAACAGGCTATCAGGTCGATAAAAATAAAGATGACGAGCTAACCGGGTTTGATT ACAAAGATGACGATGATAAG |
| | (biSQTanti-Bcl/Mcl1.(nt)BimBH2.2A.(L2)Bad mRNA sequence with FLAG epitope tag) |
| 91 | ATGATTCCCAGAGGCTTGAGTGAGGCTAAGCCCGCCACACCTGAGATCCAGGAAATC GTGGACAAGGTGAAACCCCAGCTGGAAGAAAAAACCAACGAGACCTACGGCAAACT GGAGGCTGTCCAGTACAAAACTCAAGTCCTGGCCTCAACCAACTACTACATTAAAGT TAGAGCAGGCGATAATAAATACATGCACCTTAAAGTATTCAACGGTCCTGACATGCG ACCTGAGATTTGGATAGCACAGGAAGCAAGAAGGATTGGGGATGAAGCTAATGCAT ATTACGCCCGCCGCCCGGGGCAGAATGCTGACCGTGTGCTGACCGGTTATCAGGTAG ACAAGAACAAAGACGACGAGCTAACAGGCTTCGACTACAAAGACGATGATGATAAG |
| | (SQTanti-Mcl1.(L2)BimBH3.2A mRNA sequence with FLAG epitope tag) |
| 92 | ATGGCAACACCCGCGTCCGCACCCGATACAAGAGCACTGGTTGCAGACTTCGTGGGC TACAAACTGAGGCAGAAGGGGTACGTCTGTGGTGCGGCCCCGGTGAAGGCCTGCC GCCGACCCTCTGCACCAGGCTATGCGAGCTGCAGGGGACGAGTTTGAGACACGCTTTA GACGTACATTTTCTGATTTGGCGGCTCAGCTCCACGTCACCCCTGGGAGCGCTCAGCAG CGATTTACCCAAGTAAGCGACGAGCTCTTTCAAGGAGGCCCCAATTGGGGCCGGCTGG TCGCGTTCTTTGTGTTTGGGGCCGCATTATGCGCGGAAAGCGTTAACAAAGAGATGGA ACCCTTAGTAGGACAAGTCCAGGAATGGATGGTGGCCTACCTAGAGACACAGCTAGCT GACTGGATTCATTCATCAGGGGGCTGGGCCGAGTTTACTGCCTTATATGGGGATGGGG CACTCGAAGAAGCCAGGAGACTTCGCGAAGGAATTGGGCATCAGTGAGGACTGTGC TAACTGGAGCCGTCGCCCTGGGTGCCCTGGTTACCGTGGGTGCTTTCTTTGCGTCTAAA |
| | (Bcl-w wild-type mRNA sequence without epitope tag) |
| 93 | ATGTCTCAGAGCAACAGAGAATTGGTAGTGGATTTTCTGAGTTATAAACTATCGCAGA |
| | AAGGCTACTCTTGGTCACAGTTCTCAGATGTTGAGGAGAACAGGACTGAAGCACCAGA GGGGACAGAGTCTGAGATGGAGACCCCCAGTGCTATAAACGGTAACCCCTCCTGGCAC CTAGCCGATAGCCCCGCCGTGAACGGAGCCACCGGACACAGCAGCTCCTTAGATGCCC GCGAGGTGATTCCGATGGCCGCAGTAAAGCAGGCTCTCCGAGAAGCCGGCGATGAATT TGAGCTTAGGTATAGGAGGGCCTTCTCTGATCTTACTTCACAGCTTCACATCACACCTG GCACAGCTTATCAGAGCTTCGAACAGGTGGTGAATGAACTTTTTCGAGATGGCGTAAA CTGGGGCCGCATCGTGGCCTTCTTTAGCTTCGGTGGGGCCCTGTGCGTCGAATCAGTGG ACAAAGAAATGCAAGTCCTGGTGAGCCGAATCGCAGCCTGGATGGCCACGTACTTGAA CGATCACCTGGAGCCTTGGATCCAAGAGAACGGCGGATGGGACACTTTCGTCGAGTTG TATGGCAATAATGCCGCTGCCGAAAGTCGGAAAGGGCAGGAGCGATTTAACCGCTGGT TCCTTACAGGCATGACTGTTGCTGGGGTGGTGCTCTTGGGTTCTCTCTTCAGCCGCAAA [?] |
| | (Bcl-xL Wild-type mRNA sequence without epitope tag) |
| 94 | ATGGGACCACTCGGGTCGGAGGATGACCTGTATCGCCAATCCCTGGAAATCATCTCT AGGTACCTGAGGGAGCAGGCCACCGGCTCGAAAGATAGTAAACCACTTGGCGAAGC CGGAGCTGCTGGCAGAAGGGCTCTCGAGACACTCCGGCGCGTGGGTGATGGAGTGC AACGCAATCACGAGACTGCCTTCCAGGGCATGCTCAGGAAGCTGGATATCAAAAAC GAGGATGATGTTAAGAGTCTCTCTCGAGTCATGATTCATGTGTTTTCCGACGGTGTCA CTAACTGGGGTAGAATTGTTACTCTGATAAGTTTTGGAGCATTCGTTGCCAAGCACTT GAAGACAATTAACCAGGAGTCGTGCATCGAGCCCCTAGCGGAAAGCATCACAGATGT TCTCGTGCGAACCAAGCGCGATTGGCTGGTCAAGCAGAGGGGATGGGACGGGTTTGT TGAGTTTTTCCACGTCGAGGATCTGGAAGGGGGG |
| | (Mcl-1 del.N/C mRNA sequence without epitope tag) |
| 95 | ATGGCTACACCAGCCAGTGCACCAGACACGAGGGCGCTGGTTGCCGATTTCGTGGG ATACAAGCTCAGGCAAAAAGGGTACGTAAGCGGTGCCGGGCCCGGGGAGGGACCG GCTGCCGATCCACTCCACCAGGCTATGAGGGCAGCTGGGGACGAATTCGAAACACG CTTTCGGCGGACTTTCTCCGACCTTGCCGCACAGCTACACGTCACTCCCGGGAGCGC TCAGCAGCGCTTCACGCAGGTCTCAGACGAATTGTTCCAAGGCGGCCCTAATTGGG GAAGATTGGTGGCCTTCTTCGTGTTTGGGGCAGCACTGTGCGCTGAGTCCGTGAACA AAGAAATGGAACCCCTCGTTGGCCAAGTCCAAGAATGGATGGTGGAATATCTGGAA ACACAGTTAGCGGATTGGATTCACTCATCTGGGGGCTGGGCTGAGTTTACAGCACTG TACGGGGACGGTGCCCTAGAGGAGGCCCGAAGACTGAGGGAAGGCAACTGGGCCA GCGTTCGCACGGTGTTGACCGGCGCGGTTGCTTTGGGCGCTTTGGTTACCGTCGGCG CATTCTTCGCCAGTAAA |
| | (Bcl-w (C29S/A128E)BH3 mRNA sequence without epitope tag) |
| 96 | ATGGCACACGCTGGTAGGACAGGCTACGACAACCGTGAGATTGTGATGAAGTATATT CACTATAAGCTGTCTCAAAGAGGCTACGAATGGGATGCGGGGGCCGTCGGGGCTGCC CCACCTGGTGCCGCCCCGGCTCCTGGGATTTTCTCTTCCCAGCCGGGACATACCCCTC ATCCTGCCGCTTCTAGGGACCCTGTGGCAAGAACATCCCCTCTACAGACCCCTGCCGC CCCAGGTGCGGCTGCTGGCCCAGCGTTAAGTCCCGTTCCACCAGTCGTGCACCTTACC CTTAGGCAAGCGGGCGACGATTTCTCTAGGCGATACCGGAGGGACTTTGCCGAAATGT CATCACAGCTCCATCTTACTCCATTTACTGCCCGTGGCAGATTCGCCACCGTAGTGGAA GAACTCTTTCGAGACGGCGTAAATTGGGGGAGGATCGTGGCATTTTTCGAATTTGGAG GGGTTATGTGTGTGGAGAGCGTGAATCGGGAAATGTCTCCTCTGGTTGACAATATTGC CTTGTGGATGACCGAATATCTTAACAGGCACCTCCACACCTGGATTCAAGATAATGGT GGATGGGATGCGTTTGTGGAACTTTATGGGCCTTCAATGCGT |
| | (Bcl-2(D34A)del.C32 BH3 mRNA sequence without epitope tag) |
| 97 | ATGTCCCAGAGCAACAGGGAACTCGTAGTCGATTTCTTGTCCTACAAGCTCAGTCAGA AGGGCTACTCTTGGTCCCAATTTTCCGACGTGGAAGAAAATCGGACAGAAGCCCCTGA GGGAACCGAGAGTGAGATGGAGACCCCGTCTGCAATCAACGGGAACCCTTCTTGGCA CTTGGCCGACAGTCCCGCTGTGAATGGCGCCACAGGTCACAGTTCATCACTCGACGCT AGAGAAGTTATACCCATGGCAGCAGTCAAGCAAGCTCTGCGGGAGGCCGGCGACGAG TTCGAACTTCGGTACCGCCGAGCTTTTTCTGACCTGACTAGTCAGCTGCATATTACTCC GGGCACTGCGTACCAGTCGTTCGAACAGGTGGTTAATGAACTATTTCGAGACGGCGTG AATTGGGGCAGAATTGTAGCCTTTTTCTCCTTTGGAGGTGCACTGTGTGTCGAGAGCGT CGATAAGGAAATGCAGGTGTTGGTGAGCCGTATCGCGGCCTGGATGGCCACGTATCTG AACGACCATTTGGAACCATGGATCCAAGAAAACGGGGGTTGGGATACTTTCGTGGAGT TATATGGCAACAATGCTGCAGCGGAAAGTCGCAAGGGCCAGGAACGA |
| | (Bcl-xL del.C24 mRNA sequence without epitope tag) |
| 98 | ATGGGCTCTGATGAGCTGTACCGGCAGAGCCTCGAGATCATAAGCAGATACCTGAGGG AACAAGCGACGGGGGCCAAGGATACTAAGCCCATGGGACGCTCCGGGGCAACGTCTC |
| | GCAAAGCCCTCGAGACCCTCAGAAGGTCGGCGATGGGGTACAGCGCAACCACGAGA CTGCTTTCCAGGGCATGCTCCGAAAGCTGGACATCAAGAACGAAGACGATGTGAAGT CTCTTTCCCGTGTGATGATACATGTGTTTTCTGATGGGGTTACCAACTGGGGACGCATT GTGACCCTTATTAGCTTCGGGGCGTTCGTCGCTAAGCACCTCAAGACAATCAATCAGG AAAGTTGTATTGAACCTTTAGCTGAGAGCATAACTGACGTCTTGGTCCGTACAAAGAG GGATTGGTTGGTCAAACAACGTGGTTGGGATGGTTTCGTAGAGTTCTTCCATGTCGAA GATCTTGAGGGAGGG |
| | (Mcl1 del.N/C(2010) mRNA sequence without epitope tag) |
| 99 | ATGAGCCAGTCTAACCGCGAACTTGTTGTGGACTTCCTTAGTTATAAACTCTCTCAGA AAGGGTATTCTTGGAGTCAGTTTCTGATGTTGAAGAAAATCGGACTGAAGCTCCCG AGGGGACCGAAAGTGAGATGGAGACCCCAAGCGCAATAAATGGCAACCCATCTTGG CATCTCGCCGCCTCTCCCGCAGTAAACGGAGCTACCGGTCACTCTTCTAGCCTGGACG CCCGGGAGGTGATACCCATGGCAGCCGTTAAGCAAGCCTTGCGGGAAGCAGGGGATG AGTTCGAACTTCGCTACAGGAGGGCATTTTCTGACCTCACCTCCCAGTTGCATATTAC GCCAGGGACGGCATACCAGAGCTTTGAGCAAGTGGTCAATGAACTCTTTCGAGACGG AGTCAACTGGGGACGCATTGTAGCCTTCTTTTCATTTGGGGGCGCCCTGTGTGTGGAG TCTGTCGACAAGGAGATGCAGGTGCTCGTAAGCCGCATCGCCGCCTGGATGGCGACCT ATCTTAATGATCACCTAGAACCTTGGATACAGGAGAATGGCGGATGGGACACCTTCGT TGAGCTGTACGGCAACAACGCGGCCGCAGAAAGCCGCAAGGGTCAAGAGAGG |
| | (Bcl-xL(D61A)del.C24 mRNA sequence without epitope tag) |
| 100 | ATGATTCCTCGAGGGCTATCAGAAGCTAAGCCTGCCACACCAGAGATTCAGGAGATC GTGGACAAGGTGAAGCCACAACTCGAGGAGAAAACCAACGAAACATACGGCAAGCT CGAAGCTGTCCAGTATAAAACTCAGGTCTTAGATATGCGACCAGAAATTTGGATTGC CCAGGAAGCTCGGCGCATCGGTGACGAGGCTAATGCCTATTATGCCAGAAGGGCAAG CACTAATTATTATATCAAAGTGCGAGCCGGAGATAATAAATACATGCACCTCAAGGTA TTCAACGGACCCCCTGGGCAAAACGCTGACAGAGTCTTAACCGGATATCAGGTTGACA AAAATAAGGACGACGAGCTTACCGGATTC |
| | (SQTanti-Mcl1.(L1)BimBH3.2A mRNA sequence without epitope tag) |
| 101 | ATGATACCTAGGGATATGAGGCCTGAGATTTGGATAGCACAGGAGGCACGTAGGAT AGGAGATGAAGCCAATGCATACTACGCCAGGCGCGGTCTGTCAGAGGCCAAACCGG CAACACCAGAGATCCAAGAAATTGTCGATAAAGTCAAGCCTCAGTTAGAGGAGAAG ACTAATGAGACGTATGGCAAGCTCGAAGCAGTGCAATACAAGACTCAGGTCCTGGCT TCCACCAATTATTATATCAAAGTGCGCGCCGGCGACAACAAGTACATGCACCTTAAG GTTTTTAACGGCCCTCCAGGCCAGAATGCTGACCGTGTGCTGACAGGTTACCAGGTC GACAAGAATAAGGACGATGAGTTGACCGGCTTC |
| | (SQTanti-Mcl1.(nt)BimBH3.2A mRNA sequence without epitope tag) |
| 102 | ATGATTCCTCGTGGCCTCTCTGAGGCCAAGCCTGCCACCCCCGAGATACAGGAAATC GTTGACAAAGTTAAACCTCAGCTCGAGGAAAAGACCAACGAGACTTACGGAAAACT TGAGGCAGTGCAGTATAAGACTCAGGTCCTGAAAGCTCTCGAAACTCTGAGACGCG TGGGGGATGGTGTCCAGCGTAATCACGAGACGGCCTTTGCAAGCACAAATTATTAC ATTAAGGTGCGAGCCGGCGATAACAAATACATGCACCTCAAAGTGTTTAATGGACC GCCCGGCCAGAACGCCGACCGCGTATTGACTGGGTACCAGGTAGACAAAAACAAG GATGACGAACTTACAGGGTTC |
| | (SQT.(L1)Mcl1.BH3 mRNA sequence without epitope tag) |
| 103 | ATGATACCCCGGGGGCTTTCGGAGGCAAAGCCAGCTACCCCTGAAATCCAAGAAAT CGTGGATAAGGTGAAACCTCAGCTGGAGGAGAAAACCAACGAAACATACGGAAA CTGGAGGCAGTTCAATACAAAACACAGGTTTTGGCCAGCACTAATTATTACATCAAA GTGAGAGCGGGCGACAATAAGTATATGCATTTGAAGGTGTTCAACGGGCCTAAGGCT CTCGAGACTCTACGGCGGGTCGGAGACGGCGTCCAGAGAAACCACGAGACTGCATTC CCTGGCCAGAACGCAGACCGTGTCCTGACTGGCTACCAGGTCGATAAAAACAAGGAC GATGAGCTGACGGGATTC |
| | (SQT.(L2)Mcl1.BH3 mRNA sequence without epitope tag) |
| 104 | ATGATTCCAAGGGACATGAGACCAGAGATCTGGATTGCACAGGAGGCTAGGCGCAT AGGAGATGAAGCAAATGCGTATTATGCACGACGCGGTCTTTCTGAAGCTAAGCCTG CGACGCCGGAGATTCAGGAGATAGTGGATAAAGTTAAACCTCAATTAGAGGAAAAG ACCAATGAGACCTACGGCAAACTGGAAGCCGTGCAATATAAAACTCAGGTTCTGGCC TCCACTAACTATTATATCAAAGTGAGGGCAGGAGACAACAAGTACATGCACTTAAAG GTGTTCAATGGGCCCAACCTGTGGGCGGCCCAGCGCTACGGTCGCGAGCTACGAAGG ATGTCTGACGAGTTCGTTGACAGCTTCAAGAAGGGGCCTGGACAGAACGCCGATAGA |
| | GTGTTAACAGGCTATCAGGTCGATAAAAATAAAGATGACGAGCTAACCGGGTTT |
| | (biSQTanti-Bcl/Mcl1.(nt)BimBH2.2A.(L2)Bad mRNA sequence without epitope tag) |
| 105 | ATGATTCCCAGAGGCTTGAGTGAGGCTAAGCCCGCCACACCTGAGATCCAGGAAATC GTGGACAAGGTGAAACCCCAGCTGGAAGAAAAAACCAACGAGACCTACGGCAAACT GGAGGCTGTCCAGTACAAAACTCAAGTCCTGGCCTCAACCAACTACTACATTAAAGT TAGAGCAGGCGATAATAAATACATGCACCTTAAAGTATTCAACGGTCCTGACATGCG ACCTGAGATTTGGATAGCACAGGAAGCAAGAAGGATTGGGGATGAAGCTAATGCAT ATTACGCCCGCCGCCCGGGGCAGAATGCTGACCGTGTGCTGACCGGTTATCAGGTAG ACAAGAACAAAGACGACGAGCTAACAGGCTTC |
| | (SQTanti-Mcll.(L2)BimBH3.2A mRNA sequence without epitope tag) |
| 106 | UCAACAUCAGUCUGAUAAGCUA (miR-21 sequence) |
| 107 | MIPRDMRPEIWIAQEARRIGDEANAYYARRGLSEAKPATPEIQEIVDKVKPQLEEKTNETY GKLEAVQYKTQVLASTNYYIKVRAGDNKYMHLKVFNGPPGQNADRVLTGYQVDKNKD DELTGFGKPIPNPLLGLDST |
| | (SQTanti-Mcl1.(nt)BimBH3.2A.v5) |
| 108 | MIPRKALETLRRVGDGVQRNHETAFGLSEAKPATPEIQEIVDKVKPQLEEKTNETYGK LEAVQYKTQVLASTNYYIKVRAGDNKYMHLKVFNGPPGQNADRVLTGYQVDKNKD DELTGFGKPIPNPLLGLDST |
| | (SQT.(nt)Mcl1. BH3.cv5) |
| 109 | MIPRDMRPEIWIAQEARRIGDEANAYYARRGLSEAKPATPEIQEIVDKVKPQLEEKTNETY GKLEAVQYKTQVLASTNYYIKVRAGDNKYMHLKVFNGPPGQNADRVLTGYQVDKNKD DELTGF |
| | (SQTanti-Mcll.(nt)BimBH3.2A without v5 tag) |
| 110 | MIPRKALETLRRVGDGVQRNHETAFGLSEAKPATPEIQEIVDKVKPQLEEKTNETYGK LEAVQYKTQVLASTNYYIKVRAGDNKYMHLKVFNGPPGQNADRVLTGYQVDKNKD DELTGF |
| | (SQT.(nt)Mcll.BH3 without v5 tag) |
| 111 | ATGATCCAAGAGATATGCGCCCTGAAATCTGGATTGCACAAGAAGCCCGGAGGATTGGA GATGAGGCGAACGCCTACTACGCCCGGAGAGGCCTGTCGGAAGCTAAACCGGCCACCCCC GAGATCCAGGAGATCGTGGATAAGGTCAAGCCCCAGCTTGAAGAAAAGACTAACGAAACC TATGGGAAGCTGGAGGCCGTGCAGTACAAGACTCAAGTGCTCGCGTCCACCAACTACTACA TCAAGGTCCGCGCCGGCGACAACAAGTACATGCACTTGAAAGTGTTCAATGGCCCGCCGG GACAGAACGCCGACCGGGTGCTGACTGGATACCAGGTCGACAAGAACAAGGACGACGAGC TGACCGGATTCGGGAAGCCTATTCCCAACCCTCTCCTGGGTCTGGACAGCACC |
| | (SQTanti-Mcl1.(nt)BimBH3.2A.v5) |
| 112 | ATGATCCCTAGGAAGGCCCTGGAGACCCTGAGGAGAGTGGCGACGGAGTGCAGAGGAAC CACGAGACCGCCTTCGGCCTGAGCGAGGCAAAGCCAGCAACACCCGAGATCCAGGAGATC GTGGATAAGGTGAAGCCCCAGCTGGAGGAGAAGACCAATGAGACATACGGCAAGCTGGA GGCCGTGCAGTATAAGACCCAGGTGCTGGCCTCCACAAACTACTATATCAAGGTGCGGCC GGCGACAATAAGTACATGCACCTGAAGGTGTTCAACGGACCACCTGGACAGAATGCAGAC AGGGTGCTGACCGGCTATCAGGTGGATAAGAACAAGGACGATGAGCTGACAGGCTTTGGC AAGCCAATCCCCAATCCTCTGCTGGGCCTGGATTCTACA |
| | (SQT.(nt)Mcl1. BH3.cv5) |
| 113 | UGUAGUGUUUCCUACUUUAUGGA (hsa-miR-142-3p) |
| 114 | CAUAAAGUAGAAAGCACUACU (hsa-miR-142-5p) |
| 115 | HYPWFKARLYPL (Anti-Ras peptide sequence) |
| 116 | MIPRHYPWFKARLYPLGLSEAKPATPEIQEIVDKVKPQLEEKTNETYGKLEAVQYKTQVLAST NYYIKVRAGDNKYMHLKVFNGPPGQNADRVLTGYQVDKNKDDELTGF |
| | (Anti-Ras inserted into N-terminus of scaffold) |
| 117 | MIPRGLSEAKPATPEIQEIVDKVKPQLEEKTNETYGKLEAVQYKTQVLHYPWFKARLYPLAST NYYIKVRAGDNKYMHLKVFNGPPGQNADRVLTGYQVDKNKDDELTGF |
| | (Anti-Ras inserted into Loop 1 of scaffold) |
| 118 | MIPRGLSEAKPATPEIQEIVDKVKPQLEEKTNETYGKLEAVQYKTQVLASTNYYIKVRAGDNK YMHLKVFNGPHYPWFKARLYPLPGQNADRVLTGYQVDKNKDDELTGF |
| | (Anti-Ras inserted into Loop 2 of scaffold) |
| 119 | MIPRMSRRKQAKPQHIGLSEAKPATPEIQEIVDKVKPQLEEKTNETYGKLEAVQYKTQVLAST NYYIKVRAGDNKYMHLKVFNGPPGQNADRVLTGYQVDKNKDDELTGF |
| | (SALL4-inhibitory peptide inserted into N-terminus of scaffold) |
| 120 | MIPRGLSEAKPATPEIQEIVDKVKPQLEEKTNETYGKLEAVQYKTQVLMSRRKQAKPQHIAST NYYIKVRAGDNKYMHLKVFNGPPGQNADRVLTGYQVDKNKDDELTGF |
| | (SALL4-inhibitory peptide inserted into Loop 1 of scaffold) |
| 121 | MIPRGLSEAKPATPEIQEIVDKVKPQLEEKTNETYGKLEAVQYKTQVLASTNYYIKVRAGDNK YMHLKVFNGPMSRRKQAKPQHIPGQNADRVLTGYQVDKNKDDELTGF |
| | (SALL4-inhibitory peptide inserted into Loop 2 of scaffold) |
| 122 | MIPRMSRRKQAKPQHIGLSEAKPATPEIQEIVDKVKPQLEEKTNETYGKLEAVQYKTQVLAST NYYIKVRAGDNKYMHLKVFNGPPGQNADRVLTGYQVDKNKDDELTGF GKPIPNPLLGLDST |
| | (SALL4-inhibitory peptide inserted into N-terminus of scaffold + V5 flag) |
| 123 | MIPRGLSEAKPATPEIQEIVDKVKPQLEEKTNETYGKLEAVQYKTQVLMSRRKQAKPQHIAST NYYIKVRAGDNKYMHLKVFNGPPGQNADRVLTGYQVDKNKDDELTGF GKPIPNPLLGLDST |
| | (SALL4-inhibitory peptide inserted into Loop 1 of scaffold + V5 flag) |
| 124 | MIPRGLSEAKPATPEIQEIVDKVKPQLEEKTNETYGKLEAVQYKTQVLASTNYYIKVRAGDNK YMHLKVFNGPMSRRKQAKPQHIPGQNADRVLTGYQVDKNKDDELTGF GKPIPNPLLGLDST |
| | (SALL4-inhibitory peptide inserted into Loop 2 of scaffold + V5 flag) |
| 125 | MSRRKQAKPQHI |
| | (SALL4-inhibitory peptide sequence) |
| 126 | MIPRMEGISNFKTPSKLSEKKKGLSEAKPATPEIQEIVDKVKPQLEEKTNETYGKLEAVQYKT QVLASTNYYIKVRAGDNKYMHLKVFNGPPGQNADRVLTGYQVDKNKDDELTGF |
| | (TOPK-inhibitory peptide inserted in N-terminus of scaffold) |
| 127 | MIPRGLSEAKPATPEIQEIVDKVKPQLEEKTNETYGKLEAVQYKTQVLMEGISNFKTPSKLSEK KKASTNYYIKVRAGDNKYMHLKVFNGPPGQNADRVLTGYQVDKNKDDELTGF |
| | (TOPK-inhibitory peptide inserted in Loop 1 of scaffold) |
| 128 | MIPRGLSEAKPATPEIQEIVDKVKPQLEEKTNETYGKLEAVQYKTQVLASTNYYIKVRAGDNK YMHLKVFNGPMEGISNFKTPSKLSEKKKPGQNADRVLTGYQVDKNKDDELTGF |
| | (TOPK-inhibitory peptide inserted in Loop 2 of scaffold) |
| 129 | MEGISNFKTPSKLSEKKK |
| | (TOPK-inhibitory peptide sequence) |
| 130 | TSFAEYWNLLSP |
| | (p53-inhibitory peptide sequence) |
| 131 | ETFSDLWKLLPE |
| | (p53-inhibitory peptide sequence) |
| 132 | ETFEHWWSQLLS |
| | (p53-inhibitory peptide sequence) |
| 133 | ETFEHWWAQLTS |
| | (p53-inhibitory peptide sequence) |
| 134 | LTFEHSWAQLTS |
| | (p53-inhibitory peptide sequence) |
| 135 | LTFEHWWAQLTS |
| | (p53-inhibitory peptide sequence) |
| 136 | LTFEHYWAQLTS |
| | (p53-inhibitory peptide sequence) |
| 137 | LTFEEYWAQLLSAA |
| | (p53-inhibitory peptide sequence) |
| 138 | LTAEEYTAQLLSAA |
| | (p53-inhibitory peptide sequence) |
| 139 | TNWYANLEKLLR |
| | (p53-inhibitory peptide sequence) |
| 140 | TAWYANFEKLLR |
| | (p53-inhibitory peptide sequence) |
| 141 | DWWPLAFEALLR |
| | (p53-inhibitory peptide sequence) |
| 142 | MIPRLTFEHYWAQLTSGLSEAKPATPEIQEIVDKVKPQLEEKTNETYGKLEAVQYKTQVLAST NYYIKVRAGDNKYMHLKVFNGPPGQNADRVLTGYQVDKNKDDELTGF |
| | (P53 inhibitory peptide p3848 inserted in N-term of scaffold) |
| 143 | MIPRLTFEEYWAQLLSAAGLSEAKPATPEIQEIVDKVKPQLEEKTNETYGKLEAVQYKTQVLA STNYYIKVRAGDNKYMHLKVFNGPPGQNADRVLTGYQVDKNKDDELTGF |
| | (P53 inhibitory peptide p7041 inserted in N-term of scaffold) |
| 144 | MIPRETFEHWWSQLLSGLSEAKPATPEIQEIVDKVKPQLEEKTNETYGKLEAVQYKTQVLAST NYYIKVRAGDNKYMHLKVFNGPPGQNADRVLTGYQVDKNKDDELTGF |
| | (P53 inhibitory peptide pDIQ inserted in N-term of scaffold) |
| 145 | MIPRTSFAEYWNLLSPGLSEAKPATPEIQEIVDKVKPQLEEKTNETYGKLEAVQYKTQVLASTN YYIKVRAGDNKYMHLKVFNGPPGQNADRVLTGYQVDKNKDDELTGF |
| | (P53 inhibitory peptide pMI inserted in N-term of scaffold) |
| 146 | MIPRGLSEAKPATPEIQEIVDKVKPQLEEKTNETYGKLEAVQYKTQVLASTNYYIKVRAGDNK YMHLKVFNGPLTFEHYWAQLTSPGQNADRVLTGYQVDKNKDDELTGF |
| | (P53 inhibitory peptide p3848 inserted in Loop 2 of scaffold) |
| 147 | MIPRGLSEAKPATPEIQEIVDKVKPQLEEKTNETYGKLEAVQYKTQVLASTNYYIKVRAGDNK YMHLKVFNGPETFEHWWAQLTSPGQNADRVLTGYQVDKNKDDELTGF |
| | (P53 inhibitory peptide p1E6W inserted in Loop 2 of scaffold) |
| 148 | MIPRLTFEHWWAQLTSGLSEAKPATPEIQEIVDKVKPQLEEKTNETYGKLEAVQYKTQVLAST NYYIKVRAGDNKYMHLKVFNGPPGQNADRVLTGYQVDKNKDDELTGF |
| | (P53 inhibitory peptide p6W inserted in N-term of scaffold) |
| 149 | ATGATACCTCGGCTTACATTCGAACACTGGTGGCCCAGTTGACATCAGGACTCAGCGAGG CCAAACCCGCTACACCTGAGATTCAAGAGATCGTCGATAAGGTAAAGCCCCAACTTGAAG AAAAGACAAACGAGACATATGGAAAGTTAGAGGCTGTTCAGTATAAGACACAGGTGCTTG CCTCCACCAACTACTATATCAAGGTAAGGGCCGGAGACAACAAATACATGCATCTCAAAGT ATTCAATGGGCCCCCTGGTCAGAATGCGGATCGCGTCCTGACAGGCTACCAGGTCGACAAA AATAAGGACGACGAGCTCACGGGATTT |
| | (nt seq. for P53 inhibitory peptide p3848 inserted in N-term of scaffold) |
| 150 | ATGATTCCAAGGCTGACCTTTGAAGAGTACTGGGCGCAGTTGCTTAGTGCCGCCGGCCTTT CAGAGGCAAAGCCCGCCACGCCAGAGATTCAGGAGATAGTTGATAAGGTGAAACCCCAAC TTGAGGAAAAAACTAACGAGACATATGGGAAGCTTGAGGCCGTACAATATAAGACTCAGG TGCTGGCCTCAACAAATTATTATATCAAGGTGCGGGCCGGAGATAATAAGTACATGCACCT GAAGGTTTTCAATGGCCCGCCAGGGCAGAACGCTGACAGGGTGCTGACGGGCTATCAGGT GGACAAAAATAAAGACGACGAACTGACGGGATTT |
| | (nt seq. for P53 inhibitory peptide p7041 inserted in N-term of scaffold) |
| 151 | ATGATCCCCCGCGAGACATTTGAACACTGGTGGTCACAGCTCCTTAGCGGTCTCAGTGAGG CTAAGCCTGCGACTCCAGAGATCCAGGAGATCGTGGATAAAGTAAAGCCTCAGCTGGAGG AAAAAACTAACGAGACTTACGGAAACTGGAAGCGGTGCAATACAAAACACAGGTGCTGG CCTCAACTAACTATTACATCAAAGTGCGCGCTGGTGATAACAAATACATGCATTTAAAGGT CTTCAATGGGCCACCAGGACAGAACGCAGACAGAGTGCTGACTGGCTACCAGGTGGACAA GAACAAAGATGACGAGCTTACAGGCTTT |
| | (nt seq. for P53 inhibitory peptide pDIQ inserted in N-term of scaffold) |
| 152 | ATGATCCCCAGAACATCCTTCGCAGAGTATTGGAATCTGCTGTCCCCCGGCCTTTCAGAAG CCAAACCTGCTACGCCAGAGATCCAAGAAATTGTGGACAAAGTGAAGCCCCAGCTCGAGG AGAAGACTAACGAGACATATGGTAAGTTAGAGGCCGTACAGTACAAAACGCAAGTGCTTG CTTCCACCAACTACTACATCAAAGTGAGAGCAGGTGATAATAAATACATGCACCTTAAAGT TTTTAACGGCCCTCCTGGGCAGAACGCCGACAGAGTCCTCACTGGATATCAGGTTGATAAA AACAAGGATGACGAGCTGACTGGTTTC |
| | (nt seq. for P53 inhibitory peptide pMI inserted in N-term of scaffold) |
| 153 | ATGATTCCCAGAGGCCTGTCCGAGGCTAAACCAGCCACACCTGAGATTCAGGAAATCGTGG ATAAAGTTAAGCCTCAACTCGAAGAAAAGACAAACGAGACGTATGGTAAGCTTGAAGCTG TGCAGTATAAAACTCAAGTCCTGGCCTCCACCAATTATTACATTAAGGTCAGGGCTGGAGA CAATAAGTACATGCATCTGAAAGTGTTCAACGGACCCTTGACCTTCGAACACTATTGGGCC CAACTCACCAGTCCCGGTCAGAACGCGGACCGCGTACTTACAGGTTACCAAGTGGACAAG AATAAAGATGACGAGCTGACAGGCTTC |
| | (nt seq. for P53 inhibitory peptide p3848 inserted in Loop 2 of scaffold) |
| 154 | ATGATCCCTCGCGGCCTATCCGAGGCAAAGCCAGCTACCCCTGAAATACAGGAGATCGTGG ATAAGGTAAAACCCCAACTGGAGGAGAAGACTAATGAGACATATGGCAAACTGGAAGCTG TTCAATATAAGACACAGGTGCTTGCCTCTACCAACTATTATATCAAAGTCCGGGCAGGAGA TAATAAATATATGCATTTGAAGGTATTTAATGGCCCCGAGACATTTGAACACTGGTGGGCG CAGTTAACCTCTCCGGGGCAGAATGCGGATAGGGTGCTGACCGGGTACCAGGTTGACAAG AATAAGGATGACGAACTAACCGGTTTT |
| | (nt seq. for P53 inhibitory peptide p1E6W inserted in Loop 2 of scaffold) |
| 155 | ATGATACCTCGGCTTACATTCGAACACTGGTGGCCCAGTTGACATCAGGACTCAGCGAGG CCAAACCCGCTACACCTGAGATTCAAGAGATCGTCGATAAGGTAAAGCCCCAACTTGAAG AAAAGACAAACGAGACATATGGAAAGTTAGAGGCTGTTCAGTATAAGACACAGGTGCTTG CCTCCACCAACTACTATATCAAGGTAAGGGCCGGAGACAACAAATACATGCATCTCAAAGT ATTCAATGGGCCCCCTGGTCAGAATGCGGATCGCGTCCTGACAGGCTACCAGGTCGACAAA AATAAGGACGACGAGCTCACGGGATTT |
| | (nt seq. for P53 inhibitory peptide p6W inserted in N-term of scaffold) |
| 156 | GKPIPNPLLGLDST |
| | (V5 tag) |
| 157 | TCAAGCTTTTGGACCCTCGTACAGAAGCTAATACGACTCACTATAGGGAAATAAGAGAGA AAAGAAGAGTAAGAAGAAATATAAGAGCCACCATGATCCCACGGCTGACTTTTGAGCACT ATTGGGCCCAGCTAACCTCGGGGCTTAGCGAGGCCAAACCCGCCACACCTGAGATCCAAG AAATAGTGGACAAAGTTAAACCTCAGTTAGAGGAGAAAACCAATGAAACATACGGCAAAC TCGAGGCAGTTCAATACAAGACCCAGGTATTGGCATCCACGAACTATTATATAAAGGTGAG GGCTGGCGACAACAAGTATATGCACCTGAAAGTGTTTAATGGGCCACCCGGGCAGAACGC TGACCGAGTGCTCACCGGTTACCAAGTCGATAAGAACAAGGACGATGAGCTCACAGGATT CGGGAAGCCCATTCCGAACCCTCTGCTGGGCCTGGACAGCACCTGATAATAGGCTGGAGCC TCGGTGGCCATGCTTCTTGCCCCTTGGGCCTCCCCCCAGCCCCTCCTCCCCTTCCTGCACCC GTACCCCCGTGGTCTTTGAATAAAGTCTGAGTGGGCGGC |
| | (mRNA construct seq. for P53 inhibitory peptide p3848 inserted in N-term of scaffold) |
| 158 | TCAAGCTTTTGGACCCTCGTACAGAAGCTAATACGACTCACTATAGGGAAATAAGAGAGA AAAGAAGAGTAAGAAGAAATATAAGAGCCACCATGATTCCAAGGCTGACCTTTGAAGAGT ACTGGGCGCAGTTGCTTAGTGCCGCCGGCCTTTCAGAGGCAAAGCCCGCCACGCCAGAGAT TCAGGAGATAGTTGATAAGGTGAAACCCCAACTTGAGGAAAAAACTAACGAGACATATGG GAAGCTTGAGGCCGTACAATATAAGACTCAGGTGCTGGCCTCAACAAATTATTATATCAAG GTGCGGGCCGGAGATAATAAGTACATGCACCTGAAGGTTTTCAATGGCCCGCCAGGGCAG AACGCTGACAGGGTGCTGACGGGCTATCAGGTGGACAAAAATAAAGACGACGAACTGACG GGATTTGGAAAGCCTATCCCTAATCCGCTGTTAGGACTGGATAGCACCTGATAATAGGCTG GAGCCTCGGTGGCCATGCTTCTTGCCCCTTGGGCCTCCCCCCAGCCCCTCCTCCCCTTCCTG CACCCGTACCCCCGTGGTCTTTGAATAAAGTCTGAGTGGGCGGC |
| | (mRNA construct seq. for P53 inhibitory peptide p7041 inserted in N-term of scaffold) |
| 159 | TCAAGCTTTTGGACCCTCGTACAGAAGCTAATACGACTCACTATAGGGAAATAAGAGAGA AAAGAAGAGTAAGAAGAAATATAAGAGCCACCATGATCCCCCGCGAGACATTTGAACACT GGTGGTCACAGCTCCTTAGCGGTCTCAGTGAGGCTAAGCCTGCGACTCCAGAGATCCAGGA GATCGTGGATAAAGTAAAGCCTCAGCTGGAGGAAAAAACTAACGAGACTTACGGAAACT GGAAGCGGTGCAATACAAAACACAGGTGCTGGCCTCAACTAACTATTACATCAAAGTGCG CGCTGGTGATAACAAATACATGCATTTAAAGGTCTTCAATGGGCCACCAGGACAGAACGC AGACAGAGTGCTGACTGGCTACCAGGTGGACAAGAACAAAGATGACGAGCTTACAGGCTT TGGCAAGCCTATCCCCAACCCACTCCTAGGGCTCGATTCGACGTGATAATAGGCTGGAGCC TCGGTGGCCATGCTTCTTGCCCCTTGGGCCTCCCCCCAGCCCCTCCTCCCCTTCCTGCACCC GTACCCCCGTGGTCTTTGAATAAAGTCTGAGTGGGCGGC |
| | (mRNA construct seq. for P53 inhibitory peptide pDIQ inserted in N-term of scaffold) |
| 160 | TCAAGCTTTTGGACCCTCGTACAGAAGCTAATACGACTCACTATAGGGAAATAAGAGAGA AAAGAAGAGTAAGAAGAAATATAAGAGCCACCATGATCCCCAGAACATCCTTCGCAGAGT ATTGGAATCTGCTGTCCCCCGGCCTTTCAGAAGCCAAACCTGCTACGCCAGAGATCCAAGA AATTGTGGACAAAGTGAAGCCCCAGCTCGAGGAGAAGACTAACGAGACATATGGTAAGTT AGAGGCCGTACAGTACAAAACGCAAGTGCTTGCTTCCACCAACTACTACATCAAAGTGAG AGCAGGTGATAATAAATACATGCACCTTAAAGTTTTAACGGCCCTCCTGGGCAGAACGCC GACAGAGTCCTCACTGGATATCAGGTTGATAAAAACAAGGATGACGAGCTGACTGGTTTCG GCAAACCTATCCCCAACCCTCTGCTGGGGCTGGACTCCACCTGATAATAGGCTGGAGCCTC GGTGGCCATGCTTCTTGCCCCTTGGGCCTCCCCCCAGCCCCTCCTCCCCTTCCTGCACCCGT ACCCCCGTGGTCTTTGAATAAAGTCTGAGTGGGCGGC |
| | (mRNA construct seq. for P53 inhibitory peptide pMI inserted in N-term of scaffold) |
| 161 | TCAAGCTTTTGGACCCTCGTACAGAAGCTAATACGACTCACTATAGGGAAATAAGAGAGA AAAGAAGAGTAAGAAGAAATATAAGAGCCACCATGATTCCCAGAGGCCTGTCCGAGGCTA AACCAGCCACACCTGAGATTCAGGAAATCGTGGATAAAGTTAAGCCTCAACTCGAAGAAA AGACAAACGAGACGTATGGTAAGCTTGAAGCTGTGCAGTATAAAACTCAAGTCCTGGCCTC CACCAATTATTACATTAAGGTCAGGGCTGGAGACAATAAGTACATGCATCTGAAAGTGTTC AACGGACCCTTGACCTTCGAACACTATTGGGCCCAACTCACCAGTCCCGGTCAGAACGCGG ACCGCGTACTTACAGGTTACCAAGTGGACAAGAATAAAGATGACGAGCTGACAGGCTTCG |
| | GTAAGCCAATCCCCAATCCTCTTCTCGGCCTGGACAGCACCTGATAATAGGCTGGAGCCTC GGTGGCCATGCTTCTTGCCCCTTGGGCCTCCCCCCAGCCCCTCCTCCCCTTCCTGCACCCGT ACCCCCGTGGTCTTTGAATAAAGTCTGAGTGGGCGGC |
| | (mRNA construct seq. for P53 inhibitory peptide p3848 inserted in Loop 2 of scaffold) |
| 162 | TCAAGCTTTTGGACCCTCGTACAGAAGCTAATACGACTCACTATAGGGAAATAAGAGAGA AAAGAAGAGTAAGAAGAAATATAAGAGCCACCATGATCCCTCGCGGCCTATCCGAGGCAA AGCCAGCTACCCCTGAAATACAGGAGATCGTGGATAAGGTAAAACCCCAACTGGAGGAGA AGACTAATGAGACATATGGCAAACTGGAAGCTGTTCAATATAAGACACAGGTGCTTGCCTC TACCAACTATTATATCAAAGTCCGGGCAGGAGATAATAAATATATGCATTTGAAGGTATTT AATGGCCCCGAGACATTTGAACACTGGTGGGCGCAGTTAACCTCTCCGGGGCAGAATGCG GATAGGGTGCTGACCGGGTACCAGGTTGACAAGAATAAGGATGACGAACTAACCGGTTTT GGCAAACCTATTCCTAACCCACTGCTGGGGCTGGACTCCACGTGATAATAGGCTGGAGCCT CGGTGGCCATGCTTCTTGCCCCTTGGGCCTCCCCCCAGCCCCTCCTCCCCTTCCTGCACCCG TACCCCCGTGGTCTTTGAATAAAGTCTGAGTGGGCGGC |
| | (mRNA construct seq. for P53 inhibitory peptide p1E6W inserted in Loop 2 of scaffold) |
| 163 | TCAAGCTTTTGGACCCTCGTACAGAAGCTAATACGACTCACTATAGGGAAATAAGAGAGA AAAGAAGAGTAAGAAGAAATATAAGAGCCACCATGATACCTCGGCTTACATTCGAACACT GGTGGGCCCAGTTGACATCAGGACTCAGCGAGGCCAAACCCGCTACACCTGAGATTCAAG AGATCGTCGATAAGGTAAAGCCCCAACTTGAAGAAAAGACAAACGAGACATATGGAAAGT TAGAGGCTGTTCAGTATAAGACACAGGTGCTTGCCTCCACCAACTACTATATCAAGGTAAG GGCCGGAGACAACAAATACATGCATCTCAAAGTATTCAATGGGCCCCCTGGTCAGAATGC GGATCGCGTCCTGACAGGCTACCAGGTCGACAAAAATAAGGACGACGAGCTCACGGATT TGGCAAACCTATTCCTAACCCACTCTTGGGCCTCGATTCGACTTGATAATAGGCTGGAGCCT CGGTGGCCATGCTTCTTGCCCCTTGGGCCTCCCCCCAGCCCCTCCTCCCCTTCCTGCACCCG TACCCCCGTGGTCTTTGAATAAAGTCTGAGTGGGCGGC |
| | (mRNA construct seq. for P53 inhibitory peptide p6W inserted in N-term of scaffold) |
| 164 | MFGLKRNAVIGLNLYCGGAGLGAGSGGATRPGGRLLATGAKDTKPMGRSGATSRKALETLRR VGDGVQRNHETAFQGMLRRLDIKNEDDVKSLSRVMIHVFSDGVTNWGRIVTLISFGAFVAKHL KTINQESCIEPLAESITDVLVRTKRDWLVKQRGWDGFVEFFQVEDLEGGIRNVLLAFAGVAGV GAGLAYLKRGKPIPNPLLGLDST |
| | (MCL1-ES.cV5 - endogenous MCL1-ES with v5 tag) |
| 165 | ATGTTTGGCCTCAAAAGAAACGCGGTAATCGGACTCAACCTCTACTGTGGGGGGCCGGCT TGGGGGCCGGCAGCGGCGGCGCCACCCGCCCGGGAGGGCGACTTTTGGCCACCGGCGCCA AGGACACAAAGCCAATGGGCAGGTCTGGGGCCACCAGCAGGAAGGCGCTGGAGACCTTAC GACGGGTTGGGGATGGCGTGCAGCGCAACCACGAGACGGCCTTCCAAGGCATGCTTCGGA GACTGGACATCAAAAACGAAGACGATGTGAAATCGTTGTCTCGAGTGATGATCCATGTTTT CAGCGACGGCGTAACAAACTGGGGCAGGATTGTGACTCTCATTTCTTTTGGTGCCTTTGTG GCTAAACACTTGAAGACCATAAACCAAGAAAGCTGCATCGAACCATTAGCAGAAAGTATC ACAGACGTTCTCGTAAGGACAAAACGGGACTGGCTAGTTAAACAAAGAGGCTGGGATGGG TTTGTGGAGTTCTTCCAAGTAGAGGACCTAGAAGGTGGCATCAGGAATGTGCTGCTGGCTT TTGCAGGTGTTGCTGGAGTAGGAGCTGGTTTGGCATATCTAAAAAGAGGGAAGCCTATTCC GAACCCTCTGCTGGGTCTGGACTCCACC |
| | (MCL1-ES.cV5 - endogenous MCL1-ES with v5 tag) |
| 166 | TCAAGCTTTTGGACCCTCGTACAGAAGCTAATACGACTCACTATAGGGAAATAAGAGAGA AAAGAAGAGTAAGAAGAAATATAAGAGCCACCATGATCCCAAGAGAAGAACAGTGGGCA CGCGAAATTGGAGCTCAACTCAGACGGATGGCCGACGACCTGAACGCCCAGTACGAGCGG CGGGGACTCTCAGAAGCGAAGCCTGCCACCCCCGAAATCCAGGAAATCGTCGACAAGGTC AAGCCGCAGCTTGAGGAAAAGACCAACGAGACTTACGGAAAACTGGAGGCCGTGCAGTAT AAGACCCAAGTGTTGGCCTCCACCAACTACTACATTAAGGTCCGCGCCGGCGATAACAAGT ACATGCACCTGAAGGTGTTCAATGGTCCTCCGGGACAGAACGCGGACAGGGTGCTGACTG GCTACCAAGTGGACAAGAACAAGGACGACGAGCTGACGGGCTTCGACTACAAGGATGATG ATGACAAATGATAATAGGCTGGAGCCTCGGTGGCCATGCTTCTTGCCCCTTGGGCCTCCCC CCAGCCCCTCCTCCCCTTCCTGCACCCGTACCCCCGTGGTCTTTGAATAAAGTCTGAGTGGG CGGCTCTAGA |
| | (SQT.PumaBH3.nt.flag_Hs3U-Xba1) |
| 167 | TCAAGCTTTTGGACCCTCGTACAGAAGCTAATACGACTCACTATAGGGAAATAAGAGAGA AAAGAAGAGTAAGAAGAAATATAAGAGCCACCATGATCCCAAGAGACATGCGCCCCGAA ATTTGGATCGCCCAGGAGCTCAGACGCATCGGAGATGAATTCAACGCATACTACGCTCGGC GGGGACTCTCAGAAGCGAAGCCTGCCACCCCCGAAATCCAGGAAATCGTCGACAAGGTCA AGCCGCAGCTTGAGGAAAAGACCAACGAGACTTACGGAAAACTGGAGGCCGTGCAGTATA |
| | AGACCCAAGTGTTGGCCTCCACCAACTACTACATTAAGGTCCGCGCCGGCGATAACAAGTA CATGCACCTGAAGGTGTTCAATGGTCCTCCGGACAGAACGCGGACAGGGTGCTGACTGG CTACCAAGTGGACAAGAACAAGGACGACGAGCTGACGGCTTCGACTACAAGGATGATGA TGACAAATGATAATAGGCTGGAGCCTCGGTGGCCATGCTTCTTGCCCCTTGGGCCTCCCCC CAGCCCCTCCTCCCCTTCCTGCACCCGTACCCCCGTGGTCTTTGAATAAAGTCTGAGTGGGC GGCTCTAGA |
| | (SQT.BimBH3.net.flag_Hs3U-XbaI) |

## Claims

1. A lipid nanoparticle comprising a messenger RNA (mRNA) encoding a polypeptide comprising a Stefin A (SteA) mutant polypeptide scaffold comprising at least one binding polypeptide domain, wherein optionally said mRNA comprises one or more modified nucleobases.

2. The lipid nanoparticle of claim 1, wherein the SteA mutant polypeptide scaffold is Stefin A Quadruple Mutant - Tracy (SQT) having an N-terminal insertion site, a loop 1 insertion site and a loop 2 insertion site, wherein the binding polypeptide domain is located at the N-terminal insertion site, the loop 1 insertion site, or the loop 2 insertion site, and optionally wherein the SteA mutant polypeptide scaffold comprises first and second binding polypeptides, and optionally a third binding polypeptide.

3. The lipid nanoparticle of claim 2, wherein
(i) the first and second binding polypeptide domains comprise the same amino acid sequence;
(ii) the first and second binding polypeptide domains comprise two different amino acid sequences;
(iii) the first, second, and third binding polypeptide domains comprise the same amino acid sequence;
(iv) the first, second, and third binding polypeptide domains comprise at least two different amino acid sequences; or
(v) the first, second, and third binding polypeptide domains each comprise a different amino acid sequence.

4. The lipid nanoparticle of any one of claims 1-3, wherein the at least one binding polypeptide domain:
(i) binds to a target selected from the group consisting of an oncoprotein, a viral protein, an apoptosis-inducing protein, a transcription factor, a cytokine, a receptor, an enzyme, DNA or RNA; or
(ii) is from a protein selected from the group consisting of an oncoprotein, an antibody or antigen-binding portion thereof, a ligand-binding protein, a DNA-binding protein and an RNA-binding protein.

5. The lipid nanoparticle of any one of claims 1-4, wherein the mRNA comprises a microRNA (miRNA) binding site.

6. The lipid nanoparticle of any one of claims 1-5, wherein
(i) the SteA scaffold comprises an amino acid sequence having at least 90% identity to any one of SEQ ID NOs:53-71;
(ii). the SteA scaffold comprises an amino acid sequence selected from any one of SEQ ID NOs:53-71; or
(iii) the SteA scaffold comprises the amino acid sequence of SEQ ID NO:70.

7. The lipid nanoparticle of any one of claims 1-6, wherein the lipid nanoparticle comprises a cationic lipid, optionally an ionizable cationic lipid or 2,2-dilinoleyl-4-methylaminoethyl-[1,3]-dioxolane (DLin-KC2-DMA), dilinoleyl-methyl-4-dimethylaminobutyrate (DLin-MC3-DMA) and di((Z)-non-2-en-1-yl) 9-((4-(dimethylamino)butanoyl)oxy)heptadecanedioate (L319),
wherein optionally the lipid nanoparticle comprises
(i) a PEG-modified lipid; and/or
(ii) a sterol, optionally a cholesterol; and/or
(iii) a non-cationic lipid, optionally a neutral lipid.

8. The lipid nanoparticle of claim 7, wherein the cationic lipid nanoparticle has a molar ratio of about 20-60% cationic lipid, about 5-25% non-cationic lipid, about 25-55% sterol and about 0.5-15% PEG-modified lipid.

9. The lipid nanoparticle of any one of the preceding claims; wherein at least 80% or 100% of the uracils in the open reading frame have a chemical modification and/or wherein the chemical modification is in the 5-position of the uracils.

10. A pharmaceutical composition comprising the lipid nanoparticle of any one of claims 1-9, and a pharmaceutically acceptable carrier, diluent or excipient.

11. The lipid nanoparticle of any one of claims 1-9 or the pharmaceutical composition of claim 10 for use in medicine.

12. The lipid nanoparticle of any one of claims 1-9, or the pharmaceutical composition of claim 10 for use in the treatment of cancer.

## Patentansprüche

1. Lipid-Nanopartikel, umfassend eine Messenger-RNA (mRNA), welche ein Polypeptid codiert, umfassend ein mutiertes Stefin A (SteA)-Polypeptidgerüst, umfassend wenigstens eine Polypeptidbindungsdomäne, wobei die mRNA optional eine oder mehrere modifizierte Nukleobasen umfasst.

2. Lipid-Nanopartikel nach Anspruch 1, wobei das mutierte SteA-Polypeptidgerüst Stefin A Quadruple Mutant-Tracy (SQT) mit einer N-terminalen Insertionsstelle, einer Schleife 1-Insertionsstelle und einer Schleife 2-Insertionsstelle ist, wobei die Polypeptidbindungsdomäne an der N-terminalen Insertionsstelle, der Schleife 1-Insertionsstelle oder der Schleife 2-Insertionsstelle lokalisiert ist, und wobei das mutierte SteA-Polypeptidgerüst optional ein erstes und ein zweites Bindungspolypeptid und optional ein drittes Bindungspolypeptid umfasst.

3. Lipid-Nanopartikel nach Anspruch 2, wobei
(i) die erste und die zweite Polypeptidbindungsdomäne die gleiche Aminosäuresequenz umfassen;
(ii) die erste und die zweite Polypeptidbindungsdomäne zwei unterschiedliche Aminosäuresequenzen umfassen;
(iii) die erste, die zweite und die dritte Polypeptidbindungsdomäne die gleiche Aminosäuresequenz umfassen;
(iv) die erste, die zweite und die dritte Polypeptidbindungsdomäne wenigstens zwei unterschiedliche Aminosäuresequenzen umfassen; oder
(v) die erste, die zweite und die dritte Polypeptidbindungsdomäne jeweils eine unterschiedliche Aminosäuresequenz umfassen.

4. Lipid-Nanopartikel nach irgendeinem der Ansprüche 1-3, wobei die wenigstens eine Polypeptidbindungsdomäne:
(i) an ein Ziel bindet, ausgewählt aus der Gruppe, bestehend aus einem Onkoprotein, einem Virusprotein, einem Apoptose-induzierenden Protein, einem Transkriptionsfaktor, einem Zytokin, einem Rezeptor, einem Enzym, DNA oder RNA; oder
(ii) von einem Protein stammt, ausgewählt aus der Gruppe, bestehend aus einem Onkoprotein, einem Antikörper oder Antigen-bindenden Abschnitt davon, einem Ligandenbindungsprotein, einem DNA-Bindungsprotein und einem RNA-Bindungsprotein.

5. Lipid-Nanopartikel nach irgendeinem der Ansprüche 1-4, wobei die mRNA eine microRNA (miRNA)-Bindungsstelle umfasst.

6. Lipid-Nanopartikel nach irgendeinem der Ansprüche 1-5, wobei
(i) das SteA-Gerüst eine Aminosäuresequenz umfasst, die wenigstens 90% Identität zu irgendeiner der SEQ ID NOs: 53-71 aufweist;
(ii) das SteA-Gerüst eine Aminosäuresequenz, ausgewählt aus irgendeiner von SEQ ID NOs: 53-71, umfasst; oder
(iii) das SteA-Gerüst die Aminosäuresequenz von SEQ ID NO: 70 umfasst.

7. Lipid-Nanopartikel nach irgendeinem der Ansprüche 1-6, wobei das Lipid-Nanopartikel ein kationisches Lipid umfasst, optional ein ionisierbares kationisches Lipid oder 2,2-Dilinoleyl-4-methylaminoethyl-[1,3]-dioxolan [DLin-KC2-DMA), Dilinoleyl-methyl-4-dimethylaminobutyrat (DLin-MC3-DMA) und Di (Z)-non-2-en-1-yl) 9-((4-(dimethylamino)butanoyl)oxy)heptadecandioat (L319), wobei das Lipid-Nanopartikel optional umfasst
(i) ein PEG-modifiziertes Lipid; und/oder
(ii) ein Sterin, optional ein Cholesterin; und/oder
(iii) ein nicht-kationisches Lipid, optional ein neutrales Lipid.

8. Lipid-Nanopartikel nach Anspruch 7, wobei das kationische Lipid-Nanopartikel ein molares Verhältnis von etwa 20-60% kationischem Lipid, etwa 5-25% nichtkationischem Lipid, etwa 25-55% Sterin und etwa 0,5-15% PEG-modifiziertem Lipid hat.

9. Lipid-Nanopartikel nach irgendeinem der vorangegangenen Ansprüche, wobei wenigstens 80% oder 100% der Uracile in dem offenen Leserahmen eine chemische Modifikation aufweisen und/oder wobei die chemische Modifikation an der 5-Position der Uracile liegt.

10. Pharmazeutische Zusammensetzung, umfassend das Lipid-Nanopartikel nach irgendeinem der Ansprüche 1-9 und ein(en) pharmazeutisch akzeptable(n/s) Träger, Verdünnungsmittel oder Hilfsstoff.

11. Lipid-Nanopartikel nach irgendeinem der Ansprüche 1-9 oder pharmazeutische Zusammensetzung nach Anspruch 10 zur Verwendung in der Medizin.

12. Lipid-Nanopartikel nach irgendeinem der Ansprüche 1-9 oder pharmazeutische Zusammensetzung nach Anspruch 10 zur Verwendung bei der Behandlung von Krebs.

## Revendications

1. Nanoparticule lipidique comprenant un ARN messager (ARNm) codant pour un polypeptide comprenant une structure polypeptidique mutante de Stefin A (SteA) comprenant au moins un domaine polypeptidique de liaison, dans laquelle facultativement ledit ARNm comprend une ou plusieurs bases nucléiques modifiées.

2. Nanoparticule lipidique selon la revendication 1, dans laquelle la structure polypeptidique mutante de SteA est Stefin A Quadruple Mutant - Tracy (SQT) présentant un site d'insertion N-terminal, un site d'insertion de boucle 1 et un site d'insertion de boucle 2, dans laquelle le domaine polypeptidique de liaison est situé au niveau du site d'insertion N-terminal, du site d'insertion de boucle 1 ou du site d'insertion de boucle 2, et facultativement dans laquelle la structure polypeptidique mutante de SteA comprend des premier et deuxième polypeptides de liaison, et facultativement un troisième polypeptide de liaison.

3. Nanoparticule lipidique selon la revendication 2, dans laquelle
(i) les premier et deuxième domaines polypeptides de liaison comprennent la même séquence d'acides aminés ;
(ii) les premier et deuxième domaines polypeptides de liaison comprennent deux séquences d'acides aminés différentes ;
(iii) les premier, deuxième et troisième domaines polypeptides de liaison comprennent la même séquence d'acides aminés ;
(iv) les premier, deuxième et troisième domaines polypeptides de liaison comprennent au moins deux séquences d'acides aminés différentes ;
(ii) les premier, deuxième et troisième domaines polypeptides de liaison comprennent chacun une séquence d'acides aminés différente.

4. Nanoparticule lipidique selon l'une quelconque des revendications 1 à 3, dans laquelle le au moins un domaine polypeptide de liaison :
(i) se lie à une cible sélectionnée dans le groupe consistant en une oncoprotéine, une protéine virale, une protéine induisant l'apoptose, un facteur de transcription, une cytokine, un récepteur, une enzyme, un ADN ou un ARN ; ou
(ii) provient d'une protéine sélectionnée dans le groupe consistant en une oncoprotéine, un anticorps ou une partie de celui-ci se liant à un antigène, une protéine se liant à un ligand, une protéine se liant à un ADN et une protéine se liant à un ARN.

5. Nanoparticule lipidique selon l'une quelconque des revendications 1 à 4, dans laquelle l'ARNm comprend un site de liaison à un microARN (miARN).

6. Nanoparticule lipidique selon l'une quelconque des revendications 1 à 5, dans laquelle
(i) la structure de SteA comprend une séquence d'acides aminés présentant au moins 90 % d'identité avec l'une quelconque des SEQ ID NO : 53 à 71 ;
(ii) la structure de SteA comprend une séquence d'acides aminés sélectionnée parmi l'une quelconque des SEQ ID NO : 53 à 71 ; ou
(iii) la structure de SteA comprend la séquence d'acides aminés de SEQ ID NO : 70.

7. Nanoparticule lipidique selon l'une quelconque des revendications 1 à 6, dans laquelle la nanoparticule lipidique comprend un lipide cationique, facultativement un lipide cationique ionisable ou le 2,2-dilinoléyl-4-méthylaminoéthyl-[1,3]-dioxolane (DLin-KC2-DMA), le 4-diméthylaminobutyrate de dilinoléyl-méthyle (DLin-MC3-DMA) et le 9-((4-(diméthylamino)butanoyl)oxy)heptadécanedioate de di((Z)-non-2-èn-1-yle) (L319),
dans laquelle facultativement la nanoparticule lipidique comprend
(i) un lipide modifié par PEG ; et/ou
(ii) un stérol, facultativement un cholestérol ; et/ou
(iii) un lipide non cationique, facultativement un lipide neutre.

8. Nanoparticule lipidique selon la revendication 7, dans laquelle la nanoparticule lipidique cationique présente un rapport molaire d'environ 20 à 60 % de lipide cationique, d'environ 5 à 25 % de lipide non cationique, d'environ 25 à 55 % de stérol et d'environ 0,5 à 15 % de lipide modifié par PEG.

9. Nanoparticule lipidique selon l'une quelconque des revendications précédentes, dans laquelle au moins 80% ou 100% des uraciles dans le cadre de lecture ouvert présentent une modification chimique et/ou dans laquelle la modification chimique est en position 5 des uraciles.

10. Composition pharmaceutique comprenant la nanoparticule lipidique selon l'une quelconque des revendications 1 à 9, et un support, diluant ou excipient pharmaceutiquement acceptable.

11. Nanoparticule lipidique selon l'une quelconque des revendications 1 à 9 ou la composition pharmaceutique selon la revendication 10 pour une utilisation en médecine.

12. Nanoparticule lipidique selon l'une quelconque des revendications 1 à 9 ou la composition pharmaceutique selon la revendication 10 pour une utilisation dans le traitement d'un cancer.
